# EUROPEAN PATENT APPLICATION

(11) **EP 4 382 601 A1**
(43) Date of publication of application: **12.06.2024**
(21) Application number: 22852343.7
(22) Date of filing: 05.08.2022
(51) Int. Cl.: C12N 15/11, C12N 15/864, A61K 48/00, A61P 27/02

(54) **COMPOSITION AND METHOD FOR TREATING LEBER'S HEREDITARY OPTIC NEUROPATHY CAUSED BY ND4 MUTATION**

(30) Priority: 06.08.2021 CN 202110899561
(71) Applicant: Wuhan Neurophth Biotechnology Limited Company, Wuhan, Hubei 430060 (CN)
(72) Inventor: LI, Bin, Wuhan, Hubei 430060 (CN)
(74) Representative: Cooley (UK) LLP
(86) International application number: PCT/CN2022/110545
(87) International publication number: WO 2023/011632

(57) **Abstract**

Provided is a recombinant nucleic acid comprising: a mitochondrial targeting sequence; and a mitochondrial protein coding sequence, wherein the mitochondrial protein coding sequence encodes a polypeptide comprising a mitochondrial protein; a 3' UTR nucleic acid sequence, and a polyA signal sequence. Also provided is a pharmaceutical composition comprising the recombinant nucleic acid and a method for treating Leber's hereditary optic neuropathy (LHON) using the pharmaceutical composition.

## Description

### TECHNICAL FIELD

The present invention relates to a recombinant nucleic acid encoding a mitochondrial protein and a pharmaceutical composition related thereto, and use thereof for treating an ocular disease such as Leber's hereditary optic neuropathy.

### BACKGROUND

Leber's hereditary optic neuropathy (LHON) is a mitochondrially inherited degeneration (passed from mother to offspring) of a retinal ganglion cell (RGC) and its axon, which results in acute or subacute central vision loss. This affects primarily young adult males. LHON is transmitted only through the mother as it is mainly caused by mutations in the mitochondrial (rather than nuclear) genome, and only the ovum contributes mitochondria to the embryo. LHON is usually caused by one of three point mutations of pathogenic mitochondrial DNA (mtDNA). Those mutations are at nucleotide positions 11778 G to A (G11778A), 3460 G to A (G3460A) and 14484 T to C (T14484C) in the NADH dehydrogenase subunit-4 protein (ND 4), NADH dehydrogenase subunit-1 protein (ND 1) and NADH dehydrogenase subunit-6 protein (ND 6) subunit genes of complex I of the oxidative phosphorylation chain in mitochondria, respectively. Each mutation is considered to carry a significant risk of permanent vision loss. LHON usually progresses without pain over a period of weeks to months until binocular visual acuity deteriorates below 0.1, which seriously affects the quality of life of the patient. The two LHON mutants, G3460A and T14484C, result in an 80% reduction in the activity of mitochondrial NADH dehydrogenase isolated from patient platelets. Ninety percent of Chinese LHON patients carry the G11778A mutation. The G11778A mutation changes arginine to histidine in the ND4 protein, resulting in dysfunction and optic nerve damage in LHON patients. Therefore, there is a need to develop a compositions and a method for treating LHON with improved transfection efficiency and therapeutic efficacy.

### SUMMARY

Disclosed herein is a recombinant nucleic acid comprising (sequentially from the 5' end to the 3' end) a mitochondrial targeting sequence and a mitochondrial protein coding sequence; optionally, the mitochondrial protein coding sequence encodes an ND4 protein; and optionally, the ND4 protein comprises an amino acid sequence having at least 90%, at least 95%, at least 96%, at least 97%, at least 98%, at least 99%, or 100% identity to SEQ ID NO: 160.

In some embodiments, the recombinant nucleic acid comprises a sequence having at least 90%, at least 95%, at least 96%, at least 97%, at least 98%, at least 99%, or 100% identity to any one of sequences as set forth in SEQ ID NOs: 180 and 174-176. In some embodiments, the recombinant nucleic acid comprises a sequence having at least 90%, at least 95%, at least 96%, at least 97%, at least 98%, at least 99%, or 100% identity to SEQ ID NO: 180. In some embodiments, the recombinant nucleic acid comprises a sequence as set forth in SEQ ID NO: 180.

In some embodiments, the recombinant nucleic acid comprises a Kozak sequence positioned before the 5' end of the mitochondrial targeting sequence; optionally, the Kozak sequence is SEQ ID NO: 171; and optionally, no redundant nucleotides are present between the Kozak sequence and the mitochondrial targeting sequence.

In some embodiments, the recombinant nucleic acid comprises an intron sequence; optionally, the intron sequence is positioned before the 5' end of the mitochondrial targeting sequence; optionally, the intron sequence is positioned before the 5' end of the Kozak sequence; and optionally, the intron sequence comprises a sequence having at least 90%, at least 95%, at least 96%, at least 97%, at least 98%, at least 99%, or 100% identity to a sequence as set forth in SEQ ID NO: 170.

In some embodiments, the recombinant nucleic acid comprises a promoter sequence; optionally, the promoter sequence is positioned before the 5' end of the mitochondrial targeting sequence, the Kozak sequence, and/or the intron sequence; and optionally, the promoter sequence comprises a sequence having at least 90%, at least 95%, at least 96%, at least 97%, at least 98%, at least 99%, or 100% identity to a sequence as set forth in SEQ ID NO: 169.

In some embodiments, the recombinant nucleic acid comprises a 3' UTR sequence; optionally, the 3' UTR sequence is positioned after the 3' end of the mitochondrial protein coding sequence; and optionally, the 3' UTR sequence comprises a sequence having at least 90%, at least 95%, at least 96%, at least 97%, at least 98%, at least 99%, or 100% identity to a sequence as set forth in SEQ ID NO: 13.

In some embodiments, the recombinant nucleic acid comprises a polyA signal sequence; optionally, the polyA signal sequence is positioned after the 3' end of the mitochondrial protein coding sequence and/or the 3' UTR sequence; optionally, the polyA signal sequence comprises a sequence having at least 90%, at least 95%, at least 96%, at least 97%, at least 98%, at least 99%, or 100% identity to a sequence as set forth in SEQ ID NO: 172 or 173; and optionally, the polyA signal sequence comprises a sequence having at least 90%, at least 95%, at least 96%, at least 97%, at least 98%, at least 99%, or 100% identity to a sequence as set forth in SEQ ID NO: 173.

In some embodiments, the recombinant nucleic acid comprises a sequence having at least 80%, at least 85%, at least 90%, at least 95%, or 100% identity to a spacer sequence as set forth in SEQ ID NO: 185 between the 3' UTR sequence and the polyA signal sequence.

In some embodiments, the mitochondrial targeting sequence comprises a sequence having at least 90%, at least 95%, at least 96%, at least 97%, at least 98%, at least 99%, or 100% identity to a sequence as set forth in SEQ ID NO: 1.

In some embodiments, the mitochondrial protein coding sequence comprises a sequence having at least 90%, at least 95%, at least 96%, at least 97%, at least 98%, at least 99%, or 100% identity to a sequence as set forth in SEQ ID NO: 6.

In some embodiments, the recombinant nucleic acid comprises a first inverted terminal repeat (ITR) sequence and a second ITR sequence. In some embodiments, the first ITR sequence comprises a sequence having at least 90%, at least 95%, at least 96%, at least 97%, at least 98%, at least 99%, or 100% identity to a sequence as set forth in SEQ ID NO: 178. In some embodiments, the second ITR sequence comprises a sequence having at least 90%, at least 95%, at least 96%, at least 97%, at least 98%, at least 99%, or 100% identity to a sequence as set forth in SEQ ID NO: 179.

Disclosed herein is a recombinant nucleic acid comprising (sequentially from the 5' end to the 3' end): a mitochondrial targeting sequence, a mitochondrial protein coding sequence, a 3' UTR sequence, and a polyA signal sequence, wherein the polyA signal sequence comprises a sequence having at least 90%, at least 95%, at least 96%, at least 97%, at least 98%, at least 99%, or 100% identity to a sequence as set forth in SEQ ID NO: 173.

In some embodiments, the 3' UTR sequence comprises a sequence having at least 90%, at least 95%, at least 96%, at least 97%, at least 98%, at least 99%, or 100% identity to a sequence as set forth in SEQ ID NO: 13.

In some embodiments, mRNA comprising the mitochondrial protein coding sequence produced by transcription of the recombinant nucleic acid has an expression level that is higher than mRNA of a control recombinant nucleic acid lacking the polyA signal sequence; preferably, has an expression level that is at least 10%, at least 15%, at least 20%, at least 30%, at least 40%, at least 50%, at least 60%, at least 70%, at least 80%, at least 90%, at least 100%, at least 150%, at least 200 %, or at least 300% higher than mRNA of the control recombinant nucleic acid. In some embodiments, the control recombinant nucleic acid is substituted at a position of the polyA signal sequence with a sequence comprising SEQ ID NO: 172; preferably, mRNA produced by transcription of the recombinant nucleic acid has an expression level that is at least 10%, at least 15%, or at least 20% higher than mRNA produced by the control recombinant nucleic acid.

In some embodiments, the mitochondrial protein produced by translation of the recombinant nucleic acid has an expression level that is higher than the mitochondrial protein of the control recombinant nucleic acid lacking the polyA signal sequence; preferably, has an expression level that is at least 10%, at least 15%, at least 20%, at least 30%, at least 40%, at least 50%, at least 60%, at least 70%, at least 80%, at least 90%, at least 100%, at least 150%, at least 200 %, or at least 300% higher than the mitochondrial protein of the control recombinant nucleic acid. In some embodiments, the control recombinant nucleic acid is substituted at a position of the polyA signal sequence with a sequence comprising SEQ ID NO: 172; preferably, the mitochondrial protein produced by translation of the recombinant nucleic acid has an expression level that is at least 10%, at least 15%, or at least 20% higher than the mitochondrial protein produced by the control recombinant nucleic acid.

In some embodiments, the polyA signal sequence has a length of no more than 122 base pairs, no more than 125 base pairs, no more than 130 base pairs, no more than 140 base pairs, no more than 150 base pairs, no more than 160 base pairs, no more than 170 base pairs, no more than 180 base pairs, no more than 190 base pairs, or no more than 200 base pairs.

In some embodiments, the recombinant nucleic acid comprises a Kozak sequence positioned before the 5' end of the mitochondrial targeting sequence; optionally, the Kozak sequence is SEQ ID NO: 171; and optionally, no redundant nucleotides are present between the Kozak sequence and the mitochondrial targeting sequence.

In some embodiments, the recombinant nucleic acid comprises a sequence having at least 80%, at least 85%, at least 90%, at least 95%, or 100% identity to a spacer sequence as set forth in SEQ ID NO: 185 between the 3' UTR sequence and the polyA signal sequence.

Disclosed herein is a recombinant nucleic acid comprising (sequentially from the 5' end to the 3' end): a Kozak sequence, a mitochondrial targeting sequence, a mitochondrial protein coding sequence, and a 3' UTR sequence, wherein the Kozak sequence is SEQ ID NO: 171; the 3' UTR sequence comprises a sequence having at least 90%, at least 95%, at least 96%, at least 97%, at least 98%, at least 99%, or 100% identity to a sequence as set forth in SEQ ID NO: 13; and no redundant nucleotides are present between the Kozak sequence and the mitochondrial targeting sequence.

In some embodiments, the recombinant nucleic acid comprises a polyA signal sequence, and the polyA signal sequence comprises a sequence having at least 90%, at least 95%, at least 96%, at least 97%, at least 98%, at least 99%, or 100% identity to a sequence as set forth in SEQ ID NO: 172 or 173.

In some embodiments, the recombinant nucleic acid comprises an intron sequence; optionally, the intron sequence is positioned before the 5' end of the Kozak sequence; optionally, the intron sequence comprises a sequence having at least 90%, at least 95%, at least 96%, at least 97%, at least 98%, at least 99%, or 100% identity to a sequence as set forth in SEQ ID NO: 170.

In some embodiments, the recombinant nucleic acid comprises a promoter sequence; optionally, the promoter sequence comprises a sequence having at least 90%, at least 95%, at least 96%, at least 97%, at least 98%, at least 99%, or 100% identity to a sequence as set forth in SEQ ID NO: 169. In some embodiments, the promoter sequence is positioned before the 5' end of the intron sequence.

Disclosed herein is a recombinant nucleic acid comprising (sequentially from the 5' end to the 3' end): a promoter sequence, an intron sequence, a Kozak sequence, a mitochondrial targeting sequence and a mitochondrial protein coding sequence; the intron sequence comprises a sequence having at least 90%, at least 95%, at least 96%, at least 97%, at least 98%, at least 99%, or 100% identity to a sequence as set forth in SEQ ID NO: 170; optionally, the recombinant nucleic acid further comprises a 3' UTR sequence; and optionally, the recombinant nucleic acid further comprises a polyA signal sequence.

In some embodiments, the recombinant nucleic acid further comprises a first inverted terminal repeat (ITR) sequence and a second ITR sequence. In some embodiments, the first ITR sequence comprises a sequence having at least 90%, at least 95%, at least 96%, at least 97%, at least 98%, at least 99%, or 100% identity to a sequence as set forth in SEQ ID NO: 178. In some embodiments, the second ITR sequence comprises a sequence having at least 90%, at least 95%, at least 96%, at least 97%, at least 98%, at least 99%, or 100% identity to a sequence as set forth in SEQ ID NO: 179.

In some embodiments, no redundant nucleotides are present between the mitochondrial protein coding sequence and the 3' UTR sequence.

In some embodiments, the mitochondrial targeting sequence comprises a sequence having at least 90%, at least 95%, at least 96%, at least 97%, at least 98%, at least 99%, or 100% identity to a sequence as set forth in SEQ ID NO: 1.

In some embodiments, the mitochondrial protein coding sequence comprises a sequence having at least 90%, at least 95%, at least 96%, at least 97%, at least 98%, at least 99%, or 100% identity to a sequence as set forth in SEQ ID NO: 6.

In some embodiments, no redundant nucleotides are present between the mitochondrial targeting sequence and the mitochondrial protein coding sequence.

In some embodiments, the recombinant nucleic acid comprises (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence; preferably, the first ITR sequence comprises a sequence having at least 90%, at least 95%, at least 96%, at least 97%, at least 98%, at least 99%, or 100% identity to a sequence as set forth in SEQ ID NO: 178; the promoter sequence comprises a sequence having at least 90%, at least 95%, at least 96%, at least 97%, at least 98%, at least 99%, or 100% identity to a sequence as set forth in SEQ ID NO: 169; the intron sequence comprises a sequence having at least 90%, at least 95%, at least 96%, at least 97%, at least 98%, at least 99%, or 100% identity to a sequence as set forth in SEQ ID NO: 170; the Kozak sequence is SEQ ID NO: 171; the mitochondrial targeting sequence comprises a sequence having at least 90%, at least 95%, at least 96%, at least 97%, at least 98%, at least 99%, or 100% identity to a sequence as set forth in SEQ ID NO: 1; the mitochondrial protein coding sequence comprises a sequence having at least 90%, at least 95%, at least 96%, at least 97%, at least 98%, at least 99%, or 100% identity to a sequence as set forth in SEQ ID NO: 6; the 3' UTR sequence comprises a sequence having at least 90%, at least 95%, at least 96%, at least 97%, at least 98%, at least 99%, or 100% identity to a sequence as set forth in SEQ ID NO: 13; the polyA signal sequence comprises a sequence having at least 90%, at least 95%, at least 96%, at least 97%, at least 98%, at least 99%, or 100% identity to a sequence as set forth in SEQ ID NO: 173; and the second ITR sequence comprises a sequence having at least 90%, at least 95%, at least 96%, at least 97%, at least 98%, at least 99%, or 100% identity to a sequence as set forth in SEQ ID NO: 179.

In some embodiments, the recombinant nucleic acid comprises (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence; preferably, the first ITR sequence comprises a sequence having at least 99%, at least 99.5%, or 100% identity to a sequence as set forth in SEQ ID NO: 178; the promoter sequence comprises a sequence having at least 99%, at least 99.5%, or 100% identity to a sequence as set forth in SEQ ID NO: 169; the intron sequence comprises a sequence having at least 99%, at least 99.5%, or 100% identity to a sequence as set forth in SEQ ID NO: 170; the Kozak sequence is SEQ ID NO: 171; the mitochondrial targeting sequence comprises a sequence having at least 99%, at least 99.5%, or 100% identity to a sequence as set forth in SEQ ID NO: 1; the mitochondrial protein coding sequence comprises a sequence having at least 99%, at least 99.5%, or 100% identity to a sequence as set forth in SEQ ID NO: 6; the 3' UTR sequence comprises a sequence having at least 99%, at least 99.5%, or 100% identity to a sequence as set forth in SEQ ID NO: 13; the polyA signal sequence comprises a sequence having at least 99%, at least 99.5%, or 100% identity to a sequence as set forth in SEQ ID NO: 173; and the second ITR sequence comprises a sequence having at least 99%, at least 99.5%, or 100% identity to a sequence as set forth in SEQ ID NO: 179.

In some embodiments, the recombinant nucleic acid comprises (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence; preferably, the first ITR sequence comprises a sequence as set forth in SEQ ID NO: 178; the promoter sequence comprises a sequence as set forth in SEQ ID NO: 169; the intron sequence comprises a sequence as set forth in SEQ ID NO: 170; the Kozak sequence is SEQ ID NO: 171; the mitochondrial targeting sequence comprises a sequence as set forth in SEQ ID NO: 1; the mitochondrial protein coding sequence comprises a sequence as set forth in SEQ ID NO: 6; the 3' UTR sequence comprises a sequence as set forth in SEQ ID NO: 13; the polyA signal sequence comprises a sequence as set forth in SEQ ID NO: 173; and the second ITR sequence comprises a sequence as set forth in SEQ ID NO: 179.

In some embodiments, the recombinant nucleic acid comprises a sequence having at least 90%, at least 95%, at least 96%, at least 97%, at least 98%, at least 99%, or 100% identity to any one of sequences as set forth in SEQ ID NOs: 174-176 and 180. In some embodiments, the recombinant nucleic acid comprises a sequence having at least 90%, at least 95%, at least 96%, at least 97%, at least 98%, at least 99%, or 100% identity to SEQ ID NO: 180. In some embodiments, the recombinant nucleic acid comprises a sequence as set forth in SEQ ID NO: 180.

Disclosed herein is a viral vector comprising the recombinant nucleic acid described herein. In some embodiments, the viral vector is a recombinant adeno-associated virus (rAAV) vector. In some embodiments, the rAAV vector is an rAAV2 vector.

Disclosed herein is a pharmaceutical composition comprising the recombinant nucleic acid described herein. Disclosed herein is a pharmaceutical composition comprising the viral vector described herein. Disclosed herein is a pharmaceutical composition comprising the recombinant adeno-associated virus (rAAV) vector described herein.

Disclosed herein is a pharmaceutical composition comprising the virus described herein. In some embodiments, the virus is an adeno-associated virus (AAV). In some embodiments, the virus comprises a recombinant nucleic acid described herein.

In some embodiments, the pharmaceutical composition further comprises a pharmaceutically acceptable excipient thereof. In some embodiments, the pharmaceutically acceptable excipient comprises phosphate buffered saline (PBS), α,α-trehalose dehydrate, L-histidine hydrochloride monohydrate, polysorbate 20, NaCl, NaH₂PO₄, Na₂HPO₄, KH₂PO₄, K₂HPO₄, poloxamer 188, or any combination thereof. In some embodiments, the pharmaceutically acceptable excipient is selected from phosphate buffered saline (PBS), α,α-trehalose dehydrate, L-histidine hydrochloride monohydrate, polysorbate 20, NaCl, NaH₂PO₄, Na₂HPO₄, KH₂PO₄, K₂HPO₄, poloxamer 188, and any combination thereof. In some embodiments, the pharmaceutically acceptable excipient comprises poloxamer 188. In some embodiments, the pharmaceutically acceptable excipient comprises 0.0001%-0.01% poloxamer 188. In some embodiments, the pharmaceutically acceptable excipient comprises 0.001% poloxamer 188. In some embodiments, the pharmaceutically acceptable excipient further comprises one or more salts. In some embodiments, the one or more salts comprise NaCl, Na₂HPO₄, and KH₂PO₄. In some embodiments, the pharmaceutical composition comprises, a) the NaCl at a concentration of 5-15 mg/mL; preferably, the NaCl at a concentration of 9 mg/mL; b) the KH₂PO₄ at a concentration of 0.1-0.5 mg/mL; preferably, the KH₂PO₄ at a concentration of 0.144 mg/mL; and/or c) the Na₂HPO₄ at a concentration of 0.5-1 mg/mL; preferably, the Na₂HPO₄ at a concentration of 0.795 mg/mL. In some embodiments, the pharmaceutical composition has a pH of 7.2-7.4. In some embodiments, the pharmaceutical composition has a pH of 7.3.

In some embodiments, the pharmaceutical composition has a viral titer of at least 1.0×10¹⁰ vg/mL, at least 3.0×10¹⁰ vg/mL, or at least 6.0×10¹⁰ vg/mL. In some embodiments, the pharmaceutical composition has a viral titer of at least 9.0×10¹⁰ vg/mL.

In some embodiments, the pharmaceutical composition retains at least 60% of a viral titer after five freeze/thaw cycles as compared with the viral titer prior to the freeze/thaw cycles.

Disclosed herein is use of the pharmaceutical composition described herein in the preparation of a medicament for treating an ocular disease.

Disclosed herein is a method for treating an ocular disease, which comprises administering to a patient an effective dose of the pharmaceutical composition described herein.

In some embodiments, the ocular disease is Leber's hereditary optic neuropathy (LHON). In some embodiments, the pharmaceutical composition is administered by intraocular or intravitreal injection. In some embodiments, the pharmaceutical composition is administered by intravitreal injection. In some embodiments, 0.01-0.1mL of the pharmaceutical composition is administered in each eye. In some embodiments, 0.045-0.055 mL of the pharmaceutical composition is administered in each eye. In some embodiments, the adeno-associated virus is administered at a dose of at least 1.5×10⁹ vg/eye; preferably, the adeno-associated virus is administered at a dose of at least 3.0×10⁹ vg/eye. In some embodiments, the adeno-associated virus is administered at a dose of at least 4.5×10⁹ vg/eye.

In some embodiments, the use or method comprises administering a corticosteroid to a patient. In some embodiments, the corticosteroid comprises prednisone or methylprednisolone. In some embodiments, the corticosteroid is prednisone. In some embodiments, the corticosteroid is administered beginning about 2 days prior to the administration of the pharmaceutical composition. In some embodiments, the corticosteroid is administered orally. In some embodiments, the corticosteroid is administered for about 28 consecutive days after the administration begins. In some embodiments, the steroid is administered daily after the administration begins, and at a dose that is reduced after each week of continuous administration. In some embodiments, the corticosteroid is administered at a dosage of: 40 mg daily for one week at the beginning of administration, then 30 mg daily for one week, then 20 mg daily for one week, and 10 mg daily for one week at last.

In some embodiments, the use or method comprises administering creatine phosphate sodium to the patient. In some embodiments, the creatine phosphate sodium is administered by intravenous injection. In some embodiments, the administration of the pharmaceutical composition results in a higher average recovery level of vision as compared to administration of a pharmaceutical composition without the recombinant nucleic acid.

In another aspect, disclosed herein are a recombinant nucleic acid, a pharmaceutical composition and a method for treating LHON.

In one aspect, disclosed herein is a recombinant nucleic acid comprising: a mitochondrial targeting sequence; a mitochondrial protein coding sequence comprising a sequence having at least 99% identity to a sequence selected from the group consisting of SEQ ID NOs: 7, 8, 10 and 12; and a 3' UTR nucleic acid sequence.

In some cases, the mitochondrial targeting sequence encodes a polypeptide comprising a peptide sequence having at least 90%, at least 95%, at least 97%, at least 99% or 100% identity to a sequence selected from the group consisting of SEQ ID NOs: 129-159. In some cases, the mitochondrial targeting sequence comprises a sequence having at least 90%, at least 95%, at least 97%, at least 99%, or 100% identity to a sequence as set forth in SEQ ID NO: 2. In some cases, the mitochondrial targeting sequence comprises a sequence having at least 90%, at least 95%, at least 97%, at least 99%, or 100% identity to a sequence as set forth in SEQ ID NO: 3. In some cases, the mitochondrial targeting sequence comprises a sequence having at least 90%, at least 95%, at least 97%, at least 99%, or 100% identity to a sequence as set forth in SEQ ID NO: 4. In some cases, the mitochondrial targeting sequence comprises a sequence having at least 90%, at least 95%, at least 97%, at least 99%, or 100% identity to a sequence as set forth in SEQ ID NO: 5.

In some cases, the mitochondrial protein coding sequence comprises a sequence having at least 90%, at least 95%, at least 97%, at least 99%, or 100% identity to a sequence as set forth in SEQ ID NO: 7 or 8. In some cases, the mitochondrial protein coding sequence comprises a sequence having at least 90%, at least 95%, at least 97%, at least 99%, or 100% identity to a sequence as set forth in SEQ ID NO: 10. In some cases, the mitochondrial protein coding sequence comprises a sequence having at least 90%, at least 95%, at least 97%, at least 99%, or 100% identity to a sequence as set forth in SEQ ID NO: 12.

In some cases, the 3' UTR nucleic acid sequence comprises a sequence having at least 90%, at least 95%, at least 97%, at least 99% or 100% identity to a sequence selected from the group consisting of SEQ ID NOs: 111-125. In some cases, the 3' UTR nucleic acid sequence comprises a sequence having at least 90%, at least 95%, at least 97%, at least 99%, or 100% identity to a sequence as set forth in SEQ ID NO: 13 or SEQ ID NO: 14.

In some cases, the recombinant nucleic acid comprises a sequence having at least 90%, at least 95%, at least 97%, at least 99%, or 100% identity to a sequence selected from the group consisting of SEQ ID NOs: 17-20, 23-24, 27-28, 31-34, 37-38, 41-42, 45-48, 51-52, 55-56, 59-62, 65-66, 69-70, 73-76, 79-80, and 83-84.

In another aspect, disclosed herein is a recombinant nucleic acid comprising: a mitochondrial targeting sequence comprising a sequence having at least 90% identity to a sequence selected from the group consisting of SEQ ID NOs: 2, 3, 4, and 5; a mitochondrial protein coding sequence, wherein the mitochondrial protein coding sequence encodes a polypeptide comprising a mitochondrial protein; and a 3' UTR nucleic acid sequence.

In some cases, the mitochondrial targeting sequence comprises a sequence having at least 90%, at least 95%, at least 97%, at least 99%, or 100% identity to a sequence as set forth in SEQ ID NO: 2. In some cases, the mitochondrial targeting sequence comprises a sequence having at least 90%, at least 95%, at least 97%, at least 99%, or 100% identity to a sequence as set forth in SEQ ID NO: 3. In some cases, the mitochondrial targeting sequence comprises a sequence having at least 90%, at least 95%, at least 97%, at least 99%, or 100% identity to a sequence as set forth in SEQ ID NO: 4. In some cases, the mitochondrial targeting sequence comprises a sequence having at least 90%, at least 95%, at least 97%, at least 99%, or 100% identity to a sequence as set forth in SEQ ID NO: 5.

In some cases, the mitochondrial protein is selected from the group consisting of NADH dehydrogenase 4 (ND4), NADH dehydrogenase 6 (ND6), NADH dehydrogenase 1 (ND1), and variants thereof. In some cases, the mitochondrial protein comprises NADH dehydrogenase 4 (ND4) or a variant thereof. In some cases, the mitochondrial protein comprises a peptide sequence having at least 90%, at least 95%, at least 97%, at least 99%, or 100% identity to a sequence as set forth in SEQ ID NO: 160. In some cases, the mitochondrial protein coding sequence comprises a sequence having at least 90%, at least 95%, at least 97%, at least 99%, or 100% identity to a sequence as set forth in SEQ ID NOs: 6, 7, or 8. In some cases, the mitochondrial protein comprises NADH dehydrogenase 6 (ND6) or a variant thereof. In some cases, the mitochondrial protein comprises a sequence having at least 90%, at least 95%, at least 97%, at least 99%, or 100% identity to a sequence as set forth in SEQ ID NO: 161. In some cases, the mitochondrial protein coding sequence comprises a sequence having at least 90%, at least 95%, at least 97%, at least 99%, or 100% identity to a sequence as set forth in SEQ ID NO: 9 or 10. In some cases, the mitochondrial protein comprises NADH dehydrogenase 1 (ND1) or a variant thereof. In some cases, the mitochondrial protein comprises a sequence having at least 90%, at least 95%, at least 97%, at least 99%, or 100% identity to a sequence as set forth in SEQ ID NO: 162. In some cases, the mitochondrial protein coding sequence comprises a sequence having at least 90%, at least 95%, at least 97%, at least 99%, or 100% identity to a sequence as set forth in SEQ ID NO: 11 or 12.

In some cases, the 3' UTR nucleic acid sequence is positioned at the 3' end of the mitochondrial targeting sequence. In some cases, the 3' UTR nucleic acid sequence comprises a sequence selected from the group consisting of: hsACO2, hsATP5B, hsAK2, hsALDH2, hsCOX10, hsUQCRFS 1, hsNDUFV1, hsNDUFV2, hsSOD2, hsCOX6c, hsIRP1, hsMRPS12, hsATP5J2, rnSOD2, and hsOXA1L. In some cases, the 3' UTR nucleic acid sequence comprises a sequence having at least 90%, at least 95%, at least 97%, at least 99% or 100% identity to a sequence selected from the group consisting of SEQ ID NOs: 111-125. In some cases, the 3' UTR nucleic acid sequence comprises a sequence having at least 90%, at least 95%, at least 97%, at least 99%, or 100% identity to a sequence as set forth in SEQ ID NO: 13 or SEQ ID NO: 14.

In some cases, the mitochondrial targeting sequence is positioned at the 5' end of the 3' UTR nucleic acid sequence. In some cases, the mitochondrial targeting sequence is positioned at the 3' end of the mitochondrial targeting sequence.

In some cases, the recombinant nucleic acid comprises a sequence having at least 90%, at least 95%, at least 97%, at least 99%, or 100% identity to a sequence selected from the group consisting of SEQ ID NOs: 29-84.

In another aspect, disclosed herein is a recombinant nucleic acid comprising: a mitochondrial targeting sequence; a mitochondrial protein coding sequence comprising a sequence having at least 90%, at least 95%, at least 97%, at least 99% or 100% identity to a sequence selected from the group consisting of SEQ ID NOs: 7, 8, 10 and 12; and a 3' UTR nucleic acid sequence.

In some cases, the mitochondrial targeting sequence comprises a sequence encoding a polypeptide selected from the group consisting of: hsCOX10, hsCOX8, scRPM2, lcSirt5, tbNDUS7, ncQCR2, hsATP5G2, hsLACTB, spilv1, gmCOX2, crATP6, hsOPA1, hsSDHD, hsADCK3, osP0644B06.24-2, *Neurospora crassa* ATP9 (ncATP9), hsGHITM, hsNDUFAB1, hsATP5G3, crATP6_hsADCK3, ncATP9_ncATP9, zmLOC100282174, ncATP9_zmLOC100282174_spilv1_ncATP9, zmLOC 100282174_hsADCK3_crATP6_hsATP5G3, zmLOC100282174_hsADCK3_hsATP5G3, ncATP9_zmLOC100282174, hsADCK3_zmLOC100282174_crATP6_hsATP5G3, crATP6_hsADCK3_zmLOC100282174_hsATP5G3, hsADCK3_zmLOC 100282174, hsADCK3_zmLOC100282174_crATP6, ncATP9_zmLOC100282174_spilv1_GNFP_ncATP9, and ncATP9_zmLOC_100282174_spilv1_lcSirt5_osP0644B06.24-2_hsATP5G2_ncATP9. In some cases, the mitochondrial targeting sequence encodes a polypeptide comprising a peptide sequence having at least 90%, at least 95%, at least 97%, at least 99% or 100% identity to a sequence selected from the group consisting of SEQ ID NOs: 129-159. In some cases, the mitochondrial targeting sequence comprises a sequence having at least 90%, at least 95%, at least 97%, at least 99%, or 100% identity to a sequence as set forth in SEQ ID NO: 2 or 3. In some cases, the mitochondrial targeting sequence comprises a sequence having at least 90%, at least 95%, at least 97%, at least 99%, or 100% identity to a sequence as set forth in SEQ ID NO: 4. In some cases, the mitochondrial targeting sequence comprises a sequence having at least 90%, at least 95%, at least 97%, at least 99%, or 100% identity to a sequence as set forth in SEQ ID NO: 5.

In some cases, the mitochondrial protein coding sequence comprises a sequence having at least 90%, at least 95%, at least 97%, at least 99%, or 100% identity to a sequence as set forth in SEQ ID NO: 7 or 8. In some cases, the mitochondrial protein coding sequence comprises a sequence having at least 90%, at least 95%, at least 97%, at least 99%, or 100% identity to a sequence as set forth in SEQ ID NO: 10. In some cases, the mitochondrial protein coding sequence comprises a sequence having at least 90%, at least 95%, at least 97%, at least 99%, or 100% identity to a sequence as set forth in SEQ ID NO: 12.

In some cases, the 3' UTR nucleic acid sequence is positioned at the 3' end of the mitochondrial targeting sequence. In some cases, the 3' UTR nucleic acid sequence comprises a sequence selected from the group consisting of: hsACO2, hsATP5B, hsAK2, hsALDH2, hsCOX10, hsUQCRFS1, hsNDUFV1, hsNDUFV2, hsSOD2, hsCOX6c, hsIRP1, hsMRPS12, hsATP5J2, rnSOD2, and hsOXA1L. In some cases, the 3' UTR nucleic acid sequence comprises a sequence having at least 90%, at least 95%, at least 97%, at least 99% or 100% identity to a sequence selected from the group consisting of SEQ ID NOs: 111-125. In some cases, the 3' UTR nucleic acid sequence comprises a sequence having at least 90%, at least 95%, at least 97%, at least 99%, or 100% identity to a sequence as set forth in SEQ ID NO: 13 or SEQ ID NO: 14.

In some cases, the mitochondrial targeting sequence is positioned at the 5' end of the 3' UTR nucleic acid sequence. In some cases, the mitochondrial targeting sequence is positioned at the 3' end of the mitochondrial targeting sequence.

In some cases, the recombinant nucleic acid comprises a sequence having at least 90%, at least 95%, at least 97%, at least 99%, or 100% identity to a sequence selected from the group consisting of SEQ ID NOs: 17-20, 23-24, 27-28, 31-34, 37-38, 41-42, 45-48, 51-52, 55-56, 59-62, 65-66, 69-70, 73-76, 79-80, and 83-84.

In another aspect, disclosed herein is a recombinant nucleic acid comprising a mitochondrial targeting sequence having at least 90%, at least 95%, at least 97%, at least 99%, or 100% identity to a sequence selected from the group consisting of SEQ ID NOs: 2, 3, and 4. In some cases, the mitochondrial targeting sequence comprises a sequence having at least 90%, at least 95%, at least 97%, at least 99%, or 100% identity to a sequence as set forth in SEQ ID NO: 2. In some cases, the mitochondrial targeting sequence comprises a sequence having at least 90%, at least 95%, at least 97%, at least 99%, or 100% identity to a sequence as set forth in SEQ ID NO: 3. In some cases, the mitochondrial targeting sequence comprises a sequence having at least 90%, at least 95%, at least 97%, at least 99%, or 100% identity to a sequence as set forth in SEQ ID NO: 4.

In some cases, the recombinant nucleic acid further comprises a mitochondrial protein coding sequence, wherein the mitochondrial protein coding sequence encodes a polypeptide comprising a mitochondrial protein. In some cases, the mitochondrial protein is selected from the group consisting of NADH dehydrogenase 4 (ND4), NADH dehydrogenase 6 (ND6), NADH dehydrogenase 1 (ND1), and variants thereof. In some cases, the mitochondrial protein comprises NADH dehydrogenase 4 (ND4) or a variant thereof. In some cases, the mitochondrial protein comprises a peptide sequence having at least 90%, at least 95%, at least 97%, at least 99%, or 100% identity to a sequence as set forth in SEQ ID NO: 160. In some cases, the mitochondrial protein coding sequence comprises a sequence having at least 90%, at least 95%, at least 97%, at least 99%, or 100% identity to a sequence as set forth in SEQ ID NOs: 6, 7, or 8. In some cases, the mitochondrial protein comprises NADH dehydrogenase 6 (ND6) or a variant thereof. In some cases, the mitochondrial protein comprises a sequence having at least 90%, at least 95%, at least 97%, at least 99%, or 100% identity to a sequence as set forth in SEQ ID NO: 161. In some cases, the mitochondrial protein coding sequence comprises a sequence having at least 90%, at least 95%, at least 97%, at least 99%, or 100% identity to a sequence as set forth in SEQ ID NO: 9 or 10. In some cases, the mitochondrial protein comprises NADH dehydrogenase 1 (ND1) or a variant thereof. In some cases, the mitochondrial protein comprises a sequence having at least 90%, at least 95%, at least 97%, at least 99%, or 100% identity to a sequence as set forth in SEQ ID NO: 162. In some cases, the mitochondrial protein coding sequence comprises a sequence having at least 90%, at least 95%, at least 97%, at least 99%, or 100% identity to a sequence as set forth in SEQ ID NO: 11 or 12.

In some cases, the recombinant nucleic acid further comprises a 3' UTR nucleic acid sequence. In some cases, the 3' UTR nucleic acid sequence is positioned at the 3' end of the mitochondrial targeting sequence. In some cases, the 3' UTR nucleic acid sequence comprises a sequence selected from the group consisting of: hsACO2, hsATP5B, hsAK2, hsALDH2, hsCOX10, hsUQCRFS1, hsNDUFV1, hsNDUFV2, hsSOD2, hsCOX6c, hsIRP1, hsMRPS12, hsATP5J2, rnSOD2, and hsOXA1L. In some cases, the 3' UTR nucleic acid sequence comprises a sequence having at least 90%, at least 95%, at least 97%, at least 99% or 100% identity to a sequence selected from the group consisting of SEQ ID NOs: 111-125. In some cases, the 3' UTR nucleic acid sequence comprises a sequence having at least 90%, at least 95%, at least 97%, at least 99%, or 100% identity to a sequence as set forth in SEQ ID NO: 13 or SEQ ID NO: 14. In some cases, the mitochondrial targeting sequence is positioned at the 5' end of the 3' UTR nucleic acid sequence. In some cases, the mitochondrial targeting sequence is positioned at the 3' end of the mitochondrial targeting sequence.

In some cases, the recombinant nucleic acid comprises a sequence having at least 90%, at least 95%, at least 97%, at least 99%, or 100% identity to a sequence selected from the group consisting of SEQ ID NOs: 29-70.

In another aspect, disclosed herein is a recombinant nucleic acid comprising a mitochondrial protein coding sequence, wherein the mitochondrial protein coding sequence encodes a polypeptide comprising a mitochondrial protein, wherein the mitochondrial protein coding sequence comprises a sequence having at least 90%, at least 95%, at least 97%, at least 99%, or 100% identity to a sequence selected from the group consisting of SEQ ID NOs: 7, 8, 10, and 12.

In some cases, the recombinant nucleic acid further comprises a mitochondrial targeting sequence. In some cases, the mitochondrial targeting sequence comprises a sequence encoding a polypeptide selected from the group consisting of: hsCOX10, hsCOX8, scRPM2, lcSirt5, tbNDUS7, ncQCR2, hsATP5G2, hsLACTB, spilv1, gmCOX2, crATP6, hsOPA1, hsSDHD, hsADCK3, osP0644B06.24-2, *Neurospora crassa* ATP9 (ncATP9), hsGHITM, hsNDUFAB1, hsATP5G3, crATP6_hsADCK3, ncATP9_ncATP9, zmLOC100282174, ncATP9_zmLOC100282174_spilv1_ncATP9, zmLOC100282174_hsADCK3_crATP6_hsATP5G3, zmLOC100282174_hsADCK3_hsATP5G3, ncATP9_zmLOC100282174, hsADCK3_zmLOC100282174_crATP6_hsATP5G3, crATP6_hsADCK3_zmLOC100282174_hsATP5G3, hsADCK3_zmLOC100282174, hsADCK3_zmLOC100282174_crATP6, ncATP9_zmLOC100282174_spilv1_GNFP_ncATP9, and ncATP9_zmLOC100282174_spilv1_lcSirt5_osP0644B06.24-2_hsATP5G2_ncATP9. In some cases, the mitochondrial targeting sequence encodes a polypeptide comprising a peptide sequence having at least 90%, at least 95%, at least 97%, at least 99% or 100% identity to a sequence selected from the group consisting of SEQ ID NOs: 129-159. In some cases, the mitochondrial targeting sequence comprises a sequence having at least 90%, at least 95%, at least 97%, at least 99%, or 100% identity to a sequence as set forth in SEQ ID NO: 2. In some cases, the mitochondrial targeting sequence comprises a sequence having at least 90%, at least 95%, at least 97%, at least 99%, or 100% identity to a sequence as set forth in SEQ ID NO: 3. In some cases, the mitochondrial targeting sequence comprises a sequence having at least 90%, at least 95%, at least 97%, at least 99%, or 100% identity to a sequence as set forth in SEQ ID NO: 4. In some cases, the mitochondrial targeting sequence comprises a sequence having at least 90%, at least 95%, at least 97%, at least 99%, or 100% identity to a sequence as set forth in SEQ ID NO: 5.

In some cases, the mitochondrial protein coding sequence comprises a sequence having at least 90%, at least 95%, at least 97%, at least 99%, or 100% identity to a sequence as set forth in SEQ ID NO: 7 or 8. In some cases, the mitochondrial protein coding sequence comprises a sequence having at least 90%, at least 95%, at least 97%, at least 99%, or 100% identity to a sequence as set forth in SEQ ID NO: 10. In some cases, the mitochondrial protein coding sequence comprises a sequence having at least 90%, at least 95%, at least 97%, at least 99%, or 100% identity to a sequence as set forth in SEQ ID NO: 12.

In some cases, the recombinant nucleic acid further comprises a 3' UTR nucleic acid sequence. In some cases, the 3' UTR nucleic acid sequence is positioned at the 3' end of the mitochondrial targeting sequence. In some cases, the 3' UTR nucleic acid sequence comprises a sequence selected from the group consisting of: hsACO2, hsATP5B, hsAK2, hsALDH2, hsCOX10, hsUQCRFS1, hsNDUFV1, hsNDUFV2, hsSOD2, hsCOX6c, hsIRP1, hsMRPS12, hsATP5J2, rnSOD2, and hsOXA1L. In some cases, the 3' UTR nucleic acid sequence comprises a sequence having at least 90%, at least 95%, at least 97%, at least 99% or 100% identity to a sequence selected from the group consisting of SEQ ID NOs: 111-125. In some cases, the 3' UTR nucleic acid sequence comprises a sequence having at least 90%, at least 95%, at least 97%, at least 99%, or 100% identity to a sequence as set forth in SEQ ID NO: 13 or SEQ ID NO: 14. In some cases, the mitochondrial targeting sequence is positioned at the 5' end of the 3' UTR nucleic acid sequence. In some cases, the mitochondrial targeting sequence is positioned at the 3' end of the mitochondrial targeting sequence.

In some cases, the recombinant nucleic acid comprises a sequence having at least 90%, at least 95%, at least 97%, at least 99%, or 100% identity to a sequence selected from the group consisting of SEQ ID NOs: 17-20, 23-24, 27-28, 31-34, 37-38, 41-42, 45-48, 51-52, 55-56, 59-62, 65-66, 69-70, 73-76, 79-80, and 83-84.

In another aspect, disclosed herein is a viral vector comprising a recombinant nucleic acid disclosed herein. In some cases, the viral vector is an adeno-associated virus (AAV) vector. In some cases, the AAV vector is selected from the group consisting of: AAV1, AAV2, AAV3, AAV4, AAV5, AAV6, AAV7, AAV8, AAV9, AAV10, AAV11, AAV12, AAV13, AAV14, AAV15 and AAV16 vectors. In some cases, the AAV vector is a recombinant AAV (rAAV) vector. In some cases, the rAAV vector is an rAAV2 vector.

In another aspect, disclosed herein is a method for treating an ocular disease, which comprises administering to a patient in need thereof any of the pharmaceutical compositions disclosed herein. In some cases, the ocular disease is Leber's hereditary optic neuropathy (LHON). In some cases, the method comprises administering the pharmaceutical composition to one or both eyes of the patient. In some cases, the pharmaceutical composition is administered by intraocular injection or intravitreal injection. In some cases, the pharmaceutical composition is administered by intravitreal injection. In some cases, about 0.01-0.1 mL of the pharmaceutical composition is administered by intravitreal injection. In some cases, about 0.05 mL of the pharmaceutical composition is administered by intravitreal injection.

In some cases, the method further comprises administering methylprednisolone to the patient. In some cases, methylprednisolone is administered prior to intravitreal injection of the pharmaceutical composition. In some cases, methylprednisolone is administered orally. In some cases, methylprednisolone is administered daily for at least 1 day, 2 days, 3 days, 4 days, 5 days, 6 days, or 7 days prior to intravitreal injection of the pharmaceutical composition. In some cases, methylprednisolone is administered daily. In some cases, methylprednisolone is administered at a daily dose of about 32 mg/60 kg. In some cases, methylprednisolone is administered after intravitreal injection of the pharmaceutical composition. In some cases, the method further comprises administering creatine phosphate sodium to the patient. In some cases, creatine phosphate sodium is administered intravenously. In some cases, methylprednisolone is administered intravenously or orally. In some cases, the method comprises administering methylprednisolone intravenously for at least one day, followed by administering methylprednisolone orally for at least one week. In some cases, the method comprises administering methylprednisolone intravenously for about 3 days, followed by administering methylprednisolone orally for at least about 6 weeks. In some cases, methylprednisolone is administered intravenously at a daily dose of about 80 mg/60 kg. In some cases, the administration of the pharmaceutical composition results in a higher average recovery level of vision than administration of a comparable pharmaceutical composition without the recombinant nucleic acid. In some cases, the administration of the pharmaceutical composition results in a higher average recovery level of vision than administration of a comparable pharmaceutical composition comprising the recombinant nucleic acid as set forth in SEQ ID NO: 15.

In some embodiments, the present disclosure provides a method for treating an ocular disease, which comprises administering to a patient in need thereof (a) a first pharmaceutical composition comprising an adeno-associated virus (AAV) comprising a recombinant nucleic acid, wherein the recombinant nucleic acid comprises: (i) a nucleic acid sequence encoding a mitochondrial targeting peptide; (ii) a nucleic acid sequence encoding a mitochondrial protein, wherein the nucleic acid sequence comprises a nucleic acid sequence having at least 90%, at least 95%, at least 97%, at least 99% or 100% identity to a sequence selected from the group consisting of SEQ ID NOs: 6-12; and (iii) a 3' UTR nucleic acid sequence; and (b) a second pharmaceutical composition comprising a steroid.

In some embodiments, the nucleic acid sequence encoding the mitochondrial protein encodes a polypeptide comprising an amino acid sequence having at least 90%, at least 95%, at least 97%, at least 99% or 100% identity to a sequence selected from the group consisting of SEQ ID NOs: 160-162. In some embodiments, the nucleic acid sequence encoding a mitochondrial targeting peptide encodes a polypeptide comprising an amino acid sequence having at least 90%, at least 95%, at least 97%, at least 99% or 100% identity to a sequence selected from the group consisting of SEQ ID NOs: 126-159. In some embodiments, the nucleic acid sequence encoding a mitochondrial targeting peptide comprises a nucleic acid sequence having at least 90%, at least 95%, at least 97%, at least 99%, or 100% identity to a sequence selected from the group consisting of SEQ ID NOs: 1-5. In some embodiments, the 3' UTR nucleic acid sequence comprises a nucleic acid sequence (nucleic sequence) having at least 90%, at least 95%, at least 97%, at least 99% or 100% identity to a sequence selected from the group consisting of SEQ ID NOs: 13, 14 and 111-125.

In some embodiments, the present disclosure provides a method for treating an ocular disease, which comprises administering to a patient in need thereof (a) a first pharmaceutical composition comprising an adeno-associated virus (AAV) comprising a recombinant nucleic acid, wherein the recombinant nucleic acid comprises: (i) a nucleic acid sequence encoding a mitochondrial targeting peptide comprising an amino acid sequence (amino sequence) having at least 90%, at least 95%, at least 97%, at least 99% or 100% identity to a sequence selected from the group consisting of SEQ ID NOs: 126-159; (ii) a nucleic acid sequence encoding a mitochondrial protein; and (iii) a 3' UTR nucleic acid sequence; and (b) a second pharmaceutical composition comprising a steroid.

In some embodiments, the mitochondrial protein is selected from the group consisting of: NADH dehydrogenase 4 (ND4), NADH dehydrogenase 6 (ND6), NADH dehydrogenase 1 (ND1), and variants thereof. In some embodiments, the nucleic acid sequence encoding a mitochondrial protein comprises a nucleic acid sequence having at least 90%, at least 95%, at least 97%, at least 99%, or 100% identity to a nucleic acid sequence selected from the group consisting of SEQ ID NOs: 6-12. In some embodiments, the nucleic acid sequence encoding a mitochondrial protein encodes a polypeptide comprising an amino acid sequence having at least 90%, at least 95%, at least 97%, at least 99% or 100% identity to a sequence selected from the group consisting of SEQ ID NOs: 160-162. In some embodiments, the nucleic acid sequence encoding a mitochondrial targeting peptide comprises a nucleic acid sequence having at least 90%, at least 95%, at least 97%, at least 99%, or 100% identity to a sequence selected from the group consisting of SEQ ID NOs: 1-5. In some embodiments, the 3' UTR nucleic acid sequence comprises a sequence having at least 90%, at least 95%, at least 97%, at least 99% or 100% identity to a sequence selected from the group consisting of SEQ ID NOs: 13, 14 and 111-125.

In some embodiments, the present disclosure provides a method for treating an ocular disease, which comprises administering to a patient in need thereof (a) a first pharmaceutical composition comprising an adeno-associated virus (AAV) comprising a recombinant nucleic acid, wherein the recombinant nucleic acid comprises: (i) a nucleic acid sequence encoding a mitochondrial targeting peptide comprising an amino acid sequence having at least 90%, at least 95%, at least 97%, at least 99% or 100% identity to a sequence selected from the group consisting of SEQ ID NOs: 126-159; (ii) a nucleic acid sequence encoding a mitochondrial protein comprising a nucleic acid sequence having at least 90%, at least 95%, at least 97%, at least 99% or 100% identity to a sequence selected from the group consisting of SEQ ID NOs: 6-12; and (iii) a 3' UTR nucleic acid sequence having at least 90%, at least 95%, at least 97%, at least 99% or 100% identity to a sequence selected from the group consisting of SEQ ID NOs: 13, 14 and 111-125; and (b) a second pharmaceutical composition comprising a steroid.

In some embodiments, the present disclosure provides a method for treating an ocular disease, which comprises administering to a patient in need thereof (a) a first pharmaceutical composition comprising an adeno-associated virus (AAV) comprising a recombinant nucleic acid, wherein the recombinant nucleic acid comprises: (i) a nucleic acid sequence encoding a mitochondrial targeting peptide; and (ii) a nucleic acid sequence encoding a mitochondrial protein; and (b) a second pharmaceutical composition comprising a steroid. In some embodiments, the mitochondrial protein is selected from the group consisting of: NADH dehydrogenase 4 (ND4), NADH dehydrogenase 6 (ND6), NADH dehydrogenase 1 (ND1), and variants thereof. In some embodiments, the 3' UTR nucleic acid sequence comprises a nucleic acid sequence having at least 90%, at least 95%, at least 97%, at least 99% or 100% identity to a sequence selected from the group consisting of SEQ ID NOs: 13, 14 and 111-125.

In some embodiments, the recombinant nucleic acid comprises a sequence having at least 90%, at least 95%, at least 97%, at least 99%, or 100% identity to a sequence selected from the group consisting of SEQ ID NOs: 15-84. In some embodiments, the recombinant nucleic acid comprises a sequence having at least 90%, at least 95%, at least 97%, at least 99%, or 100% identity to SEQ ID NO: 15.

In some embodiments, the first pharmaceutical composition is administered by intraocular injection or intravitreal injection. In some embodiments, about 0.01-0.1 mL of the first pharmaceutical composition is administered by intravitreal injection. In some embodiments, about 0.05 mL of the first pharmaceutical composition is administered by intravitreal injection. In some embodiments, the first pharmaceutical composition is administered to one or both eyes of the patient.

In some embodiments, the steroid is selected from the group consisting of: alclometasone dipropionate, amcinonide, beclomethasone dipropionate, beclomethasone, betamethasone benzoate, betamethasone dipropionate, betamethasone sodium phosphate, betamethasone sodium phosphate and sodium acetate, betamethasone valerate, clobetasol propionate, clocortolone pivalate, cortisol (hydrocortisone), cortisol acetate (hydrocortisone), cortisone butyrate (hydrocortisone), cortisol cypionate (hydrocortisone), cortisol (hydrocortisone) sodium phosphate, cortisol (hydrocortisone) sodium succinate, cortisol valerate (hydrocortisone), cortisone acetate, desonide, desoximetasone, dexamethasone, dexamethasone acetate, dexamethasone sodium phosphate, diflorasone diacetate, fludrocortisone acetate, flunisolide, fluocinonid acetate, fluocinonide, fluorometholone, flurandrenolide, halcinonide, medrysone, methylprednisolone, methylprednisolone acetate, methylprednisolone sodium succinate, mometasone furoate, paramethasone acetate, prednisolone, prednisolone acetate, prednisolone sodium phosphate, prednisolone butylacetate, prednisone, triamcinolone, triamcinolone acetonide, triamcinolone diacetate, and triamcinolone hexaacetonide or synthetic analogs thereof.

In some embodiments, the steroid is a glucocorticoid. In some embodiments, the glucocorticoid is methylprednisolone or prednisone.

In some embodiments, methylprednisolone is formulated as a tablet or liquid for intravenous administration. In some embodiments, the steroid is administered orally or intravenously.

In some embodiments, the steroid is administered prior to the administration of the first pharmaceutical composition. In some embodiments, the steroid is administered daily for at least 1 day, 2 days, 3 days, 4 days, 5 days, 6 days, or 7 days prior to the administration of the first pharmaceutical composition. In some embodiments, the steroid is methylprednisolone, and is administered at a daily dose of about 30 mg/60 kg to about 40 mg/60 kg or about 30 mg to about 40 mg. In some embodiments, the daily dose of methylprednisolone is about 32 mg/60 kg or 32 mg. In some embodiments, the steroid is prednisone and is administered at a daily dose of about 50 mg/60 kg to about 70 mg/60 kg. In some embodiments, the daily dose of prednisone is about 60 mg/60 kg.

In some embodiments, the steroid is administered after the administration of the first pharmaceutical composition. In some embodiments, the steroid is administered daily for at least 1 day, at least 2 days, at least 3 days, at least 4 days, at least 5 days, at least 6 days, at least 7 days, at least 8 days, at least 9 days, at least 10 days, at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, or at least 15 weeks after the administration of the first pharmaceutical composition.

In some embodiments, the steroid is methylprednisolone and is administered at a daily dose of between about 70 mg/60 kg and 90 mg/60 kg or between about 70 mg and 90 mg. In some embodiments, the daily dose of the methylprednisolone is about 80 mg/60 kg or 80 mg. In some embodiments, the methylprednisolone is administered for at least two days after the administration of the first pharmaceutical composition. In some embodiments, subsequent doses of the methylprednisolone are administered daily for at least 7 weeks after the administration of the first pharmaceutical composition, wherein the dose of the methylprednisolone is reduced week by week.

In some embodiments, the steroid is prednisone and is administered at a daily dose of between about 50 mg/60 kg and 70 mg/60 kg or between about 50 mg and about 70 mg. In some embodiments, the daily dose of the prednisone is about 60 mg/60 kg or 60 mg. In some embodiments, the prednisone is administered for at least seven days after the administration of the first pharmaceutical composition. In some embodiments, wherein after seven days, the prednisone is administered at a daily dose of between about 30 mg/60 kg and about 50 mg/60 kg or between about 30 mg and 50 mg. In some embodiments, the daily dose of the prednisone is about 40 mg/60 kg or 40 mg. In some embodiments, subsequent doses of the prednisone are administered daily for at least 4 days, wherein the dose of the prednisone is reduced daily.

In some embodiments, the steroid is administered before and after the administration of the first pharmaceutical composition.

In some embodiments, the steroid is methylprednisolone and is administered daily for at least seven days prior to the administration of the first pharmaceutical composition and administered daily for at least 7 weeks after the administration of the first pharmaceutical composition. In some embodiments, the methylprednisolone is administered at a daily dose of about 32 mg/60 kg or 32 mg prior to the administration of the first pharmaceutical composition. In some embodiments, the methylprednisolone is administered at a daily dose of about 80 mg/60 kg or 80 mg for at least 2 days after the administration of the first pharmaceutical composition. In some embodiments, the methylprednisolone is administered at a daily dose of about 40 mg/60 kg or 40 mg for at least 4 days beginning three days after the administration of the first pharmaceutical composition. In some embodiments, the methylprednisolone is administered at a daily dose of about 32 mg/60 kg or 32 mg for at least one week beginning one week after the administration of the first pharmaceutical composition. In some embodiments, the methylprednisolone is administered at a daily dose of about 24 mg/60 kg or 24 mg for at least one week beginning two weeks after the administration of the first pharmaceutical composition. In some embodiments, the methylprednisolone is administered at a daily dose of about 16 mg/60 kg or 16 mg for at least one week beginning three weeks after the administration of the first pharmaceutical composition. In some embodiments, the methylprednisolone is administered at a daily dose of about 8 mg/60 kg or 8 mg for at least one week beginning four weeks after the administration of the first pharmaceutical composition. In some embodiments, the methylprednisolone is administered at a daily dose of about 6 mg/60 kg or 6 mg for at least one week beginning five weeks after the administration of the first pharmaceutical composition. In some embodiments, the methylprednisolone is administered at a daily dose of about 4 mg/60 kg or 4 mg for at least one week beginning six weeks after the administration of the first pharmaceutical composition.

In some embodiments, the steroid is prednisone and is administered daily for at least two days prior to the administration of the first pharmaceutical composition and administered daily for at least eleven days after the administration of the first pharmaceutical composition. In some embodiments, the prednisone is administered at a daily dose of about 60 mg/60 kg or 60 mg prior to the administration of the first pharmaceutical composition. In some embodiments, the prednisone is administered at a daily dose of about 60 mg/60 kg or 60 mg for at least seven days after the administration of the first pharmaceutical composition. In some embodiments, the prednisone is administered at a daily dose of about 40 mg/60 kg or 40 mg for at least one day eight days after the administration of the first pharmaceutical composition. In some embodiments, the prednisone is administered at a daily dose of about 20 mg/60 kg or 20 mg for at least one day nine days after the administration of the first pharmaceutical composition. In some embodiments, the prednisone is administered at a daily dose of about 10 mg/60 kg or 10 mg for at least one day ten days after the administration of the first pharmaceutical composition.

In some embodiments, the method further comprises administering creatine phosphate sodium to the patient. In some embodiments, the creatine phosphate sodium is administered intravenously before and/or after the administration of the first pharmaceutical composition.

In some embodiments, the administration of the first pharmaceutical composition and the second pharmaceutical composition results in a higher average recovery level of vision than a comparable pharmaceutical composition administered without the second pharmaceutical composition. In some embodiments, the administration of the first pharmaceutical composition and the second pharmaceutical composition results in a lower incidence of adverse events than the comparable pharmaceutical composition administered without the second pharmaceutical composition. In some embodiments, the adverse events are selected from anterior chamber inflammation, vitritis, ocular hypertension, cataract removal, keratitis, vitreous hemorrhage, allergic conjunctivitis and ophthalmalgia. In some embodiments, the higher average recovery level of vision and the lower incidence of adverse events are determined in a patient population with the ocular disease. In some embodiments, the patient population is ethnically matched. In some embodiments, the patient population is Chinese or Argentine.

In some embodiments, the ocular disease is Leber's hereditary optic neuropathy (LHON). In some embodiments, the AAV is selected from AAV1, AAV2, AAV3, AAV4, AAV5, AAV6, AAV7, AA8, AAV9, and AAV10. In some embodiments, the AAV is the AAV2.

In some embodiments, the present disclosure provides a method for screening and treating a patient with an ocular disease, which comprises: (a) obtaining a serum sample from the patient; (b) culturing a target cell population together with a composition comprising an adeno-associated virus (AAV) in the presence of the serum sample, wherein the AAV comprises a recombinant nucleic acid encoding a detectable marker; and (c) detecting an expression level of the detectable marker in the target cell population after culturing, wherein the patient is selected for the treatment if the expression level of the detectable marker in the target cell population is higher than a predetermined threshold.

In some embodiments, the present disclosure provides a method for screening and treating a patient with an ocular disease, which comprises: (a) culturing a target cell population together with a composition comprising an adeno-associated virus (AAV) in the presence of a serum sample from the patient, wherein the AAV comprises a recombinant nucleic acid encoding a detectable marker; and (b) detecting an expression level of the detectable marker in the target cell population after culturing, wherein the patient is selected for the treatment if the expression level of the detectable marker in the target cell population is higher than a predetermined threshold.

In some embodiments, the present disclosure provides a method for treating an ocular disease in a patient in need thereof, which comprises: (a) obtaining a serum sample from the patient; (b) culturing a target cell population together with a composition comprising a first adeno-associated virus (AAV) in the presence of the serum sample, wherein the first AAV comprises a first recombinant nucleic acid encoding a detectable marker; (c) detecting an expression level of the detectable marker in the target cell population; and (d) administering a pharmaceutical composition comprising a second AAV to the patient, wherein the second AAV comprises a second recombinant nucleic acid, wherein the expression level of the detectable marker in the target cell population is higher than a predetermined threshold.

In some embodiments, the present disclosure provides a method for treating an ocular disease in a patient in need thereof, including: (a) culturing a target cell population together with a composition comprising a first adeno-associated virus (AAV) in the presence of a serum sample from the patient, wherein the first AAV comprises a first recombinant nucleic acid encoding a detectable marker; (b) detecting an expression level of the detectable marker in the target cell population; and (c) administering a pharmaceutical composition comprising a second AAV to the patient, wherein the second AAV comprises a second recombinant nucleic acid, wherein the expression level of the detectable marker in the target cell population is higher than a predetermined threshold.

In some embodiments, the detectable marker is a fluorescent protein. In some embodiments, the fluorescent protein is a green fluorescent protein (GFP). In some embodiments, the detectable marker is detected by flow cytometry or qPCR. In some embodiments, the step of culturing is performed for at least 12 hours, 1 day, 2 days, 3 days, 4 days, 5 days, 6 days, 7 days, or more.

In some embodiments, the predetermined threshold is about 40% of cells expressing the detectable marker when detection is performed by the flow cytometry. In some embodiments, the predetermined threshold is about 0.6 of a relative expression level of the detectable marker when detection is performed by the qPCR. In some embodiments, the target cells are HEK-293 T cells.

In some embodiments, the treatment is a recombinant AAV comprising a nucleic acid sequence encoding a mitochondrial protein. In some embodiments, the mitochondrial protein is selected from the group consisting of: NADH dehydrogenase 4 (ND4), NADH dehydrogenase 6 (ND6), NADH dehydrogenase 1 (ND1), and variants thereof. In some embodiments, the patient comprises a mutation selected from G11778A in an ND4 gene, G3460A in an ND1 gene, and T14484C in an ND6 gene.

In some embodiments, the present disclosure provides a kit, which comprises comprising an adeno-associated virus (AAV), wherein the AVV comprises a recombinant nucleic acid encoding a detectable marker; a target cell population; and one or more reagents for detecting the detectable marker. In some embodiments, the kit further comprises a transfection reagent for transfecting the target cell population with the AAV. In some embodiments, the kit further comprises a second AAV comprising a recombinant nucleic acid encoding a mitochondrial protein. In some embodiments, the one or more reagents for detecting the detectable marker are selected from antibodies binding to the detectable marker and one or more primer oligonucleotides specific to the recombinant nucleic acid encoding the detectable marker.

### BRIEF DESCRIPTION OF THE DRAWINGS

The novelty features of the present invention are set forth with particularity in the appended claims. The features and advantages of the present invention will be better understood by reference to the following detailed description and accompanying drawings enumerating illustrative embodiments (in which the principles of the present invention are utilized), wherein:
FIG. 1 shows a map of a vector pAAV-CMV-ND4-3'Flag-COX10UTR-SV40;
FIG. 2 shows a map of a vector pAAV-CMV-ND4-3'Flag-COX10UTR-bGH;
FIG. 3 shows a map of a vector pAAV-CMV-ND4-3'Flag-COX10UTR;
FIG. 4 shows expression efficiency—mRNA of plasmids in HEK293 cells;
FIG. 5 shows results of the experiment for testing the stability of formulas at 2-8°C;
FIG. 6 shows results of the freeze/thaw experiment for testing the stability of formulas;

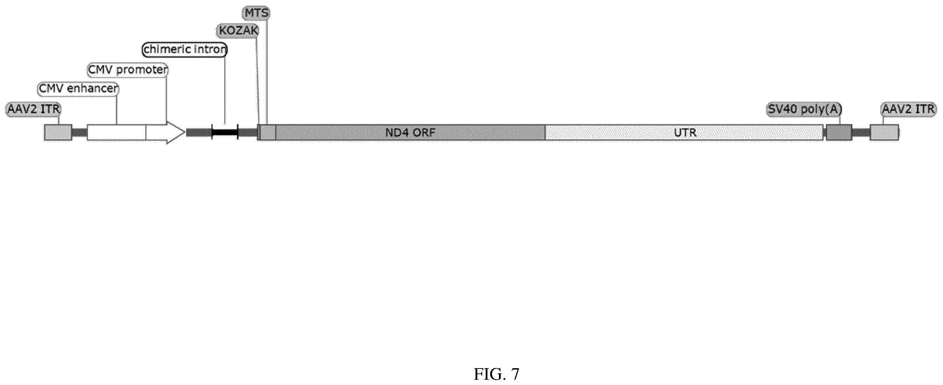

FIG. 7 shows design of an ND4 expression cassette;
FIG. 8 shows a first exemplary dosing regimen for a steroid; and
FIG. 9 shows a second exemplary dosing regimen for a steroid.

### DETAILED DESCRIPTION

### Definitions

Unless defined otherwise, all technical and scientific terms used herein have the same meaning as commonly understood by those of ordinary skill in the art to which the present disclosure belongs. Although any methods and materials similar or identical to those described herein can be used during implementing or testing of preparations or unit doses herein, some methods and materials are now described. Unless otherwise stated, techniques employed or contemplated herein are standard methodologies. The materials, methods and examples are illustrative only and not restrictive.

As used herein and in the appended claims, the singular forms a "(an)", "and" and "the (said)" include plural referents, unless the context specifies clearly otherwise. Thus, for example, reference to "a compound" includes a plurality of such agents, and reference to "the salt" includes reference to one or more salts (or reference to a plurality of salts) and equivalents thereof known to those skilled in the art, etc.

As used herein, unless otherwise indicated, the term "or" may be a conjunction or a disjunctive conjunction. As used herein, unless otherwise indicated, any embodiment may be combined with any other embodiment.

As used herein, unless otherwise indicated, some inventive embodiments herein contemplate numerical ranges. When a range is present, the range includes range endpoints. Additionally, each subrange and value within the range is present as if explicitly written out.

The term "about" and its grammatical equivalents in relation to a reference value as well as its grammatical equivalents as used herein may include a range of values plus or minus 10% of the value, such as a range of values plus or minus 10%, 9%, 8%, 7%, 6%, 5%, 4%, 3%, 2%, or 1% of the value. For example, the amount "about 10" includes amounts from 9 to 11.

The term "comprising" (and related terms such as "comprises" or "having" or "including") is not intended to exclude that certain other embodiments (e.g., embodiments of any material components, compositions, methods or processes, etc., described herein) may "consist of" or "essentially consist of' the features described.

The term "subject" refers to a mammal who has been or will be the subject of treatment, observation, or experimentation. The term "mammal" is intended to have its standard meaning and encompasses, for example, humans, dogs, cats, sheep and cows. The methods described herein can be used in human treatment and veterinary applications. In some embodiments, the subject is a human.

The term "treatment" encompasses the administration of at least one compound disclosed herein or pharmaceutically acceptable salts thereof to a mammalian subject, particularly a human subject, in need of such administration, and includes (i) suppression of development of clinical symptoms of diseases such as a cancer, (ii) regression of clinical symptoms of diseases such as a cancer, and/or (iii) prophylactic treatment to prevent attack of diseases such as a cancer.

The term "therapeutically effective amount" of a chemical entity described herein refers to an amount effective to provide a therapeutic benefit, such as ameliorating symptoms, slowing disease progression, or preventing diseases, when administered to a human or non-human subject.

As used herein, unless otherwise indicated, the terms "nucleic acid" and "polynucleotide" are used interchangeably.

As used herein, unless otherwise indicated, a drug dose of X mg/60 kg refers to the use of X mg of a drug per 60 kg of patient body weight. For example, a drug dose of 100 mg/60 kg refers to that a patient weighing 60 kg is required to take 100 mg of the drug, and correspondingly, another patient weighing 30 kg is required to take 50 mg of the drug.

As used herein, unless otherwise indicated, the term "operably linked" refers to a functional linkage between two or more sequences. For example, an operable linkage between a polynucleotide of interest and a regulatory sequence (e.g., a promoter) is a functional linkage that allows expression of the polynucleotide of interest. In this sense, the term "operably linked" refers to the positioning of a regulatory region and a coding sequence to be transcribed such that the regulatory region is effective for regulating the transcription or translation of the coding sequence of interest. In some embodiments disclosed herein, the term "operably linked" refers to a configuration in which a regulatory sequence is positioned in place relative to a sequence encoding a polypeptide or functional RNA such that a control sequence directs or modulates the expression or cellular localization of mRNA encoding the polypeptide, the polypeptide and/or the functional RNA, and a promoter is operably linked to a nucleic acid sequence if it can mediate transcription of the nucleic acid sequence. Operably linked elements may be contiguous or non-contiguous. Basic techniques for operably linking two or more DNA sequences are familiar to those of ordinary skill in the art, and these methods have been described in many books for standard molecular biology procedures (see for example, Maniatis et al., "Molecular Cloning: A Laboratory Manual" 2nd ed. Cold Spring Harbor Laboratory Press, Cold Spring Harbor, NY).

### Nucleic acid and polypeptide sequence

In one aspect herein, provided is a recombinant nucleic acid, which comprises one or more sequences selected from a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence. In some embodiments, the recombinant nucleic acid comprises all of those sequences. In some embodiments, the recombinant nucleic acid comprises (sequentially from the 5' end to the 3' end): a first ITR sequence, a promoter sequence, an intron sequence, a Kozak sequence, a mitochondrial targeting sequence, a mitochondrial protein coding sequence, 3' UTR sequence, a polyA signal sequence, and a second ITR sequence. In some embodiments, the various sequences are operably linked to each other. In some embodiments, the various regulatory sequences are operably linked to the mitochondrial protein coding sequence.

Table 1 is nucleic acid and polypeptide sequences disclosed herein. A first column shows SEQ ID NO for each sequence. A second column describes nucleic acid or polypeptide constructs. For example, a construct COX10-ND4-3'UTR (SEQ ID NO: 15) is a nucleic acid that binds to nucleic acid sequences of COX10 (SEQ ID NO: 1), ND4 (SEQ ID NO: 6) and 3'UTR (SEQ ID NO: 13).

**Table 1: Nucleic acid and polypeptide sequences and SEQ ID NO**

| **SEQ ID NO** | **Description** | **Sequences** |
|---|---|---|
| 1 | COX10 | ATGGCCGCATCTCCGCACACTCTCTCCTCACGCCTCCTGACAGGTTGCGTAGG AGGCTCTGTCTGGTATCTTGAAAGAAGAACT |
| 2 | opt_COX10 | ATGGCCGCCTCTCCACACACACTGAGTAGCAGACTGCTGACCGGCTGTGTTG GCGGCTCTGTGTGGTATCTGGAACGGCGGACA |
| 3 | opt_COX10* | ATGGCCGCCAGCCCCCACACCCTGAGCAGCCGCCTGCTGACCGGCTGCGTGG GCGGCAGCGTGTGGTACCTGGAGCGCCGCACC |
| 4 | COX8 | ATGTCCGTCCTGACGCGCCTGCTGCTGCGGGGCTTGACACGGCTCGGCTCGG CGGCTCCAGTGCGGCGCGCCAGAATCCATTCGTTG |
| 5 | OPA1 | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTG |
| 6 | ND4 | ATGCTAAAACTAATCGTCCCAACAATTATGTTACTACCACTGACATGGCTTTC CAAAAAACACATGATTTGGATCAACACAACCACCCACAGCCTAATTATTAGC ATCATCCCTCTACTATTTTTTAACCAAATCAACAACAACCTATTTAGCTGTTC CCCAACCTTTTCCTCCGACCCCCTAACAACCCCCCTCCTAATGCTAACTACCT GGCTCCTACCCCTCACAATCATGGCAAGCCAACGCCACTTATCCAGTGAACC ACTATCACGAAAAAAACTCTACCTCTCTATGCTAATCTCCCTACAAATCTCCT TAATTATGACATTCACAGCCACAGAACTAATCATGTTTTATATCTTCTTCGAA ACCACACTTATCCCCACCTTGGCTATCATCACCCGATGGGGCAACCAGCCAG AACGCCTGAACGCAGGCACATACTTCCTATTCTACACCCTAGTAGGCTCCCTT CCCCTACTCATCGCACTAATTTACACTCACAACACCCTAGGCTCACTAAACAT |
| | | TCTACTACTCACTCTCACTGCCCAAGAACTATCAAACTCCTGGGCCAACAACT TAATGTGGCTAGCTTACACAATGGCTTTTATGGTAAAGATGCCTCTTTACGGA CTCCACTTATGGCTCCCTAAAGCCCATGTCGAAGCCCCCATCGCTGGGTCAAT GGTACTTGCCGCAGTACTCTTAAAACTAGGCGGCTATGGTATGATGCGCCTC ACACTCATTCTCAACCCCCTGACAAAACACATGGCCTACCCCTTCCTTGTACT ATCCCTATGGGGCATGATTATGACAAGCTCCATCTGCCTACGACAAACAGAC CTAAAATCGCTCATTGCATACTCTTCAATCAGCCACATGGCCCTCGTAGTAAC AGCCATTCTCATCCAAACCCCCTGGAGCTTCACCGGCGCAGTCATTCTCATGA TCGCCCACGGGCTTACATCCTCATTACTATTCTGCCTAGCAAACTCAAACTAC GAACGCACTCACAGTCGCATCATGATCCTCTCTCAAGGACTTCAAACTCTACT CCCACTAATGGCTTTTTGGTGGCTTCTAGCAAGCCTCGCTAACCTCGCCTTAC CCCCCACTATTAACCTACTGGGAGAACTCTCTGTGCTAGTAACCACGTTCTCC TGGTCAAATATCACTCTCCTACTTACAGGACTCAACATGCTAGTCACAGCCCT ATACTCCCTCTACATGTTTACCACAACACAATGGGGCTCACTCACCCACCAC ATTAACAACATGAAACCCTCATTCACACGAGAAAACACCCTCATGTTCATGC ACCTATCCCCCATTCTCCTCCTATCCCTCAACCCCGACATCATTACCGGGTTT TCCTCTTAA |
| 7 | opt_ND4 | ATGCTGAAGCTGATCGTGCCCACCATCATGCTGCTGCCTCTGACCTGGCTGA GCAAGAAACACATGATCTGGATCAACACCACCACGCACAGCCTGATCATCAG CATCATCCCTCTGCTGTTCTTCAACCAGATCAACAACAACCTGTTCAGCTGCA GCCCCACCTTCAGCAGCGACCCTCTGACAACACCTCTGCTGATGCTGACCAC CTGGCTGCTGCCCCTCACAATCATGGCCTCTCAGAGACACCTGAGCAGCGAG CCCCTGAGCCGGAAGAAACTGTACCTGAGCATGCTGATCTCCCTGCAGATCT CTCTGATCATGACCTTCACCGCCACCGAGCTGATCATGTTCTACATCTTTTTC GAGACAACGCTGATCCCCACACTGGCCATCATCACCAGATGGGGCAACCAGC CTGAGAGACTGAACGCCGGCACCTACTTTCTGTTCTACACCCTCGTGGGCAG CCTGCCACTGCTGATTGCCCTGATCTACACCCACAACACCCTGGGCTCCCTGA ACATCCTGCTGCTGACACTGACAGCCCAAGAGCTGAGCAACAGCTGGGCCAA CAATCTGATGTGGCTGGCCTACACAATGGCCTTCATGGTCAAGATGCCCCTG TACGGCCTGCACCTGTGGCTGCCTAAAGCTCATGTGGAAGCCCCTATCGCCG GCTCTATGGTGCTGGCTGCAGTGCTGCTGAAACTCGGCGGCTACGGCATGAT GCGGCTGACCCTGATTCTGAATCCCCTGACCAAGCACATGGCCTATCCATTTC TGGTGCTGAGCCTGTGGGGCATGATTATGACCAGCAGCATCTGCCTGCGGCA GACCGATCTGAAGTCCCTGATCGCCTACAGCTCCATCAGCCACATGGCCCTG GTGGTCACCGCCATCCTGATTCAGACCCCTTGGAGCTTTACAGGCGCCGTGA TCCTGATGATTGCCCACGGCCTGACAAGCAGCCTGCTGTTTTGTCTGGCCAAC AGCAACTACGAGCGGACCCACAGCAGAATCATGATCCTGTCTCAGGGCCTGC AGACCCTCCTGCCTCTTATGGCTTTTTGGTGGCTGCTGGCCTCTCTGGCCAAT CTGGCACTGCCTCCTACCATCAATCTGCTGGGCGAGCTGAGCGTGCTGGTCA CCACATTCAGCTGGTCCAATATCACCCTGCTGCTCACCGGCCTGAACATGCTG |
| | | GTTACAGCCCTGTACTCCCTGTACATGTTCACCACCACACAGTGGGGAAGCC TGACACACCACATCAACAATATGAAGCCCAGCTTCACCCGCGAGAACACCCT GATGTTCATGCATCTGAGCCCCATTCTGCTGCTGTCCCTGAATCCTGATATCA TCACCGGCTTCTCCAGCTGA |
| 8 | opt_ND4* | ATGCTGAAGCTGATCGTGCCCACCATCATGCTGCTGCCCCTGACCTGGCTGA GCAAGAAGCACATGATCTGGATCAACACCACCACCCACAGCCTGATCATCAG CATCATCCCCCTGCTGTTCTTCAACCAGATCAACAACAACCTGTTCAGCTGCA GCCCCACCTTCAGCAGCGACCCCCTGACCACCCCCCTGCTGATGCTGACCAC CTGGCTGCTGCCCCTGACCATCATGGCCAGCCAGCGCCACCTGAGCAGCGAG CCCCTGAGCCGCAAGAAGCTGTACCTGAGCATGCTGATCAGCCTGCAGATCA GCCTGATCATGACCTTCACCGCCACCGAGCTGATCATGTTCTACATCTTCTTC GAGACCACCCTGATCCCCACCCTGGCCATCATCACCCGCTGGGGCAACCAGC CCGAGCGCCTGAACGCCGGCACCTACTTCCTGTTCTACACCCTGGTGGGCAG CCTGCCCCTGCTGATCGCCCTGATCTACACCCACAACACCCTGGGCAGCCTG AACATCCTGCTGCTGACCCTGACCGCCCAGGAGCTGAGCAACAGCTGGGCCA ACAACCTGATGTGGCTGGCCTACACCATGGCCTTCATGGTGAAGATGCCCCT GTACGGCCTGCACCTGTGGCTGCCCAAGGCCCACGTGGAGGCCCCCATCGCC GGCAGCATGGTGCTGGCCGCCGTGCTGCTGAAGCTGGGCGGCTACGGCATGA TGCGCCTGACCCTGATCCTGAACCCCCTGACCAAGCACATGGCCTACCCCTTC CTGGTGCTGAGCCTGTGGGGCATGATCATGACCAGCAGCATCTGCCTGCGCC AGACCGACCTGAAGAGCCTGATCGCCTACAGCAGCATCAGCCACATGGCCCT GGTGGTGACCGCCATCCTGATCCAGACCCCCTGGAGCTTCACCGGCGCCGTG ATCCTGATGATCGCCCACGGCCTGACCAGCAGCCTGCTGTTCTGCCTGGCCA ACAGCAACTACGAGCGCACCCACAGCCGCATCATGATCCTGAGCCAGGGCCT GCAGACCCTGCTGCCCCTGATGGCCTTCTGGTGGCTGCTGGCCAGCCTGGCC AACCTGGCCCTGCCCCCCACCATCAACCTGCTGGGCGAGCTGAGCGTGCTGG TGACCACCTTCAGCTGGAGCAACATCACCCTGCTGCTGACCGGCCTGAACAT GCTGGTGACCGCCCTGTACAGCCTGTACATGTTCACCACCACCCAGTGGGGC AGCCTGACCCACCACATCAACAACATGAAGCCCAGCTTCACCCGCGAGAACA CCCTGATGTTCATGCACCTGAGCCCCATCCTGCTGCTGAGCCTGAACCCCGAC ATCATCACCGGCTTCAGCAGCTAA |
| 9 | ND6 | ATGATGTATGCTTTGTTTCTGTTGAGTGTGGGTTTAGTAATGGGGTTTGTGGG GTTTTCTTCTAAGCCTTCTCCTATTTATGGGGGTTTAGTATTGATTGTTAGCGG TGTGGTCGGGTGTGTTATTATTCTGAATTTTGGGGGAGGTTATATGGGTTTAA TGGTTTTTTTAATTTATTTAGGGGGAATGATGGTTGTCTTTGGATATACTACA GCGATGGCTATTGAGGAGTATCCTGAGGCATGGGGGTCAGGGGTTGAGGTCT TGGTGAGTGTTTTAGTGGGGTTAGCGATGGAGGTAGGATTGGTGCTGTGGGT GAAAGAGTATGATGGGGTGGTGGTTGTGGTAAACTTTAATAGTGTAGGAAGC TGGATGATTTATGAAGGAGAGGGGTCAGGGTTGATTCGGGAGGATCCTATTG |
| | | GTGCGGGGGCTTTGTATGATTATGGGCGTTGGTTAGTAGTAGTTACTGGTTGG ACATTGTTTGTTGGTGTATATATTGTAATTGAGATTGCTCGGGGGAATTAG |
| 10 | opt_ND6 | ATGATGTACGCCCTGTTCCTGCTGAGCGTGGGCCTGGTGATGGGCTTCGTGG GCTTCAGCAGCAAGCCCAGCCCCATCTACGGCGGCCTGGTGCTGATCGTGAG CGGCGTGGTGGGCTGCGTGATCATCCTGAACTTCGGCGGCGGCTACATGGGC CTGATGGTGTTCCTGATCTACCTGGGCGGCATGATGGTGGTGTTCGGCTACAC CACCGCCATGGCCATCGAGGAGTACCCCGAGGCCTGGGGCAGCGGCGTGGA GGTGCTGGTGAGCGTGCTGGTGGGCCTGGCCATGGAGGTGGGCCTGGTGCTG TGGGTGAAGGAGTACGACGGCGTGGTGGTGGTGGTGAACTTCAACAGCGTG GGCAGCTGGATGATCTACGAGGGCGAGGGCAGCGGCCTGATCCGCGAGGAC CCCATCGGCGCCGGCGCCCTGTACGACTACGGCCGCTGGCTGGTGGTGGTGA CCGGCTGGACCCTGTTCGTGGGCGTGTACATCGTGATCGAGATCGCCCGCGG CAACTAA |
| 11 | ND1 | ATGGCCAACCTCCTACTCCTCATTGTACCCATTCTAATCGCAATGGCATTCCT AATGCTTACCGAACGAAAAATTCTAGGCTATATGCAACTACGCAAAGGCCCC AACGTTGTAGGCCCCTACGGGCTACTACAACCCTTCGCTGACGCCATAAAAC TCTTCACCAAAGAGCCCCTAAAACCCGCCACATCTACCATCACCCTCTACATC ACCGCCCCGACCTTAGCTCTCACCATCGCTCTTCTACTATGGACCCCCCTCCC CATGCCCAACCCCCTGGTCAACCTCAACCTAGGCCTCCTATTTATTCTAGCCA CCTCTAGCCTAGCCGTTTACTCAATCCTCTGGTCAGGGTGGGCATCAAACTCA AACTACGCCCTGATCGGCGCACTGCGAGCAGTAGCCCAAACAATCTCATATG AAGTCACCCTAGCCATCATTCTACTATCAACATTACTAATGAGTGGCTCCTTT AACCTCTCCACCCTTATCACAACACAAGAACACCTCTGGTTACTCCTGCCATC ATGGCCCTTGGCCATGATGTGGTTTATCTCCACACTAGCAGAGACCAACCGA ACCCCCTTCGACCTTGCCGAAGGGGAGTCCGAACTAGTCTCAGGCTTCAACA TCGAATACGCCGCAGGCCCCTTCGCCCTATTCTTCATGGCCGAATACACAAA CATTATTATGATGAACACCCTCACCACTACAATCTTCCTAGGAACAACATAT GACGCACTCTCCCCTGAACTCTACACAACATATTTTGTCACCAAGACCCTACT TCTAACCTCCCTGTTCTTATGGATTCGAACAGCATACCCCCGATTCCGCTACG ACCAACTCATGCACCTCCTATGGAAAAACTTCCTACCACTCACCCTAGCATTA CTTATGTGGTATGTCTCCATGCCCATTACAATCTCCAGCATTCCCCCTCAAAC CTAA |
| 12 | opt_ND1 | ATGGCCAACCTGCTGCTGCTGATCGTGCCCATCCTGATCGCCATGGCCTTCCT GATGCTGACCGAGCGCAAGATCCTGGGCTACATGCAGCTGCGCAAGGGCCCC AACGTGGTGGGCCCCTACGGCCTGCTGCAGCCCTTCGCCGACGCCATCAAGC TGTTCACCAAGGAGCCCCTGAAGCCCGCCACCAGCACCATCACCCTGTACAT CACCGCCCCCACCCTGGCCCTGACCATCGCCCTGCTGCTGTGGACCCCCCTGC CCATGCCCAACCCCCTGGTGAACCTGAACCTGGGCCTGCTGTTCATCCTGGCC ACCAGCAGCCTGGCCGTGTACAGCATCCTGTGGAGCGGCTGGGCCAGCAACA |
| | | GCAACTACGCCCTGATCGGCGCCCTGCGCGCCGTGGCCCAGACCATCAGCTA CGAGGTGACCCTGGCCATCATCCTGCTGAGCACCCTGCTGATGAGCGGCAGC TTCAACCTGAGCACCCTGATCACCACCCAGGAGCACCTGTGGCTGCTGCTGC CCAGCTGGCCCCTGGCCATGATGTGGTTCATCAGCACCCTGGCCGAGACCAA CCGCACCCCCTTCGACCTGGCCGAGGGCGAGAGCGAGCTGGTGAGCGGCTTC AACATCGAGTACGCCGCCGGCCCCTTCGCCCTGTTCTTCATGGCCGAGTACA CCAACATCATCATGATGAACACCCTGACCACCACCATCTTCCTGGGCACCAC CTACGACGCCCTGAGCCCCGAGCTGTACACCACCTACTTCGTGACCAAGACC CTGCTGCTGACCAGCCTGTTCCTGTGGATCCGCACCGCCTACCCCCGCTTCCG CTACGACCAGCTGATGCACCTGCTGTGGAAGAACTTCCTGCCCCTGACCCTG GCCCTGCTGATGTGGTACGTGAGCATGCCCATCACCATCAGCAGCATCCCCC CCCAGACCTAA |
| 13 | 3'UTR | GAGCACTGGGACGCCCACCGCCCCTTTCCCTCCGCTGCCAGGCGAGCATGTT GTGGTAATTCTGGAACACAAGAAGAGAAATTGCTGGGTTTAGAACAAGATTA TAAACGAATTCGGTGCTCAGTGATCACTTGACAGTTTTTTTTTTTTTTAAATAT TACCCAAAATGCTCCCCAAATAAGAAATGCATCAGCTCAGTCAGTGAATACA AAAAAGGAATTATTTTTCCCTTTGAGGGTCTTTTATACATCTCTCCTCCAACC CCACCCTCTATTCTGTTTCTTCCTCCTCACATGGGGGTACACATACACAGCTT CCTCTTTTGGTTCCATCCTTACCACCACACCACACGCACACTCCACATGCCCA GCAGAGTGGCACTTGGTGGCCAGAAAGTGTGAGCCTCATGATCTGCTGTCTG TAGTTCTGTGAGCTCAGGTCCCTCAAAGGCCTCGGAGCACCCCCTTCCTTGTG ACTGAGCCAGGGCCTGCATTTTTGGTTTTCCCCACCCCACACATTCTCAACCA TAGTCCTTCTAACAATACCAATAGCTAGGACCCGGCTGCTGTGCACTGGGAC TGGGGATTCCACATGTTTGCCTTGGGAGTCTCAAGCTGGACTGCCAGCCCCT GTCCTCCCTTCACCCCCATTGCGTATGAGCATTTCAGAACTCCAAGGAGTCAC AGGCATCTTTATAGTTCACGTTAACATATAGACACTGTTGGAAGCAGTTCCTT CTAAAAGGGTAGCCCTGGACTTAATACCAGCCGGATACCTCTGGCCCCCACC CCATTACTGTACCTCTGGAGTCACTACTGTGGGTCGCCACTCCTCTGCTACAC AGCACGGCTTTTTCAAGGCTGTATTGAGAAGGGAAGTTAGGAAGAAGGGTGT GCTGGGCTAACCAGCCCACAGAGCTCACATTCCTGTCCCTTGGGTGAAAAAT ACATGTCCATCCTGATATCTCCTGAATTCAGAAATTAGCCTCCACATGTGCAA TGGCTTTAAGAGCCAGAAGCAGGGTTCTGGGAATTTTGCAAGTTACCTGTGG CCAGGTGTGGTCTCGGTTACCAAATACGGTTACCTGCAGCTTTTTAGTCCTTT GTGCTCCCACGGGTCTACAGAGTCCCATCTGCCCAAAGGTCTTGAAGCTTGA CAGGATGTTTTCGATTACTCAGTCTCCCAGGGCACTACTGGTCCGTAGGATTC GATTGGTCGGGGTAGGAGAGTTAAACAACATTTAAACAGAGTTCTCTCAAAA ATGTCTAAAGGGATTGTAGGTAGATAACATCCAATCACTGTTTGCACTTATCT GAAATCTTCCCTCTTGGCTGCCCCCAGGTATTTACTGTGGAGAACATTGCATA GGAATGTCTGGAAAAAGCTTCTACAACTTGTTACAGCCTTCACATTTGTAGA AGCTTT |
| 14 | 3'UTR* | GAGCACTGGGACGCCCACCGCCCCTTTCCCTCCGCTGCCAGGCGAGCATGTT GTGGTAATTCTGGAACACAAGAAGAGAAATTGCTGGGTTTAGAACAAGATTA TAAACGAATTCGGTGCTCAGTGATCACTTGACAGTTTTTTTTTTTTTTAAATAT TACCCAAAATGCTCCCCAAATAAGAAATGCATCAGCTCAGTCAGTGAATACA AAAAAGGAATTATTTTTCCCTTTGAGGGTCTTTTATACATCTCTCCTCCAACC CCACCCTCTATTCTGTTTCTTCCTCCTCACATGGGGGTACACATACACAGCTT CCTCTTTTGGTTCCATCCTTACCACCACACCACACGCACACTCCACATGCCCA GCAGAGTGGCACTTGGTGGCCAGAAAGTGTGAGCCTCATGATCTGCTGTCTG TAGTTCTGTGAGCTCAGGTCCCTCAAAGGCCTCGGAGCACCCCCTTCCTTGTG ACTGAGCCAGGGCCTGCATTTTTGGTTTTCCCCACCCCACACATTCTCAACCA TAGTCCTTCTAACAATACCAATAGCTAGGACCCGGCTGCTGTGCACTGGGAC TGGGGATTCCACATGTTTGCCTTGGGAGTCTCAAGCTGGACTGCCA |
| 15 | COX10-ND4-3 'UTR | ATGGCCGCATCTCCGCACACTCTCTCCTCACGCCTCCTGACAGGTTGCGTAGG AGGCTCTGTCTGGTATCTTGAAAGAAGAACTATGCTAAAACTAATCGTCCCA ACAATTATGTTACTACCACTGACATGGCTTTCCAAAAAACACATGATTTGGA TCAACACAACCACCCACAGCCTAATTATTAGCATCATCCCTCTACTATTTTTT AACCAAATCAACAACAACCTATTTAGCTGTTCCCCAACCTTTTCCTCCGACCC CCTAACAACCCCCCTCCTAATGCTAACTACCTGGCTCCTACCCCTCACAATCA TGGCAAGCCAACGCCACTTATCCAGTGAACCACTATCACGAAAAAAACTCTA CCTCTCTATGCTAATCTCCCTACAAATCTCCTTAATTATGACATTCACAGCCA CAGAACTAATCATGTTTTATATCTTCTTCGAAACCACACTTATCCCCACCTTG GCTATCATCACCCGATGGGGCAACCAGCCAGAACGCCTGAACGCAGGCACA TACTTCCTATTCTACACCCTAGTAGGCTCCCTTCCCCTACTCATCGCACTAATT TACACTCACAACACCCTAGGCTCACTAAACATTCTACTACTCACTCTCACTGC CCAAGAACTATCAAACTCCTGGGCCAACAACTTAATGTGGCTAGCTTACACA ATGGCTTTTATGGTAAAGATGCCTCTTTACGGACTCCACTTATGGCTCCCTAA AGCCCATGTCGAAGCCCCCATCGCTGGGTCAATGGTACTTGCCGCAGTACTC TTAAAACTAGGCGGCTATGGTATGATGCGCCTCACACTCATTCTCAACCCCCT GACAAAACACATGGCCTACCCCTTCCTTGTACTATCCCTATGGGGCATGATTA TGACAAGCTCCATCTGCCTACGACAAACAGACCTAAAATCGCTCATTGCATA CTCTTCAATCAGCCACATGGCCCTCGTAGTAACAGCCATTCTCATCCAAACCC CCTGGAGCTTCACCGGCGCAGTCATTCTCATGATCGCCCACGGGCTTACATCC TCATTACTATTCTGCCTAGCAAACTCAAACTACGAACGCACTCACAGTCGCA TCATGATCCTCTCTCAAGGACTTCAAACTCTACTCCCACTAATGGCTTTTTGG TGGCTTCTAGCAAGCCTCGCTAACCTCGCCTTACCCCCCACTATTAACCTACT GGGAGAACTCTCTGTGCTAGTAACCACGTTCTCCTGGTCAAATATCACTCTCC TACTTACAGGACTCAACATGCTAGTCACAGCCCTATACTCCCTCTACATGTTT ACCACAACACAATGGGGCTCACTCACCCACCACATTAACAACATGAAACCCT CATTCACACGAGAAAACACCCTCATGTTCATGCACCTATCCCCCATTCTCCTC CTATCCCTCAACCCCGACATCATTACCGGGTTTTCCTCTTAAGAGCACTGGGA |
| | | CGCCCACCGCCCCTTTCCCTCCGCTGCCAGGCGAGCATGTTGTGGTAATTCTG GAACACAAGAAGAGAAATTGCTGGGTTTAGAACAAGATTATAAACGAATTC GGTGCTCAGTGATCACTTGACAGTTTTTTTTTTTTTTAAATATTACCCAAAATG CTCCCCAAATAAGAAATGCATCAGCTCAGTCAGTGAATACAAAAAAGGAATT ATTTTTCCCTTTGAGGGTCTTTTATACATCTCTCCTCCAACCCCACCCTCTATT CTGTTTCTTCCTCCTCACATGGGGGTACACATACACAGCTTCCTCTTTTGGTTC CATCCTTACCACCACACCACACGCACACTCCACATGCCCAGCAGAGTGGCAC TTGGTGGCCAGAAAGTGTGAGCCTCATGATCTGCTGTCTGTAGTTCTGTGAGC TCAGGTCCCTCAAAGGCCTCGGAGCACCCCCTTCCTTGTGACTGAGCCAGGG CCTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCAGCCCCTGTCCTCCCTTCACC CCCATTGCGTATGAGCATTTCAGAACTCCAAGGAGTCACAGGCATCTTTATA GTTCACGTTAACATATAGACACTGTTGGAAGCAGTTCCTTCTAAAAGGGTAG CCCTGGACTTAATACCAGCCGGATACCTCTGGCCCCCACCCCATTACTGTACC TCTGGAGTCACTACTGTGGGTCGCCACTCCTCTGCTACACAGCACGGCTTTTT CAAGGCTGTATTGAGAAGGGAAGTTAGGAAGAAGGGTGTGCTGGGCTAACC AGCCCACAGAGCTCACATTCCTGTCCCTTGGGTGAAAAATACATGTCCATCC TGATATCTCCTGAATTCAGAAATTAGCCTCCACATGTGCAATGGCTTTAAGA GCCAGAAGCAGGGTTCTGGGAATTTTGCAAGTTACCTGTGGCCAGGTGTGGT CTCGGTTACCAAATACGGTTACCTGCAGCTTTTTAGTCCTTTGTGCTCCCACG GGTCTACAGAGTCCCATCTGCCCAAAGGTCTTGAAGCTTGACAGGATGTTTT CGATTACTCAGTCTCCCAGGGCACTACTGGTCCGTAGGATTCGATTGGTCGG GGTAGGAGAGTTAAACAACATTTAAACAGAGTTCTCTCAAAAATGTCTAAAG GGATTGTAGGTAGATAACATCCAATCACTGTTTGCACTTATCTGAAATCTTCC CTCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 16 | COX10-ND4-3 'UTR* | ATGGCCGCATCTCCGCACACTCTCTCCTCACGCCTCCTGACAGGTTGCGTAGG AGGCTCTGTCTGGTATCTTGAAAGAAGAACTATGCTAAAACTAATCGTCCCA ACAATTATGTTACTACCACTGACATGGCTTTCCAAAAAACACATGATTTGGA TCAACACAACCACCCACAGCCTAATTATTAGCATCATCCCTCTACTATTTTTT AACCAAATCAACAACAACCTATTTAGCTGTTCCCCAACCTTTTCCTCCGACCC CCTAACAACCCCCCTCCTAATGCTAACTACCTGGCTCCTACCCCTCACAATCA TGGCAAGCCAACGCCACTTATCCAGTGAACCACTATCACGAAAAAAACTCTA CCTCTCTATGCTAATCTCCCTACAAATCTCCTTAATTATGACATTCACAGCCA CAGAACTAATCATGTTTTATATCTTCTTCGAAACCACACTTATCCCCACCTTG GCTATCATCACCCGATGGGGCAACCAGCCAGAACGCCTGAACGCAGGCACA TACTTCCTATTCTACACCCTAGTAGGCTCCCTTCCCCTACTCATCGCACTAATT TACACTCACAACACCCTAGGCTCACTAAACATTCTACTACTCACTCTCACTGC CCAAGAACTATCAAACTCCTGGGCCAACAACTTAATGTGGCTAGCTTACACA |
| | | ATGGCTTTTATGGTAAAGATGCCTCTTTACGGACTCCACTTATGGCTCCCTAA AGCCCATGTCGAAGCCCCCATCGCTGGGTCAATGGTACTTGCCGCAGTACTC TTAAAACTAGGCGGCTATGGTATGATGCGCCTCACACTCATTCTCAACCCCCT GACAAAACACATGGCCTACCCCTTCCTTGTACTATCCCTATGGGGCATGATTA TGACAAGCTCCATCTGCCTACGACAAACAGACCTAAAATCGCTCATTGCATA CTCTTCAATCAGCCACATGGCCCTCGTAGTAACAGCCATTCTCATCCAAACCC CCTGGAGCTTCACCGGCGCAGTCATTCTCATGATCGCCCACGGGCTTACATCC TCATTACTATTCTGCCTAGCAAACTCAAACTACGAACGCACTCACAGTCGCA TCATGATCCTCTCTCAAGGACTTCAAACTCTACTCCCACTAATGGCTTTTTGG TGGCTTCTAGCAAGCCTCGCTAACCTCGCCTTACCCCCCACTATTAACCTACT GGGAGAACTCTCTGTGCTAGTAACCACGTTCTCCTGGTCAAATATCACTCTCC TACTTACAGGACTCAACATGCTAGTCACAGCCCTATACTCCCTCTACATGTTT ACCACAACACAATGGGGCTCACTCACCCACCACATTAACAACATGAAACCCT CATTCACACGAGAAAACACCCTCATGTTCATGCACCTATCCCCCATTCTCCTC CTATCCCTCAACCCCGACATCATTACCGGGTTTTCCTCTTAAGAGCACTGGGA CGCCCACCGCCCCTTTCCCTCCGCTGCCAGGCGAGCATGTTGTGGTAATTCTG GAACACAAGAAGAGAAATTGCTGGGTTTAGAACAAGATTATAAACGAATTC GGTGCTCAGTGATCACTTGACAGTTTTTTTTTTTTTTAAATATTACCCAAAATG CTCCCCAAATAAGAAATGCATCAGCTCAGTCAGTGAATACAAAAAAGGAATT ATTTTTCCCTTTGAGGGTCTTTTATACATCTCTCCTCCAACCCCACCCTCTATT CTGTTTCTTCCTCCTCACATGGGGGTACACATACACAGCTTCCTCTTTTGGTTC CATCCTTACCACCACACCACACGCACACTCCACATGCCCAGCAGAGTGGCAC TTGGTGGCCAGAAAGTGTGAGCCTCATGATCTGCTGTCTGTAGTTCTGTGAGC TCAGGTCCCTCAAAGGCCTCGGAGCACCCCCTTCCTTGTGACTGAGCCAGGG CCTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCA |
| 17 | COX10-opt_N D4-3' UTR | ATGGCCGCATCTCCGCACACTCTCTCCTCACGCCTCCTGACAGGTTGCGTAGG AGGCTCTGTCTGGTATCTTGAAAGAAGAACTATGCTGAAGCTGATCGTGCCC ACCATCATGCTGCTGCCTCTGACCTGGCTGAGCAAGAAACACATGATCTGGA TCAACACCACCACGCACAGCCTGATCATCAGCATCATCCCTCTGCTGTTCTTC AACCAGATCAACAACAACCTGTTCAGCTGCAGCCCCACCTTCAGCAGCGACC CTCTGACAACACCTCTGCTGATGCTGACCACCTGGCTGCTGCCCCTCACAATC ATGGCCTCTCAGAGACACCTGAGCAGCGAGCCCCTGAGCCGGAAGAAACTG TACCTGAGCATGCTGATCTCCCTGCAGATCTCTCTGATCATGACCTTCACCGC CACCGAGCTGATCATGTTCTACATCTTTTTCGAGACAACGCTGATCCCCACAC TGGCCATCATCACCAGATGGGGCAACCAGCCTGAGAGACTGAACGCCGGCA CCTACTTTCTGTTCTACACCCTCGTGGGCAGCCTGCCACTGCTGATTGCCCTG ATCTACACCCACAACACCCTGGGCTCCCTGAACATCCTGCTGCTGACACTGA CAGCCCAAGAGCTGAGCAACAGCTGGGCCAACAATCTGATGTGGCTGGCCTA |
| | | CACAATGGCCTTCATGGTCAAGATGCCCCTGTACGGCCTGCACCTGTGGCTG CCTAAAGCTCATGTGGAAGCCCCTATCGCCGGCTCTATGGTGCTGGCTGCAG TGCTGCTGAAACTCGGCGGCTACGGCATGATGCGGCTGACCCTGATTCTGAA TCCCCTGACCAAGCACATGGCCTATCCATTTCTGGTGCTGAGCCTGTGGGGC ATGATTATGACCAGCAGCATCTGCCTGCGGCAGACCGATCTGAAGTCCCTGA TCGCCTACAGCTCCATCAGCCACATGGCCCTGGTGGTCACCGCCATCCTGATT CAGACCCCTTGGAGCTTTACAGGCGCCGTGATCCTGATGATTGCCCACGGCC TGACAAGCAGCCTGCTGTTTTGTCTGGCCAACAGCAACTACGAGCGGACCCA CAGCAGAATCATGATCCTGTCTCAGGGCCTGCAGACCCTCCTGCCTCTTATGG CTTTTTGGTGGCTGCTGGCCTCTCTGGCCAATCTGGCACTGCCTCCTACCATC AATCTGCTGGGCGAGCTGAGCGTGCTGGTCACCACATTCAGCTGGTCCAATA TCACCCTGCTGCTCACCGGCCTGAACATGCTGGTTACAGCCCTGTACTCCCTG TACATGTTCACCACCACACAGTGGGGAAGCCTGACACACCACATCAACAATA TGAAGCCCAGCTTCACCCGCGAGAACACCCTGATGTTCATGCATCTGAGCCC CATTCTGCTGCTGTCCCTGAATCCTGATATCATCACCGGCTTCTCCAGCTGAG AGCACTGGGACGCCCACCGCCCCTTTCCCTCCGCTGCCAGGCGAGCATGTTG TGGTAATTCTGGAACACAAGAAGAGAAATTGCTGGGTTTAGAACAAGATTAT AAACGAATTCGGTGCTCAGTGATCACTTGACAGTTTTTTTTTTTTTTAAATATT ACCCAAAATGCTCCCCAAATAAGAAATGCATCAGCTCAGTCAGTGAATACAA AAAAGGAATTATTTTTCCCTTTGAGGGTCTTTTATACATCTCTCCTCCAACCC CACCCTCTATTCTGTTTCTTCCTCCTCACATGGGGGTACACATACACAGCTTC CTCTTTTGGTTCCATCCTTACCACCACACCACACGCACACTCCACATGCCCAG CAGAGTGGCACTTGGTGGCCAGAAAGTGTGAGCCTCATGATCTGCTGTCTGT AGTTCTGTGAGCTCAGGTCCCTCAAAGGCCTCGGAGCACCCCCTTCCTTGTGA CTGAGCCAGGGCCTGCATTTTTGGTTTTCCCCACCCCACACATTCTCAACCAT AGTCCTTCTAACAATACCAATAGCTAGGACCCGGCTGCTGTGCACTGGGACT GGGGATTCCACATGTTTGCCTTGGGAGTCTCAAGCTGGACTGCCAGCCCCTG TCCTCCCTTCACCCCCATTGCGTATGAGCATTTCAGAACTCCAAGGAGTCACA GGCATCTTTATAGTTCACGTTAACATATAGACACTGTTGGAAGCAGTTCCTTC TAAAAGGGTAGCCCTGGACTTAATACCAGCCGGATACCTCTGGCCCCCACCC CATTACTGTACCTCTGGAGTCACTACTGTGGGTCGCCACTCCTCTGCTACACA GCACGGCTTTTTCAAGGCTGTATTGAGAAGGGAAGTTAGGAAGAAGGGTGTG CTGGGCTAACCAGCCCACAGAGCTCACATTCCTGTCCCTTGGGTGAAAAATA CATGTCCATCCTGATATCTCCTGAATTCAGAAATTAGCCTCCACATGTGCAAT GGCTTTAAGAGCCAGAAGCAGGGTTCTGGGAATTTTGCAAGTTACCTGTGGC CAGGTGTGGTCTCGGTTACCAAATACGGTTACCTGCAGCTTTTTAGTCCTTTG TGCTCCCACGGGTCTACAGAGTCCCATCTGCCCAAAGGTCTTGAAGCTTGAC AGGATGTTTTCGATTACTCAGTCTCCCAGGGCACTACTGGTCCGTAGGATTCG ATTGGTCGGGGTAGGAGAGTTAAACAACATTTAAACAGAGTTCTCTCAAAAA TGTCTAAAGGGATTGTAGGTAGATAACATCCAATCACTGTTTGCACTTATCTG |
| | | AAATCTTCCCTCTTGGCTGCCCCCAGGTATTTACTGTGGAGAACATTGCATAG GAATGTCTGGAAAAAGCTTCTACAACTTGTTACAGCCTTCACATTTGTAGAA GCTTT |
| 18 | COX10-opt_N D4-3' UTR* | ATGGCCGCATCTCCGCACACTCTCTCCTCACGCCTCCTGACAGGTTGCGTAGG AGGCTCTGTCTGGTATCTTGAAAGAAGAACTATGCTGAAGCTGATCGTGCCC ACCATCATGCTGCTGCCTCTGACCTGGCTGAGCAAGAAACACATGATCTGGA TCAACACCACCACGCACAGCCTGATCATCAGCATCATCCCTCTGCTGTTCTTC AACCAGATCAACAACAACCTGTTCAGCTGCAGCCCCACCTTCAGCAGCGACC CTCTGACAACACCTCTGCTGATGCTGACCACCTGGCTGCTGCCCCTCACAATC ATGGCCTCTCAGAGACACCTGAGCAGCGAGCCCCTGAGCCGGAAGAAACTG TACCTGAGCATGCTGATCTCCCTGCAGATCTCTCTGATCATGACCTTCACCGC CACCGAGCTGATCATGTTCTACATCTTTTTCGAGACAACGCTGATCCCCACAC TGGCCATCATCACCAGATGGGGCAACCAGCCTGAGAGACTGAACGCCGGCA CCTACTTTCTGTTCTACACCCTCGTGGGCAGCCTGCCACTGCTGATTGCCCTG ATCTACACCCACAACACCCTGGGCTCCCTGAACATCCTGCTGCTGACACTGA CAGCCCAAGAGCTGAGCAACAGCTGGGCCAACAATCTGATGTGGCTGGCCTA CACAATGGCCTTCATGGTCAAGATGCCCCTGTACGGCCTGCACCTGTGGCTG CCTAAAGCTCATGTGGAAGCCCCTATCGCCGGCTCTATGGTGCTGGCTGCAG TGCTGCTGAAACTCGGCGGCTACGGCATGATGCGGCTGACCCTGATTCTGAA TCCCCTGACCAAGCACATGGCCTATCCATTTCTGGTGCTGAGCCTGTGGGGC ATGATTATGACCAGCAGCATCTGCCTGCGGCAGACCGATCTGAAGTCCCTGA TCGCCTACAGCTCCATCAGCCACATGGCCCTGGTGGTCACCGCCATCCTGATT CAGACCCCTTGGAGCTTTACAGGCGCCGTGATCCTGATGATTGCCCACGGCC TGACAAGCAGCCTGCTGTTTTGTCTGGCCAACAGCAACTACGAGCGGACCCA CAGCAGAATCATGATCCTGTCTCAGGGCCTGCAGACCCTCCTGCCTCTTATGG CTTTTTGGTGGCTGCTGGCCTCTCTGGCCAATCTGGCACTGCCTCCTACCATC AATCTGCTGGGCGAGCTGAGCGTGCTGGTCACCACATTCAGCTGGTCCAATA TCACCCTGCTGCTCACCGGCCTGAACATGCTGGTTACAGCCCTGTACTCCCTG TACATGTTCACCACCACACAGTGGGGAAGCCTGACACACCACATCAACAATA TGAAGCCCAGCTTCACCCGCGAGAACACCCTGATGTTCATGCATCTGAGCCC CATTCTGCTGCTGTCCCTGAATCCTGATATCATCACCGGCTTCTCCAGCTGAG AGCACTGGGACGCCCACCGCCCCTTTCCCTCCGCTGCCAGGCGAGCATGTTG TGGTAATTCTGGAACACAAGAAGAGAAATTGCTGGGTTTAGAACAAGATTAT AAACGAATTCGGTGCTCAGTGATCACTTGACAGTTTTTTTTTTTTTTAAATATT ACCCAAAATGCTCCCCAAATAAGAAATGCATCAGCTCAGTCAGTGAATACAA AAAAGGAATTATTTTTCCCTTTGAGGGTCTTTTATACATCTCTCCTCCAACCC CACCCTCTATTCTGTTTCTTCCTCCTCACATGGGGGTACACATACACAGCTTC CTCTTTTGGTTCCATCCTTACCACCACACCACACGCACACTCCACATGCCCAG CAGAGTGGCACTTGGTGGCCAGAAAGTGTGAGCCTCATGATCTGCTGTCTGT AGTTCTGTGAGCTCAGGTCCCTCAAAGGCCTCGGAGCACCCCCTTCCTTGTGA |
| | | CTGAGCCAGGGCCTGCATTTTTGGTTTTCCCCACCCCACACATTCTCAACCAT AGTCCTTCTAACAATACCAATAGCTAGGACCCGGCTGCTGTGCACTGGGACT GGGGATTCCACATGTTTGCCTTGGGAGTCTCAAGCTGGACTGCCA |
| 19 | COX10-opt_N D4*-3' UTR | ATGGCCGCATCTCCGCACACTCTCTCCTCACGCCTCCTGACAGGTTGCGTAGG AGGCTCTGTCTGGTATCTTGAAAGAAGAACTATGCTGAAGCTGATCGTGCCC ACCATCATGCTGCTGCCCCTGACCTGGCTGAGCAAGAAGCACATGATCTGGA TCAACACCACCACCCACAGCCTGATCATCAGCATCATCCCCCTGCTGTTCTTC AACCAGATCAACAACAACCTGTTCAGCTGCAGCCCCACCTTCAGCAGCGACC CCCTGACCACCCCCCTGCTGATGCTGACCACCTGGCTGCTGCCCCTGACCATC ATGGCCAGCCAGCGCCACCTGAGCAGCGAGCCCCTGAGCCGCAAGAAGCTG TACCTGAGCATGCTGATCAGCCTGCAGATCAGCCTGATCATGACCTTCACCG CCACCGAGCTGATCATGTTCTACATCTTCTTCGAGACCACCCTGATCCCCACC CTGGCCATCATCACCCGCTGGGGCAACCAGCCCGAGCGCCTGAACGCCGGCA CCTACTTCCTGTTCTACACCCTGGTGGGCAGCCTGCCCCTGCTGATCGCCCTG ATCTACACCCACAACACCCTGGGCAGCCTGAACATCCTGCTGCTGACCCTGA CCGCCCAGGAGCTGAGCAACAGCTGGGCCAACAACCTGATGTGGCTGGCCTA CACCATGGCCTTCATGGTGAAGATGCCCCTGTACGGCCTGCACCTGTGGCTG CCCAAGGCCCACGTGGAGGCCCCCATCGCCGGCAGCATGGTGCTGGCCGCCG TGCTGCTGAAGCTGGGCGGCTACGGCATGATGCGCCTGACCCTGATCCTGAA CCCCCTGACCAAGCACATGGCCTACCCCTTCCTGGTGCTGAGCCTGTGGGGC ATGATCATGACCAGCAGCATCTGCCTGCGCCAGACCGACCTGAAGAGCCTGA TCGCCTACAGCAGCATCAGCCACATGGCCCTGGTGGTGACCGCCATCCTGAT CCAGACCCCCTGGAGCTTCACCGGCGCCGTGATCCTGATGATCGCCCACGGC CTGACCAGCAGCCTGCTGTTCTGCCTGGCCAACAGCAACTACGAGCGCACCC ACAGCCGCATCATGATCCTGAGCCAGGGCCTGCAGACCCTGCTGCCCCTGAT GGCCTTCTGGTGGCTGCTGGCCAGCCTGGCCAACCTGGCCCTGCCCCCCACC ATCAACCTGCTGGGCGAGCTGAGCGTGCTGGTGACCACCTTCAGCTGGAGCA ACATCACCCTGCTGCTGACCGGCCTGAACATGCTGGTGACCGCCCTGTACAG CCTGTACATGTTCACCACCACCCAGTGGGGCAGCCTGACCCACCACATCAAC AACATGAAGCCCAGCTTCACCCGCGAGAACACCCTGATGTTCATGCACCTGA GCCCCATCCTGCTGCTGAGCCTGAACCCCGACATCATCACCGGCTTCAGCAG CTAAGAGCACTGGGACGCCCACCGCCCCTTTCCCTCCGCTGCCAGGCGAGCA TGTTGTGGTAATTCTGGAACACAAGAAGAGAAATTGCTGGGTTTAGAACAAG ATTATAAACGAATTCGGTGCTCAGTGATCACTTGACAGTTTTTTTTTTTTTTAA ATATTACCCAAAATGCTCCCCAAATAAGAAATGCATCAGCTCAGTCAGTGAA TACAAAAAAGGAATTATTTTTCCCTTTGAGGGTCTTTTATACATCTCTCCTCC AACCCCACCCTCTATTCTGTTTCTTCCTCCTCACATGGGGGTACACATACACA GCTTCCTCTTTTGGTTCCATCCTTACCACCACACCACACGCACACTCCACATG CCCAGCAGAGTGGCACTTGGTGGCCAGAAAGTGTGAGCCTCATGATCTGCTG TCTGTAGTTCTGTGAGCTCAGGTCCCTCAAAGGCCTCGGAGCACCCCCTTCCT |
| | | TGTGACTGAGCCAGGGCCTGCATTTTTGGTTTTCCCCACCCCACACATTCTCA ACCATAGTCCTTCTAACAATACCAATAGCTAGGACCCGGCTGCTGTGCACTG GGACTGGGGATTCCACATGTTTGCCTTGGGAGTCTCAAGCTGGACTGCCAGC CCCTGTCCTCCCTTCACCCCCATTGCGTATGAGCATTTCAGAACTCCAAGGAG TCACAGGCATCTTTATAGTTCACGTTAACATATAGACACTGTTGGAAGCAGTT CCTTCTAAAAGGGTAGCCCTGGACTTAATACCAGCCGGATACCTCTGGCCCC CACCCCATTACTGTACCTCTGGAGTCACTACTGTGGGTCGCCACTCCTCTGCT ACACAGCACGGCTTTTTCAAGGCTGTATTGAGAAGGGAAGTTAGGAAGAAG GGTGTGCTGGGCTAACCAGCCCACAGAGCTCACATTCCTGTCCCTTGGGTGA AAAATACATGTCCATCCTGATATCTCCTGAATTCAGAAATTAGCCTCCACATG TGCAATGGCTTTAAGAGCCAGAAGCAGGGTTCTGGGAATTTTGCAAGTTACC TGTGGCCAGGTGTGGTCTCGGTTACCAAATACGGTTACCTGCAGCTTTTTAGT CCTTTGTGCTCCCACGGGTCTACAGAGTCCCATCTGCCCAAAGGTCTTGAAGC TTGACAGGATGTTTTCGATTACTCAGTCTCCCAGGGCACTACTGGTCCGTAGG ATTCGATTGGTCGGGGTAGGAGAGTTAAACAACATTTAAACAGAGTTCTCTC AAAAATGTCTAAAGGGATTGTAGGTAGATAACATCCAATCACTGTTTGCACT TATCTGAAATCTTCCCTCTTGGCTGCCCCCAGGTATTTACTGTGGAGAACATT GCATAGGAATGTCTGGAAAAAGCTTCTACAACTTGTTACAGCCTTCACATTT GTAGAAGCTTT |
| 20 | COX10-opt_N D4*-3' UTR* | ATGGCCGCATCTCCGCACACTCTCTCCTCACGCCTCCTGACAGGTTGCGTAGG AGGCTCTGTCTGGTATCTTGAAAGAAGAACTATGCTGAAGCTGATCGTGCCC ACCATCATGCTGCTGCCCCTGACCTGGCTGAGCAAGAAGCACATGATCTGGA TCAACACCACCACCCACAGCCTGATCATCAGCATCATCCCCCTGCTGTTCTTC AACCAGATCAACAACAACCTGTTCAGCTGCAGCCCCACCTTCAGCAGCGACC CCCTGACCACCCCCCTGCTGATGCTGACCACCTGGCTGCTGCCCCTGACCATC ATGGCCAGCCAGCGCCACCTGAGCAGCGAGCCCCTGAGCCGCAAGAAGCTG TACCTGAGCATGCTGATCAGCCTGCAGATCAGCCTGATCATGACCTTCACCG CCACCGAGCTGATCATGTTCTACATCTTCTTCGAGACCACCCTGATCCCCACC CTGGCCATCATCACCCGCTGGGGCAACCAGCCCGAGCGCCTGAACGCCGGCA CCTACTTCCTGTTCTACACCCTGGTGGGCAGCCTGCCCCTGCTGATCGCCCTG ATCTACACCCACAACACCCTGGGCAGCCTGAACATCCTGCTGCTGACCCTGA CCGCCCAGGAGCTGAGCAACAGCTGGGCCAACAACCTGATGTGGCTGGCCTA CACCATGGCCTTCATGGTGAAGATGCCCCTGTACGGCCTGCACCTGTGGCTG CCCAAGGCCCACGTGGAGGCCCCCATCGCCGGCAGCATGGTGCTGGCCGCCG TGCTGCTGAAGCTGGGCGGCTACGGCATGATGCGCCTGACCCTGATCCTGAA CCCCCTGACCAAGCACATGGCCTACCCCTTCCTGGTGCTGAGCCTGTGGGGC ATGATCATGACCAGCAGCATCTGCCTGCGCCAGACCGACCTGAAGAGCCTGA TCGCCTACAGCAGCATCAGCCACATGGCCCTGGTGGTGACCGCCATCCTGAT CCAGACCCCCTGGAGCTTCACCGGCGCCGTGATCCTGATGATCGCCCACGGC CTGACCAGCAGCCTGCTGTTCTGCCTGGCCAACAGCAACTACGAGCGCACCC |
| | | ACAGCCGCATCATGATCCTGAGCCAGGGCCTGCAGACCCTGCTGCCCCTGAT GGCCTTCTGGTGGCTGCTGGCCAGCCTGGCCAACCTGGCCCTGCCCCCCACC ATCAACCTGCTGGGCGAGCTGAGCGTGCTGGTGACCACCTTCAGCTGGAGCA ACATCACCCTGCTGCTGACCGGCCTGAACATGCTGGTGACCGCCCTGTACAG CCTGTACATGTTCACCACCACCCAGTGGGGCAGCCTGACCCACCACATCAAC AACATGAAGCCCAGCTTCACCCGCGAGAACACCCTGATGTTCATGCACCTGA GCCCCATCCTGCTGCTGAGCCTGAACCCCGACATCATCACCGGCTTCAGCAG CTAAGAGCACTGGGACGCCCACCGCCCCTTTCCCTCCGCTGCCAGGCGAGCA TGTTGTGGTAATTCTGGAACACAAGAAGAGAAATTGCTGGGTTTAGAACAAG ATTATAAACGAATTCGGTGCTCAGTGATCACTTGACAGTTTTTTTTTTTTTTAA ATATTACCCAAAATGCTCCCCAAATAAGAAATGCATCAGCTCAGTCAGTGAA TACAAAAAAGGAATTATTTTTCCCTTTGAGGGTCTTTTATACATCTCTCCTCC AACCCCACCCTCTATTCTGTTTCTTCCTCCTCACATGGGGGTACACATACACA GCTTCCTCTTTTGGTTCCATCCTTACCACCACACCACACGCACACTCCACATG CCCAGCAGAGTGGCACTTGGTGGCCAGAAAGTGTGAGCCTCATGATCTGCTG TCTGTAGTTCTGTGAGCTCAGGTCCCTCAAAGGCCTCGGAGCACCCCCTTCCT TGTGACTGAGCCAGGGCCTGCATTTTTGGTTTTCCCCACCCCACACATTCTCA ACCATAGTCCTTCTAACAATACCAATAGCTAGGACCCGGCTGCTGTGCACTG GGACTGGGGATTCCACATGTTTGCCTTGGGAGTCTCAAGCTGGACTGCCA |
| 21 | COX10-ND6-3 'UTR | ATGGCCGCATCTCCGCACACTCTCTCCTCACGCCTCCTGACAGGTTGCGTAGG AGGCTCTGTCTGGTATCTTGAAAGAAGAACTATGATGTATGCTTTGTTTCTGT TGAGTGTGGGTTTAGTAATGGGGTTTGTGGGGTTTTCTTCTAAGCCTTCTCCT ATTTATGGGGGTTTAGTATTGATTGTTAGCGGTGTGGTCGGGTGTGTTATTAT TCTGAATTTTGGGGGAGGTTATATGGGTTTAATGGTTTTTTTAATTTATTTAG GGGGAATGATGGTTGTCTTTGGATATACTACAGCGATGGCTATTGAGGAGTA TCCTGAGGCATGGGGGTCAGGGGTTGAGGTCTTGGTGAGTGTTTTAGTGGGG TTAGCGATGGAGGTAGGATTGGTGCTGTGGGTGAAAGAGTATGATGGGGTGG TGGTTGTGGTAAACTTTAATAGTGTAGGAAGCTGGATGATTTATGAAGGAGA GGGGTCAGGGTTGATTCGGGAGGATCCTATTGGTGCGGGGGCTTTGTATGAT TATGGGCGTTGGTTAGTAGTAGTTACTGGTTGGACATTGTTTGTTGGTGTATA TATTGTAATTGAGATTGCTCGGGGGAATTAGGAGCACTGGGACGCCCACCGC CCCTTTCCCTCCGCTGCCAGGCGAGCATGTTGTGGTAATTCTGGAACACAAG AAGAGAAATTGCTGGGTTTAGAACAAGATTATAAACGAATTCGGTGCTCAGT GATCACTTGACAGTTTTTTTTTTTTTTAAATATTACCCAAAATGCTCCCCAAAT AAGAAATGCATCAGCTCAGTCAGTGAATACAAAAAAGGAATTATTTTTCCCT TTGAGGGTCTTTTATACATCTCTCCTCCAACCCCACCCTCTATTCTGTTTCTTC CTCCTCACATGGGGGTACACATACACAGCTTCCTCTTTTGGTTCCATCCTTAC CACCACACCACACGCACACTCCACATGCCCAGCAGAGTGGCACTTGGTGGCC AGAAAGTGTGAGCCTCATGATCTGCTGTCTGTAGTTCTGTGAGCTCAGGTCCC TCAAAGGCCTCGGAGCACCCCCTTCCTTGTGACTGAGCCAGGGCCTGCATTTT |
| | | TGGTTTTCCCCACCCCACACATTCTCAACCATAGTCCTTCTAACAATACCAAT AGCTAGGACCCGGCTGCTGTGCACTGGGACTGGGGATTCCACATGTTTGCCT TGGGAGTCTCAAGCTGGACTGCCAGCCCCTGTCCTCCCTTCACCCCCATTGCG TATGAGCATTTCAGAACTCCAAGGAGTCACAGGCATCTTTATAGTTCACGTT AACATATAGACACTGTTGGAAGCAGTTCCTTCTAAAAGGGTAGCCCTGGACT TAATACCAGCCGGATACCTCTGGCCCCCACCCCATTACTGTACCTCTGGAGTC ACTACTGTGGGTCGCCACTCCTCTGCTACACAGCACGGCTTTTTCAAGGCTGT ATTGAGAAGGGAAGTTAGGAAGAAGGGTGTGCTGGGCTAACCAGCCCACAG AGCTCACATTCCTGTCCCTTGGGTGAAAAATACATGTCCATCCTGATATCTCC TGAATTCAGAAATTAGCCTCCACATGTGCAATGGCTTTAAGAGCCAGAAGCA GGGTTCTGGGAATTTTGCAAGTTACCTGTGGCCAGGTGTGGTCTCGGTTACCA AATACGGTTACCTGCAGCTTTTTAGTCCTTTGTGCTCCCACGGGTCTACAGAG TCCCATCTGCCCAAAGGTCTTGAAGCTTGACAGGATGTTTTCGATTACTCAGT CTCCCAGGGCACTACTGGTCCGTAGGATTCGATTGGTCGGGGTAGGAGAGTT AAACAACATTTAAACAGAGTTCTCTCAAAAATGTCTAAAGGGATTGTAGGTA GATAACATCCAATCACTGTTTGCACTTATCTGAAATCTTCCCTCTTGGCTGCC CCCAGGTATTTACTGTGGAGAACATTGCATAGGAATGTCTGGAAAAAGCTTC TACAACTTGTTACAGCCTTCACATTTGTAGAAGCTTT |
| 22 | COX10-ND6-3 'UTR* | ATGGCCGCATCTCCGCACACTCTCTCCTCACGCCTCCTGACAGGTTGCGTAGG AGGCTCTGTCTGGTATCTTGAAAGAAGAACTATGATGTATGCTTTGTTTCTGT TGAGTGTGGGTTTAGTAATGGGGTTTGTGGGGTTTTCTTCTAAGCCTTCTCCT ATTTATGGGGGTTTAGTATTGATTGTTAGCGGTGTGGTCGGGTGTGTTATTAT TCTGAATTTTGGGGGAGGTTATATGGGTTTAATGGTTTTTTTAATTTATTTAG GGGGAATGATGGTTGTCTTTGGATATACTACAGCGATGGCTATTGAGGAGTA TCCTGAGGCATGGGGGTCAGGGGTTGAGGTCTTGGTGAGTGTTTTAGTGGGG TTAGCGATGGAGGTAGGATTGGTGCTGTGGGTGAAAGAGTATGATGGGGTGG TGGTTGTGGTAAACTTTAATAGTGTAGGAAGCTGGATGATTTATGAAGGAGA GGGGTCAGGGTTGATTCGGGAGGATCCTATTGGTGCGGGGGCTTTGTATGAT TATGGGCGTTGGTTAGTAGTAGTTACTGGTTGGACATTGTTTGTTGGTGTATA TATTGTAATTGAGATTGCTCGGGGGAATTAGGAGCACTGGGACGCCCACCGC CCCTTTCCCTCCGCTGCCAGGCGAGCATGTTGTGGTAATTCTGGAACACAAG AAGAGAAATTGCTGGGTTTAGAACAAGATTATAAACGAATTCGGTGCTCAGT GATCACTTGACAGTTTTTTTTTTTTTTAAATATTACCCAAAATGCTCCCCAAAT AAGAAATGCATCAGCTCAGTCAGTGAATACAAAAAAGGAATTATTTTTCCCT TTGAGGGTCTTTTATACATCTCTCCTCCAACCCCACCCTCTATTCTGTTTCTTC CTCCTCACATGGGGGTACACATACACAGCTTCCTCTTTTGGTTCCATCCTTAC CACCACACCACACGCACACTCCACATGCCCAGCAGAGTGGCACTTGGTGGCC AGAAAGTGTGAGCCTCATGATCTGCTGTCTGTAGTTCTGTGAGCTCAGGTCCC TCAAAGGCCTCGGAGCACCCCCTTCCTTGTGACTGAGCCAGGGCCTGCATTTT TGGTTTTCCCCACCCCACACATTCTCAACCATAGTCCTTCTAACAATACCAAT |
| | | AGCTAGGACCCGGCTGCTGTGCACTGGGACTGGGGATTCCACATGTTTGCCT TGGGAGTCTCAAGCTGGACTGCCA |
| 23 | COX10-opt_N D6-3' UTR | ATGGCCGCATCTCCGCACACTCTCTCCTCACGCCTCCTGACAGGTTGCGTAGG AGGCTCTGTCTGGTATCTTGAAAGAAGAACTATGATGTACGCCCTGTTCCTGC TGAGCGTGGGCCTGGTGATGGGCTTCGTGGGCTTCAGCAGCAAGCCCAGCCC CATCTACGGCGGCCTGGTGCTGATCGTGAGCGGCGTGGTGGGCTGCGTGATC ATCCTGAACTTCGGCGGCGGCTACATGGGCCTGATGGTGTTCCTGATCTACCT GGGCGGCATGATGGTGGTGTTCGGCTACACCACCGCCATGGCCATCGAGGAG TACCCCGAGGCCTGGGGCAGCGGCGTGGAGGTGCTGGTGAGCGTGCTGGTG GGCCTGGCCATGGAGGTGGGCCTGGTGCTGTGGGTGAAGGAGTACGACGGC GTGGTGGTGGTGGTGAACTTCAACAGCGTGGGCAGCTGGATGATCTACGAGG GCGAGGGCAGCGGCCTGATCCGCGAGGACCCCATCGGCGCCGGCGCCCTGT ACGACTACGGCCGCTGGCTGGTGGTGGTGACCGGCTGGACCCTGTTCGTGGG CGTGTACATCGTGATCGAGATCGCCCGCGGCAACTAAGAGCACTGGGACGCC CACCGCCCCTTTCCCTCCGCTGCCAGGCGAGCATGTTGTGGTAATTCTGGAAC ACAAGAAGAGAAATTGCTGGGTTTAGAACAAGATTATAAACGAATTCGGTG CTCAGTGATCACTTGACAGTTTTTTTTTTTTTTAAATATTACCCAAAATGCTCC CCAAATAAGAAATGCATCAGCTCAGTCAGTGAATACAAAAAAGGAATTATTT TTCCCTTTGAGGGTCTTTTATACATCTCTCCTCCAACCCCACCCTCTATTCTGT TTCTTCCTCCTCACATGGGGGTACACATACACAGCTTCCTCTTTTGGTTCCAT CCTTACCACCACACCACACGCACACTCCACATGCCCAGCAGAGTGGCACTTG GTGGCCAGAAAGTGTGAGCCTCATGATCTGCTGTCTGTAGTTCTGTGAGCTC AGGTCCCTCAAAGGCCTCGGAGCACCCCCTTCCTTGTGACTGAGCCAGGGCC TGCATTTTTGGTTTTCCCCACCCCACACATTCTCAACCATAGTCCTTCTAACA ATACCAATAGCTAGGACCCGGCTGCTGTGCACTGGGACTGGGGATTCCACAT GTTTGCCTTGGGAGTCTCAAGCTGGACTGCCAGCCCCTGTCCTCCCTTCACCC CCATTGCGTATGAGCATTTCAGAACTCCAAGGAGTCACAGGCATCTTTATAG TTCACGTTAACATATAGACACTGTTGGAAGCAGTTCCTTCTAAAAGGGTAGC CCTGGACTTAATACCAGCCGGATACCTCTGGCCCCCACCCCATTACTGTACCT CTGGAGTCACTACTGTGGGTCGCCACTCCTCTGCTACACAGCACGGCTTTTTC AAGGCTGTATTGAGAAGGGAAGTTAGGAAGAAGGGTGTGCTGGGCTAACCA GCCCACAGAGCTCACATTCCTGTCCCTTGGGTGAAAAATACATGTCCATCCT GATATCTCCTGAATTCAGAAATTAGCCTCCACATGTGCAATGGCTTTAAGAG CCAGAAGCAGGGTTCTGGGAATTTTGCAAGTTACCTGTGGCCAGGTGTGGTC TCGGTTACCAAATACGGTTACCTGCAGCTTTTTAGTCCTTTGTGCTCCCACGG GTCTACAGAGTCCCATCTGCCCAAAGGTCTTGAAGCTTGACAGGATGTTTTC GATTACTCAGTCTCCCAGGGCACTACTGGTCCGTAGGATTCGATTGGTCGGG GTAGGAGAGTTAAACAACATTTAAACAGAGTTCTCTCAAAAATGTCTAAAGG GATTGTAGGTAGATAACATCCAATCACTGTTTGCACTTATCTGAAATCTTCCC TCTTGGCTGCCCCCAGGTATTTACTGTGGAGAACATTGCATAGGAATGTCTG |
| | | GAAAAAGCTTCTACAACTTGTTACAGCCTTCACATTTGTAGAAGCTTT |
| 24 | COX10-opt_N D6-3' UTR* | ATGGCCGCATCTCCGCACACTCTCTCCTCACGCCTCCTGACAGGTTGCGTAGG AGGCTCTGTCTGGTATCTTGAAAGAAGAACTATGATGTACGCCCTGTTCCTGC TGAGCGTGGGCCTGGTGATGGGCTTCGTGGGCTTCAGCAGCAAGCCCAGCCC CATCTACGGCGGCCTGGTGCTGATCGTGAGCGGCGTGGTGGGCTGCGTGATC ATCCTGAACTTCGGCGGCGGCTACATGGGCCTGATGGTGTTCCTGATCTACCT GGGCGGCATGATGGTGGTGTTCGGCTACACCACCGCCATGGCCATCGAGGAG TACCCCGAGGCCTGGGGCAGCGGCGTGGAGGTGCTGGTGAGCGTGCTGGTG GGCCTGGCCATGGAGGTGGGCCTGGTGCTGTGGGTGAAGGAGTACGACGGC GTGGTGGTGGTGGTGAACTTCAACAGCGTGGGCAGCTGGATGATCTACGAGG GCGAGGGCAGCGGCCTGATCCGCGAGGACCCCATCGGCGCCGGCGCCCTGT ACGACTACGGCCGCTGGCTGGTGGTGGTGACCGGCTGGACCCTGTTCGTGGG CGTGTACATCGTGATCGAGATCGCCCGCGGCAACTAAGAGCACTGGGACGCC CACCGCCCCTTTCCCTCCGCTGCCAGGCGAGCATGTTGTGGTAATTCTGGAAC ACAAGAAGAGAAATTGCTGGGTTTAGAACAAGATTATAAACGAATTCGGTG CTCAGTGATCACTTGACAGTTTTTTTTTTTTTTAAATATTACCCAAAATGCTCC CCAAATAAGAAATGCATCAGCTCAGTCAGTGAATACAAAAAAGGAATTATTT TTCCCTTTGAGGGTCTTTTATACATCTCTCCTCCAACCCCACCCTCTATTCTGT TTCTTCCTCCTCACATGGGGGTACACATACACAGCTTCCTCTTTTGGTTCCAT CCTTACCACCACACCACACGCACACTCCACATGCCCAGCAGAGTGGCACTTG GTGGCCAGAAAGTGTGAGCCTCATGATCTGCTGTCTGTAGTTCTGTGAGCTC AGGTCCCTCAAAGGCCTCGGAGCACCCCCTTCCTTGTGACTGAGCCAGGGCC TGCATTTTTGGTTTTCCCCACCCCACACATTCTCAACCATAGTCCTTCTAACA ATACCAATAGCTAGGACCCGGCTGCTGTGCACTGGGACTGGGGATTCCACAT GTTTGCCTTGGGAGTCTCAAGCTGGACTGCCA |
| 25 | COX10-ND1-3 'UTR | ATGGCCGCATCTCCGCACACTCTCTCCTCACGCCTCCTGACAGGTTGCGTAGG AGGCTCTGTCTGGTATCTTGAAAGAAGAACTATGGCCAACCTCCTACTCCTC ATTGTACCCATTCTAATCGCAATGGCATTCCTAATGCTTACCGAACGAAAAA TTCTAGGCTATATGCAACTACGCAAAGGCCCCAACGTTGTAGGCCCCTACGG GCTACTACAACCCTTCGCTGACGCCATAAAACTCTTCACCAAAGAGCCCCTA AAACCCGCCACATCTACCATCACCCTCTACATCACCGCCCCGACCTTAGCTCT CACCATCGCTCTTCTACTATGGACCCCCCTCCCCATGCCCAACCCCCTGGTCA ACCTCAACCTAGGCCTCCTATTTATTCTAGCCACCTCTAGCCTAGCCGTTTAC TCAATCCTCTGGTCAGGGTGGGCATCAAACTCAAACTACGCCCTGATCGGCG CACTGCGAGCAGTAGCCCAAACAATCTCATATGAAGTCACCCTAGCCATCAT TCTACTATCAACATTACTAATGAGTGGCTCCTTTAACCTCTCCACCCTTATCA CAACACAAGAACACCTCTGGTTACTCCTGCCATCATGGCCCTTGGCCATGAT GTGGTTTATCTCCACACTAGCAGAGACCAACCGAACCCCCTTCGACCTTGCC GAAGGGGAGTCCGAACTAGTCTCAGGCTTCAACATCGAATACGCCGCAGGCC |
| | | CCTTCGCCCTATTCTTCATGGCCGAATACACAAACATTATTATGATGAACACC CTCACCACTACAATCTTCCTAGGAACAACATATGACGCACTCTCCCCTGAACT CTACACAACATATTTTGTCACCAAGACCCTACTTCTAACCTCCCTGTTCTTAT GGATTCGAACAGCATACCCCCGATTCCGCTACGACCAACTCATGCACCTCCT ATGGAAAAACTTCCTACCACTCACCCTAGCATTACTTATGTGGTATGTCTCCA TGCCCATTACAATCTCCAGCATTCCCCCTCAAACCTAAGAGCACTGGGACGC CCACCGCCCCTTTCCCTCCGCTGCCAGGCGAGCATGTTGTGGTAATTCTGGAA CACAAGAAGAGAAATTGCTGGGTTTAGAACAAGATTATAAACGAATTCGGT GCTCAGTGATCACTTGACAGTTTTTTTTTTTTTTAAATATTACCCAAAATGCTC CCCAAATAAGAAATGCATCAGCTCAGTCAGTGAATACAAAAAAGGAATTATT TTTCCCTTTGAGGGTCTTTTATACATCTCTCCTCCAACCCCACCCTCTATTCTG TTTCTTCCTCCTCACATGGGGGTACACATACACAGCTTCCTCTTTTGGTTCCAT CCTTACCACCACACCACACGCACACTCCACATGCCCAGCAGAGTGGCACTTG GTGGCCAGAAAGTGTGAGCCTCATGATCTGCTGTCTGTAGTTCTGTGAGCTC AGGTCCCTCAAAGGCCTCGGAGCACCCCCTTCCTTGTGACTGAGCCAGGGCC TGCATTTTTGGTTTTCCCCACCCCACACATTCTCAACCATAGTCCTTCTAACA ATACCAATAGCTAGGACCCGGCTGCTGTGCACTGGGACTGGGGATTCCACAT GTTTGCCTTGGGAGTCTCAAGCTGGACTGCCAGCCCCTGTCCTCCCTTCACCC CCATTGCGTATGAGCATTTCAGAACTCCAAGGAGTCACAGGCATCTTTATAG TTCACGTTAACATATAGACACTGTTGGAAGCAGTTCCTTCTAAAAGGGTAGC CCTGGACTTAATACCAGCCGGATACCTCTGGCCCCCACCCCATTACTGTACCT CTGGAGTCACTACTGTGGGTCGCCACTCCTCTGCTACACAGCACGGCTTTTTC AAGGCTGTATTGAGAAGGGAAGTTAGGAAGAAGGGTGTGCTGGGCTAACCA GCCCACAGAGCTCACATTCCTGTCCCTTGGGTGAAAAATACATGTCCATCCT GATATCTCCTGAATTCAGAAATTAGCCTCCACATGTGCAATGGCTTTAAGAG CCAGAAGCAGGGTTCTGGGAATTTTGCAAGTTACCTGTGGCCAGGTGTGGTC TCGGTTACCAAATACGGTTACCTGCAGCTTTTTAGTCCTTTGTGCTCCCACGG GTCTACAGAGTCCCATCTGCCCAAAGGTCTTGAAGCTTGACAGGATGTTTTC GATTACTCAGTCTCCCAGGGCACTACTGGTCCGTAGGATTCGATTGGTCGGG GTAGGAGAGTTAAACAACATTTAAACAGAGTTCTCTCAAAAATGTCTAAAGG GATTGTAGGTAGATAACATCCAATCACTGTTTGCACTTATCTGAAATCTTCCC TCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 26 | COX10-ND1-3 'UTR* | ATGGCCGCATCTCCGCACACTCTCTCCTCACGCCTCCTGACAGGTTGCGTAGG AGGCTCTGTCTGGTATCTTGAAAGAAGAACTATGGCCAACCTCCTACTCCTC ATTGTACCCATTCTAATCGCAATGGCATTCCTAATGCTTACCGAACGAAAAA TTCTAGGCTATATGCAACTACGCAAAGGCCCCAACGTTGTAGGCCCCTACGG GCTACTACAACCCTTCGCTGACGCCATAAAACTCTTCACCAAAGAGCCCCTA AAACCCGCCACATCTACCATCACCCTCTACATCACCGCCCCGACCTTAGCTCT CACCATCGCTCTTCTACTATGGACCCCCCTCCCCATGCCCAACCCCCTGGTCA |
| | | ACCTCAACCTAGGCCTCCTATTTATTCTAGCCACCTCTAGCCTAGCCGTTTAC TCAATCCTCTGGTCAGGGTGGGCATCAAACTCAAACTACGCCCTGATCGGCG CACTGCGAGCAGTAGCCCAAACAATCTCATATGAAGTCACCCTAGCCATCAT TCTACTATCAACATTACTAATGAGTGGCTCCTTTAACCTCTCCACCCTTATCA CAACACAAGAACACCTCTGGTTACTCCTGCCATCATGGCCCTTGGCCATGAT GTGGTTTATCTCCACACTAGCAGAGACCAACCGAACCCCCTTCGACCTTGCC GAAGGGGAGTCCGAACTAGTCTCAGGCTTCAACATCGAATACGCCGCAGGCC CCTTCGCCCTATTCTTCATGGCCGAATACACAAACATTATTATGATGAACACC CTCACCACTACAATCTTCCTAGGAACAACATATGACGCACTCTCCCCTGAACT CTACACAACATATTTTGTCACCAAGACCCTACTTCTAACCTCCCTGTTCTTAT GGATTCGAACAGCATACCCCCGATTCCGCTACGACCAACTCATGCACCTCCT ATGGAAAAACTTCCTACCACTCACCCTAGCATTACTTATGTGGTATGTCTCCA TGCCCATTACAATCTCCAGCATTCCCCCTCAAACCTAAGAGCACTGGGACGC CCACCGCCCCTTTCCCTCCGCTGCCAGGCGAGCATGTTGTGGTAATTCTGGAA CACAAGAAGAGAAATTGCTGGGTTTAGAACAAGATTATAAACGAATTCGGT GCTCAGTGATCACTTGACAGTTTTTTTTTTTTTTAAATATTACCCAAAATGCTC CCCAAATAAGAAATGCATCAGCTCAGTCAGTGAATACAAAAAAGGAATTATT TTTCCCTTTGAGGGTCTTTTATACATCTCTCCTCCAACCCCACCCTCTATTCTG TTTCTTCCTCCTCACATGGGGGTACACATACACAGCTTCCTCTTTTGGTTCCAT CCTTACCACCACACCACACGCACACTCCACATGCCCAGCAGAGTGGCACTTG GTGGCCAGAAAGTGTGAGCCTCATGATCTGCTGTCTGTAGTTCTGTGAGCTC AGGTCCCTCAAAGGCCTCGGAGCACCCCCTTCCTTGTGACTGAGCCAGGGCC TGCATTTTTGGTTTTCCCCACCCCACACATTCTCAACCATAGTCCTTCTAACA ATACCAATAGCTAGGACCCGGCTGCTGTGCACTGGGACTGGGGATTCCACAT GTTTGCCTTGGGAGTCTCAAGCTGGACTGCCA |
| 27 | COX10-opt_N D1-3'UTR | ATGGCCGCATCTCCGCACACTCTCTCCTCACGCCTCCTGACAGGTTGCGTAGG AGGCTCTGTCTGGTATCTTGAAAGAAGAACTATGGCCAACCTGCTGCTGCTG ATCGTGCCCATCCTGATCGCCATGGCCTTCCTGATGCTGACCGAGCGCAAGA TCCTGGGCTACATGCAGCTGCGCAAGGGCCCCAACGTGGTGGGCCCCTACGG CCTGCTGCAGCCCTTCGCCGACGCCATCAAGCTGTTCACCAAGGAGCCCCTG AAGCCCGCCACCAGCACCATCACCCTGTACATCACCGCCCCCACCCTGGCCC TGACCATCGCCCTGCTGCTGTGGACCCCCCTGCCCATGCCCAACCCCCTGGTG AACCTGAACCTGGGCCTGCTGTTCATCCTGGCCACCAGCAGCCTGGCCGTGT ACAGCATCCTGTGGAGCGGCTGGGCCAGCAACAGCAACTACGCCCTGATCGG CGCCCTGCGCGCCGTGGCCCAGACCATCAGCTACGAGGTGACCCTGGCCATC ATCCTGCTGAGCACCCTGCTGATGAGCGGCAGCTTCAACCTGAGCACCCTGA TCACCACCCAGGAGCACCTGTGGCTGCTGCTGCCCAGCTGGCCCCTGGCCAT GATGTGGTTCATCAGCACCCTGGCCGAGACCAACCGCACCCCCTTCGACCTG GCCGAGGGCGAGAGCGAGCTGGTGAGCGGCTTCAACATCGAGTACGCCGCC GGCCCCTTCGCCCTGTTCTTCATGGCCGAGTACACCAACATCATCATGATGAA |
| | | CACCCTGACCACCACCATCTTCCTGGGCACCACCTACGACGCCCTGAGCCCC GAGCTGTACACCACCTACTTCGTGACCAAGACCCTGCTGCTGACCAGCCTGT TCCTGTGGATCCGCACCGCCTACCCCCGCTTCCGCTACGACCAGCTGATGCAC CTGCTGTGGAAGAACTTCCTGCCCCTGACCCTGGCCCTGCTGATGTGGTACGT GAGCATGCCCATCACCATCAGCAGCATCCCCCCCCAGACCTAAGAGCACTGG GACGCCCACCGCCCCTTTCCCTCCGCTGCCAGGCGAGCATGTTGTGGTAATTC TGGAACACAAGAAGAGAAATTGCTGGGTTTAGAACAAGATTATAAACGAAT TCGGTGCTCAGTGATCACTTGACAGTTTTTTTTTTTTTTAAATATTACCCAAAA TGCTCCCCAAATAAGAAATGCATCAGCTCAGTCAGTGAATACAAAAAAGGA ATTATTTTTCCCTTTGAGGGTCTTTTATACATCTCTCCTCCAACCCCACCCTCT ATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCAGCCCCTGTCCTCCCT TCACCCCCATTGCGTATGAGCATTTCAGAACTCCAAGGAGTCACAGGCATCT TTATAGTTCACGTTAACATATAGACACTGTTGGAAGCAGTTCCTTCTAAAAG GGTAGCCCTGGACTTAATACCAGCCGGATACCTCTGGCCCCCACCCCATTAC TGTACCTCTGGAGTCACTACTGTGGGTCGCCACTCCTCTGCTACACAGCACGG CTTTTTCAAGGCTGTATTGAGAAGGGAAGTTAGGAAGAAGGGTGTGCTGGGC TAACCAGCCCACAGAGCTCACATTCCTGTCCCTTGGGTGAAAAATACATGTC CATCCTGATATCTCCTGAATTCAGAAATTAGCCTCCACATGTGCAATGGCTTT AAGAGCCAGAAGCAGGGTTCTGGGAATTTTGCAAGTTACCTGTGGCCAGGTG TGGTCTCGGTTACCAAATACGGTTACCTGCAGCTTTTTAGTCCTTTGTGCTCC CACGGGTCTACAGAGTCCCATCTGCCCAAAGGTCTTGAAGCTTGACAGGATG TTTTCGATTACTCAGTCTCCCAGGGCACTACTGGTCCGTAGGATTCGATTGGT CGGGGTAGGAGAGTTAAACAACATTTAAACAGAGTTCTCTCAAAAATGTCTA AAGGGATTGTAGGTAGATAACATCCAATCACTGTTTGCACTTATCTGAAATC TTCCCTCTTGGCTGCCCCCAGGTATTTACTGTGGAGAACATTGCATAGGAATG TCTGGAAAAAGCTTCTACAACTTGTTACAGCCTTCACATTTGTAGAAGCTTT |
| 28 | COX10-opt_N D1-3'UTR* | ATGGCCGCATCTCCGCACACTCTCTCCTCACGCCTCCTGACAGGTTGCGTAGG AGGCTCTGTCTGGTATCTTGAAAGAAGAACTATGGCCAACCTGCTGCTGCTG ATCGTGCCCATCCTGATCGCCATGGCCTTCCTGATGCTGACCGAGCGCAAGA TCCTGGGCTACATGCAGCTGCGCAAGGGCCCCAACGTGGTGGGCCCCTACGG CCTGCTGCAGCCCTTCGCCGACGCCATCAAGCTGTTCACCAAGGAGCCCCTG AAGCCCGCCACCAGCACCATCACCCTGTACATCACCGCCCCCACCCTGGCCC TGACCATCGCCCTGCTGCTGTGGACCCCCCTGCCCATGCCCAACCCCCTGGTG AACCTGAACCTGGGCCTGCTGTTCATCCTGGCCACCAGCAGCCTGGCCGTGT |
| | | ACAGCATCCTGTGGAGCGGCTGGGCCAGCAACAGCAACTACGCCCTGATCGG CGCCCTGCGCGCCGTGGCCCAGACCATCAGCTACGAGGTGACCCTGGCCATC ATCCTGCTGAGCACCCTGCTGATGAGCGGCAGCTTCAACCTGAGCACCCTGA TCACCACCCAGGAGCACCTGTGGCTGCTGCTGCCCAGCTGGCCCCTGGCCAT GATGTGGTTCATCAGCACCCTGGCCGAGACCAACCGCACCCCCTTCGACCTG GCCGAGGGCGAGAGCGAGCTGGTGAGCGGCTTCAACATCGAGTACGCCGCC GGCCCCTTCGCCCTGTTCTTCATGGCCGAGTACACCAACATCATCATGATGAA CACCCTGACCACCACCATCTTCCTGGGCACCACCTACGACGCCCTGAGCCCC GAGCTGTACACCACCTACTTCGTGACCAAGACCCTGCTGCTGACCAGCCTGT TCCTGTGGATCCGCACCGCCTACCCCCGCTTCCGCTACGACCAGCTGATGCAC CTGCTGTGGAAGAACTTCCTGCCCCTGACCCTGGCCCTGCTGATGTGGTACGT GAGCATGCCCATCACCATCAGCAGCATCCCCCCCCAGACCTAAGAGCACTGG GACGCCCACCGCCCCTTTCCCTCCGCTGCCAGGCGAGCATGTTGTGGTAATTC TGGAACACAAGAAGAGAAATTGCTGGGTTTAGAACAAGATTATAAACGAAT TCGGTGCTCAGTGATCACTTGACAGTTTTTTTTTTTTTTAAATATTACCCAAAA TGCTCCCCAAATAAGAAATGCATCAGCTCAGTCAGTGAATACAAAAAAGGA ATTATTTTTCCCTTTGAGGGTCTTTTATACATCTCTCCTCCAACCCCACCCTCT ATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCA |
| 29 | opt_COX10-N D4-3' UTR | ATGGCCGCCTCTCCACACACACTGAGTAGCAGACTGCTGACCGGCTGTGTTG GCGGCTCTGTGTGGTATCTGGAACGGCGGACAATGCTAAAACTAATCGTCCC AACAATTATGTTACTACCACTGACATGGCTTTCCAAAAAACACATGATTTGG ATCAACACAACCACCCACAGCCTAATTATTAGCATCATCCCTCTACTATTTTT TAACCAAATCAACAACAACCTATTTAGCTGTTCCCCAACCTTTTCCTCCGACC CCCTAACAACCCCCCTCCTAATGCTAACTACCTGGCTCCTACCCCTCACAATC ATGGCAAGCCAACGCCACTTATCCAGTGAACCACTATCACGAAAAAAACTCT ACCTCTCTATGCTAATCTCCCTACAAATCTCCTTAATTATGACATTCACAGCC ACAGAACTAATCATGTTTTATATCTTCTTCGAAACCACACTTATCCCCACCTT GGCTATCATCACCCGATGGGGCAACCAGCCAGAACGCCTGAACGCAGGCAC ATACTTCCTATTCTACACCCTAGTAGGCTCCCTTCCCCTACTCATCGCACTAA TTTACACTCACAACACCCTAGGCTCACTAAACATTCTACTACTCACTCTCACT GCCCAAGAACTATCAAACTCCTGGGCCAACAACTTAATGTGGCTAGCTTACA CAATGGCTTTTATGGTAAAGATGCCTCTTTACGGACTCCACTTATGGCTCCCT AAAGCCCATGTCGAAGCCCCCATCGCTGGGTCAATGGTACTTGCCGCAGTAC TCTTAAAACTAGGCGGCTATGGTATGATGCGCCTCACACTCATTCTCAACCCC |
| | | CTGACAAAACACATGGCCTACCCCTTCCTTGTACTATCCCTATGGGGCATGAT TATGACAAGCTCCATCTGCCTACGACAAACAGACCTAAAATCGCTCATTGCA TACTCTTCAATCAGCCACATGGCCCTCGTAGTAACAGCCATTCTCATCCAAAC CCCCTGGAGCTTCACCGGCGCAGTCATTCTCATGATCGCCCACGGGCTTACAT CCTCATTACTATTCTGCCTAGCAAACTCAAACTACGAACGCACTCACAGTCG CATCATGATCCTCTCTCAAGGACTTCAAACTCTACTCCCACTAATGGCTTTTT GGTGGCTTCTAGCAAGCCTCGCTAACCTCGCCTTACCCCCCACTATTAACCTA CTGGGAGAACTCTCTGTGCTAGTAACCACGTTCTCCTGGTCAAATATCACTCT CCTACTTACAGGACTCAACATGCTAGTCACAGCCCTATACTCCCTCTACATGT TTACCACAACACAATGGGGCTCACTCACCCACCACATTAACAACATGAAACC CTCATTCACACGAGAAAACACCCTCATGTTCATGCACCTATCCCCCATTCTCC TCCTATCCCTCAACCCCGACATCATTACCGGGTTTTCCTCTTAAGAGCACTGG GACGCCCACCGCCCCTTTCCCTCCGCTGCCAGGCGAGCATGTTGTGGTAATTC TGGAACACAAGAAGAGAAATTGCTGGGTTTAGAACAAGATTATAAACGAAT TCGGTGCTCAGTGATCACTTGACAGTTTTTTTTTTTTTTAAATATTACCCAAAA TGCTCCCCAAATAAGAAATGCATCAGCTCAGTCAGTGAATACAAAAAAGGA ATTATTTTTCCCTTTGAGGGTCTTTTATACATCTCTCCTCCAACCCCACCCTCT ATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCAGCCCCTGTCCTCCCT TCACCCCCATTGCGTATGAGCATTTCAGAACTCCAAGGAGTCACAGGCATCT TTATAGTTCACGTTAACATATAGACACTGTTGGAAGCAGTTCCTTCTAAAAG GGTAGCCCTGGACTTAATACCAGCCGGATACCTCTGGCCCCCACCCCATTAC TGTACCTCTGGAGTCACTACTGTGGGTCGCCACTCCTCTGCTACACAGCACGG CTTTTTCAAGGCTGTATTGAGAAGGGAAGTTAGGAAGAAGGGTGTGCTGGGC TAACCAGCCCACAGAGCTCACATTCCTGTCCCTTGGGTGAAAAATACATGTC CATCCTGATATCTCCTGAATTCAGAAATTAGCCTCCACATGTGCAATGGCTTT AAGAGCCAGAAGCAGGGTTCTGGGAATTTTGCAAGTTACCTGTGGCCAGGTG TGGTCTCGGTTACCAAATACGGTTACCTGCAGCTTTTTAGTCCTTTGTGCTCC CACGGGTCTACAGAGTCCCATCTGCCCAAAGGTCTTGAAGCTTGACAGGATG TTTTCGATTACTCAGTCTCCCAGGGCACTACTGGTCCGTAGGATTCGATTGGT CGGGGTAGGAGAGTTAAACAACATTTAAACAGAGTTCTCTCAAAAATGTCTA AAGGGATTGTAGGTAGATAACATCCAATCACTGTTTGCACTTATCTGAAATC TTCCCTCTTGGCTGCCCCCAGGTATTTACTGTGGAGAACATTGCATAGGAATG TCTGGAAAAAGCTTCTACAACTTGTTACAGCCTTCACATTTGTAGAAGCTTT |
| 30 | opt_COX10-N | ATGGCCGCCTCTCCACACACACTGAGTAGCAGACTGCTGACCGGCTGTGTTG |
| | D4-3'UTR* | GCGGCTCTGTGTGGTATCTGGAACGGCGGACAATGCTAAAACTAATCGTCCC AACAATTATGTTACTACCACTGACATGGCTTTCCAAAAAACACATGATTTGG ATCAACACAACCACCCACAGCCTAATTATTAGCATCATCCCTCTACTATTTTT TAACCAAATCAACAACAACCTATTTAGCTGTTCCCCAACCTTTTCCTCCGACC CCCTAACAACCCCCCTCCTAATGCTAACTACCTGGCTCCTACCCCTCACAATC ATGGCAAGCCAACGCCACTTATCCAGTGAACCACTATCACGAAAAAAACTCT ACCTCTCTATGCTAATCTCCCTACAAATCTCCTTAATTATGACATTCACAGCC ACAGAACTAATCATGTTTTATATCTTCTTCGAAACCACACTTATCCCCACCTT GGCTATCATCACCCGATGGGGCAACCAGCCAGAACGCCTGAACGCAGGCAC ATACTTCCTATTCTACACCCTAGTAGGCTCCCTTCCCCTACTCATCGCACTAA TTTACACTCACAACACCCTAGGCTCACTAAACATTCTACTACTCACTCTCACT GCCCAAGAACTATCAAACTCCTGGGCCAACAACTTAATGTGGCTAGCTTACA CAATGGCTTTTATGGTAAAGATGCCTCTTTACGGACTCCACTTATGGCTCCCT AAAGCCCATGTCGAAGCCCCCATCGCTGGGTCAATGGTACTTGCCGCAGTAC TCTTAAAACTAGGCGGCTATGGTATGATGCGCCTCACACTCATTCTCAACCCC CTGACAAAACACATGGCCTACCCCTTCCTTGTACTATCCCTATGGGGCATGAT TATGACAAGCTCCATCTGCCTACGACAAACAGACCTAAAATCGCTCATTGCA TACTCTTCAATCAGCCACATGGCCCTCGTAGTAACAGCCATTCTCATCCAAAC CCCCTGGAGCTTCACCGGCGCAGTCATTCTCATGATCGCCCACGGGCTTACAT CCTCATTACTATTCTGCCTAGCAAACTCAAACTACGAACGCACTCACAGTCG CATCATGATCCTCTCTCAAGGACTTCAAACTCTACTCCCACTAATGGCTTTTT GGTGGCTTCTAGCAAGCCTCGCTAACCTCGCCTTACCCCCCACTATTAACCTA CTGGGAGAACTCTCTGTGCTAGTAACCACGTTCTCCTGGTCAAATATCACTCT CCTACTTACAGGACTCAACATGCTAGTCACAGCCCTATACTCCCTCTACATGT TTACCACAACACAATGGGGCTCACTCACCCACCACATTAACAACATGAAACC CTCATTCACACGAGAAAACACCCTCATGTTCATGCACCTATCCCCCATTCTCC TCCTATCCCTCAACCCCGACATCATTACCGGGTTTTCCTCTTAAGAGCACTGG GACGCCCACCGCCCCTTTCCCTCCGCTGCCAGGCGAGCATGTTGTGGTAATTC TGGAACACAAGAAGAGAAATTGCTGGGTTTAGAACAAGATTATAAACGAAT TCGGTGCTCAGTGATCACTTGACAGTTTTTTTTTTTTTTAAATATTACCCAAAA TGCTCCCCAAATAAGAAATGCATCAGCTCAGTCAGTGAATACAAAAAAGGA ATTATTTTTCCCTTTGAGGGTCTTTTATACATCTCTCCTCCAACCCCACCCTCT ATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCA |
| 31 | opt_COX10-op | ATGGCCGCCTCTCCACACACACTGAGTAGCAGACTGCTGACCGGCTGTGTTG |
| | t_ND4-3'UTR | GCGGCTCTGTGTGGTATCTGGAACGGCGGACAATGCTGAAGCTGATCGTGCC CACCATCATGCTGCTGCCTCTGACCTGGCTGAGCAAGAAACACATGATCTGG ATCAACACCACCACGCACAGCCTGATCATCAGCATCATCCCTCTGCTGTTCTT CAACCAGATCAACAACAACCTGTTCAGCTGCAGCCCCACCTTCAGCAGCGAC CCTCTGACAACACCTCTGCTGATGCTGACCACCTGGCTGCTGCCCCTCACAAT CATGGCCTCTCAGAGACACCTGAGCAGCGAGCCCCTGAGCCGGAAGAAACT GTACCTGAGCATGCTGATCTCCCTGCAGATCTCTCTGATCATGACCTTCACCG CCACCGAGCTGATCATGTTCTACATCTTTTTCGAGACAACGCTGATCCCCACA CTGGCCATCATCACCAGATGGGGCAACCAGCCTGAGAGACTGAACGCCGGC ACCTACTTTCTGTTCTACACCCTCGTGGGCAGCCTGCCACTGCTGATTGCCCT GATCTACACCCACAACACCCTGGGCTCCCTGAACATCCTGCTGCTGACACTG ACAGCCCAAGAGCTGAGCAACAGCTGGGCCAACAATCTGATGTGGCTGGCCT ACACAATGGCCTTCATGGTCAAGATGCCCCTGTACGGCCTGCACCTGTGGCT GCCTAAAGCTCATGTGGAAGCCCCTATCGCCGGCTCTATGGTGCTGGCTGCA GTGCTGCTGAAACTCGGCGGCTACGGCATGATGCGGCTGACCCTGATTCTGA ATCCCCTGACCAAGCACATGGCCTATCCATTTCTGGTGCTGAGCCTGTGGGG CATGATTATGACCAGCAGCATCTGCCTGCGGCAGACCGATCTGAAGTCCCTG ATCGCCTACAGCTCCATCAGCCACATGGCCCTGGTGGTCACCGCCATCCTGA TTCAGACCCCTTGGAGCTTTACAGGCGCCGTGATCCTGATGATTGCCCACGG CCTGACAAGCAGCCTGCTGTTTTGTCTGGCCAACAGCAACTACGAGCGGACC CACAGCAGAATCATGATCCTGTCTCAGGGCCTGCAGACCCTCCTGCCTCTTAT GGCTTTTTGGTGGCTGCTGGCCTCTCTGGCCAATCTGGCACTGCCTCCTACCA TCAATCTGCTGGGCGAGCTGAGCGTGCTGGTCACCACATTCAGCTGGTCCAA TATCACCCTGCTGCTCACCGGCCTGAACATGCTGGTTACAGCCCTGTACTCCC TGTACATGTTCACCACCACACAGTGGGGAAGCCTGACACACCACATCAACAA TATGAAGCCCAGCTTCACCCGCGAGAACACCCTGATGTTCATGCATCTGAGC CCCATTCTGCTGCTGTCCCTGAATCCTGATATCATCACCGGCTTCTCCAGCTG AGAGCACTGGGACGCCCACCGCCCCTTTCCCTCCGCTGCCAGGCGAGCATGT TGTGGTAATTCTGGAACACAAGAAGAGAAATTGCTGGGTTTAGAACAAGATT ATAAACGAATTCGGTGCTCAGTGATCACTTGACAGTTTTTTTTTTTTTTAAAT ATTACCCAAAATGCTCCCCAAATAAGAAATGCATCAGCTCAGTCAGTGAATA CAAAAAAGGAATTATTTTTCCCTTTGAGGGTCTTTTATACATCTCTCCTCCAA CCCCACCCTCTATTCTGTTTCTTCCTCCTCACATGGGGGTACACATACACAGC TTCCTCTTTTGGTTCCATCCTTACCACCACACCACACGCACACTCCACATGCC CAGCAGAGTGGCACTTGGTGGCCAGAAAGTGTGAGCCTCATGATCTGCTGTC TGTAGTTCTGTGAGCTCAGGTCCCTCAAAGGCCTCGGAGCACCCCCTTCCTTG TGACTGAGCCAGGGCCTGCATTTTTGGTTTTCCCCACCCCACACATTCTCAAC CATAGTCCTTCTAACAATACCAATAGCTAGGACCCGGCTGCTGTGCACTGGG ACTGGGGATTCCACATGTTTGCCTTGGGAGTCTCAAGCTGGACTGCCAGCCC CTGTCCTCCCTTCACCCCCATTGCGTATGAGCATTTCAGAACTCCAAGGAGTC |
| | | ACAGGCATCTTTATAGTTCACGTTAACATATAGACACTGTTGGAAGCAGTTC CTTCTAAAAGGGTAGCCCTGGACTTAATACCAGCCGGATACCTCTGGCCCCC ACCCCATTACTGTACCTCTGGAGTCACTACTGTGGGTCGCCACTCCTCTGCTA CACAGCACGGCTTTTTCAAGGCTGTATTGAGAAGGGAAGTTAGGAAGAAGG GTGTGCTGGGCTAACCAGCCCACAGAGCTCACATTCCTGTCCCTTGGGTGAA AAATACATGTCCATCCTGATATCTCCTGAATTCAGAAATTAGCCTCCACATGT GCAATGGCTTTAAGAGCCAGAAGCAGGGTTCTGGGAATTTTGCAAGTTACCT GTGGCCAGGTGTGGTCTCGGTTACCAAATACGGTTACCTGCAGCTTTTTAGTC CTTTGTGCTCCCACGGGTCTACAGAGTCCCATCTGCCCAAAGGTCTTGAAGCT TGACAGGATGTTTTCGATTACTCAGTCTCCCAGGGCACTACTGGTCCGTAGG ATTCGATTGGTCGGGGTAGGAGAGTTAAACAACATTTAAACAGAGTTCTCTC AAAAATGTCTAAAGGGATTGTAGGTAGATAACATCCAATCACTGTTTGCACT TATCTGAAATCTTCCCTCTTGGCTGCCCCCAGGTATTTACTGTGGAGAACATT GCATAGGAATGTCTGGAAAAAGCTTCTACAACTTGTTACAGCCTTCACATTT GTAGAAGCTTT |
| 32 | opt_COX10-op t_ND4-3'UTR * | ATGGCCGCCTCTCCACACACACTGAGTAGCAGACTGCTGACCGGCTGTGTTG GCGGCTCTGTGTGGTATCTGGAACGGCGGACAATGCTGAAGCTGATCGTGCC CACCATCATGCTGCTGCCTCTGACCTGGCTGAGCAAGAAACACATGATCTGG ATCAACACCACCACGCACAGCCTGATCATCAGCATCATCCCTCTGCTGTTCTT CAACCAGATCAACAACAACCTGTTCAGCTGCAGCCCCACCTTCAGCAGCGAC CCTCTGACAACACCTCTGCTGATGCTGACCACCTGGCTGCTGCCCCTCACAAT CATGGCCTCTCAGAGACACCTGAGCAGCGAGCCCCTGAGCCGGAAGAAACT GTACCTGAGCATGCTGATCTCCCTGCAGATCTCTCTGATCATGACCTTCACCG CCACCGAGCTGATCATGTTCTACATCTTTTTCGAGACAACGCTGATCCCCACA CTGGCCATCATCACCAGATGGGGCAACCAGCCTGAGAGACTGAACGCCGGC ACCTACTTTCTGTTCTACACCCTCGTGGGCAGCCTGCCACTGCTGATTGCCCT GATCTACACCCACAACACCCTGGGCTCCCTGAACATCCTGCTGCTGACACTG ACAGCCCAAGAGCTGAGCAACAGCTGGGCCAACAATCTGATGTGGCTGGCCT ACACAATGGCCTTCATGGTCAAGATGCCCCTGTACGGCCTGCACCTGTGGCT GCCTAAAGCTCATGTGGAAGCCCCTATCGCCGGCTCTATGGTGCTGGCTGCA GTGCTGCTGAAACTCGGCGGCTACGGCATGATGCGGCTGACCCTGATTCTGA ATCCCCTGACCAAGCACATGGCCTATCCATTTCTGGTGCTGAGCCTGTGGGG CATGATTATGACCAGCAGCATCTGCCTGCGGCAGACCGATCTGAAGTCCCTG ATCGCCTACAGCTCCATCAGCCACATGGCCCTGGTGGTCACCGCCATCCTGA TTCAGACCCCTTGGAGCTTTACAGGCGCCGTGATCCTGATGATTGCCCACGG CCTGACAAGCAGCCTGCTGTTTTGTCTGGCCAACAGCAACTACGAGCGGACC CACAGCAGAATCATGATCCTGTCTCAGGGCCTGCAGACCCTCCTGCCTCTTAT GGCTTTTTGGTGGCTGCTGGCCTCTCTGGCCAATCTGGCACTGCCTCCTACCA TCAATCTGCTGGGCGAGCTGAGCGTGCTGGTCACCACATTCAGCTGGTCCAA TATCACCCTGCTGCTCACCGGCCTGAACATGCTGGTTACAGCCCTGTACTCCC |
| | | TGTACATGTTCACCACCACACAGTGGGGAAGCCTGACACACCACATCAACAA TATGAAGCCCAGCTTCACCCGCGAGAACACCCTGATGTTCATGCATCTGAGC CCCATTCTGCTGCTGTCCCTGAATCCTGATATCATCACCGGCTTCTCCAGCTG AGAGCACTGGGACGCCCACCGCCCCTTTCCCTCCGCTGCCAGGCGAGCATGT TGTGGTAATTCTGGAACACAAGAAGAGAAATTGCTGGGTTTAGAACAAGATT ATAAACGAATTCGGTGCTCAGTGATCACTTGACAGTTTTTTTTTTTTTTAAAT ATTACCCAAAATGCTCCCCAAATAAGAAATGCATCAGCTCAGTCAGTGAATA CAAAAAAGGAATTATTTTTCCCTTTGAGGGTCTTTTATACATCTCTCCTCCAA CCCCACCCTCTATTCTGTTTCTTCCTCCTCACATGGGGGTACACATACACAGC TTCCTCTTTTGGTTCCATCCTTACCACCACACCACACGCACACTCCACATGCC CAGCAGAGTGGCACTTGGTGGCCAGAAAGTGTGAGCCTCATGATCTGCTGTC TGTAGTTCTGTGAGCTCAGGTCCCTCAAAGGCCTCGGAGCACCCCCTTCCTTG TGACTGAGCCAGGGCCTGCATTTTTGGTTTTCCCCACCCCACACATTCTCAAC CATAGTCCTTCTAACAATACCAATAGCTAGGACCCGGCTGCTGTGCACTGGG ACTGGGGATTCCACATGTTTGCCTTGGGAGTCTCAAGCTGGACTGCCA |
| 33 | opt_COX10-op t_ND4*-3'UT R | ATGGCCGCCTCTCCACACACACTGAGTAGCAGACTGCTGACCGGCTGTGTTG GCGGCTCTGTGTGGTATCTGGAACGGCGGACAATGCTGAAGCTGATCGTGCC CACCATCATGCTGCTGCCCCTGACCTGGCTGAGCAAGAAGCACATGATCTGG ATCAACACCACCACCCACAGCCTGATCATCAGCATCATCCCCCTGCTGTTCTT CAACCAGATCAACAACAACCTGTTCAGCTGCAGCCCCACCTTCAGCAGCGAC CCCCTGACCACCCCCCTGCTGATGCTGACCACCTGGCTGCTGCCCCTGACCAT CATGGCCAGCCAGCGCCACCTGAGCAGCGAGCCCCTGAGCCGCAAGAAGCT GTACCTGAGCATGCTGATCAGCCTGCAGATCAGCCTGATCATGACCTTCACC GCCACCGAGCTGATCATGTTCTACATCTTCTTCGAGACCACCCTGATCCCCAC CCTGGCCATCATCACCCGCTGGGGCAACCAGCCCGAGCGCCTGAACGCCGGC ACCTACTTCCTGTTCTACACCCTGGTGGGCAGCCTGCCCCTGCTGATCGCCCT GATCTACACCCACAACACCCTGGGCAGCCTGAACATCCTGCTGCTGACCCTG ACCGCCCAGGAGCTGAGCAACAGCTGGGCCAACAACCTGATGTGGCTGGCCT ACACCATGGCCTTCATGGTGAAGATGCCCCTGTACGGCCTGCACCTGTGGCT GCCCAAGGCCCACGTGGAGGCCCCCATCGCCGGCAGCATGGTGCTGGCCGCC GTGCTGCTGAAGCTGGGCGGCTACGGCATGATGCGCCTGACCCTGATCCTGA ACCCCCTGACCAAGCACATGGCCTACCCCTTCCTGGTGCTGAGCCTGTGGGG CATGATCATGACCAGCAGCATCTGCCTGCGCCAGACCGACCTGAAGAGCCTG ATCGCCTACAGCAGCATCAGCCACATGGCCCTGGTGGTGACCGCCATCCTGA TCCAGACCCCCTGGAGCTTCACCGGCGCCGTGATCCTGATGATCGCCCACGG CCTGACCAGCAGCCTGCTGTTCTGCCTGGCCAACAGCAACTACGAGCGCACC CACAGCCGCATCATGATCCTGAGCCAGGGCCTGCAGACCCTGCTGCCCCTGA TGGCCTTCTGGTGGCTGCTGGCCAGCCTGGCCAACCTGGCCCTGCCCCCCACC ATCAACCTGCTGGGCGAGCTGAGCGTGCTGGTGACCACCTTCAGCTGGAGCA ACATCACCCTGCTGCTGACCGGCCTGAACATGCTGGTGACCGCCCTGTACAG |
| | | CCTGTACATGTTCACCACCACCCAGTGGGGCAGCCTGACCCACCACATCAAC AACATGAAGCCCAGCTTCACCCGCGAGAACACCCTGATGTTCATGCACCTGA GCCCCATCCTGCTGCTGAGCCTGAACCCCGACATCATCACCGGCTTCAGCAG CTAAGAGCACTGGGACGCCCACCGCCCCTTTCCCTCCGCTGCCAGGCGAGCA TGTTGTGGTAATTCTGGAACACAAGAAGAGAAATTGCTGGGTTTAGAACAAG ATTATAAACGAATTCGGTGCTCAGTGATCACTTGACAGTTTTTTTTTTTTTTAA ATATTACCCAAAATGCTCCCCAAATAAGAAATGCATCAGCTCAGTCAGTGAA TACAAAAAAGGAATTATTTTTCCCTTTGAGGGTCTTTTATACATCTCTCCTCC AACCCCACCCTCTATTCTGTTTCTTCCTCCTCACATGGGGGTACACATACACA GCTTCCTCTTTTGGTTCCATCCTTACCACCACACCACACGCACACTCCACATG CCCAGCAGAGTGGCACTTGGTGGCCAGAAAGTGTGAGCCTCATGATCTGCTG TCTGTAGTTCTGTGAGCTCAGGTCCCTCAAAGGCCTCGGAGCACCCCCTTCCT TGTGACTGAGCCAGGGCCTGCATTTTTGGTTTTCCCCACCCCACACATTCTCA ACCATAGTCCTTCTAACAATACCAATAGCTAGGACCCGGCTGCTGTGCACTG GGACTGGGGATTCCACATGTTTGCCTTGGGAGTCTCAAGCTGGACTGCCAGC CCCTGTCCTCCCTTCACCCCCATTGCGTATGAGCATTTCAGAACTCCAAGGAG TCACAGGCATCTTTATAGTTCACGTTAACATATAGACACTGTTGGAAGCAGTT CCTTCTAAAAGGGTAGCCCTGGACTTAATACCAGCCGGATACCTCTGGCCCC CACCCCATTACTGTACCTCTGGAGTCACTACTGTGGGTCGCCACTCCTCTGCT ACACAGCACGGCTTTTTCAAGGCTGTATTGAGAAGGGAAGTTAGGAAGAAG GGTGTGCTGGGCTAACCAGCCCACAGAGCTCACATTCCTGTCCCTTGGGTGA AAAATACATGTCCATCCTGATATCTCCTGAATTCAGAAATTAGCCTCCACATG TGCAATGGCTTTAAGAGCCAGAAGCAGGGTTCTGGGAATTTTGCAAGTTACC TGTGGCCAGGTGTGGTCTCGGTTACCAAATACGGTTACCTGCAGCTTTTTAGT CCTTTGTGCTCCCACGGGTCTACAGAGTCCCATCTGCCCAAAGGTCTTGAAGC TTGACAGGATGTTTTCGATTACTCAGTCTCCCAGGGCACTACTGGTCCGTAGG ATTCGATTGGTCGGGGTAGGAGAGTTAAACAACATTTAAACAGAGTTCTCTC AAAAATGTCTAAAGGGATTGTAGGTAGATAACATCCAATCACTGTTTGCACT TATCTGAAATCTTCCCTCTTGGCTGCCCCCAGGTATTTACTGTGGAGAACATT GCATAGGAATGTCTGGAAAAAGCTTCTACAACTTGTTACAGCCTTCACATTT GTAGAAGCTTT |
| 34 | opt_COX10-op t_ND4*-3'UT R* | ATGGCCGCCTCTCCACACACACTGAGTAGCAGACTGCTGACCGGCTGTGTTG GCGGCTCTGTGTGGTATCTGGAACGGCGGACAATGCTGAAGCTGATCGTGCC CACCATCATGCTGCTGCCCCTGACCTGGCTGAGCAAGAAGCACATGATCTGG ATCAACACCACCACCCACAGCCTGATCATCAGCATCATCCCCCTGCTGTTCTT CAACCAGATCAACAACAACCTGTTCAGCTGCAGCCCCACCTTCAGCAGCGAC CCCCTGACCACCCCCCTGCTGATGCTGACCACCTGGCTGCTGCCCCTGACCAT CATGGCCAGCCAGCGCCACCTGAGCAGCGAGCCCCTGAGCCGCAAGAAGCT GTACCTGAGCATGCTGATCAGCCTGCAGATCAGCCTGATCATGACCTTCACC GCCACCGAGCTGATCATGTTCTACATCTTCTTCGAGACCACCCTGATCCCCAC |
| | | CCTGGCCATCATCACCCGCTGGGGCAACCAGCCCGAGCGCCTGAACGCCGGC ACCTACTTCCTGTTCTACACCCTGGTGGGCAGCCTGCCCCTGCTGATCGCCCT GATCTACACCCACAACACCCTGGGCAGCCTGAACATCCTGCTGCTGACCCTG ACCGCCCAGGAGCTGAGCAACAGCTGGGCCAACAACCTGATGTGGCTGGCCT ACACCATGGCCTTCATGGTGAAGATGCCCCTGTACGGCCTGCACCTGTGGCT GCCCAAGGCCCACGTGGAGGCCCCCATCGCCGGCAGCATGGTGCTGGCCGCC GTGCTGCTGAAGCTGGGCGGCTACGGCATGATGCGCCTGACCCTGATCCTGA ACCCCCTGACCAAGCACATGGCCTACCCCTTCCTGGTGCTGAGCCTGTGGGG CATGATCATGACCAGCAGCATCTGCCTGCGCCAGACCGACCTGAAGAGCCTG ATCGCCTACAGCAGCATCAGCCACATGGCCCTGGTGGTGACCGCCATCCTGA TCCAGACCCCCTGGAGCTTCACCGGCGCCGTGATCCTGATGATCGCCCACGG CCTGACCAGCAGCCTGCTGTTCTGCCTGGCCAACAGCAACTACGAGCGCACC CACAGCCGCATCATGATCCTGAGCCAGGGCCTGCAGACCCTGCTGCCCCTGA TGGCCTTCTGGTGGCTGCTGGCCAGCCTGGCCAACCTGGCCCTGCCCCCCACC ATCAACCTGCTGGGCGAGCTGAGCGTGCTGGTGACCACCTTCAGCTGGAGCA ACATCACCCTGCTGCTGACCGGCCTGAACATGCTGGTGACCGCCCTGTACAG CCTGTACATGTTCACCACCACCCAGTGGGGCAGCCTGACCCACCACATCAAC AACATGAAGCCCAGCTTCACCCGCGAGAACACCCTGATGTTCATGCACCTGA GCCCCATCCTGCTGCTGAGCCTGAACCCCGACATCATCACCGGCTTCAGCAG CTAAGAGCACTGGGACGCCCACCGCCCCTTTCCCTCCGCTGCCAGGCGAGCA TGTTGTGGTAATTCTGGAACACAAGAAGAGAAATTGCTGGGTTTAGAACAAG ATTATAAACGAATTCGGTGCTCAGTGATCACTTGACAGTTTTTTTTTTTTTTAA ATATTACCCAAAATGCTCCCCAAATAAGAAATGCATCAGCTCAGTCAGTGAA TACAAAAAAGGAATTATTTTTCCCTTTGAGGGTCTTTTATACATCTCTCCTCC AACCCCACCCTCTATTCTGTTTCTTCCTCCTCACATGGGGGTACACATACACA GCTTCCTCTTTTGGTTCCATCCTTACCACCACACCACACGCACACTCCACATG CCCAGCAGAGTGGCACTTGGTGGCCAGAAAGTGTGAGCCTCATGATCTGCTG TCTGTAGTTCTGTGAGCTCAGGTCCCTCAAAGGCCTCGGAGCACCCCCTTCCT TGTGACTGAGCCAGGGCCTGCATTTTTGGTTTTCCCCACCCCACACATTCTCA ACCATAGTCCTTCTAACAATACCAATAGCTAGGACCCGGCTGCTGTGCACTG GGACTGGGGATTCCACATGTTTGCCTTGGGAGTCTCAAGCTGGACTGCCA |
| 35 | opt_COX10-N D6-3'UTR | ATGGCCGCCTCTCCACACACACTGAGTAGCAGACTGCTGACCGGCTGTGTTG GCGGCTCTGTGTGGTATCTGGAACGGCGGACAATGATGTATGCTTTGTTTCTG TTGAGTGTGGGTTTAGTAATGGGGTTTGTGGGGTTTTCTTCTAAGCCTTCTCC TATTTATGGGGGTTTAGTATTGATTGTTAGCGGTGTGGTCGGGTGTGTTATTA TTCTGAATTTTGGGGGAGGTTATATGGGTTTAATGGTTTTTTTAATTTATTTAG GGGGAATGATGGTTGTCTTTGGATATACTACAGCGATGGCTATTGAGGAGTA TCCTGAGGCATGGGGGTCAGGGGTTGAGGTCTTGGTGAGTGTTTTAGTGGGG TTAGCGATGGAGGTAGGATTGGTGCTGTGGGTGAAAGAGTATGATGGGGTGG TGGTTGTGGTAAACTTTAATAGTGTAGGAAGCTGGATGATTTATGAAGGAGA |
| | | GGGGTCAGGGTTGATTCGGGAGGATCCTATTGGTGCGGGGGCTTTGTATGAT TATGGGCGTTGGTTAGTAGTAGTTACTGGTTGGACATTGTTTGTTGGTGTATA TATTGTAATTGAGATTGCTCGGGGGAATTAGGAGCACTGGGACGCCCACCGC CCCTTTCCCTCCGCTGCCAGGCGAGCATGTTGTGGTAATTCTGGAACACAAG AAGAGAAATTGCTGGGTTTAGAACAAGATTATAAACGAATTCGGTGCTCAGT GATCACTTGACAGTTTTTTTTTTTTTTAAATATTACCCAAAATGCTCCCCAAAT AAGAAATGCATCAGCTCAGTCAGTGAATACAAAAAAGGAATTATTTTTCCCT TTGAGGGTCTTTTATACATCTCTCCTCCAACCCCACCCTCTATTCTGTTTCTTC CTCCTCACATGGGGGTACACATACACAGCTTCCTCTTTTGGTTCCATCCTTAC CACCACACCACACGCACACTCCACATGCCCAGCAGAGTGGCACTTGGTGGCC AGAAAGTGTGAGCCTCATGATCTGCTGTCTGTAGTTCTGTGAGCTCAGGTCCC TCAAAGGCCTCGGAGCACCCCCTTCCTTGTGACTGAGCCAGGGCCTGCATTTT TGGTTTTCCCCACCCCACACATTCTCAACCATAGTCCTTCTAACAATACCAAT AGCTAGGACCCGGCTGCTGTGCACTGGGACTGGGGATTCCACATGTTTGCCT TGGGAGTCTCAAGCTGGACTGCCAGCCCCTGTCCTCCCTTCACCCCCATTGCG TATGAGCATTTCAGAACTCCAAGGAGTCACAGGCATCTTTATAGTTCACGTT AACATATAGACACTGTTGGAAGCAGTTCCTTCTAAAAGGGTAGCCCTGGACT TAATACCAGCCGGATACCTCTGGCCCCCACCCCATTACTGTACCTCTGGAGTC ACTACTGTGGGTCGCCACTCCTCTGCTACACAGCACGGCTTTTTCAAGGCTGT ATTGAGAAGGGAAGTTAGGAAGAAGGGTGTGCTGGGCTAACCAGCCCACAG AGCTCACATTCCTGTCCCTTGGGTGAAAAATACATGTCCATCCTGATATCTCC TGAATTCAGAAATTAGCCTCCACATGTGCAATGGCTTTAAGAGCCAGAAGCA GGGTTCTGGGAATTTTGCAAGTTACCTGTGGCCAGGTGTGGTCTCGGTTACCA AATACGGTTACCTGCAGCTTTTTAGTCCTTTGTGCTCCCACGGGTCTACAGAG TCCCATCTGCCCAAAGGTCTTGAAGCTTGACAGGATGTTTTCGATTACTCAGT CTCCCAGGGCACTACTGGTCCGTAGGATTCGATTGGTCGGGGTAGGAGAGTT AAACAACATTTAAACAGAGTTCTCTCAAAAATGTCTAAAGGGATTGTAGGTA GATAACATCCAATCACTGTTTGCACTTATCTGAAATCTTCCCTCTTGGCTGCC CCCAGGTATTTACTGTGGAGAACATTGCATAGGAATGTCTGGAAAAAGCTTC TACAACTTGTTACAGCCTTCACATTTGTAGAAGCTTT |
| 36 | opt_COX10-N D6-3'UTR* | ATGGCCGCCTCTCCACACACACTGAGTAGCAGACTGCTGACCGGCTGTGTTG GCGGCTCTGTGTGGTATCTGGAACGGCGGACAATGATGTATGCTTTGTTTCTG TTGAGTGTGGGTTTAGTAATGGGGTTTGTGGGGTTTTCTTCTAAGCCTTCTCC TATTTATGGGGGTTTAGTATTGATTGTTAGCGGTGTGGTCGGGTGTGTTATTA TTCTGAATTTTGGGGGAGGTTATATGGGTTTAATGGTTTTTTTAATTTATTTAG GGGGAATGATGGTTGTCTTTGGATATACTACAGCGATGGCTATTGAGGAGTA TCCTGAGGCATGGGGGTCAGGGGTTGAGGTCTTGGTGAGTGTTTTAGTGGGG TTAGCGATGGAGGTAGGATTGGTGCTGTGGGTGAAAGAGTATGATGGGGTGG TGGTTGTGGTAAACTTTAATAGTGTAGGAAGCTGGATGATTTATGAAGGAGA GGGGTCAGGGTTGATTCGGGAGGATCCTATTGGTGCGGGGGCTTTGTATGAT |
| | | TATGGGCGTTGGTTAGTAGTAGTTACTGGTTGGACATTGTTTGTTGGTGTATA TATTGTAATTGAGATTGCTCGGGGGAATTAGGAGCACTGGGACGCCCACCGC CCCTTTCCCTCCGCTGCCAGGCGAGCATGTTGTGGTAATTCTGGAACACAAG AAGAGAAATTGCTGGGTTTAGAACAAGATTATAAACGAATTCGGTGCTCAGT GATCACTTGACAGTTTTTTTTTTTTTTAAATATTACCCAAAATGCTCCCCAAAT AAGAAATGCATCAGCTCAGTCAGTGAATACAAAAAAGGAATTATTTTTCCCT TTGAGGGTCTTTTATACATCTCTCCTCCAACCCCACCCTCTATTCTGTTTCTTC CTCCTCACATGGGGGTACACATACACAGCTTCCTCTTTTGGTTCCATCCTTAC CACCACACCACACGCACACTCCACATGCCCAGCAGAGTGGCACTTGGTGGCC AGAAAGTGTGAGCCTCATGATCTGCTGTCTGTAGTTCTGTGAGCTCAGGTCCC TCAAAGGCCTCGGAGCACCCCCTTCCTTGTGACTGAGCCAGGGCCTGCATTTT TGGTTTTCCCCACCCCACACATTCTCAACCATAGTCCTTCTAACAATACCAAT AGCTAGGACCCGGCTGCTGTGCACTGGGACTGGGGATTCCACATGTTTGCCT TGGGAGTCTCAAGCTGGACTGCCA |
| 37 | opt_COX10-op t_ND6-3'UTR | ATGGCCGCCTCTCCACACACACTGAGTAGCAGACTGCTGACCGGCTGTGTTG GCGGCTCTGTGTGGTATCTGGAACGGCGGACAATGATGTACGCCCTGTTCCT GCTGAGCGTGGGCCTGGTGATGGGCTTCGTGGGCTTCAGCAGCAAGCCCAGC CCCATCTACGGCGGCCTGGTGCTGATCGTGAGCGGCGTGGTGGGCTGCGTGA TCATCCTGAACTTCGGCGGCGGCTACATGGGCCTGATGGTGTTCCTGATCTAC CTGGGCGGCATGATGGTGGTGTTCGGCTACACCACCGCCATGGCCATCGAGG AGTACCCCGAGGCCTGGGGCAGCGGCGTGGAGGTGCTGGTGAGCGTGCTGG TGGGCCTGGCCATGGAGGTGGGCCTGGTGCTGTGGGTGAAGGAGTACGACG GCGTGGTGGTGGTGGTGAACTTCAACAGCGTGGGCAGCTGGATGATCTACGA GGGCGAGGGCAGCGGCCTGATCCGCGAGGACCCCATCGGCGCCGGCGCCCT GTACGACTACGGCCGCTGGCTGGTGGTGGTGACCGGCTGGACCCTGTTCGTG GGCGTGTACATCGTGATCGAGATCGCCCGCGGCAACTAAGAGCACTGGGAC GCCCACCGCCCCTTTCCCTCCGCTGCCAGGCGAGCATGTTGTGGTAATTCTGG AACACAAGAAGAGAAATTGCTGGGTTTAGAACAAGATTATAAACGAATTCG GTGCTCAGTGATCACTTGACAGTTTTTTTTTTTTTTAAATATTACCCAAAATGC TCCCCAAATAAGAAATGCATCAGCTCAGTCAGTGAATACAAAAAAGGAATT ATTTTTCCCTTTGAGGGTCTTTTATACATCTCTCCTCCAACCCCACCCTCTATT CTGTTTCTTCCTCCTCACATGGGGGTACACATACACAGCTTCCTCTTTTGGTTC CATCCTTACCACCACACCACACGCACACTCCACATGCCCAGCAGAGTGGCAC TTGGTGGCCAGAAAGTGTGAGCCTCATGATCTGCTGTCTGTAGTTCTGTGAGC TCAGGTCCCTCAAAGGCCTCGGAGCACCCCCTTCCTTGTGACTGAGCCAGGG CCTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCAGCCCCTGTCCTCCCTTCACC CCCATTGCGTATGAGCATTTCAGAACTCCAAGGAGTCACAGGCATCTTTATA GTTCACGTTAACATATAGACACTGTTGGAAGCAGTTCCTTCTAAAAGGGTAG |
| | | CCCTGGACTTAATACCAGCCGGATACCTCTGGCCCCCACCCCATTACTGTACC TCTGGAGTCACTACTGTGGGTCGCCACTCCTCTGCTACACAGCACGGCTTTTT CAAGGCTGTATTGAGAAGGGAAGTTAGGAAGAAGGGTGTGCTGGGCTAACC AGCCCACAGAGCTCACATTCCTGTCCCTTGGGTGAAAAATACATGTCCATCC TGATATCTCCTGAATTCAGAAATTAGCCTCCACATGTGCAATGGCTTTAAGA GCCAGAAGCAGGGTTCTGGGAATTTTGCAAGTTACCTGTGGCCAGGTGTGGT CTCGGTTACCAAATACGGTTACCTGCAGCTTTTTAGTCCTTTGTGCTCCCACG GGTCTACAGAGTCCCATCTGCCCAAAGGTCTTGAAGCTTGACAGGATGTTTT CGATTACTCAGTCTCCCAGGGCACTACTGGTCCGTAGGATTCGATTGGTCGG GGTAGGAGAGTTAAACAACATTTAAACAGAGTTCTCTCAAAAATGTCTAAAG GGATTGTAGGTAGATAACATCCAATCACTGTTTGCACTTATCTGAAATCTTCC CTCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 38 | opt_COX10-op t_ND6-3'UTR * | ATGGCCGCCTCTCCACACACACTGAGTAGCAGACTGCTGACCGGCTGTGTTG GCGGCTCTGTGTGGTATCTGGAACGGCGGACAATGATGTACGCCCTGTTCCT GCTGAGCGTGGGCCTGGTGATGGGCTTCGTGGGCTTCAGCAGCAAGCCCAGC CCCATCTACGGCGGCCTGGTGCTGATCGTGAGCGGCGTGGTGGGCTGCGTGA TCATCCTGAACTTCGGCGGCGGCTACATGGGCCTGATGGTGTTCCTGATCTAC CTGGGCGGCATGATGGTGGTGTTCGGCTACACCACCGCCATGGCCATCGAGG AGTACCCCGAGGCCTGGGGCAGCGGCGTGGAGGTGCTGGTGAGCGTGCTGG TGGGCCTGGCCATGGAGGTGGGCCTGGTGCTGTGGGTGAAGGAGTACGACG GCGTGGTGGTGGTGGTGAACTTCAACAGCGTGGGCAGCTGGATGATCTACGA GGGCGAGGGCAGCGGCCTGATCCGCGAGGACCCCATCGGCGCCGGCGCCCT GTACGACTACGGCCGCTGGCTGGTGGTGGTGACCGGCTGGACCCTGTTCGTG GGCGTGTACATCGTGATCGAGATCGCCCGCGGCAACTAAGAGCACTGGGAC GCCCACCGCCCCTTTCCCTCCGCTGCCAGGCGAGCATGTTGTGGTAATTCTGG AACACAAGAAGAGAAATTGCTGGGTTTAGAACAAGATTATAAACGAATTCG GTGCTCAGTGATCACTTGACAGTTTTTTTTTTTTTTAAATATTACCCAAAATGC TCCCCAAATAAGAAATGCATCAGCTCAGTCAGTGAATACAAAAAAGGAATT ATTTTTCCCTTTGAGGGTCTTTTATACATCTCTCCTCCAACCCCACCCTCTATT CTGTTTCTTCCTCCTCACATGGGGGTACACATACACAGCTTCCTCTTTTGGTTC CATCCTTACCACCACACCACACGCACACTCCACATGCCCAGCAGAGTGGCAC TTGGTGGCCAGAAAGTGTGAGCCTCATGATCTGCTGTCTGTAGTTCTGTGAGC TCAGGTCCCTCAAAGGCCTCGGAGCACCCCCTTCCTTGTGACTGAGCCAGGG CCTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCA |
| 39 | opt_COX10-N D1-3'UTR | ATGGCCGCCTCTCCACACACACTGAGTAGCAGACTGCTGACCGGCTGTGTTG GCGGCTCTGTGTGGTATCTGGAACGGCGGACAATGGCCAACCTCCTACTCCT |
| | | CATTGTACCCATTCTAATCGCAATGGCATTCCTAATGCTTACCGAACGAAAA ATTCTAGGCTATATGCAACTACGCAAAGGCCCCAACGTTGTAGGCCCCTACG GGCTACTACAACCCTTCGCTGACGCCATAAAACTCTTCACCAAAGAGCCCCT AAAACCCGCCACATCTACCATCACCCTCTACATCACCGCCCCGACCTTAGCTC TCACCATCGCTCTTCTACTATGGACCCCCCTCCCCATGCCCAACCCCCTGGTC AACCTCAACCTAGGCCTCCTATTTATTCTAGCCACCTCTAGCCTAGCCGTTTA CTCAATCCTCTGGTCAGGGTGGGCATCAAACTCAAACTACGCCCTGATCGGC GCACTGCGAGCAGTAGCCCAAACAATCTCATATGAAGTCACCCTAGCCATCA TTCTACTATCAACATTACTAATGAGTGGCTCCTTTAACCTCTCCACCCTTATC ACAACACAAGAACACCTCTGGTTACTCCTGCCATCATGGCCCTTGGCCATGA TGTGGTTTATCTCCACACTAGCAGAGACCAACCGAACCCCCTTCGACCTTGCC GAAGGGGAGTCCGAACTAGTCTCAGGCTTCAACATCGAATACGCCGCAGGCC CCTTCGCCCTATTCTTCATGGCCGAATACACAAACATTATTATGATGAACACC CTCACCACTACAATCTTCCTAGGAACAACATATGACGCACTCTCCCCTGAACT CTACACAACATATTTTGTCACCAAGACCCTACTTCTAACCTCCCTGTTCTTAT GGATTCGAACAGCATACCCCCGATTCCGCTACGACCAACTCATGCACCTCCT ATGGAAAAACTTCCTACCACTCACCCTAGCATTACTTATGTGGTATGTCTCCA TGCCCATTACAATCTCCAGCATTCCCCCTCAAACCTAAGAGCACTGGGACGC CCACCGCCCCTTTCCCTCCGCTGCCAGGCGAGCATGTTGTGGTAATTCTGGAA CACAAGAAGAGAAATTGCTGGGTTTAGAACAAGATTATAAACGAATTCGGT GCTCAGTGATCACTTGACAGTTTTTTTTTTTTTTAAATATTACCCAAAATGCTC CCCAAATAAGAAATGCATCAGCTCAGTCAGTGAATACAAAAAAGGAATTATT TTTCCCTTTGAGGGTCTTTTATACATCTCTCCTCCAACCCCACCCTCTATTCTG TTTCTTCCTCCTCACATGGGGGTACACATACACAGCTTCCTCTTTTGGTTCCAT CCTTACCACCACACCACACGCACACTCCACATGCCCAGCAGAGTGGCACTTG GTGGCCAGAAAGTGTGAGCCTCATGATCTGCTGTCTGTAGTTCTGTGAGCTC AGGTCCCTCAAAGGCCTCGGAGCACCCCCTTCCTTGTGACTGAGCCAGGGCC TGCATTTTTGGTTTTCCCCACCCCACACATTCTCAACCATAGTCCTTCTAACA ATACCAATAGCTAGGACCCGGCTGCTGTGCACTGGGACTGGGGATTCCACAT GTTTGCCTTGGGAGTCTCAAGCTGGACTGCCAGCCCCTGTCCTCCCTTCACCC CCATTGCGTATGAGCATTTCAGAACTCCAAGGAGTCACAGGCATCTTTATAG TTCACGTTAACATATAGACACTGTTGGAAGCAGTTCCTTCTAAAAGGGTAGC CCTGGACTTAATACCAGCCGGATACCTCTGGCCCCCACCCCATTACTGTACCT CTGGAGTCACTACTGTGGGTCGCCACTCCTCTGCTACACAGCACGGCTTTTTC AAGGCTGTATTGAGAAGGGAAGTTAGGAAGAAGGGTGTGCTGGGCTAACCA GCCCACAGAGCTCACATTCCTGTCCCTTGGGTGAAAAATACATGTCCATCCT GATATCTCCTGAATTCAGAAATTAGCCTCCACATGTGCAATGGCTTTAAGAG CCAGAAGCAGGGTTCTGGGAATTTTGCAAGTTACCTGTGGCCAGGTGTGGTC TCGGTTACCAAATACGGTTACCTGCAGCTTTTTAGTCCTTTGTGCTCCCACGG GTCTACAGAGTCCCATCTGCCCAAAGGTCTTGAAGCTTGACAGGATGTTTTC |
| | | GATTACTCAGTCTCCCAGGGCACTACTGGTCCGTAGGATTCGATTGGTCGGG GTAGGAGAGTTAAACAACATTTAAACAGAGTTCTCTCAAAAATGTCTAAAGG GATTGTAGGTAGATAACATCCAATCACTGTTTGCACTTATCTGAAATCTTCCC TCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 40 | opt_COX10-N D 1-3' UTR* | ATGGCCGCCTCTCCACACACACTGAGTAGCAGACTGCTGACCGGCTGTGTTG GCGGCTCTGTGTGGTATCTGGAACGGCGGACAATGGCCAACCTCCTACTCCT CATTGTACCCATTCTAATCGCAATGGCATTCCTAATGCTTACCGAACGAAAA ATTCTAGGCTATATGCAACTACGCAAAGGCCCCAACGTTGTAGGCCCCTACG GGCTACTACAACCCTTCGCTGACGCCATAAAACTCTTCACCAAAGAGCCCCT AAAACCCGCCACATCTACCATCACCCTCTACATCACCGCCCCGACCTTAGCTC TCACCATCGCTCTTCTACTATGGACCCCCCTCCCCATGCCCAACCCCCTGGTC AACCTCAACCTAGGCCTCCTATTTATTCTAGCCACCTCTAGCCTAGCCGTTTA CTCAATCCTCTGGTCAGGGTGGGCATCAAACTCAAACTACGCCCTGATCGGC GCACTGCGAGCAGTAGCCCAAACAATCTCATATGAAGTCACCCTAGCCATCA TTCTACTATCAACATTACTAATGAGTGGCTCCTTTAACCTCTCCACCCTTATC ACAACACAAGAACACCTCTGGTTACTCCTGCCATCATGGCCCTTGGCCATGA TGTGGTTTATCTCCACACTAGCAGAGACCAACCGAACCCCCTTCGACCTTGCC GAAGGGGAGTCCGAACTAGTCTCAGGCTTCAACATCGAATACGCCGCAGGCC CCTTCGCCCTATTCTTCATGGCCGAATACACAAACATTATTATGATGAACACC CTCACCACTACAATCTTCCTAGGAACAACATATGACGCACTCTCCCCTGAACT CTACACAACATATTTTGTCACCAAGACCCTACTTCTAACCTCCCTGTTCTTAT GGATTCGAACAGCATACCCCCGATTCCGCTACGACCAACTCATGCACCTCCT ATGGAAAAACTTCCTACCACTCACCCTAGCATTACTTATGTGGTATGTCTCCA TGCCCATTACAATCTCCAGCATTCCCCCTCAAACCTAAGAGCACTGGGACGC CCACCGCCCCTTTCCCTCCGCTGCCAGGCGAGCATGTTGTGGTAATTCTGGAA CACAAGAAGAGAAATTGCTGGGTTTAGAACAAGATTATAAACGAATTCGGT GCTCAGTGATCACTTGACAGTTTTTTTTTTTTTTAAATATTACCCAAAATGCTC CCCAAATAAGAAATGCATCAGCTCAGTCAGTGAATACAAAAAAGGAATTATT TTTCCCTTTGAGGGTCTTTTATACATCTCTCCTCCAACCCCACCCTCTATTCTG TTTCTTCCTCCTCACATGGGGGTACACATACACAGCTTCCTCTTTTGGTTCCAT CCTTACCACCACACCACACGCACACTCCACATGCCCAGCAGAGTGGCACTTG GTGGCCAGAAAGTGTGAGCCTCATGATCTGCTGTCTGTAGTTCTGTGAGCTC AGGTCCCTCAAAGGCCTCGGAGCACCCCCTTCCTTGTGACTGAGCCAGGGCC TGCATTTTTGGTTTTCCCCACCCCACACATTCTCAACCATAGTCCTTCTAACA ATACCAATAGCTAGGACCCGGCTGCTGTGCACTGGGACTGGGGATTCCACAT GTTTGCCTTGGGAGTCTCAAGCTGGACTGCCA |
| 41 | opt_COX10-op t_ND1-3'UTR | ATGGCCGCCTCTCCACACACACTGAGTAGCAGACTGCTGACCGGCTGTGTTG GCGGCTCTGTGTGGTATCTGGAACGGCGGACAATGGCCAACCTGCTGCTGCT |
| | | GATCGTGCCCATCCTGATCGCCATGGCCTTCCTGATGCTGACCGAGCGCAAG ATCCTGGGCTACATGCAGCTGCGCAAGGGCCCCAACGTGGTGGGCCCCTACG GCCTGCTGCAGCCCTTCGCCGACGCCATCAAGCTGTTCACCAAGGAGCCCCT GAAGCCCGCCACCAGCACCATCACCCTGTACATCACCGCCCCCACCCTGGCC CTGACCATCGCCCTGCTGCTGTGGACCCCCCTGCCCATGCCCAACCCCCTGGT GAACCTGAACCTGGGCCTGCTGTTCATCCTGGCCACCAGCAGCCTGGCCGTG TACAGCATCCTGTGGAGCGGCTGGGCCAGCAACAGCAACTACGCCCTGATCG GCGCCCTGCGCGCCGTGGCCCAGACCATCAGCTACGAGGTGACCCTGGCCAT CATCCTGCTGAGCACCCTGCTGATGAGCGGCAGCTTCAACCTGAGCACCCTG ATCACCACCCAGGAGCACCTGTGGCTGCTGCTGCCCAGCTGGCCCCTGGCCA TGATGTGGTTCATCAGCACCCTGGCCGAGACCAACCGCACCCCCTTCGACCT GGCCGAGGGCGAGAGCGAGCTGGTGAGCGGCTTCAACATCGAGTACGCCGC CGGCCCCTTCGCCCTGTTCTTCATGGCCGAGTACACCAACATCATCATGATGA ACACCCTGACCACCACCATCTTCCTGGGCACCACCTACGACGCCCTGAGCCC CGAGCTGTACACCACCTACTTCGTGACCAAGACCCTGCTGCTGACCAGCCTG TTCCTGTGGATCCGCACCGCCTACCCCCGCTTCCGCTACGACCAGCTGATGCA CCTGCTGTGGAAGAACTTCCTGCCCCTGACCCTGGCCCTGCTGATGTGGTACG TGAGCATGCCCATCACCATCAGCAGCATCCCCCCCCAGACCTAAGAGCACTG GGACGCCCACCGCCCCTTTCCCTCCGCTGCCAGGCGAGCATGTTGTGGTAATT CTGGAACACAAGAAGAGAAATTGCTGGGTTTAGAACAAGATTATAAACGAA TTCGGTGCTCAGTGATCACTTGACAGTTTTTTTTTTTTTTAAATATTACCCAAA ATGCTCCCCAAATAAGAAATGCATCAGCTCAGTCAGTGAATACAAAAAAGG AATTATTTTTCCCTTTGAGGGTCTTTTATACATCTCTCCTCCAACCCCACCCTC TATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCAGCCCCTGTCCTCCCT TCACCCCCATTGCGTATGAGCATTTCAGAACTCCAAGGAGTCACAGGCATCT TTATAGTTCACGTTAACATATAGACACTGTTGGAAGCAGTTCCTTCTAAAAG GGTAGCCCTGGACTTAATACCAGCCGGATACCTCTGGCCCCCACCCCATTAC TGTACCTCTGGAGTCACTACTGTGGGTCGCCACTCCTCTGCTACACAGCACGG CTTTTTCAAGGCTGTATTGAGAAGGGAAGTTAGGAAGAAGGGTGTGCTGGGC TAACCAGCCCACAGAGCTCACATTCCTGTCCCTTGGGTGAAAAATACATGTC CATCCTGATATCTCCTGAATTCAGAAATTAGCCTCCACATGTGCAATGGCTTT AAGAGCCAGAAGCAGGGTTCTGGGAATTTTGCAAGTTACCTGTGGCCAGGTG TGGTCTCGGTTACCAAATACGGTTACCTGCAGCTTTTTAGTCCTTTGTGCTCC CACGGGTCTACAGAGTCCCATCTGCCCAAAGGTCTTGAAGCTTGACAGGATG |
| | | TTTTCGATTACTCAGTCTCCCAGGGCACTACTGGTCCGTAGGATTCGATTGGT CGGGGTAGGAGAGTTAAACAACATTTAAACAGAGTTCTCTCAAAAATGTCTA AAGGGATTGTAGGTAGATAACATCCAATCACTGTTTGCACTTATCTGAAATC TTCCCTCTTGGCTGCCCCCAGGTATTTACTGTGGAGAACATTGCATAGGAATG TCTGGAAAAAGCTTCTACAACTTGTTACAGCCTTCACATTTGTAGAAGCTTT |
| 42 | opt_COX10-op t_ND1-3'UTR * | ATGGCCGCCTCTCCACACACACTGAGTAGCAGACTGCTGACCGGCTGTGTTG GCGGCTCTGTGTGGTATCTGGAACGGCGGACAATGGCCAACCTGCTGCTGCT GATCGTGCCCATCCTGATCGCCATGGCCTTCCTGATGCTGACCGAGCGCAAG ATCCTGGGCTACATGCAGCTGCGCAAGGGCCCCAACGTGGTGGGCCCCTACG GCCTGCTGCAGCCCTTCGCCGACGCCATCAAGCTGTTCACCAAGGAGCCCCT GAAGCCCGCCACCAGCACCATCACCCTGTACATCACCGCCCCCACCCTGGCC CTGACCATCGCCCTGCTGCTGTGGACCCCCCTGCCCATGCCCAACCCCCTGGT GAACCTGAACCTGGGCCTGCTGTTCATCCTGGCCACCAGCAGCCTGGCCGTG TACAGCATCCTGTGGAGCGGCTGGGCCAGCAACAGCAACTACGCCCTGATCG GCGCCCTGCGCGCCGTGGCCCAGACCATCAGCTACGAGGTGACCCTGGCCAT CATCCTGCTGAGCACCCTGCTGATGAGCGGCAGCTTCAACCTGAGCACCCTG ATCACCACCCAGGAGCACCTGTGGCTGCTGCTGCCCAGCTGGCCCCTGGCCA TGATGTGGTTCATCAGCACCCTGGCCGAGACCAACCGCACCCCCTTCGACCT GGCCGAGGGCGAGAGCGAGCTGGTGAGCGGCTTCAACATCGAGTACGCCGC CGGCCCCTTCGCCCTGTTCTTCATGGCCGAGTACACCAACATCATCATGATGA ACACCCTGACCACCACCATCTTCCTGGGCACCACCTACGACGCCCTGAGCCC CGAGCTGTACACCACCTACTTCGTGACCAAGACCCTGCTGCTGACCAGCCTG TTCCTGTGGATCCGCACCGCCTACCCCCGCTTCCGCTACGACCAGCTGATGCA CCTGCTGTGGAAGAACTTCCTGCCCCTGACCCTGGCCCTGCTGATGTGGTACG TGAGCATGCCCATCACCATCAGCAGCATCCCCCCCCAGACCTAAGAGCACTG GGACGCCCACCGCCCCTTTCCCTCCGCTGCCAGGCGAGCATGTTGTGGTAATT CTGGAACACAAGAAGAGAAATTGCTGGGTTTAGAACAAGATTATAAACGAA TTCGGTGCTCAGTGATCACTTGACAGTTTTTTTTTTTTTTAAATATTACCCAAA ATGCTCCCCAAATAAGAAATGCATCAGCTCAGTCAGTGAATACAAAAAAGG AATTATTTTTCCCTTTGAGGGTCTTTTATACATCTCTCCTCCAACCCCACCCTC TATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCA |
| 43 | opt_COX10*-ND4-3'UTR | ATGGCCGCCAGCCCCCACACCCTGAGCAGCCGCCTGCTGACCGGCTGCGTGG GCGGCAGCGTGTGGTACCTGGAGCGCCGCACCATGCTAAAACTAATCGTCCC |
| | | AACAATTATGTTACTACCACTGACATGGCTTTCCAAAAAACACATGATTTGG ATCAACACAACCACCCACAGCCTAATTATTAGCATCATCCCTCTACTATTTTT TAACCAAATCAACAACAACCTATTTAGCTGTTCCCCAACCTTTTCCTCCGACC CCCTAACAACCCCCCTCCTAATGCTAACTACCTGGCTCCTACCCCTCACAATC ATGGCAAGCCAACGCCACTTATCCAGTGAACCACTATCACGAAAAAAACTCT ACCTCTCTATGCTAATCTCCCTACAAATCTCCTTAATTATGACATTCACAGCC ACAGAACTAATCATGTTTTATATCTTCTTCGAAACCACACTTATCCCCACCTT GGCTATCATCACCCGATGGGGCAACCAGCCAGAACGCCTGAACGCAGGCAC ATACTTCCTATTCTACACCCTAGTAGGCTCCCTTCCCCTACTCATCGCACTAA TTTACACTCACAACACCCTAGGCTCACTAAACATTCTACTACTCACTCTCACT GCCCAAGAACTATCAAACTCCTGGGCCAACAACTTAATGTGGCTAGCTTACA CAATGGCTTTTATGGTAAAGATGCCTCTTTACGGACTCCACTTATGGCTCCCT AAAGCCCATGTCGAAGCCCCCATCGCTGGGTCAATGGTACTTGCCGCAGTAC TCTTAAAACTAGGCGGCTATGGTATGATGCGCCTCACACTCATTCTCAACCCC CTGACAAAACACATGGCCTACCCCTTCCTTGTACTATCCCTATGGGGCATGAT TATGACAAGCTCCATCTGCCTACGACAAACAGACCTAAAATCGCTCATTGCA TACTCTTCAATCAGCCACATGGCCCTCGTAGTAACAGCCATTCTCATCCAAAC CCCCTGGAGCTTCACCGGCGCAGTCATTCTCATGATCGCCCACGGGCTTACAT CCTCATTACTATTCTGCCTAGCAAACTCAAACTACGAACGCACTCACAGTCG CATCATGATCCTCTCTCAAGGACTTCAAACTCTACTCCCACTAATGGCTTTTT GGTGGCTTCTAGCAAGCCTCGCTAACCTCGCCTTACCCCCCACTATTAACCTA CTGGGAGAACTCTCTGTGCTAGTAACCACGTTCTCCTGGTCAAATATCACTCT CCTACTTACAGGACTCAACATGCTAGTCACAGCCCTATACTCCCTCTACATGT TTACCACAACACAATGGGGCTCACTCACCCACCACATTAACAACATGAAACC CTCATTCACACGAGAAAACACCCTCATGTTCATGCACCTATCCCCCATTCTCC TCCTATCCCTCAACCCCGACATCATTACCGGGTTTTCCTCTTAAGAGCACTGG GACGCCCACCGCCCCTTTCCCTCCGCTGCCAGGCGAGCATGTTGTGGTAATTC TGGAACACAAGAAGAGAAATTGCTGGGTTTAGAACAAGATTATAAACGAAT TCGGTGCTCAGTGATCACTTGACAGTTTTTTTTTTTTTTAAATATTACCCAAAA TGCTCCCCAAATAAGAAATGCATCAGCTCAGTCAGTGAATACAAAAAAGGA ATTATTTTTCCCTTTGAGGGTCTTTTATACATCTCTCCTCCAACCCCACCCTCT ATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCAGCCCCTGTCCTCCCT TCACCCCCATTGCGTATGAGCATTTCAGAACTCCAAGGAGTCACAGGCATCT TTATAGTTCACGTTAACATATAGACACTGTTGGAAGCAGTTCCTTCTAAAAG |
| | | GGTAGCCCTGGACTTAATACCAGCCGGATACCTCTGGCCCCCACCCCATTAC TGTACCTCTGGAGTCACTACTGTGGGTCGCCACTCCTCTGCTACACAGCACGG CTTTTTCAAGGCTGTATTGAGAAGGGAAGTTAGGAAGAAGGGTGTGCTGGGC TAACCAGCCCACAGAGCTCACATTCCTGTCCCTTGGGTGAAAAATACATGTC CATCCTGATATCTCCTGAATTCAGAAATTAGCCTCCACATGTGCAATGGCTTT AAGAGCCAGAAGCAGGGTTCTGGGAATTTTGCAAGTTACCTGTGGCCAGGTG TGGTCTCGGTTACCAAATACGGTTACCTGCAGCTTTTTAGTCCTTTGTGCTCC CACGGGTCTACAGAGTCCCATCTGCCCAAAGGTCTTGAAGCTTGACAGGATG TTTTCGATTACTCAGTCTCCCAGGGCACTACTGGTCCGTAGGATTCGATTGGT CGGGGTAGGAGAGTTAAACAACATTTAAACAGAGTTCTCTCAAAAATGTCTA AAGGGATTGTAGGTAGATAACATCCAATCACTGTTTGCACTTATCTGAAATC TTCCCTCTTGGCTGCCCCCAGGTATTTACTGTGGAGAACATTGCATAGGAATG TCTGGAAAAAGCTTCTACAACTTGTTACAGCCTTCACATTTGTAGAAGCTTT |
| 44 | opt_COX10*-ND4-3'UTR* | ATGGCCGCCAGCCCCCACACCCTGAGCAGCCGCCTGCTGACCGGCTGCGTGG GCGGCAGCGTGTGGTACCTGGAGCGCCGCACCATGCTAAAACTAATCGTCCC AACAATTATGTTACTACCACTGACATGGCTTTCCAAAAAACACATGATTTGG ATCAACACAACCACCCACAGCCTAATTATTAGCATCATCCCTCTACTATTTTT TAACCAAATCAACAACAACCTATTTAGCTGTTCCCCAACCTTTTCCTCCGACC CCCTAACAACCCCCCTCCTAATGCTAACTACCTGGCTCCTACCCCTCACAATC ATGGCAAGCCAACGCCACTTATCCAGTGAACCACTATCACGAAAAAAACTCT ACCTCTCTATGCTAATCTCCCTACAAATCTCCTTAATTATGACATTCACAGCC ACAGAACTAATCATGTTTTATATCTTCTTCGAAACCACACTTATCCCCACCTT GGCTATCATCACCCGATGGGGCAACCAGCCAGAACGCCTGAACGCAGGCAC ATACTTCCTATTCTACACCCTAGTAGGCTCCCTTCCCCTACTCATCGCACTAA TTTACACTCACAACACCCTAGGCTCACTAAACATTCTACTACTCACTCTCACT GCCCAAGAACTATCAAACTCCTGGGCCAACAACTTAATGTGGCTAGCTTACA CAATGGCTTTTATGGTAAAGATGCCTCTTTACGGACTCCACTTATGGCTCCCT AAAGCCCATGTCGAAGCCCCCATCGCTGGGTCAATGGTACTTGCCGCAGTAC TCTTAAAACTAGGCGGCTATGGTATGATGCGCCTCACACTCATTCTCAACCCC CTGACAAAACACATGGCCTACCCCTTCCTTGTACTATCCCTATGGGGCATGAT TATGACAAGCTCCATCTGCCTACGACAAACAGACCTAAAATCGCTCATTGCA TACTCTTCAATCAGCCACATGGCCCTCGTAGTAACAGCCATTCTCATCCAAAC CCCCTGGAGCTTCACCGGCGCAGTCATTCTCATGATCGCCCACGGGCTTACAT CCTCATTACTATTCTGCCTAGCAAACTCAAACTACGAACGCACTCACAGTCG CATCATGATCCTCTCTCAAGGACTTCAAACTCTACTCCCACTAATGGCTTTTT GGTGGCTTCTAGCAAGCCTCGCTAACCTCGCCTTACCCCCCACTATTAACCTA CTGGGAGAACTCTCTGTGCTAGTAACCACGTTCTCCTGGTCAAATATCACTCT CCTACTTACAGGACTCAACATGCTAGTCACAGCCCTATACTCCCTCTACATGT TTACCACAACACAATGGGGCTCACTCACCCACCACATTAACAACATGAAACC CTCATTCACACGAGAAAACACCCTCATGTTCATGCACCTATCCCCCATTCTCC |
| | | TCCTATCCCTCAACCCCGACATCATTACCGGGTTTTCCTCTTAAGAGCACTGG GACGCCCACCGCCCCTTTCCCTCCGCTGCCAGGCGAGCATGTTGTGGTAATTC TGGAACACAAGAAGAGAAATTGCTGGGTTTAGAACAAGATTATAAACGAAT TCGGTGCTCAGTGATCACTTGACAGTTTTTTTTTTTTTTAAATATTACCCAAAA TGCTCCCCAAATAAGAAATGCATCAGCTCAGTCAGTGAATACAAAAAAGGA ATTATTTTTCCCTTTGAGGGTCTTTTATACATCTCTCCTCCAACCCCACCCTCT ATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCA |
| 45 | opt_COX10*-o pt_ND4-3'UT R | ATGGCCGCCAGCCCCCACACCCTGAGCAGCCGCCTGCTGACCGGCTGCGTGG GCGGCAGCGTGTGGTACCTGGAGCGCCGCACCATGCTGAAGCTGATCGTGCC CACCATCATGCTGCTGCCTCTGACCTGGCTGAGCAAGAAACACATGATCTGG ATCAACACCACCACGCACAGCCTGATCATCAGCATCATCCCTCTGCTGTTCTT CAACCAGATCAACAACAACCTGTTCAGCTGCAGCCCCACCTTCAGCAGCGAC CCTCTGACAACACCTCTGCTGATGCTGACCACCTGGCTGCTGCCCCTCACAAT CATGGCCTCTCAGAGACACCTGAGCAGCGAGCCCCTGAGCCGGAAGAAACT GTACCTGAGCATGCTGATCTCCCTGCAGATCTCTCTGATCATGACCTTCACCG CCACCGAGCTGATCATGTTCTACATCTTTTTCGAGACAACGCTGATCCCCACA CTGGCCATCATCACCAGATGGGGCAACCAGCCTGAGAGACTGAACGCCGGC ACCTACTTTCTGTTCTACACCCTCGTGGGCAGCCTGCCACTGCTGATTGCCCT GATCTACACCCACAACACCCTGGGCTCCCTGAACATCCTGCTGCTGACACTG ACAGCCCAAGAGCTGAGCAACAGCTGGGCCAACAATCTGATGTGGCTGGCCT ACACAATGGCCTTCATGGTCAAGATGCCCCTGTACGGCCTGCACCTGTGGCT GCCTAAAGCTCATGTGGAAGCCCCTATCGCCGGCTCTATGGTGCTGGCTGCA GTGCTGCTGAAACTCGGCGGCTACGGCATGATGCGGCTGACCCTGATTCTGA ATCCCCTGACCAAGCACATGGCCTATCCATTTCTGGTGCTGAGCCTGTGGGG CATGATTATGACCAGCAGCATCTGCCTGCGGCAGACCGATCTGAAGTCCCTG ATCGCCTACAGCTCCATCAGCCACATGGCCCTGGTGGTCACCGCCATCCTGA TTCAGACCCCTTGGAGCTTTACAGGCGCCGTGATCCTGATGATTGCCCACGG CCTGACAAGCAGCCTGCTGTTTTGTCTGGCCAACAGCAACTACGAGCGGACC CACAGCAGAATCATGATCCTGTCTCAGGGCCTGCAGACCCTCCTGCCTCTTAT GGCTTTTTGGTGGCTGCTGGCCTCTCTGGCCAATCTGGCACTGCCTCCTACCA TCAATCTGCTGGGCGAGCTGAGCGTGCTGGTCACCACATTCAGCTGGTCCAA TATCACCCTGCTGCTCACCGGCCTGAACATGCTGGTTACAGCCCTGTACTCCC TGTACATGTTCACCACCACACAGTGGGGAAGCCTGACACACCACATCAACAA TATGAAGCCCAGCTTCACCCGCGAGAACACCCTGATGTTCATGCATCTGAGC |
| | | CCCATTCTGCTGCTGTCCCTGAATCCTGATATCATCACCGGCTTCTCCAGCTG AGAGCACTGGGACGCCCACCGCCCCTTTCCCTCCGCTGCCAGGCGAGCATGT TGTGGTAATTCTGGAACACAAGAAGAGAAATTGCTGGGTTTAGAACAAGATT ATAAACGAATTCGGTGCTCAGTGATCACTTGACAGTTTTTTTTTTTTTTAAAT ATTACCCAAAATGCTCCCCAAATAAGAAATGCATCAGCTCAGTCAGTGAATA CAAAAAAGGAATTATTTTTCCCTTTGAGGGTCTTTTATACATCTCTCCTCCAA CCCCACCCTCTATTCTGTTTCTTCCTCCTCACATGGGGGTACACATACACAGC TTCCTCTTTTGGTTCCATCCTTACCACCACACCACACGCACACTCCACATGCC CAGCAGAGTGGCACTTGGTGGCCAGAAAGTGTGAGCCTCATGATCTGCTGTC TGTAGTTCTGTGAGCTCAGGTCCCTCAAAGGCCTCGGAGCACCCCCTTCCTTG TGACTGAGCCAGGGCCTGCATTTTTGGTTTTCCCCACCCCACACATTCTCAAC CATAGTCCTTCTAACAATACCAATAGCTAGGACCCGGCTGCTGTGCACTGGG ACTGGGGATTCCACATGTTTGCCTTGGGAGTCTCAAGCTGGACTGCCAGCCC CTGTCCTCCCTTCACCCCCATTGCGTATGAGCATTTCAGAACTCCAAGGAGTC ACAGGCATCTTTATAGTTCACGTTAACATATAGACACTGTTGGAAGCAGTTC CTTCTAAAAGGGTAGCCCTGGACTTAATACCAGCCGGATACCTCTGGCCCCC ACCCCATTACTGTACCTCTGGAGTCACTACTGTGGGTCGCCACTCCTCTGCTA CACAGCACGGCTTTTTCAAGGCTGTATTGAGAAGGGAAGTTAGGAAGAAGG GTGTGCTGGGCTAACCAGCCCACAGAGCTCACATTCCTGTCCCTTGGGTGAA AAATACATGTCCATCCTGATATCTCCTGAATTCAGAAATTAGCCTCCACATGT GCAATGGCTTTAAGAGCCAGAAGCAGGGTTCTGGGAATTTTGCAAGTTACCT GTGGCCAGGTGTGGTCTCGGTTACCAAATACGGTTACCTGCAGCTTTTTAGTC CTTTGTGCTCCCACGGGTCTACAGAGTCCCATCTGCCCAAAGGTCTTGAAGCT TGACAGGATGTTTTCGATTACTCAGTCTCCCAGGGCACTACTGGTCCGTAGG ATTCGATTGGTCGGGGTAGGAGAGTTAAACAACATTTAAACAGAGTTCTCTC AAAAATGTCTAAAGGGATTGTAGGTAGATAACATCCAATCACTGTTTGCACT TATCTGAAATCTTCCCTCTTGGCTGCCCCCAGGTATTTACTGTGGAGAACATT GCATAGGAATGTCTGGAAAAAGCTTCTACAACTTGTTACAGCCTTCACATTT GTAGAAGCTTT |
| 46 | opt_COX10*-o pt_ND4-3'UT R* | ATGGCCGCCAGCCCCCACACCCTGAGCAGCCGCCTGCTGACCGGCTGCGTGG GCGGCAGCGTGTGGTACCTGGAGCGCCGCACCATGCTGAAGCTGATCGTGCC CACCATCATGCTGCTGCCTCTGACCTGGCTGAGCAAGAAACACATGATCTGG ATCAACACCACCACGCACAGCCTGATCATCAGCATCATCCCTCTGCTGTTCTT CAACCAGATCAACAACAACCTGTTCAGCTGCAGCCCCACCTTCAGCAGCGAC CCTCTGACAACACCTCTGCTGATGCTGACCACCTGGCTGCTGCCCCTCACAAT CATGGCCTCTCAGAGACACCTGAGCAGCGAGCCCCTGAGCCGGAAGAAACT GTACCTGAGCATGCTGATCTCCCTGCAGATCTCTCTGATCATGACCTTCACCG CCACCGAGCTGATCATGTTCTACATCTTTTTCGAGACAACGCTGATCCCCACA CTGGCCATCATCACCAGATGGGGCAACCAGCCTGAGAGACTGAACGCCGGC ACCTACTTTCTGTTCTACACCCTCGTGGGCAGCCTGCCACTGCTGATTGCCCT |
| | | GATCTACACCCACAACACCCTGGGCTCCCTGAACATCCTGCTGCTGACACTG ACAGCCCAAGAGCTGAGCAACAGCTGGGCCAACAATCTGATGTGGCTGGCCT ACACAATGGCCTTCATGGTCAAGATGCCCCTGTACGGCCTGCACCTGTGGCT GCCTAAAGCTCATGTGGAAGCCCCTATCGCCGGCTCTATGGTGCTGGCTGCA GTGCTGCTGAAACTCGGCGGCTACGGCATGATGCGGCTGACCCTGATTCTGA ATCCCCTGACCAAGCACATGGCCTATCCATTTCTGGTGCTGAGCCTGTGGGG CATGATTATGACCAGCAGCATCTGCCTGCGGCAGACCGATCTGAAGTCCCTG ATCGCCTACAGCTCCATCAGCCACATGGCCCTGGTGGTCACCGCCATCCTGA TTCAGACCCCTTGGAGCTTTACAGGCGCCGTGATCCTGATGATTGCCCACGG CCTGACAAGCAGCCTGCTGTTTTGTCTGGCCAACAGCAACTACGAGCGGACC CACAGCAGAATCATGATCCTGTCTCAGGGCCTGCAGACCCTCCTGCCTCTTAT GGCTTTTTGGTGGCTGCTGGCCTCTCTGGCCAATCTGGCACTGCCTCCTACCA TCAATCTGCTGGGCGAGCTGAGCGTGCTGGTCACCACATTCAGCTGGTCCAA TATCACCCTGCTGCTCACCGGCCTGAACATGCTGGTTACAGCCCTGTACTCCC TGTACATGTTCACCACCACACAGTGGGGAAGCCTGACACACCACATCAACAA TATGAAGCCCAGCTTCACCCGCGAGAACACCCTGATGTTCATGCATCTGAGC CCCATTCTGCTGCTGTCCCTGAATCCTGATATCATCACCGGCTTCTCCAGCTG AGAGCACTGGGACGCCCACCGCCCCTTTCCCTCCGCTGCCAGGCGAGCATGT TGTGGTAATTCTGGAACACAAGAAGAGAAATTGCTGGGTTTAGAACAAGATT ATAAACGAATTCGGTGCTCAGTGATCACTTGACAGTTTTTTTTTTTTTTAAAT ATTACCCAAAATGCTCCCCAAATAAGAAATGCATCAGCTCAGTCAGTGAATA CAAAAAAGGAATTATTTTTCCCTTTGAGGGTCTTTTATACATCTCTCCTCCAA CCCCACCCTCTATTCTGTTTCTTCCTCCTCACATGGGGGTACACATACACAGC TTCCTCTTTTGGTTCCATCCTTACCACCACACCACACGCACACTCCACATGCC CAGCAGAGTGGCACTTGGTGGCCAGAAAGTGTGAGCCTCATGATCTGCTGTC TGTAGTTCTGTGAGCTCAGGTCCCTCAAAGGCCTCGGAGCACCCCCTTCCTTG TGACTGAGCCAGGGCCTGCATTTTTGGTTTTCCCCACCCCACACATTCTCAAC CATAGTCCTTCTAACAATACCAATAGCTAGGACCCGGCTGCTGTGCACTGGG ACTGGGGATTCCACATGTTTGCCTTGGGAGTCTCAAGCTGGACTGCCA |
| 47 | opt_COX10*-o pt_ND4*-3'UT R | ATGGCCGCCAGCCCCCACACCCTGAGCAGCCGCCTGCTGACCGGCTGCGTGG GCGGCAGCGTGTGGTACCTGGAGCGCCGCACCATGCTGAAGCTGATCGTGCC CACCATCATGCTGCTGCCCCTGACCTGGCTGAGCAAGAAGCACATGATCTGG ATCAACACCACCACCCACAGCCTGATCATCAGCATCATCCCCCTGCTGTTCTT CAACCAGATCAACAACAACCTGTTCAGCTGCAGCCCCACCTTCAGCAGCGAC CCCCTGACCACCCCCCTGCTGATGCTGACCACCTGGCTGCTGCCCCTGACCAT CATGGCCAGCCAGCGCCACCTGAGCAGCGAGCCCCTGAGCCGCAAGAAGCT GTACCTGAGCATGCTGATCAGCCTGCAGATCAGCCTGATCATGACCTTCACC GCCACCGAGCTGATCATGTTCTACATCTTCTTCGAGACCACCCTGATCCCCAC CCTGGCCATCATCACCCGCTGGGGCAACCAGCCCGAGCGCCTGAACGCCGGC ACCTACTTCCTGTTCTACACCCTGGTGGGCAGCCTGCCCCTGCTGATCGCCCT |
| | | GATCTACACCCACAACACCCTGGGCAGCCTGAACATCCTGCTGCTGACCCTG ACCGCCCAGGAGCTGAGCAACAGCTGGGCCAACAACCTGATGTGGCTGGCCT ACACCATGGCCTTCATGGTGAAGATGCCCCTGTACGGCCTGCACCTGTGGCT GCCCAAGGCCCACGTGGAGGCCCCCATCGCCGGCAGCATGGTGCTGGCCGCC GTGCTGCTGAAGCTGGGCGGCTACGGCATGATGCGCCTGACCCTGATCCTGA ACCCCCTGACCAAGCACATGGCCTACCCCTTCCTGGTGCTGAGCCTGTGGGG CATGATCATGACCAGCAGCATCTGCCTGCGCCAGACCGACCTGAAGAGCCTG ATCGCCTACAGCAGCATCAGCCACATGGCCCTGGTGGTGACCGCCATCCTGA TCCAGACCCCCTGGAGCTTCACCGGCGCCGTGATCCTGATGATCGCCCACGG CCTGACCAGCAGCCTGCTGTTCTGCCTGGCCAACAGCAACTACGAGCGCACC CACAGCCGCATCATGATCCTGAGCCAGGGCCTGCAGACCCTGCTGCCCCTGA TGGCCTTCTGGTGGCTGCTGGCCAGCCTGGCCAACCTGGCCCTGCCCCCCACC ATCAACCTGCTGGGCGAGCTGAGCGTGCTGGTGACCACCTTCAGCTGGAGCA ACATCACCCTGCTGCTGACCGGCCTGAACATGCTGGTGACCGCCCTGTACAG CCTGTACATGTTCACCACCACCCAGTGGGGCAGCCTGACCCACCACATCAAC AACATGAAGCCCAGCTTCACCCGCGAGAACACCCTGATGTTCATGCACCTGA GCCCCATCCTGCTGCTGAGCCTGAACCCCGACATCATCACCGGCTTCAGCAG CTAAGAGCACTGGGACGCCCACCGCCCCTTTCCCTCCGCTGCCAGGCGAGCA TGTTGTGGTAATTCTGGAACACAAGAAGAGAAATTGCTGGGTTTAGAACAAG ATTATAAACGAATTCGGTGCTCAGTGATCACTTGACAGTTTTTTTTTTTTTTAA ATATTACCCAAAATGCTCCCCAAATAAGAAATGCATCAGCTCAGTCAGTGAA TACAAAAAAGGAATTATTTTTCCCTTTGAGGGTCTTTTATACATCTCTCCTCC AACCCCACCCTCTATTCTGTTTCTTCCTCCTCACATGGGGGTACACATACACA GCTTCCTCTTTTGGTTCCATCCTTACCACCACACCACACGCACACTCCACATG CCCAGCAGAGTGGCACTTGGTGGCCAGAAAGTGTGAGCCTCATGATCTGCTG TCTGTAGTTCTGTGAGCTCAGGTCCCTCAAAGGCCTCGGAGCACCCCCTTCCT TGTGACTGAGCCAGGGCCTGCATTTTTGGTTTTCCCCACCCCACACATTCTCA ACCATAGTCCTTCTAACAATACCAATAGCTAGGACCCGGCTGCTGTGCACTG GGACTGGGGATTCCACATGTTTGCCTTGGGAGTCTCAAGCTGGACTGCCAGC CCCTGTCCTCCCTTCACCCCCATTGCGTATGAGCATTTCAGAACTCCAAGGAG TCACAGGCATCTTTATAGTTCACGTTAACATATAGACACTGTTGGAAGCAGTT CCTTCTAAAAGGGTAGCCCTGGACTTAATACCAGCCGGATACCTCTGGCCCC CACCCCATTACTGTACCTCTGGAGTCACTACTGTGGGTCGCCACTCCTCTGCT ACACAGCACGGCTTTTTCAAGGCTGTATTGAGAAGGGAAGTTAGGAAGAAG GGTGTGCTGGGCTAACCAGCCCACAGAGCTCACATTCCTGTCCCTTGGGTGA AAAATACATGTCCATCCTGATATCTCCTGAATTCAGAAATTAGCCTCCACATG TGCAATGGCTTTAAGAGCCAGAAGCAGGGTTCTGGGAATTTTGCAAGTTACC TGTGGCCAGGTGTGGTCTCGGTTACCAAATACGGTTACCTGCAGCTTTTTAGT CCTTTGTGCTCCCACGGGTCTACAGAGTCCCATCTGCCCAAAGGTCTTGAAGC TTGACAGGATGTTTTCGATTACTCAGTCTCCCAGGGCACTACTGGTCCGTAGG |
| | | ATTCGATTGGTCGGGGTAGGAGAGTTAAACAACATTTAAACAGAGTTCTCTC AAAAATGTCTAAAGGGATTGTAGGTAGATAACATCCAATCACTGTTTGCACT TATCTGAAATCTTCCCTCTTGGCTGCCCCCAGGTATTTACTGTGGAGAACATT GCATAGGAATGTCTGGAAAAAGCTTCTACAACTTGTTACAGCCTTCACATTT GTAGAAGCTTT |
| 48 | opt_COX10*-o pt_ND4*-3'UT R* | ATGGCCGCCAGCCCCCACACCCTGAGCAGCCGCCTGCTGACCGGCTGCGTGG GCGGCAGCGTGTGGTACCTGGAGCGCCGCACCATGCTGAAGCTGATCGTGCC CACCATCATGCTGCTGCCCCTGACCTGGCTGAGCAAGAAGCACATGATCTGG ATCAACACCACCACCCACAGCCTGATCATCAGCATCATCCCCCTGCTGTTCTT CAACCAGATCAACAACAACCTGTTCAGCTGCAGCCCCACCTTCAGCAGCGAC CCCCTGACCACCCCCCTGCTGATGCTGACCACCTGGCTGCTGCCCCTGACCAT CATGGCCAGCCAGCGCCACCTGAGCAGCGAGCCCCTGAGCCGCAAGAAGCT GTACCTGAGCATGCTGATCAGCCTGCAGATCAGCCTGATCATGACCTTCACC GCCACCGAGCTGATCATGTTCTACATCTTCTTCGAGACCACCCTGATCCCCAC CCTGGCCATCATCACCCGCTGGGGCAACCAGCCCGAGCGCCTGAACGCCGGC ACCTACTTCCTGTTCTACACCCTGGTGGGCAGCCTGCCCCTGCTGATCGCCCT GATCTACACCCACAACACCCTGGGCAGCCTGAACATCCTGCTGCTGACCCTG ACCGCCCAGGAGCTGAGCAACAGCTGGGCCAACAACCTGATGTGGCTGGCCT ACACCATGGCCTTCATGGTGAAGATGCCCCTGTACGGCCTGCACCTGTGGCT GCCCAAGGCCCACGTGGAGGCCCCCATCGCCGGCAGCATGGTGCTGGCCGCC GTGCTGCTGAAGCTGGGCGGCTACGGCATGATGCGCCTGACCCTGATCCTGA ACCCCCTGACCAAGCACATGGCCTACCCCTTCCTGGTGCTGAGCCTGTGGGG CATGATCATGACCAGCAGCATCTGCCTGCGCCAGACCGACCTGAAGAGCCTG ATCGCCTACAGCAGCATCAGCCACATGGCCCTGGTGGTGACCGCCATCCTGA TCCAGACCCCCTGGAGCTTCACCGGCGCCGTGATCCTGATGATCGCCCACGG CCTGACCAGCAGCCTGCTGTTCTGCCTGGCCAACAGCAACTACGAGCGCACC CACAGCCGCATCATGATCCTGAGCCAGGGCCTGCAGACCCTGCTGCCCCTGA TGGCCTTCTGGTGGCTGCTGGCCAGCCTGGCCAACCTGGCCCTGCCCCCCACC ATCAACCTGCTGGGCGAGCTGAGCGTGCTGGTGACCACCTTCAGCTGGAGCA ACATCACCCTGCTGCTGACCGGCCTGAACATGCTGGTGACCGCCCTGTACAG CCTGTACATGTTCACCACCACCCAGTGGGGCAGCCTGACCCACCACATCAAC AACATGAAGCCCAGCTTCACCCGCGAGAACACCCTGATGTTCATGCACCTGA GCCCCATCCTGCTGCTGAGCCTGAACCCCGACATCATCACCGGCTTCAGCAG CTAAGAGCACTGGGACGCCCACCGCCCCTTTCCCTCCGCTGCCAGGCGAGCA TGTTGTGGTAATTCTGGAACACAAGAAGAGAAATTGCTGGGTTTAGAACAAG ATTATAAACGAATTCGGTGCTCAGTGATCACTTGACAGTTTTTTTTTTTTTTAA ATATTACCCAAAATGCTCCCCAAATAAGAAATGCATCAGCTCAGTCAGTGAA TACAAAAAAGGAATTATTTTTCCCTTTGAGGGTCTTTTATACATCTCTCCTCC AACCCCACCCTCTATTCTGTTTCTTCCTCCTCACATGGGGGTACACATACACA GCTTCCTCTTTTGGTTCCATCCTTACCACCACACCACACGCACACTCCACATG |
| | | CCCAGCAGAGTGGCACTTGGTGGCCAGAAAGTGTGAGCCTCATGATCTGCTG TCTGTAGTTCTGTGAGCTCAGGTCCCTCAAAGGCCTCGGAGCACCCCCTTCCT TGTGACTGAGCCAGGGCCTGCATTTTTGGTTTTCCCCACCCCACACATTCTCA ACCATAGTCCTTCTAACAATACCAATAGCTAGGACCCGGCTGCTGTGCACTG GGACTGGGGATTCCACATGTTTGCCTTGGGAGTCTCAAGCTGGACTGCCA |
| 49 | opt_COX10*-ND6-3'UTR | ATGGCCGCCAGCCCCCACACCCTGAGCAGCCGCCTGCTGACCGGCTGCGTGG GCGGCAGCGTGTGGTACCTGGAGCGCCGCACCATGATGTATGCTTTGTTTCT GTTGAGTGTGGGTTTAGTAATGGGGTTTGTGGGGTTTTCTTCTAAGCCTTCTC CTATTTATGGGGGTTTAGTATTGATTGTTAGCGGTGTGGTCGGGTGTGTTATT ATTCTGAATTTTGGGGGAGGTTATATGGGTTTAATGGTTTTTTTAATTTATTTA GGGGGAATGATGGTTGTCTTTGGATATACTACAGCGATGGCTATTGAGGAGT ATCCTGAGGCATGGGGGTCAGGGGTTGAGGTCTTGGTGAGTGTTTTAGTGGG GTTAGCGATGGAGGTAGGATTGGTGCTGTGGGTGAAAGAGTATGATGGGGTG GTGGTTGTGGTAAACTTTAATAGTGTAGGAAGCTGGATGATTTATGAAGGAG AGGGGTCAGGGTTGATTCGGGAGGATCCTATTGGTGCGGGGGCTTTGTATGA TTATGGGCGTTGGTTAGTAGTAGTTACTGGTTGGACATTGTTTGTTGGTGTAT ATATTGTAATTGAGATTGCTCGGGGGAATTAGGAGCACTGGGACGCCCACCG CCCCTTTCCCTCCGCTGCCAGGCGAGCATGTTGTGGTAATTCTGGAACACAA GAAGAGAAATTGCTGGGTTTAGAACAAGATTATAAACGAATTCGGTGCTCAG TGATCACTTGACAGTTTTTTTTTTTTTTAAATATTACCCAAAATGCTCCCCAAA TAAGAAATGCATCAGCTCAGTCAGTGAATACAAAAAAGGAATTATTTTTCCC TTTGAGGGTCTTTTATACATCTCTCCTCCAACCCCACCCTCTATTCTGTTTCTT CCTCCTCACATGGGGGTACACATACACAGCTTCCTCTTTTGGTTCCATCCTTA CCACCACACCACACGCACACTCCACATGCCCAGCAGAGTGGCACTTGGTGGC CAGAAAGTGTGAGCCTCATGATCTGCTGTCTGTAGTTCTGTGAGCTCAGGTCC CTCAAAGGCCTCGGAGCACCCCCTTCCTTGTGACTGAGCCAGGGCCTGCATT TTTGGTTTTCCCCACCCCACACATTCTCAACCATAGTCCTTCTAACAATACCA ATAGCTAGGACCCGGCTGCTGTGCACTGGGACTGGGGATTCCACATGTTTGC CTTGGGAGTCTCAAGCTGGACTGCCAGCCCCTGTCCTCCCTTCACCCCCATTG CGTATGAGCATTTCAGAACTCCAAGGAGTCACAGGCATCTTTATAGTTCACG TTAACATATAGACACTGTTGGAAGCAGTTCCTTCTAAAAGGGTAGCCCTGGA CTTAATACCAGCCGGATACCTCTGGCCCCCACCCCATTACTGTACCTCTGGAG TCACTACTGTGGGTCGCCACTCCTCTGCTACACAGCACGGCTTTTTCAAGGCT GTATTGAGAAGGGAAGTTAGGAAGAAGGGTGTGCTGGGCTAACCAGCCCAC AGAGCTCACATTCCTGTCCCTTGGGTGAAAAATACATGTCCATCCTGATATCT CCTGAATTCAGAAATTAGCCTCCACATGTGCAATGGCTTTAAGAGCCAGAAG CAGGGTTCTGGGAATTTTGCAAGTTACCTGTGGCCAGGTGTGGTCTCGGTTAC CAAATACGGTTACCTGCAGCTTTTTAGTCCTTTGTGCTCCCACGGGTCTACAG AGTCCCATCTGCCCAAAGGTCTTGAAGCTTGACAGGATGTTTTCGATTACTCA GTCTCCCAGGGCACTACTGGTCCGTAGGATTCGATTGGTCGGGGTAGGAGAG |
| | | TTAAACAACATTTAAACAGAGTTCTCTCAAAAATGTCTAAAGGGATTGTAGG TAGATAACATCCAATCACTGTTTGCACTTATCTGAAATCTTCCCTCTTGGCTG CCCCCAGGTATTTACTGTGGAGAACATTGCATAGGAATGTCTGGAAAAAGCT TCTACAACTTGTTACAGCCTTCACATTTGTAGAAGCTTT |
| 50 | opt_COX10*-ND6-3'UTR* | ATGGCCGCCAGCCCCCACACCCTGAGCAGCCGCCTGCTGACCGGCTGCGTGG GCGGCAGCGTGTGGTACCTGGAGCGCCGCACCATGATGTATGCTTTGTTTCT GTTGAGTGTGGGTTTAGTAATGGGGTTTGTGGGGTTTTCTTCTAAGCCTTCTC CTATTTATGGGGGTTTAGTATTGATTGTTAGCGGTGTGGTCGGGTGTGTTATT ATTCTGAATTTTGGGGGAGGTTATATGGGTTTAATGGTTTTTTTAATTTATTTA GGGGGAATGATGGTTGTCTTTGGATATACTACAGCGATGGCTATTGAGGAGT ATCCTGAGGCATGGGGGTCAGGGGTTGAGGTCTTGGTGAGTGTTTTAGTGGG GTTAGCGATGGAGGTAGGATTGGTGCTGTGGGTGAAAGAGTATGATGGGGTG GTGGTTGTGGTAAACTTTAATAGTGTAGGAAGCTGGATGATTTATGAAGGAG AGGGGTCAGGGTTGATTCGGGAGGATCCTATTGGTGCGGGGGCTTTGTATGA TTATGGGCGTTGGTTAGTAGTAGTTACTGGTTGGACATTGTTTGTTGGTGTAT ATATTGTAATTGAGATTGCTCGGGGGAATTAGGAGCACTGGGACGCCCACCG CCCCTTTCCCTCCGCTGCCAGGCGAGCATGTTGTGGTAATTCTGGAACACAA GAAGAGAAATTGCTGGGTTTAGAACAAGATTATAAACGAATTCGGTGCTCAG TGATCACTTGACAGTTTTTTTTTTTTTTAAATATTACCCAAAATGCTCCCCAAA TAAGAAATGCATCAGCTCAGTCAGTGAATACAAAAAAGGAATTATTTTTCCC TTTGAGGGTCTTTTATACATCTCTCCTCCAACCCCACCCTCTATTCTGTTTCTT CCTCCTCACATGGGGGTACACATACACAGCTTCCTCTTTTGGTTCCATCCTTA CCACCACACCACACGCACACTCCACATGCCCAGCAGAGTGGCACTTGGTGGC CAGAAAGTGTGAGCCTCATGATCTGCTGTCTGTAGTTCTGTGAGCTCAGGTCC CTCAAAGGCCTCGGAGCACCCCCTTCCTTGTGACTGAGCCAGGGCCTGCATT TTTGGTTTTCCCCACCCCACACATTCTCAACCATAGTCCTTCTAACAATACCA ATAGCTAGGACCCGGCTGCTGTGCACTGGGACTGGGGATTCCACATGTTTGC CTTGGGAGTCTCAAGCTGGACTGCCA |
| 51 | opt_COX10*-o pt_ND6-3'UT R | ATGGCCGCCAGCCCCCACACCCTGAGCAGCCGCCTGCTGACCGGCTGCGTGG GCGGCAGCGTGTGGTACCTGGAGCGCCGCACCATGATGTACGCCCTGTTCCT GCTGAGCGTGGGCCTGGTGATGGGCTTCGTGGGCTTCAGCAGCAAGCCCAGC CCCATCTACGGCGGCCTGGTGCTGATCGTGAGCGGCGTGGTGGGCTGCGTGA TCATCCTGAACTTCGGCGGCGGCTACATGGGCCTGATGGTGTTCCTGATCTAC CTGGGCGGCATGATGGTGGTGTTCGGCTACACCACCGCCATGGCCATCGAGG AGTACCCCGAGGCCTGGGGCAGCGGCGTGGAGGTGCTGGTGAGCGTGCTGG TGGGCCTGGCCATGGAGGTGGGCCTGGTGCTGTGGGTGAAGGAGTACGACG GCGTGGTGGTGGTGGTGAACTTCAACAGCGTGGGCAGCTGGATGATCTACGA GGGCGAGGGCAGCGGCCTGATCCGCGAGGACCCCATCGGCGCCGGCGCCCT GTACGACTACGGCCGCTGGCTGGTGGTGGTGACCGGCTGGACCCTGTTCGTG |
| | | GGCGTGTACATCGTGATCGAGATCGCCCGCGGCAACTAAGAGCACTGGGAC GCCCACCGCCCCTTTCCCTCCGCTGCCAGGCGAGCATGTTGTGGTAATTCTGG AACACAAGAAGAGAAATTGCTGGGTTTAGAACAAGATTATAAACGAATTCG GTGCTCAGTGATCACTTGACAGTTTTTTTTTTTTTTAAATATTACCCAAAATGC TCCCCAAATAAGAAATGCATCAGCTCAGTCAGTGAATACAAAAAAGGAATT ATTTTTCCCTTTGAGGGTCTTTTATACATCTCTCCTCCAACCCCACCCTCTATT CTGTTTCTTCCTCCTCACATGGGGGTACACATACACAGCTTCCTCTTTTGGTTC CATCCTTACCACCACACCACACGCACACTCCACATGCCCAGCAGAGTGGCAC TTGGTGGCCAGAAAGTGTGAGCCTCATGATCTGCTGTCTGTAGTTCTGTGAGC TCAGGTCCCTCAAAGGCCTCGGAGCACCCCCTTCCTTGTGACTGAGCCAGGG CCTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCAGCCCCTGTCCTCCCTTCACC CCCATTGCGTATGAGCATTTCAGAACTCCAAGGAGTCACAGGCATCTTTATA GTTCACGTTAACATATAGACACTGTTGGAAGCAGTTCCTTCTAAAAGGGTAG CCCTGGACTTAATACCAGCCGGATACCTCTGGCCCCCACCCCATTACTGTACC TCTGGAGTCACTACTGTGGGTCGCCACTCCTCTGCTACACAGCACGGCTTTTT CAAGGCTGTATTGAGAAGGGAAGTTAGGAAGAAGGGTGTGCTGGGCTAACC AGCCCACAGAGCTCACATTCCTGTCCCTTGGGTGAAAAATACATGTCCATCC TGATATCTCCTGAATTCAGAAATTAGCCTCCACATGTGCAATGGCTTTAAGA GCCAGAAGCAGGGTTCTGGGAATTTTGCAAGTTACCTGTGGCCAGGTGTGGT CTCGGTTACCAAATACGGTTACCTGCAGCTTTTTAGTCCTTTGTGCTCCCACG GGTCTACAGAGTCCCATCTGCCCAAAGGTCTTGAAGCTTGACAGGATGTTTT CGATTACTCAGTCTCCCAGGGCACTACTGGTCCGTAGGATTCGATTGGTCGG GGTAGGAGAGTTAAACAACATTTAAACAGAGTTCTCTCAAAAATGTCTAAAG GGATTGTAGGTAGATAACATCCAATCACTGTTTGCACTTATCTGAAATCTTCC CTCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 52 | opt_COX10*-o pt_ND6-3'UT R* | ATGGCCGCCAGCCCCCACACCCTGAGCAGCCGCCTGCTGACCGGCTGCGTGG GCGGCAGCGTGTGGTACCTGGAGCGCCGCACCATGATGTACGCCCTGTTCCT GCTGAGCGTGGGCCTGGTGATGGGCTTCGTGGGCTTCAGCAGCAAGCCCAGC CCCATCTACGGCGGCCTGGTGCTGATCGTGAGCGGCGTGGTGGGCTGCGTGA TCATCCTGAACTTCGGCGGCGGCTACATGGGCCTGATGGTGTTCCTGATCTAC CTGGGCGGCATGATGGTGGTGTTCGGCTACACCACCGCCATGGCCATCGAGG AGTACCCCGAGGCCTGGGGCAGCGGCGTGGAGGTGCTGGTGAGCGTGCTGG TGGGCCTGGCCATGGAGGTGGGCCTGGTGCTGTGGGTGAAGGAGTACGACG GCGTGGTGGTGGTGGTGAACTTCAACAGCGTGGGCAGCTGGATGATCTACGA GGGCGAGGGCAGCGGCCTGATCCGCGAGGACCCCATCGGCGCCGGCGCCCT GTACGACTACGGCCGCTGGCTGGTGGTGGTGACCGGCTGGACCCTGTTCGTG GGCGTGTACATCGTGATCGAGATCGCCCGCGGCAACTAAGAGCACTGGGAC |
| | | GCCCACCGCCCCTTTCCCTCCGCTGCCAGGCGAGCATGTTGTGGTAATTCTGG AACACAAGAAGAGAAATTGCTGGGTTTAGAACAAGATTATAAACGAATTCG GTGCTCAGTGATCACTTGACAGTTTTTTTTTTTTTTAAATATTACCCAAAATGC TCCCCAAATAAGAAATGCATCAGCTCAGTCAGTGAATACAAAAAAGGAATT ATTTTTCCCTTTGAGGGTCTTTTATACATCTCTCCTCCAACCCCACCCTCTATT CTGTTTCTTCCTCCTCACATGGGGGTACACATACACAGCTTCCTCTTTTGGTTC CATCCTTACCACCACACCACACGCACACTCCACATGCCCAGCAGAGTGGCAC TTGGTGGCCAGAAAGTGTGAGCCTCATGATCTGCTGTCTGTAGTTCTGTGAGC TCAGGTCCCTCAAAGGCCTCGGAGCACCCCCTTCCTTGTGACTGAGCCAGGG CCTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCA |
| 53 | opt_COX10*-ND 1-3' UTR | ATGGCCGCCAGCCCCCACACCCTGAGCAGCCGCCTGCTGACCGGCTGCGTGG GCGGCAGCGTGTGGTACCTGGAGCGCCGCACCATGGCCAACCTCCTACTCCT CATTGTACCCATTCTAATCGCAATGGCATTCCTAATGCTTACCGAACGAAAA ATTCTAGGCTATATGCAACTACGCAAAGGCCCCAACGTTGTAGGCCCCTACG GGCTACTACAACCCTTCGCTGACGCCATAAAACTCTTCACCAAAGAGCCCCT AAAACCCGCCACATCTACCATCACCCTCTACATCACCGCCCCGACCTTAGCTC TCACCATCGCTCTTCTACTATGGACCCCCCTCCCCATGCCCAACCCCCTGGTC AACCTCAACCTAGGCCTCCTATTTATTCTAGCCACCTCTAGCCTAGCCGTTTA CTCAATCCTCTGGTCAGGGTGGGCATCAAACTCAAACTACGCCCTGATCGGC GCACTGCGAGCAGTAGCCCAAACAATCTCATATGAAGTCACCCTAGCCATCA TTCTACTATCAACATTACTAATGAGTGGCTCCTTTAACCTCTCCACCCTTATC ACAACACAAGAACACCTCTGGTTACTCCTGCCATCATGGCCCTTGGCCATGA TGTGGTTTATCTCCACACTAGCAGAGACCAACCGAACCCCCTTCGACCTTGCC GAAGGGGAGTCCGAACTAGTCTCAGGCTTCAACATCGAATACGCCGCAGGCC CCTTCGCCCTATTCTTCATGGCCGAATACACAAACATTATTATGATGAACACC CTCACCACTACAATCTTCCTAGGAACAACATATGACGCACTCTCCCCTGAACT CTACACAACATATTTTGTCACCAAGACCCTACTTCTAACCTCCCTGTTCTTAT GGATTCGAACAGCATACCCCCGATTCCGCTACGACCAACTCATGCACCTCCT ATGGAAAAACTTCCTACCACTCACCCTAGCATTACTTATGTGGTATGTCTCCA TGCCCATTACAATCTCCAGCATTCCCCCTCAAACCTAAGAGCACTGGGACGC CCACCGCCCCTTTCCCTCCGCTGCCAGGCGAGCATGTTGTGGTAATTCTGGAA CACAAGAAGAGAAATTGCTGGGTTTAGAACAAGATTATAAACGAATTCGGT GCTCAGTGATCACTTGACAGTTTTTTTTTTTTTTAAATATTACCCAAAATGCTC CCCAAATAAGAAATGCATCAGCTCAGTCAGTGAATACAAAAAAGGAATTATT TTTCCCTTTGAGGGTCTTTTATACATCTCTCCTCCAACCCCACCCTCTATTCTG TTTCTTCCTCCTCACATGGGGGTACACATACACAGCTTCCTCTTTTGGTTCCAT CCTTACCACCACACCACACGCACACTCCACATGCCCAGCAGAGTGGCACTTG GTGGCCAGAAAGTGTGAGCCTCATGATCTGCTGTCTGTAGTTCTGTGAGCTC |
| | | AGGTCCCTCAAAGGCCTCGGAGCACCCCCTTCCTTGTGACTGAGCCAGGGCC TGCATTTTTGGTTTTCCCCACCCCACACATTCTCAACCATAGTCCTTCTAACA ATACCAATAGCTAGGACCCGGCTGCTGTGCACTGGGACTGGGGATTCCACAT GTTTGCCTTGGGAGTCTCAAGCTGGACTGCCAGCCCCTGTCCTCCCTTCACCC CCATTGCGTATGAGCATTTCAGAACTCCAAGGAGTCACAGGCATCTTTATAG TTCACGTTAACATATAGACACTGTTGGAAGCAGTTCCTTCTAAAAGGGTAGC CCTGGACTTAATACCAGCCGGATACCTCTGGCCCCCACCCCATTACTGTACCT CTGGAGTCACTACTGTGGGTCGCCACTCCTCTGCTACACAGCACGGCTTTTTC AAGGCTGTATTGAGAAGGGAAGTTAGGAAGAAGGGTGTGCTGGGCTAACCA GCCCACAGAGCTCACATTCCTGTCCCTTGGGTGAAAAATACATGTCCATCCT GATATCTCCTGAATTCAGAAATTAGCCTCCACATGTGCAATGGCTTTAAGAG CCAGAAGCAGGGTTCTGGGAATTTTGCAAGTTACCTGTGGCCAGGTGTGGTC TCGGTTACCAAATACGGTTACCTGCAGCTTTTTAGTCCTTTGTGCTCCCACGG GTCTACAGAGTCCCATCTGCCCAAAGGTCTTGAAGCTTGACAGGATGTTTTC GATTACTCAGTCTCCCAGGGCACTACTGGTCCGTAGGATTCGATTGGTCGGG GTAGGAGAGTTAAACAACATTTAAACAGAGTTCTCTCAAAAATGTCTAAAGG GATTGTAGGTAGATAACATCCAATCACTGTTTGCACTTATCTGAAATCTTCCC TCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 54 | opt_COX10*-ND 1-3' UTR* | ATGGCCGCCAGCCCCCACACCCTGAGCAGCCGCCTGCTGACCGGCTGCGTGG GCGGCAGCGTGTGGTACCTGGAGCGCCGCACCATGGCCAACCTCCTACTCCT CATTGTACCCATTCTAATCGCAATGGCATTCCTAATGCTTACCGAACGAAAA ATTCTAGGCTATATGCAACTACGCAAAGGCCCCAACGTTGTAGGCCCCTACG GGCTACTACAACCCTTCGCTGACGCCATAAAACTCTTCACCAAAGAGCCCCT AAAACCCGCCACATCTACCATCACCCTCTACATCACCGCCCCGACCTTAGCTC TCACCATCGCTCTTCTACTATGGACCCCCCTCCCCATGCCCAACCCCCTGGTC AACCTCAACCTAGGCCTCCTATTTATTCTAGCCACCTCTAGCCTAGCCGTTTA CTCAATCCTCTGGTCAGGGTGGGCATCAAACTCAAACTACGCCCTGATCGGC GCACTGCGAGCAGTAGCCCAAACAATCTCATATGAAGTCACCCTAGCCATCA TTCTACTATCAACATTACTAATGAGTGGCTCCTTTAACCTCTCCACCCTTATC ACAACACAAGAACACCTCTGGTTACTCCTGCCATCATGGCCCTTGGCCATGA TGTGGTTTATCTCCACACTAGCAGAGACCAACCGAACCCCCTTCGACCTTGCC GAAGGGGAGTCCGAACTAGTCTCAGGCTTCAACATCGAATACGCCGCAGGCC CCTTCGCCCTATTCTTCATGGCCGAATACACAAACATTATTATGATGAACACC CTCACCACTACAATCTTCCTAGGAACAACATATGACGCACTCTCCCCTGAACT CTACACAACATATTTTGTCACCAAGACCCTACTTCTAACCTCCCTGTTCTTAT GGATTCGAACAGCATACCCCCGATTCCGCTACGACCAACTCATGCACCTCCT ATGGAAAAACTTCCTACCACTCACCCTAGCATTACTTATGTGGTATGTCTCCA TGCCCATTACAATCTCCAGCATTCCCCCTCAAACCTAAGAGCACTGGGACGC CCACCGCCCCTTTCCCTCCGCTGCCAGGCGAGCATGTTGTGGTAATTCTGGAA |
| | | CACAAGAAGAGAAATTGCTGGGTTTAGAACAAGATTATAAACGAATTCGGT GCTCAGTGATCACTTGACAGTTTTTTTTTTTTTTAAATATTACCCAAAATGCTC CCCAAATAAGAAATGCATCAGCTCAGTCAGTGAATACAAAAAAGGAATTATT TTTCCCTTTGAGGGTCTTTTATACATCTCTCCTCCAACCCCACCCTCTATTCTG TTTCTTCCTCCTCACATGGGGGTACACATACACAGCTTCCTCTTTTGGTTCCAT CCTTACCACCACACCACACGCACACTCCACATGCCCAGCAGAGTGGCACTTG GTGGCCAGAAAGTGTGAGCCTCATGATCTGCTGTCTGTAGTTCTGTGAGCTC AGGTCCCTCAAAGGCCTCGGAGCACCCCCTTCCTTGTGACTGAGCCAGGGCC TGCATTTTTGGTTTTCCCCACCCCACACATTCTCAACCATAGTCCTTCTAACA ATACCAATAGCTAGGACCCGGCTGCTGTGCACTGGGACTGGGGATTCCACAT GTTTGCCTTGGGAGTCTCAAGCTGGACTGCCA |
| 55 | opt_COX10*-o pt_ND1-3'UT R | ATGGCCGCCAGCCCCCACACCCTGAGCAGCCGCCTGCTGACCGGCTGCGTGG GCGGCAGCGTGTGGTACCTGGAGCGCCGCACCATGGCCAACCTGCTGCTGCT GATCGTGCCCATCCTGATCGCCATGGCCTTCCTGATGCTGACCGAGCGCAAG ATCCTGGGCTACATGCAGCTGCGCAAGGGCCCCAACGTGGTGGGCCCCTACG GCCTGCTGCAGCCCTTCGCCGACGCCATCAAGCTGTTCACCAAGGAGCCCCT GAAGCCCGCCACCAGCACCATCACCCTGTACATCACCGCCCCCACCCTGGCC CTGACCATCGCCCTGCTGCTGTGGACCCCCCTGCCCATGCCCAACCCCCTGGT GAACCTGAACCTGGGCCTGCTGTTCATCCTGGCCACCAGCAGCCTGGCCGTG TACAGCATCCTGTGGAGCGGCTGGGCCAGCAACAGCAACTACGCCCTGATCG GCGCCCTGCGCGCCGTGGCCCAGACCATCAGCTACGAGGTGACCCTGGCCAT CATCCTGCTGAGCACCCTGCTGATGAGCGGCAGCTTCAACCTGAGCACCCTG ATCACCACCCAGGAGCACCTGTGGCTGCTGCTGCCCAGCTGGCCCCTGGCCA TGATGTGGTTCATCAGCACCCTGGCCGAGACCAACCGCACCCCCTTCGACCT GGCCGAGGGCGAGAGCGAGCTGGTGAGCGGCTTCAACATCGAGTACGCCGC CGGCCCCTTCGCCCTGTTCTTCATGGCCGAGTACACCAACATCATCATGATGA ACACCCTGACCACCACCATCTTCCTGGGCACCACCTACGACGCCCTGAGCCC CGAGCTGTACACCACCTACTTCGTGACCAAGACCCTGCTGCTGACCAGCCTG TTCCTGTGGATCCGCACCGCCTACCCCCGCTTCCGCTACGACCAGCTGATGCA CCTGCTGTGGAAGAACTTCCTGCCCCTGACCCTGGCCCTGCTGATGTGGTACG TGAGCATGCCCATCACCATCAGCAGCATCCCCCCCCAGACCTAAGAGCACTG GGACGCCCACCGCCCCTTTCCCTCCGCTGCCAGGCGAGCATGTTGTGGTAATT CTGGAACACAAGAAGAGAAATTGCTGGGTTTAGAACAAGATTATAAACGAA TTCGGTGCTCAGTGATCACTTGACAGTTTTTTTTTTTTTTAAATATTACCCAAA ATGCTCCCCAAATAAGAAATGCATCAGCTCAGTCAGTGAATACAAAAAAGG AATTATTTTTCCCTTTGAGGGTCTTTTATACATCTCTCCTCCAACCCCACCCTC TATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC |
| | | AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCAGCCCCTGTCCTCCCT TCACCCCCATTGCGTATGAGCATTTCAGAACTCCAAGGAGTCACAGGCATCT TTATAGTTCACGTTAACATATAGACACTGTTGGAAGCAGTTCCTTCTAAAAG GGTAGCCCTGGACTTAATACCAGCCGGATACCTCTGGCCCCCACCCCATTAC TGTACCTCTGGAGTCACTACTGTGGGTCGCCACTCCTCTGCTACACAGCACGG CTTTTTCAAGGCTGTATTGAGAAGGGAAGTTAGGAAGAAGGGTGTGCTGGGC TAACCAGCCCACAGAGCTCACATTCCTGTCCCTTGGGTGAAAAATACATGTC CATCCTGATATCTCCTGAATTCAGAAATTAGCCTCCACATGTGCAATGGCTTT AAGAGCCAGAAGCAGGGTTCTGGGAATTTTGCAAGTTACCTGTGGCCAGGTG TGGTCTCGGTTACCAAATACGGTTACCTGCAGCTTTTTAGTCCTTTGTGCTCC CACGGGTCTACAGAGTCCCATCTGCCCAAAGGTCTTGAAGCTTGACAGGATG TTTTCGATTACTCAGTCTCCCAGGGCACTACTGGTCCGTAGGATTCGATTGGT CGGGGTAGGAGAGTTAAACAACATTTAAACAGAGTTCTCTCAAAAATGTCTA AAGGGATTGTAGGTAGATAACATCCAATCACTGTTTGCACTTATCTGAAATC TTCCCTCTTGGCTGCCCCCAGGTATTTACTGTGGAGAACATTGCATAGGAATG TCTGGAAAAAGCTTCTACAACTTGTTACAGCCTTCACATTTGTAGAAGCTTT |
| 56 | opt_COX10*-o pt_ND1-3'UT R* | ATGGCCGCCAGCCCCCACACCCTGAGCAGCCGCCTGCTGACCGGCTGCGTGG GCGGCAGCGTGTGGTACCTGGAGCGCCGCACCATGGCCAACCTGCTGCTGCT GATCGTGCCCATCCTGATCGCCATGGCCTTCCTGATGCTGACCGAGCGCAAG ATCCTGGGCTACATGCAGCTGCGCAAGGGCCCCAACGTGGTGGGCCCCTACG GCCTGCTGCAGCCCTTCGCCGACGCCATCAAGCTGTTCACCAAGGAGCCCCT GAAGCCCGCCACCAGCACCATCACCCTGTACATCACCGCCCCCACCCTGGCC CTGACCATCGCCCTGCTGCTGTGGACCCCCCTGCCCATGCCCAACCCCCTGGT GAACCTGAACCTGGGCCTGCTGTTCATCCTGGCCACCAGCAGCCTGGCCGTG TACAGCATCCTGTGGAGCGGCTGGGCCAGCAACAGCAACTACGCCCTGATCG GCGCCCTGCGCGCCGTGGCCCAGACCATCAGCTACGAGGTGACCCTGGCCAT CATCCTGCTGAGCACCCTGCTGATGAGCGGCAGCTTCAACCTGAGCACCCTG ATCACCACCCAGGAGCACCTGTGGCTGCTGCTGCCCAGCTGGCCCCTGGCCA TGATGTGGTTCATCAGCACCCTGGCCGAGACCAACCGCACCCCCTTCGACCT GGCCGAGGGCGAGAGCGAGCTGGTGAGCGGCTTCAACATCGAGTACGCCGC CGGCCCCTTCGCCCTGTTCTTCATGGCCGAGTACACCAACATCATCATGATGA ACACCCTGACCACCACCATCTTCCTGGGCACCACCTACGACGCCCTGAGCCC CGAGCTGTACACCACCTACTTCGTGACCAAGACCCTGCTGCTGACCAGCCTG TTCCTGTGGATCCGCACCGCCTACCCCCGCTTCCGCTACGACCAGCTGATGCA CCTGCTGTGGAAGAACTTCCTGCCCCTGACCCTGGCCCTGCTGATGTGGTACG TGAGCATGCCCATCACCATCAGCAGCATCCCCCCCCAGACCTAAGAGCACTG GGACGCCCACCGCCCCTTTCCCTCCGCTGCCAGGCGAGCATGTTGTGGTAATT CTGGAACACAAGAAGAGAAATTGCTGGGTTTAGAACAAGATTATAAACGAA |
| | | TTCGGTGCTCAGTGATCACTTGACAGTTTTTTTTTTTTTTAAATATTACCCAAA ATGCTCCCCAAATAAGAAATGCATCAGCTCAGTCAGTGAATACAAAAAAGG AATTATTTTTCCCTTTGAGGGTCTTTTATACATCTCTCCTCCAACCCCACCCTC TATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCA |
| 57 | COX8-ND4-3' UTR | ATGTCCGTCCTGACGCGCCTGCTGCTGCGGGGCTTGACACGGCTCGGCTCGG CGGCTCCAGTGCGGCGCGCCAGAATCCATTCGTTGATGCTAAAACTAATCGT CCCAACAATTATGTTACTACCACTGACATGGCTTTCCAAAAAACACATGATTT GGATCAACACAACCACCCACAGCCTAATTATTAGCATCATCCCTCTACTATTT TTTAACCAAATCAACAACAACCTATTTAGCTGTTCCCCAACCTTTTCCTCCGA CCCCCTAACAACCCCCCTCCTAATGCTAACTACCTGGCTCCTACCCCTCACAA TCATGGCAAGCCAACGCCACTTATCCAGTGAACCACTATCACGAAAAAAACT CTACCTCTCTATGCTAATCTCCCTACAAATCTCCTTAATTATGACATTCACAG CCACAGAACTAATCATGTTTTATATCTTCTTCGAAACCACACTTATCCCCACC TTGGCTATCATCACCCGATGGGGCAACCAGCCAGAACGCCTGAACGCAGGCA CATACTTCCTATTCTACACCCTAGTAGGCTCCCTTCCCCTACTCATCGCACTA ATTTACACTCACAACACCCTAGGCTCACTAAACATTCTACTACTCACTCTCAC TGCCCAAGAACTATCAAACTCCTGGGCCAACAACTTAATGTGGCTAGCTTAC ACAATGGCTTTTATGGTAAAGATGCCTCTTTACGGACTCCACTTATGGCTCCC TAAAGCCCATGTCGAAGCCCCCATCGCTGGGTCAATGGTACTTGCCGCAGTA CTCTTAAAACTAGGCGGCTATGGTATGATGCGCCTCACACTCATTCTCAACCC CCTGACAAAACACATGGCCTACCCCTTCCTTGTACTATCCCTATGGGGCATGA TTATGACAAGCTCCATCTGCCTACGACAAACAGACCTAAAATCGCTCATTGC ATACTCTTCAATCAGCCACATGGCCCTCGTAGTAACAGCCATTCTCATCCAAA CCCCCTGGAGCTTCACCGGCGCAGTCATTCTCATGATCGCCCACGGGCTTAC ATCCTCATTACTATTCTGCCTAGCAAACTCAAACTACGAACGCACTCACAGTC GCATCATGATCCTCTCTCAAGGACTTCAAACTCTACTCCCACTAATGGCTTTT TGGTGGCTTCTAGCAAGCCTCGCTAACCTCGCCTTACCCCCCACTATTAACCT ACTGGGAGAACTCTCTGTGCTAGTAACCACGTTCTCCTGGTCAAATATCACTC TCCTACTTACAGGACTCAACATGCTAGTCACAGCCCTATACTCCCTCTACATG TTTACCACAACACAATGGGGCTCACTCACCCACCACATTAACAACATGAAAC CCTCATTCACACGAGAAAACACCCTCATGTTCATGCACCTATCCCCCATTCTC CTCCTATCCCTCAACCCCGACATCATTACCGGGTTTTCCTCTTAAGAGCACTG GGACGCCCACCGCCCCTTTCCCTCCGCTGCCAGGCGAGCATGTTGTGGTAATT CTGGAACACAAGAAGAGAAATTGCTGGGTTTAGAACAAGATTATAAACGAA |
| | | TTCGGTGCTCAGTGATCACTTGACAGTTTTTTTTTTTTTTAAATATTACCCAAA ATGCTCCCCAAATAAGAAATGCATCAGCTCAGTCAGTGAATACAAAAAAGG AATTATTTTTCCCTTTGAGGGTCTTTTATACATCTCTCCTCCAACCCCACCCTC TATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCAGCCCCTGTCCTCCCT TCACCCCCATTGCGTATGAGCATTTCAGAACTCCAAGGAGTCACAGGCATCT TTATAGTTCACGTTAACATATAGACACTGTTGGAAGCAGTTCCTTCTAAAAG GGTAGCCCTGGACTTAATACCAGCCGGATACCTCTGGCCCCCACCCCATTAC TGTACCTCTGGAGTCACTACTGTGGGTCGCCACTCCTCTGCTACACAGCACGG CTTTTTCAAGGCTGTATTGAGAAGGGAAGTTAGGAAGAAGGGTGTGCTGGGC TAACCAGCCCACAGAGCTCACATTCCTGTCCCTTGGGTGAAAAATACATGTC CATCCTGATATCTCCTGAATTCAGAAATTAGCCTCCACATGTGCAATGGCTTT AAGAGCCAGAAGCAGGGTTCTGGGAATTTTGCAAGTTACCTGTGGCCAGGTG TGGTCTCGGTTACCAAATACGGTTACCTGCAGCTTTTTAGTCCTTTGTGCTCC CACGGGTCTACAGAGTCCCATCTGCCCAAAGGTCTTGAAGCTTGACAGGATG TTTTCGATTACTCAGTCTCCCAGGGCACTACTGGTCCGTAGGATTCGATTGGT CGGGGTAGGAGAGTTAAACAACATTTAAACAGAGTTCTCTCAAAAATGTCTA AAGGGATTGTAGGTAGATAACATCCAATCACTGTTTGCACTTATCTGAAATC TTCCCTCTTGGCTGCCCCCAGGTATTTACTGTGGAGAACATTGCATAGGAATG TCTGGAAAAAGCTTCTACAACTTGTTACAGCCTTCACATTTGTAGAAGCTTT |
| 58 | COX8-ND4-3' UTR* | ATGTCCGTCCTGACGCGCCTGCTGCTGCGGGGCTTGACACGGCTCGGCTCGG CGGCTCCAGTGCGGCGCGCCAGAATCCATTCGTTGATGCTAAAACTAATCGT CCCAACAATTATGTTACTACCACTGACATGGCTTTCCAAAAAACACATGATTT GGATCAACACAACCACCCACAGCCTAATTATTAGCATCATCCCTCTACTATTT TTTAACCAAATCAACAACAACCTATTTAGCTGTTCCCCAACCTTTTCCTCCGA CCCCCTAACAACCCCCCTCCTAATGCTAACTACCTGGCTCCTACCCCTCACAA TCATGGCAAGCCAACGCCACTTATCCAGTGAACCACTATCACGAAAAAAACT CTACCTCTCTATGCTAATCTCCCTACAAATCTCCTTAATTATGACATTCACAG CCACAGAACTAATCATGTTTTATATCTTCTTCGAAACCACACTTATCCCCACC TTGGCTATCATCACCCGATGGGGCAACCAGCCAGAACGCCTGAACGCAGGCA CATACTTCCTATTCTACACCCTAGTAGGCTCCCTTCCCCTACTCATCGCACTA ATTTACACTCACAACACCCTAGGCTCACTAAACATTCTACTACTCACTCTCAC TGCCCAAGAACTATCAAACTCCTGGGCCAACAACTTAATGTGGCTAGCTTAC ACAATGGCTTTTATGGTAAAGATGCCTCTTTACGGACTCCACTTATGGCTCCC TAAAGCCCATGTCGAAGCCCCCATCGCTGGGTCAATGGTACTTGCCGCAGTA |
| | | CTCTTAAAACTAGGCGGCTATGGTATGATGCGCCTCACACTCATTCTCAACCC CCTGACAAAACACATGGCCTACCCCTTCCTTGTACTATCCCTATGGGGCATGA TTATGACAAGCTCCATCTGCCTACGACAAACAGACCTAAAATCGCTCATTGC ATACTCTTCAATCAGCCACATGGCCCTCGTAGTAACAGCCATTCTCATCCAAA CCCCCTGGAGCTTCACCGGCGCAGTCATTCTCATGATCGCCCACGGGCTTAC ATCCTCATTACTATTCTGCCTAGCAAACTCAAACTACGAACGCACTCACAGTC GCATCATGATCCTCTCTCAAGGACTTCAAACTCTACTCCCACTAATGGCTTTT TGGTGGCTTCTAGCAAGCCTCGCTAACCTCGCCTTACCCCCCACTATTAACCT ACTGGGAGAACTCTCTGTGCTAGTAACCACGTTCTCCTGGTCAAATATCACTC TCCTACTTACAGGACTCAACATGCTAGTCACAGCCCTATACTCCCTCTACATG TTTACCACAACACAATGGGGCTCACTCACCCACCACATTAACAACATGAAAC CCTCATTCACACGAGAAAACACCCTCATGTTCATGCACCTATCCCCCATTCTC CTCCTATCCCTCAACCCCGACATCATTACCGGGTTTTCCTCTTAAGAGCACTG GGACGCCCACCGCCCCTTTCCCTCCGCTGCCAGGCGAGCATGTTGTGGTAATT CTGGAACACAAGAAGAGAAATTGCTGGGTTTAGAACAAGATTATAAACGAA TTCGGTGCTCAGTGATCACTTGACAGTTTTTTTTTTTTTTAAATATTACCCAAA ATGCTCCCCAAATAAGAAATGCATCAGCTCAGTCAGTGAATACAAAAAAGG AATTATTTTTCCCTTTGAGGGTCTTTTATACATCTCTCCTCCAACCCCACCCTC TATTCTGTTTCTTCCTCCTCACATGGGGGTACACATACACAGCTTCCTCTTTTG GTTCCATCCTTACCACCACACCACACGCACACTCCACATGCCCAGCAGAGTG GCACTTGGTGGCCAGAAAGTGTGAGCCTCATGATCTGCTGTCTGTAGTTCTGT GAGCTCAGGTCCCTCAAAGGCCTCGGAGCACCCCCTTCCTTGTGACTGAGCC AGGGCCTGCATTTTTGGTTTTCCCCACCCCACACATTCTCAACCATAGTCCTT CTAACAATACCAATAGCTAGGACCCGGCTGCTGTGCACTGGGACTGGGGATT CCACATGTTTGCCTTGGGAGTCTCAAGCTGGACTGCCA |
| 59 | COX8-opt_ND 4-3'UTR | ATGTCCGTCCTGACGCGCCTGCTGCTGCGGGGCTTGACACGGCTCGGCTCGG CGGCTCCAGTGCGGCGCGCCAGAATCCATTCGTTGATGCTGAAGCTGATCGT GCCCACCATCATGCTGCTGCCTCTGACCTGGCTGAGCAAGAAACACATGATC TGGATCAACACCACCACGCACAGCCTGATCATCAGCATCATCCCTCTGCTGTT CTTCAACCAGATCAACAACAACCTGTTCAGCTGCAGCCCCACCTTCAGCAGC GACCCTCTGACAACACCTCTGCTGATGCTGACCACCTGGCTGCTGCCCCTCAC AATCATGGCCTCTCAGAGACACCTGAGCAGCGAGCCCCTGAGCCGGAAGAA ACTGTACCTGAGCATGCTGATCTCCCTGCAGATCTCTCTGATCATGACCTTCA CCGCCACCGAGCTGATCATGTTCTACATCTTTTTCGAGACAACGCTGATCCCC ACACTGGCCATCATCACCAGATGGGGCAACCAGCCTGAGAGACTGAACGCC GGCACCTACTTTCTGTTCTACACCCTCGTGGGCAGCCTGCCACTGCTGATTGC CCTGATCTACACCCACAACACCCTGGGCTCCCTGAACATCCTGCTGCTGACA CTGACAGCCCAAGAGCTGAGCAACAGCTGGGCCAACAATCTGATGTGGCTG GCCTACACAATGGCCTTCATGGTCAAGATGCCCCTGTACGGCCTGCACCTGT GGCTGCCTAAAGCTCATGTGGAAGCCCCTATCGCCGGCTCTATGGTGCTGGC |
| | | TGCAGTGCTGCTGAAACTCGGCGGCTACGGCATGATGCGGCTGACCCTGATT CTGAATCCCCTGACCAAGCACATGGCCTATCCATTTCTGGTGCTGAGCCTGTG GGGCATGATTATGACCAGCAGCATCTGCCTGCGGCAGACCGATCTGAAGTCC CTGATCGCCTACAGCTCCATCAGCCACATGGCCCTGGTGGTCACCGCCATCCT GATTCAGACCCCTTGGAGCTTTACAGGCGCCGTGATCCTGATGATTGCCCAC GGCCTGACAAGCAGCCTGCTGTTTTGTCTGGCCAACAGCAACTACGAGCGGA CCCACAGCAGAATCATGATCCTGTCTCAGGGCCTGCAGACCCTCCTGCCTCTT ATGGCTTTTTGGTGGCTGCTGGCCTCTCTGGCCAATCTGGCACTGCCTCCTAC CATCAATCTGCTGGGCGAGCTGAGCGTGCTGGTCACCACATTCAGCTGGTCC AATATCACCCTGCTGCTCACCGGCCTGAACATGCTGGTTACAGCCCTGTACTC CCTGTACATGTTCACCACCACACAGTGGGGAAGCCTGACACACCACATCAAC AATATGAAGCCCAGCTTCACCCGCGAGAACACCCTGATGTTCATGCATCTGA GCCCCATTCTGCTGCTGTCCCTGAATCCTGATATCATCACCGGCTTCTCCAGC TGAGAGCACTGGGACGCCCACCGCCCCTTTCCCTCCGCTGCCAGGCGAGCAT GTTGTGGTAATTCTGGAACACAAGAAGAGAAATTGCTGGGTTTAGAACAAGA TTATAAACGAATTCGGTGCTCAGTGATCACTTGACAGTTTTTTTTTTTTTTAAA TATTACCCAAAATGCTCCCCAAATAAGAAATGCATCAGCTCAGTCAGTGAAT ACAAAAAAGGAATTATTTTTCCCTTTGAGGGTCTTTTATACATCTCTCCTCCA ACCCCACCCTCTATTCTGTTTCTTCCTCCTCACATGGGGGTACACATACACAG CTTCCTCTTTTGGTTCCATCCTTACCACCACACCACACGCACACTCCACATGC CCAGCAGAGTGGCACTTGGTGGCCAGAAAGTGTGAGCCTCATGATCTGCTGT CTGTAGTTCTGTGAGCTCAGGTCCCTCAAAGGCCTCGGAGCACCCCCTTCCTT GTGACTGAGCCAGGGCCTGCATTTTTGGTTTTCCCCACCCCACACATTCTCAA CCATAGTCCTTCTAACAATACCAATAGCTAGGACCCGGCTGCTGTGCACTGG GACTGGGGATTCCACATGTTTGCCTTGGGAGTCTCAAGCTGGACTGCCAGCC CCTGTCCTCCCTTCACCCCCATTGCGTATGAGCATTTCAGAACTCCAAGGAGT CACAGGCATCTTTATAGTTCACGTTAACATATAGACACTGTTGGAAGCAGTT CCTTCTAAAAGGGTAGCCCTGGACTTAATACCAGCCGGATACCTCTGGCCCC CACCCCATTACTGTACCTCTGGAGTCACTACTGTGGGTCGCCACTCCTCTGCT ACACAGCACGGCTTTTTCAAGGCTGTATTGAGAAGGGAAGTTAGGAAGAAG GGTGTGCTGGGCTAACCAGCCCACAGAGCTCACATTCCTGTCCCTTGGGTGA AAAATACATGTCCATCCTGATATCTCCTGAATTCAGAAATTAGCCTCCACATG TGCAATGGCTTTAAGAGCCAGAAGCAGGGTTCTGGGAATTTTGCAAGTTACC TGTGGCCAGGTGTGGTCTCGGTTACCAAATACGGTTACCTGCAGCTTTTTAGT CCTTTGTGCTCCCACGGGTCTACAGAGTCCCATCTGCCCAAAGGTCTTGAAGC TTGACAGGATGTTTTCGATTACTCAGTCTCCCAGGGCACTACTGGTCCGTAGG ATTCGATTGGTCGGGGTAGGAGAGTTAAACAACATTTAAACAGAGTTCTCTC AAAAATGTCTAAAGGGATTGTAGGTAGATAACATCCAATCACTGTTTGCACT TATCTGAAATCTTCCCTCTTGGCTGCCCCCAGGTATTTACTGTGGAGAACATT GCATAGGAATGTCTGGAAAAAGCTTCTACAACTTGTTACAGCCTTCACATTT |
| | | GTAGAAGCTTT |
| 60 | COX8-opt_ND 4-3'UTR* | ATGTCCGTCCTGACGCGCCTGCTGCTGCGGGGCTTGACACGGCTCGGCTCGG CGGCTCCAGTGCGGCGCGCCAGAATCCATTCGTTGATGCTGAAGCTGATCGT GCCCACCATCATGCTGCTGCCTCTGACCTGGCTGAGCAAGAAACACATGATC TGGATCAACACCACCACGCACAGCCTGATCATCAGCATCATCCCTCTGCTGTT CTTCAACCAGATCAACAACAACCTGTTCAGCTGCAGCCCCACCTTCAGCAGC GACCCTCTGACAACACCTCTGCTGATGCTGACCACCTGGCTGCTGCCCCTCAC AATCATGGCCTCTCAGAGACACCTGAGCAGCGAGCCCCTGAGCCGGAAGAA ACTGTACCTGAGCATGCTGATCTCCCTGCAGATCTCTCTGATCATGACCTTCA CCGCCACCGAGCTGATCATGTTCTACATCTTTTTCGAGACAACGCTGATCCCC ACACTGGCCATCATCACCAGATGGGGCAACCAGCCTGAGAGACTGAACGCC GGCACCTACTTTCTGTTCTACACCCTCGTGGGCAGCCTGCCACTGCTGATTGC CCTGATCTACACCCACAACACCCTGGGCTCCCTGAACATCCTGCTGCTGACA CTGACAGCCCAAGAGCTGAGCAACAGCTGGGCCAACAATCTGATGTGGCTG GCCTACACAATGGCCTTCATGGTCAAGATGCCCCTGTACGGCCTGCACCTGT GGCTGCCTAAAGCTCATGTGGAAGCCCCTATCGCCGGCTCTATGGTGCTGGC TGCAGTGCTGCTGAAACTCGGCGGCTACGGCATGATGCGGCTGACCCTGATT CTGAATCCCCTGACCAAGCACATGGCCTATCCATTTCTGGTGCTGAGCCTGTG GGGCATGATTATGACCAGCAGCATCTGCCTGCGGCAGACCGATCTGAAGTCC CTGATCGCCTACAGCTCCATCAGCCACATGGCCCTGGTGGTCACCGCCATCCT GATTCAGACCCCTTGGAGCTTTACAGGCGCCGTGATCCTGATGATTGCCCAC GGCCTGACAAGCAGCCTGCTGTTTTGTCTGGCCAACAGCAACTACGAGCGGA CCCACAGCAGAATCATGATCCTGTCTCAGGGCCTGCAGACCCTCCTGCCTCTT ATGGCTTTTTGGTGGCTGCTGGCCTCTCTGGCCAATCTGGCACTGCCTCCTAC CATCAATCTGCTGGGCGAGCTGAGCGTGCTGGTCACCACATTCAGCTGGTCC AATATCACCCTGCTGCTCACCGGCCTGAACATGCTGGTTACAGCCCTGTACTC CCTGTACATGTTCACCACCACACAGTGGGGAAGCCTGACACACCACATCAAC AATATGAAGCCCAGCTTCACCCGCGAGAACACCCTGATGTTCATGCATCTGA GCCCCATTCTGCTGCTGTCCCTGAATCCTGATATCATCACCGGCTTCTCCAGC TGAGAGCACTGGGACGCCCACCGCCCCTTTCCCTCCGCTGCCAGGCGAGCAT GTTGTGGTAATTCTGGAACACAAGAAGAGAAATTGCTGGGTTTAGAACAAGA TTATAAACGAATTCGGTGCTCAGTGATCACTTGACAGTTTTTTTTTTTTTTAAA TATTACCCAAAATGCTCCCCAAATAAGAAATGCATCAGCTCAGTCAGTGAAT ACAAAAAAGGAATTATTTTTCCCTTTGAGGGTCTTTTATACATCTCTCCTCCA ACCCCACCCTCTATTCTGTTTCTTCCTCCTCACATGGGGGTACACATACACAG CTTCCTCTTTTGGTTCCATCCTTACCACCACACCACACGCACACTCCACATGC CCAGCAGAGTGGCACTTGGTGGCCAGAAAGTGTGAGCCTCATGATCTGCTGT CTGTAGTTCTGTGAGCTCAGGTCCCTCAAAGGCCTCGGAGCACCCCCTTCCTT GTGACTGAGCCAGGGCCTGCATTTTTGGTTTTCCCCACCCCACACATTCTCAA CCATAGTCCTTCTAACAATACCAATAGCTAGGACCCGGCTGCTGTGCACTGG |
| | | GACTGGGGATTCCACATGTTTGCCTTGGGAGTCTCAAGCTGGACTGCCA |
| 61 | COX8-opt_ND 4*-3'UTR | ATGTCCGTCCTGACGCGCCTGCTGCTGCGGGGCTTGACACGGCTCGGCTCGG CGGCTCCAGTGCGGCGCGCCAGAATCCATTCGTTGATGCTGAAGCTGATCGT GCCCACCATCATGCTGCTGCCCCTGACCTGGCTGAGCAAGAAGCACATGATC TGGATCAACACCACCACCCACAGCCTGATCATCAGCATCATCCCCCTGCTGTT CTTCAACCAGATCAACAACAACCTGTTCAGCTGCAGCCCCACCTTCAGCAGC GACCCCCTGACCACCCCCCTGCTGATGCTGACCACCTGGCTGCTGCCCCTGAC CATCATGGCCAGCCAGCGCCACCTGAGCAGCGAGCCCCTGAGCCGCAAGAA GCTGTACCTGAGCATGCTGATCAGCCTGCAGATCAGCCTGATCATGACCTTC ACCGCCACCGAGCTGATCATGTTCTACATCTTCTTCGAGACCACCCTGATCCC CACCCTGGCCATCATCACCCGCTGGGGCAACCAGCCCGAGCGCCTGAACGCC GGCACCTACTTCCTGTTCTACACCCTGGTGGGCAGCCTGCCCCTGCTGATCGC CCTGATCTACACCCACAACACCCTGGGCAGCCTGAACATCCTGCTGCTGACC CTGACCGCCCAGGAGCTGAGCAACAGCTGGGCCAACAACCTGATGTGGCTG GCCTACACCATGGCCTTCATGGTGAAGATGCCCCTGTACGGCCTGCACCTGT GGCTGCCCAAGGCCCACGTGGAGGCCCCCATCGCCGGCAGCATGGTGCTGGC CGCCGTGCTGCTGAAGCTGGGCGGCTACGGCATGATGCGCCTGACCCTGATC CTGAACCCCCTGACCAAGCACATGGCCTACCCCTTCCTGGTGCTGAGCCTGT GGGGCATGATCATGACCAGCAGCATCTGCCTGCGCCAGACCGACCTGAAGA GCCTGATCGCCTACAGCAGCATCAGCCACATGGCCCTGGTGGTGACCGCCAT CCTGATCCAGACCCCCTGGAGCTTCACCGGCGCCGTGATCCTGATGATCGCC CACGGCCTGACCAGCAGCCTGCTGTTCTGCCTGGCCAACAGCAACTACGAGC GCACCCACAGCCGCATCATGATCCTGAGCCAGGGCCTGCAGACCCTGCTGCC CCTGATGGCCTTCTGGTGGCTGCTGGCCAGCCTGGCCAACCTGGCCCTGCCCC CCACCATCAACCTGCTGGGCGAGCTGAGCGTGCTGGTGACCACCTTCAGCTG GAGCAACATCACCCTGCTGCTGACCGGCCTGAACATGCTGGTGACCGCCCTG TACAGCCTGTACATGTTCACCACCACCCAGTGGGGCAGCCTGACCCACCACA TCAACAACATGAAGCCCAGCTTCACCCGCGAGAACACCCTGATGTTCATGCA CCTGAGCCCCATCCTGCTGCTGAGCCTGAACCCCGACATCATCACCGGCTTC AGCAGCTAAGAGCACTGGGACGCCCACCGCCCCTTTCCCTCCGCTGCCAGGC GAGCATGTTGTGGTAATTCTGGAACACAAGAAGAGAAATTGCTGGGTTTAGA ACAAGATTATAAACGAATTCGGTGCTCAGTGATCACTTGACAGTTTTTTTTT TTTTTAAATATTACCCAAAATGCTCCCCAAATAAGAAATGCATCAGCTCAGTC AGTGAATACAAAAAAGGAATTATTTTTCCCTTTGAGGGTCTTTTATACATCTC TCCTCCAACCCCACCCTCTATTCTGTTTCTTCCTCCTCACATGGGGGTACACA TACACAGCTTCCTCTTTTGGTTCCATCCTTACCACCACACCACACGCACACTC CACATGCCCAGCAGAGTGGCACTTGGTGGCCAGAAAGTGTGAGCCTCATGAT CTGCTGTCTGTAGTTCTGTGAGCTCAGGTCCCTCAAAGGCCTCGGAGCACCCC CTTCCTTGTGACTGAGCCAGGGCCTGCATTTTTGGTTTTCCCCACCCCACACA TTCTCAACCATAGTCCTTCTAACAATACCAATAGCTAGGACCCGGCTGCTGTG |
| | | CACTGGGACTGGGGATTCCACATGTTTGCCTTGGGAGTCTCAAGCTGGACTG CCAGCCCCTGTCCTCCCTTCACCCCCATTGCGTATGAGCATTTCAGAACTCCA AGGAGTCACAGGCATCTTTATAGTTCACGTTAACATATAGACACTGTTGGAA GCAGTTCCTTCTAAAAGGGTAGCCCTGGACTTAATACCAGCCGGATACCTCT GGCCCCCACCCCATTACTGTACCTCTGGAGTCACTACTGTGGGTCGCCACTCC TCTGCTACACAGCACGGCTTTTTCAAGGCTGTATTGAGAAGGGAAGTTAGGA AGAAGGGTGTGCTGGGCTAACCAGCCCACAGAGCTCACATTCCTGTCCCTTG GGTGAAAAATACATGTCCATCCTGATATCTCCTGAATTCAGAAATTAGCCTC CACATGTGCAATGGCTTTAAGAGCCAGAAGCAGGGTTCTGGGAATTTTGCAA GTTACCTGTGGCCAGGTGTGGTCTCGGTTACCAAATACGGTTACCTGCAGCTT TTTAGTCCTTTGTGCTCCCACGGGTCTACAGAGTCCCATCTGCCCAAAGGTCT TGAAGCTTGACAGGATGTTTTCGATTACTCAGTCTCCCAGGGCACTACTGGTC CGTAGGATTCGATTGGTCGGGGTAGGAGAGTTAAACAACATTTAAACAGAGT TCTCTCAAAAATGTCTAAAGGGATTGTAGGTAGATAACATCCAATCACTGTT TGCACTTATCTGAAATCTTCCCTCTTGGCTGCCCCCAGGTATTTACTGTGGAG AACATTGCATAGGAATGTCTGGAAAAAGCTTCTACAACTTGTTACAGCCTTC ACATTTGTAGAAGCTTT |
| 62 | COX8-opt_ND 4*-3'UTR* | ATGTCCGTCCTGACGCGCCTGCTGCTGCGGGGCTTGACACGGCTCGGCTCGG CGGCTCCAGTGCGGCGCGCCAGAATCCATTCGTTGATGCTGAAGCTGATCGT GCCCACCATCATGCTGCTGCCCCTGACCTGGCTGAGCAAGAAGCACATGATC TGGATCAACACCACCACCCACAGCCTGATCATCAGCATCATCCCCCTGCTGTT CTTCAACCAGATCAACAACAACCTGTTCAGCTGCAGCCCCACCTTCAGCAGC GACCCCCTGACCACCCCCCTGCTGATGCTGACCACCTGGCTGCTGCCCCTGAC CATCATGGCCAGCCAGCGCCACCTGAGCAGCGAGCCCCTGAGCCGCAAGAA GCTGTACCTGAGCATGCTGATCAGCCTGCAGATCAGCCTGATCATGACCTTC ACCGCCACCGAGCTGATCATGTTCTACATCTTCTTCGAGACCACCCTGATCCC CACCCTGGCCATCATCACCCGCTGGGGCAACCAGCCCGAGCGCCTGAACGCC GGCACCTACTTCCTGTTCTACACCCTGGTGGGCAGCCTGCCCCTGCTGATCGC CCTGATCTACACCCACAACACCCTGGGCAGCCTGAACATCCTGCTGCTGACC CTGACCGCCCAGGAGCTGAGCAACAGCTGGGCCAACAACCTGATGTGGCTG GCCTACACCATGGCCTTCATGGTGAAGATGCCCCTGTACGGCCTGCACCTGT GGCTGCCCAAGGCCCACGTGGAGGCCCCCATCGCCGGCAGCATGGTGCTGGC CGCCGTGCTGCTGAAGCTGGGCGGCTACGGCATGATGCGCCTGACCCTGATC CTGAACCCCCTGACCAAGCACATGGCCTACCCCTTCCTGGTGCTGAGCCTGT GGGGCATGATCATGACCAGCAGCATCTGCCTGCGCCAGACCGACCTGAAGA GCCTGATCGCCTACAGCAGCATCAGCCACATGGCCCTGGTGGTGACCGCCAT CCTGATCCAGACCCCCTGGAGCTTCACCGGCGCCGTGATCCTGATGATCGCC CACGGCCTGACCAGCAGCCTGCTGTTCTGCCTGGCCAACAGCAACTACGAGC GCACCCACAGCCGCATCATGATCCTGAGCCAGGGCCTGCAGACCCTGCTGCC CCTGATGGCCTTCTGGTGGCTGCTGGCCAGCCTGGCCAACCTGGCCCTGCCCC |
| | | CCACCATCAACCTGCTGGGCGAGCTGAGCGTGCTGGTGACCACCTTCAGCTG GAGCAACATCACCCTGCTGCTGACCGGCCTGAACATGCTGGTGACCGCCCTG TACAGCCTGTACATGTTCACCACCACCCAGTGGGGCAGCCTGACCCACCACA TCAACAACATGAAGCCCAGCTTCACCCGCGAGAACACCCTGATGTTCATGCA CCTGAGCCCCATCCTGCTGCTGAGCCTGAACCCCGACATCATCACCGGCTTC AGCAGCTAAGAGCACTGGGACGCCCACCGCCCCTTTCCCTCCGCTGCCAGGC GAGCATGTTGTGGTAATTCTGGAACACAAGAAGAGAAATTGCTGGGTTTAGA ACAAGATTATAAACGAATTCGGTGCTCAGTGATCACTTGACAGTTTTTTTTTT TTTTAAATATTACCCAAAATGCTCCCCAAATAAGAAATGCATCAGCTCAGTC AGTGAATACAAAAAAGGAATTATTTTTCCCTTTGAGGGTCTTTTATACATCTC TCCTCCAACCCCACCCTCTATTCTGTTTCTTCCTCCTCACATGGGGGTACACA TACACAGCTTCCTCTTTTGGTTCCATCCTTACCACCACACCACACGCACACTC CACATGCCCAGCAGAGTGGCACTTGGTGGCCAGAAAGTGTGAGCCTCATGAT CTGCTGTCTGTAGTTCTGTGAGCTCAGGTCCCTCAAAGGCCTCGGAGCACCCC CTTCCTTGTGACTGAGCCAGGGCCTGCATTTTTGGTTTTCCCCACCCCACACA TTCTCAACCATAGTCCTTCTAACAATACCAATAGCTAGGACCCGGCTGCTGTG CACTGGGACTGGGGATTCCACATGTTTGCCTTGGGAGTCTCAAGCTGGACTG CCA |
| 63 | COX8-ND6-3' UTR | ATGTCCGTCCTGACGCGCCTGCTGCTGCGGGGCTTGACACGGCTCGGCTCGG CGGCTCCAGTGCGGCGCGCCAGAATCCATTCGTTGATGATGTATGCTTTGTTT CTGTTGAGTGTGGGTTTAGTAATGGGGTTTGTGGGGTTTTCTTCTAAGCCTTC TCCTATTTATGGGGGTTTAGTATTGATTGTTAGCGGTGTGGTCGGGTGTGTTA TTATTCTGAATTTTGGGGGAGGTTATATGGGTTTAATGGTTTTTTTAATTTATT TAGGGGGAATGATGGTTGTCTTTGGATATACTACAGCGATGGCTATTGAGGA GTATCCTGAGGCATGGGGGTCAGGGGTTGAGGTCTTGGTGAGTGTTTTAGTG GGGTTAGCGATGGAGGTAGGATTGGTGCTGTGGGTGAAAGAGTATGATGGG GTGGTGGTTGTGGTAAACTTTAATAGTGTAGGAAGCTGGATGATTTATGAAG GAGAGGGGTCAGGGTTGATTCGGGAGGATCCTATTGGTGCGGGGGCTTTGTA TGATTATGGGCGTTGGTTAGTAGTAGTTACTGGTTGGACATTGTTTGTTGGTG TATATATTGTAATTGAGATTGCTCGGGGGAATTAGGAGCACTGGGACGCCCA CCGCCCCTTTCCCTCCGCTGCCAGGCGAGCATGTTGTGGTAATTCTGGAACAC AAGAAGAGAAATTGCTGGGTTTAGAACAAGATTATAAACGAATTCGGTGCTC AGTGATCACTTGACAGTTTTTTTTTTTTTTAAATATTACCCAAAATGCTCCCCA AATAAGAAATGCATCAGCTCAGTCAGTGAATACAAAAAAGGAATTATTTTTC CCTTTGAGGGTCTTTTATACATCTCTCCTCCAACCCCACCCTCTATTCTGTTTC TTCCTCCTCACATGGGGGTACACATACACAGCTTCCTCTTTTGGTTCCATCCT TACCACCACACCACACGCACACTCCACATGCCCAGCAGAGTGGCACTTGGTG GCCAGAAAGTGTGAGCCTCATGATCTGCTGTCTGTAGTTCTGTGAGCTCAGG TCCCTCAAAGGCCTCGGAGCACCCCCTTCCTTGTGACTGAGCCAGGGCCTGC ATTTTTGGTTTTCCCCACCCCACACATTCTCAACCATAGTCCTTCTAACAATA |
| | | CCAATAGCTAGGACCCGGCTGCTGTGCACTGGGACTGGGGATTCCACATGTT TGCCTTGGGAGTCTCAAGCTGGACTGCCAGCCCCTGTCCTCCCTTCACCCCCA TTGCGTATGAGCATTTCAGAACTCCAAGGAGTCACAGGCATCTTTATAGTTC ACGTTAACATATAGACACTGTTGGAAGCAGTTCCTTCTAAAAGGGTAGCCCT GGACTTAATACCAGCCGGATACCTCTGGCCCCCACCCCATTACTGTACCTCTG GAGTCACTACTGTGGGTCGCCACTCCTCTGCTACACAGCACGGCTTTTTCAAG GCTGTATTGAGAAGGGAAGTTAGGAAGAAGGGTGTGCTGGGCTAACCAGCC CACAGAGCTCACATTCCTGTCCCTTGGGTGAAAAATACATGTCCATCCTGAT ATCTCCTGAATTCAGAAATTAGCCTCCACATGTGCAATGGCTTTAAGAGCCA GAAGCAGGGTTCTGGGAATTTTGCAAGTTACCTGTGGCCAGGTGTGGTCTCG GTTACCAAATACGGTTACCTGCAGCTTTTTAGTCCTTTGTGCTCCCACGGGTC TACAGAGTCCCATCTGCCCAAAGGTCTTGAAGCTTGACAGGATGTTTTCGATT ACTCAGTCTCCCAGGGCACTACTGGTCCGTAGGATTCGATTGGTCGGGGTAG GAGAGTTAAACAACATTTAAACAGAGTTCTCTCAAAAATGTCTAAAGGGATT GTAGGTAGATAACATCCAATCACTGTTTGCACTTATCTGAAATCTTCCCTCTT GGCTGCCCCCAGGTATTTACTGTGGAGAACATTGCATAGGAATGTCTGGAAA AAGCTTCTACAACTTGTTACAGCCTTCACATTTGTAGAAGCTTT |
| 64 | COX8-ND6-3' UTR* | ATGTCCGTCCTGACGCGCCTGCTGCTGCGGGGCTTGACACGGCTCGGCTCGG CGGCTCCAGTGCGGCGCGCCAGAATCCATTCGTTGATGATGTATGCTTTGTTT CTGTTGAGTGTGGGTTTAGTAATGGGGTTTGTGGGGTTTTCTTCTAAGCCTTC TCCTATTTATGGGGGTTTAGTATTGATTGTTAGCGGTGTGGTCGGGTGTGTTA TTATTCTGAATTTTGGGGGAGGTTATATGGGTTTAATGGTTTTTTTAATTTATT TAGGGGGAATGATGGTTGTCTTTGGATATACTACAGCGATGGCTATTGAGGA GTATCCTGAGGCATGGGGGTCAGGGGTTGAGGTCTTGGTGAGTGTTTTAGTG GGGTTAGCGATGGAGGTAGGATTGGTGCTGTGGGTGAAAGAGTATGATGGG GTGGTGGTTGTGGTAAACTTTAATAGTGTAGGAAGCTGGATGATTTATGAAG GAGAGGGGTCAGGGTTGATTCGGGAGGATCCTATTGGTGCGGGGGCTTTGTA TGATTATGGGCGTTGGTTAGTAGTAGTTACTGGTTGGACATTGTTTGTTGGTG TATATATTGTAATTGAGATTGCTCGGGGGAATTAGGAGCACTGGGACGCCCA CCGCCCCTTTCCCTCCGCTGCCAGGCGAGCATGTTGTGGTAATTCTGGAACAC AAGAAGAGAAATTGCTGGGTTTAGAACAAGATTATAAACGAATTCGGTGCTC AGTGATCACTTGACAGTTTTTTTTTTTTTTAAATATTACCCAAAATGCTCCCCA AATAAGAAATGCATCAGCTCAGTCAGTGAATACAAAAAAGGAATTATTTTTC CCTTTGAGGGTCTTTTATACATCTCTCCTCCAACCCCACCCTCTATTCTGTTTC TTCCTCCTCACATGGGGGTACACATACACAGCTTCCTCTTTTGGTTCCATCCT TACCACCACACCACACGCACACTCCACATGCCCAGCAGAGTGGCACTTGGTG GCCAGAAAGTGTGAGCCTCATGATCTGCTGTCTGTAGTTCTGTGAGCTCAGG TCCCTCAAAGGCCTCGGAGCACCCCCTTCCTTGTGACTGAGCCAGGGCCTGC ATTTTTGGTTTTCCCCACCCCACACATTCTCAACCATAGTCCTTCTAACAATA CCAATAGCTAGGACCCGGCTGCTGTGCACTGGGACTGGGGATTCCACATGTT |
| | | TGCCTTGGGAGTCTCAAGCTGGACTGCCA |
| 65 | COX8-opt_ND 6-3'UTR | ATGTCCGTCCTGACGCGCCTGCTGCTGCGGGGCTTGACACGGCTCGGCTCGG CGGCTCCAGTGCGGCGCGCCAGAATCCATTCGTTGATGATGTACGCCCTGTT CCTGCTGAGCGTGGGCCTGGTGATGGGCTTCGTGGGCTTCAGCAGCAAGCCC AGCCCCATCTACGGCGGCCTGGTGCTGATCGTGAGCGGCGTGGTGGGCTGCG TGATCATCCTGAACTTCGGCGGCGGCTACATGGGCCTGATGGTGTTCCTGATC TACCTGGGCGGCATGATGGTGGTGTTCGGCTACACCACCGCCATGGCCATCG AGGAGTACCCCGAGGCCTGGGGCAGCGGCGTGGAGGTGCTGGTGAGCGTGC TGGTGGGCCTGGCCATGGAGGTGGGCCTGGTGCTGTGGGTGAAGGAGTACG ACGGCGTGGTGGTGGTGGTGAACTTCAACAGCGTGGGCAGCTGGATGATCTA CGAGGGCGAGGGCAGCGGCCTGATCCGCGAGGACCCCATCGGCGCCGGCGC CCTGTACGACTACGGCCGCTGGCTGGTGGTGGTGACCGGCTGGACCCTGTTC GTGGGCGTGTACATCGTGATCGAGATCGCCCGCGGCAACTAAGAGCACTGGG ACGCCCACCGCCCCTTTCCCTCCGCTGCCAGGCGAGCATGTTGTGGTAATTCT GGAACACAAGAAGAGAAATTGCTGGGTTTAGAACAAGATTATAAACGAATT CGGTGCTCAGTGATCACTTGACAGTTTTTTTTTTTTTTAAATATTACCCAAAAT GCTCCCCAAATAAGAAATGCATCAGCTCAGTCAGTGAATACAAAAAAGGAA TTATTTTTCCCTTTGAGGGTCTTTTATACATCTCTCCTCCAACCCCACCCTCTA TTCTGTTTCTTCCTCCTCACATGGGGGTACACATACACAGCTTCCTCTTTTGGT TCCATCCTTACCACCACACCACACGCACACTCCACATGCCCAGCAGAGTGGC ACTTGGTGGCCAGAAAGTGTGAGCCTCATGATCTGCTGTCTGTAGTTCTGTGA GCTCAGGTCCCTCAAAGGCCTCGGAGCACCCCCTTCCTTGTGACTGAGCCAG GGCCTGCATTTTTGGTTTTCCCCACCCCACACATTCTCAACCATAGTCCTTCT AACAATACCAATAGCTAGGACCCGGCTGCTGTGCACTGGGACTGGGGATTCC ACATGTTTGCCTTGGGAGTCTCAAGCTGGACTGCCAGCCCCTGTCCTCCCTTC ACCCCCATTGCGTATGAGCATTTCAGAACTCCAAGGAGTCACAGGCATCTTT ATAGTTCACGTTAACATATAGACACTGTTGGAAGCAGTTCCTTCTAAAAGGG TAGCCCTGGACTTAATACCAGCCGGATACCTCTGGCCCCCACCCCATTACTGT ACCTCTGGAGTCACTACTGTGGGTCGCCACTCCTCTGCTACACAGCACGGCTT TTTCAAGGCTGTATTGAGAAGGGAAGTTAGGAAGAAGGGTGTGCTGGGCTAA CCAGCCCACAGAGCTCACATTCCTGTCCCTTGGGTGAAAAATACATGTCCAT CCTGATATCTCCTGAATTCAGAAATTAGCCTCCACATGTGCAATGGCTTTAAG AGCCAGAAGCAGGGTTCTGGGAATTTTGCAAGTTACCTGTGGCCAGGTGTGG TCTCGGTTACCAAATACGGTTACCTGCAGCTTTTTAGTCCTTTGTGCTCCCAC GGGTCTACAGAGTCCCATCTGCCCAAAGGTCTTGAAGCTTGACAGGATGTTT TCGATTACTCAGTCTCCCAGGGCACTACTGGTCCGTAGGATTCGATTGGTCGG GGTAGGAGAGTTAAACAACATTTAAACAGAGTTCTCTCAAAAATGTCTAAAG GGATTGTAGGTAGATAACATCCAATCACTGTTTGCACTTATCTGAAATCTTCC CTCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 66 | COX8-opt_ND 6-3'UTR* | ATGTCCGTCCTGACGCGCCTGCTGCTGCGGGGCTTGACACGGCTCGGCTCGG CGGCTCCAGTGCGGCGCGCCAGAATCCATTCGTTGATGATGTACGCCCTGTT CCTGCTGAGCGTGGGCCTGGTGATGGGCTTCGTGGGCTTCAGCAGCAAGCCC AGCCCCATCTACGGCGGCCTGGTGCTGATCGTGAGCGGCGTGGTGGGCTGCG TGATCATCCTGAACTTCGGCGGCGGCTACATGGGCCTGATGGTGTTCCTGATC TACCTGGGCGGCATGATGGTGGTGTTCGGCTACACCACCGCCATGGCCATCG AGGAGTACCCCGAGGCCTGGGGCAGCGGCGTGGAGGTGCTGGTGAGCGTGC TGGTGGGCCTGGCCATGGAGGTGGGCCTGGTGCTGTGGGTGAAGGAGTACG ACGGCGTGGTGGTGGTGGTGAACTTCAACAGCGTGGGCAGCTGGATGATCTA CGAGGGCGAGGGCAGCGGCCTGATCCGCGAGGACCCCATCGGCGCCGGCGC CCTGTACGACTACGGCCGCTGGCTGGTGGTGGTGACCGGCTGGACCCTGTTC GTGGGCGTGTACATCGTGATCGAGATCGCCCGCGGCAACTAAGAGCACTGGG ACGCCCACCGCCCCTTTCCCTCCGCTGCCAGGCGAGCATGTTGTGGTAATTCT GGAACACAAGAAGAGAAATTGCTGGGTTTAGAACAAGATTATAAACGAATT CGGTGCTCAGTGATCACTTGACAGTTTTTTTTTTTTTTAAATATTACCCAAAAT GCTCCCCAAATAAGAAATGCATCAGCTCAGTCAGTGAATACAAAAAAGGAA TTATTTTTCCCTTTGAGGGTCTTTTATACATCTCTCCTCCAACCCCACCCTCTA TTCTGTTTCTTCCTCCTCACATGGGGGTACACATACACAGCTTCCTCTTTTGGT TCCATCCTTACCACCACACCACACGCACACTCCACATGCCCAGCAGAGTGGC ACTTGGTGGCCAGAAAGTGTGAGCCTCATGATCTGCTGTCTGTAGTTCTGTGA GCTCAGGTCCCTCAAAGGCCTCGGAGCACCCCCTTCCTTGTGACTGAGCCAG GGCCTGCATTTTTGGTTTTCCCCACCCCACACATTCTCAACCATAGTCCTTCT AACAATACCAATAGCTAGGACCCGGCTGCTGTGCACTGGGACTGGGGATTCC ACATGTTTGCCTTGGGAGTCTCAAGCTGGACTGCCA |
| 67 | COX8-ND1-3' UTR | ATGTCCGTCCTGACGCGCCTGCTGCTGCGGGGCTTGACACGGCTCGGCTCGG CGGCTCCAGTGCGGCGCGCCAGAATCCATTCGTTGATGGCCAACCTCCTACT CCTCATTGTACCCATTCTAATCGCAATGGCATTCCTAATGCTTACCGAACGAA AAATTCTAGGCTATATGCAACTACGCAAAGGCCCCAACGTTGTAGGCCCCTA CGGGCTACTACAACCCTTCGCTGACGCCATAAAACTCTTCACCAAAGAGCCC CTAAAACCCGCCACATCTACCATCACCCTCTACATCACCGCCCCGACCTTAGC TCTCACCATCGCTCTTCTACTATGGACCCCCCTCCCCATGCCCAACCCCCTGG TCAACCTCAACCTAGGCCTCCTATTTATTCTAGCCACCTCTAGCCTAGCCGTT TACTCAATCCTCTGGTCAGGGTGGGCATCAAACTCAAACTACGCCCTGATCG GCGCACTGCGAGCAGTAGCCCAAACAATCTCATATGAAGTCACCCTAGCCAT CATTCTACTATCAACATTACTAATGAGTGGCTCCTTTAACCTCTCCACCCTTA TCACAACACAAGAACACCTCTGGTTACTCCTGCCATCATGGCCCTTGGCCAT GATGTGGTTTATCTCCACACTAGCAGAGACCAACCGAACCCCCTTCGACCTT GCCGAAGGGGAGTCCGAACTAGTCTCAGGCTTCAACATCGAATACGCCGCAG GCCCCTTCGCCCTATTCTTCATGGCCGAATACACAAACATTATTATGATGAAC ACCCTCACCACTACAATCTTCCTAGGAACAACATATGACGCACTCTCCCCTG |
| | | AACTCTACACAACATATTTTGTCACCAAGACCCTACTTCTAACCTCCCTGTTC TTATGGATTCGAACAGCATACCCCCGATTCCGCTACGACCAACTCATGCACC TCCTATGGAAAAACTTCCTACCACTCACCCTAGCATTACTTATGTGGTATGTC TCCATGCCCATTACAATCTCCAGCATTCCCCCTCAAACCTAAGAGCACTGGG ACGCCCACCGCCCCTTTCCCTCCGCTGCCAGGCGAGCATGTTGTGGTAATTCT GGAACACAAGAAGAGAAATTGCTGGGTTTAGAACAAGATTATAAACGAATT CGGTGCTCAGTGATCACTTGACAGTTTTTTTTTTTTTTAAATATTACCCAAAAT GCTCCCCAAATAAGAAATGCATCAGCTCAGTCAGTGAATACAAAAAAGGAA TTATTTTTCCCTTTGAGGGTCTTTTATACATCTCTCCTCCAACCCCACCCTCTA TTCTGTTTCTTCCTCCTCACATGGGGGTACACATACACAGCTTCCTCTTTTGGT TCCATCCTTACCACCACACCACACGCACACTCCACATGCCCAGCAGAGTGGC ACTTGGTGGCCAGAAAGTGTGAGCCTCATGATCTGCTGTCTGTAGTTCTGTGA GCTCAGGTCCCTCAAAGGCCTCGGAGCACCCCCTTCCTTGTGACTGAGCCAG GGCCTGCATTTTTGGTTTTCCCCACCCCACACATTCTCAACCATAGTCCTTCT AACAATACCAATAGCTAGGACCCGGCTGCTGTGCACTGGGACTGGGGATTCC ACATGTTTGCCTTGGGAGTCTCAAGCTGGACTGCCAGCCCCTGTCCTCCCTTC ACCCCCATTGCGTATGAGCATTTCAGAACTCCAAGGAGTCACAGGCATCTTT ATAGTTCACGTTAACATATAGACACTGTTGGAAGCAGTTCCTTCTAAAAGGG TAGCCCTGGACTTAATACCAGCCGGATACCTCTGGCCCCCACCCCATTACTGT ACCTCTGGAGTCACTACTGTGGGTCGCCACTCCTCTGCTACACAGCACGGCTT TTTCAAGGCTGTATTGAGAAGGGAAGTTAGGAAGAAGGGTGTGCTGGGCTAA CCAGCCCACAGAGCTCACATTCCTGTCCCTTGGGTGAAAAATACATGTCCAT CCTGATATCTCCTGAATTCAGAAATTAGCCTCCACATGTGCAATGGCTTTAAG AGCCAGAAGCAGGGTTCTGGGAATTTTGCAAGTTACCTGTGGCCAGGTGTGG TCTCGGTTACCAAATACGGTTACCTGCAGCTTTTTAGTCCTTTGTGCTCCCAC GGGTCTACAGAGTCCCATCTGCCCAAAGGTCTTGAAGCTTGACAGGATGTTT TCGATTACTCAGTCTCCCAGGGCACTACTGGTCCGTAGGATTCGATTGGTCGG GGTAGGAGAGTTAAACAACATTTAAACAGAGTTCTCTCAAAAATGTCTAAAG GGATTGTAGGTAGATAACATCCAATCACTGTTTGCACTTATCTGAAATCTTCC CTCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 68 | COX8-ND1-3' UTR* | ATGTCCGTCCTGACGCGCCTGCTGCTGCGGGGCTTGACACGGCTCGGCTCGG CGGCTCCAGTGCGGCGCGCCAGAATCCATTCGTTGATGGCCAACCTCCTACT CCTCATTGTACCCATTCTAATCGCAATGGCATTCCTAATGCTTACCGAACGAA AAATTCTAGGCTATATGCAACTACGCAAAGGCCCCAACGTTGTAGGCCCCTA CGGGCTACTACAACCCTTCGCTGACGCCATAAAACTCTTCACCAAAGAGCCC CTAAAACCCGCCACATCTACCATCACCCTCTACATCACCGCCCCGACCTTAGC TCTCACCATCGCTCTTCTACTATGGACCCCCCTCCCCATGCCCAACCCCCTGG TCAACCTCAACCTAGGCCTCCTATTTATTCTAGCCACCTCTAGCCTAGCCGTT TACTCAATCCTCTGGTCAGGGTGGGCATCAAACTCAAACTACGCCCTGATCG |
| | | GCGCACTGCGAGCAGTAGCCCAAACAATCTCATATGAAGTCACCCTAGCCAT CATTCTACTATCAACATTACTAATGAGTGGCTCCTTTAACCTCTCCACCCTTA TCACAACACAAGAACACCTCTGGTTACTCCTGCCATCATGGCCCTTGGCCAT GATGTGGTTTATCTCCACACTAGCAGAGACCAACCGAACCCCCTTCGACCTT GCCGAAGGGGAGTCCGAACTAGTCTCAGGCTTCAACATCGAATACGCCGCAG GCCCCTTCGCCCTATTCTTCATGGCCGAATACACAAACATTATTATGATGAAC ACCCTCACCACTACAATCTTCCTAGGAACAACATATGACGCACTCTCCCCTG AACTCTACACAACATATTTTGTCACCAAGACCCTACTTCTAACCTCCCTGTTC TTATGGATTCGAACAGCATACCCCCGATTCCGCTACGACCAACTCATGCACC TCCTATGGAAAAACTTCCTACCACTCACCCTAGCATTACTTATGTGGTATGTC TCCATGCCCATTACAATCTCCAGCATTCCCCCTCAAACCTAAGAGCACTGGG ACGCCCACCGCCCCTTTCCCTCCGCTGCCAGGCGAGCATGTTGTGGTAATTCT GGAACACAAGAAGAGAAATTGCTGGGTTTAGAACAAGATTATAAACGAATT CGGTGCTCAGTGATCACTTGACAGTTTTTTTTTTTTTTAAATATTACCCAAAAT GCTCCCCAAATAAGAAATGCATCAGCTCAGTCAGTGAATACAAAAAAGGAA TTATTTTTCCCTTTGAGGGTCTTTTATACATCTCTCCTCCAACCCCACCCTCTA TTCTGTTTCTTCCTCCTCACATGGGGGTACACATACACAGCTTCCTCTTTTGGT TCCATCCTTACCACCACACCACACGCACACTCCACATGCCCAGCAGAGTGGC ACTTGGTGGCCAGAAAGTGTGAGCCTCATGATCTGCTGTCTGTAGTTCTGTGA GCTCAGGTCCCTCAAAGGCCTCGGAGCACCCCCTTCCTTGTGACTGAGCCAG GGCCTGCATTTTTGGTTTTCCCCACCCCACACATTCTCAACCATAGTCCTTCT AACAATACCAATAGCTAGGACCCGGCTGCTGTGCACTGGGACTGGGGATTCC ACATGTTTGCCTTGGGAGTCTCAAGCTGGACTGCCA |
| 69 | COX8-opt_ND 1-3'UTR | ATGTCCGTCCTGACGCGCCTGCTGCTGCGGGGCTTGACACGGCTCGGCTCGG CGGCTCCAGTGCGGCGCGCCAGAATCCATTCGTTGATGGCCAACCTGCTGCT GCTGATCGTGCCCATCCTGATCGCCATGGCCTTCCTGATGCTGACCGAGCGC AAGATCCTGGGCTACATGCAGCTGCGCAAGGGCCCCAACGTGGTGGGCCCCT ACGGCCTGCTGCAGCCCTTCGCCGACGCCATCAAGCTGTTCACCAAGGAGCC CCTGAAGCCCGCCACCAGCACCATCACCCTGTACATCACCGCCCCCACCCTG GCCCTGACCATCGCCCTGCTGCTGTGGACCCCCCTGCCCATGCCCAACCCCCT GGTGAACCTGAACCTGGGCCTGCTGTTCATCCTGGCCACCAGCAGCCTGGCC GTGTACAGCATCCTGTGGAGCGGCTGGGCCAGCAACAGCAACTACGCCCTGA TCGGCGCCCTGCGCGCCGTGGCCCAGACCATCAGCTACGAGGTGACCCTGGC CATCATCCTGCTGAGCACCCTGCTGATGAGCGGCAGCTTCAACCTGAGCACC CTGATCACCACCCAGGAGCACCTGTGGCTGCTGCTGCCCAGCTGGCCCCTGG CCATGATGTGGTTCATCAGCACCCTGGCCGAGACCAACCGCACCCCCTTCGA CCTGGCCGAGGGCGAGAGCGAGCTGGTGAGCGGCTTCAACATCGAGTACGC CGCCGGCCCCTTCGCCCTGTTCTTCATGGCCGAGTACACCAACATCATCATGA TGAACACCCTGACCACCACCATCTTCCTGGGCACCACCTACGACGCCCTGAG CCCCGAGCTGTACACCACCTACTTCGTGACCAAGACCCTGCTGCTGACCAGC |
| | | CTGTTCCTGTGGATCCGCACCGCCTACCCCCGCTTCCGCTACGACCAGCTGAT GCACCTGCTGTGGAAGAACTTCCTGCCCCTGACCCTGGCCCTGCTGATGTGGT ACGTGAGCATGCCCATCACCATCAGCAGCATCCCCCCCCAGACCTAAGAGCA CTGGGACGCCCACCGCCCCTTTCCCTCCGCTGCCAGGCGAGCATGTTGTGGT AATTCTGGAACACAAGAAGAGAAATTGCTGGGTTTAGAACAAGATTATAAA CGAATTCGGTGCTCAGTGATCACTTGACAGTTTTTTTTTTTTTTAAATATTACC CAAAATGCTCCCCAAATAAGAAATGCATCAGCTCAGTCAGTGAATACAAAA AAGGAATTATTTTTCCCTTTGAGGGTCTTTTATACATCTCTCCTCCAACCCCA CCCTCTATTCTGTTTCTTCCTCCTCACATGGGGGTACACATACACAGCTTCCT CTTTTGGTTCCATCCTTACCACCACACCACACGCACACTCCACATGCCCAGCA GAGTGGCACTTGGTGGCCAGAAAGTGTGAGCCTCATGATCTGCTGTCTGTAG TTCTGTGAGCTCAGGTCCCTCAAAGGCCTCGGAGCACCCCCTTCCTTGTGACT GAGCCAGGGCCTGCATTTTTGGTTTTCCCCACCCCACACATTCTCAACCATAG TCCTTCTAACAATACCAATAGCTAGGACCCGGCTGCTGTGCACTGGGACTGG GGATTCCACATGTTTGCCTTGGGAGTCTCAAGCTGGACTGCCAGCCCCTGTCC TCCCTTCACCCCCATTGCGTATGAGCATTTCAGAACTCCAAGGAGTCACAGG CATCTTTATAGTTCACGTTAACATATAGACACTGTTGGAAGCAGTTCCTTCTA AAAGGGTAGCCCTGGACTTAATACCAGCCGGATACCTCTGGCCCCCACCCCA TTACTGTACCTCTGGAGTCACTACTGTGGGTCGCCACTCCTCTGCTACACAGC ACGGCTTTTTCAAGGCTGTATTGAGAAGGGAAGTTAGGAAGAAGGGTGTGCT GGGCTAACCAGCCCACAGAGCTCACATTCCTGTCCCTTGGGTGAAAAATACA TGTCCATCCTGATATCTCCTGAATTCAGAAATTAGCCTCCACATGTGCAATGG CTTTAAGAGCCAGAAGCAGGGTTCTGGGAATTTTGCAAGTTACCTGTGGCCA GGTGTGGTCTCGGTTACCAAATACGGTTACCTGCAGCTTTTTAGTCCTTTGTG CTCCCACGGGTCTACAGAGTCCCATCTGCCCAAAGGTCTTGAAGCTTGACAG GATGTTTTCGATTACTCAGTCTCCCAGGGCACTACTGGTCCGTAGGATTCGAT TGGTCGGGGTAGGAGAGTTAAACAACATTTAAACAGAGTTCTCTCAAAAATG TCTAAAGGGATTGTAGGTAGATAACATCCAATCACTGTTTGCACTTATCTGA AATCTTCCCTCTTGGCTGCCCCCAGGTATTTACTGTGGAGAACATTGCATAGG AATGTCTGGAAAAAGCTTCTACAACTTGTTACAGCCTTCACATTTGTAGAAG CTTT |
| 70 | COX8-opt_ND 1-3'UTR* | ATGTCCGTCCTGACGCGCCTGCTGCTGCGGGGCTTGACACGGCTCGGCTCGG CGGCTCCAGTGCGGCGCGCCAGAATCCATTCGTTGATGGCCAACCTGCTGCT GCTGATCGTGCCCATCCTGATCGCCATGGCCTTCCTGATGCTGACCGAGCGC AAGATCCTGGGCTACATGCAGCTGCGCAAGGGCCCCAACGTGGTGGGCCCCT ACGGCCTGCTGCAGCCCTTCGCCGACGCCATCAAGCTGTTCACCAAGGAGCC CCTGAAGCCCGCCACCAGCACCATCACCCTGTACATCACCGCCCCCACCCTG GCCCTGACCATCGCCCTGCTGCTGTGGACCCCCCTGCCCATGCCCAACCCCCT GGTGAACCTGAACCTGGGCCTGCTGTTCATCCTGGCCACCAGCAGCCTGGCC GTGTACAGCATCCTGTGGAGCGGCTGGGCCAGCAACAGCAACTACGCCCTGA |
| | | TCGGCGCCCTGCGCGCCGTGGCCCAGACCATCAGCTACGAGGTGACCCTGGC CATCATCCTGCTGAGCACCCTGCTGATGAGCGGCAGCTTCAACCTGAGCACC CTGATCACCACCCAGGAGCACCTGTGGCTGCTGCTGCCCAGCTGGCCCCTGG CCATGATGTGGTTCATCAGCACCCTGGCCGAGACCAACCGCACCCCCTTCGA CCTGGCCGAGGGCGAGAGCGAGCTGGTGAGCGGCTTCAACATCGAGTACGC CGCCGGCCCCTTCGCCCTGTTCTTCATGGCCGAGTACACCAACATCATCATGA TGAACACCCTGACCACCACCATCTTCCTGGGCACCACCTACGACGCCCTGAG CCCCGAGCTGTACACCACCTACTTCGTGACCAAGACCCTGCTGCTGACCAGC CTGTTCCTGTGGATCCGCACCGCCTACCCCCGCTTCCGCTACGACCAGCTGAT GCACCTGCTGTGGAAGAACTTCCTGCCCCTGACCCTGGCCCTGCTGATGTGGT ACGTGAGCATGCCCATCACCATCAGCAGCATCCCCCCCCAGACCTAAGAGCA CTGGGACGCCCACCGCCCCTTTCCCTCCGCTGCCAGGCGAGCATGTTGTGGT AATTCTGGAACACAAGAAGAGAAATTGCTGGGTTTAGAACAAGATTATAAA CGAATTCGGTGCTCAGTGATCACTTGACAGTTTTTTTTTTTTTTAAATATTACC CAAAATGCTCCCCAAATAAGAAATGCATCAGCTCAGTCAGTGAATACAAAA AAGGAATTATTTTTCCCTTTGAGGGTCTTTTATACATCTCTCCTCCAACCCCA CCCTCTATTCTGTTTCTTCCTCCTCACATGGGGGTACACATACACAGCTTCCT CTTTTGGTTCCATCCTTACCACCACACCACACGCACACTCCACATGCCCAGCA GAGTGGCACTTGGTGGCCAGAAAGTGTGAGCCTCATGATCTGCTGTCTGTAG TTCTGTGAGCTCAGGTCCCTCAAAGGCCTCGGAGCACCCCCTTCCTTGTGACT GAGCCAGGGCCTGCATTTTTGGTTTTCCCCACCCCACACATTCTCAACCATAG TCCTTCTAACAATACCAATAGCTAGGACCCGGCTGCTGTGCACTGGGACTGG GGATTCCACATGTTTGCCTTGGGAGTCTCAAGCTGGACTGCCA |
| 71 | OPAl-ND4-3' UTR | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGCTAAAACTAATCGTCCCAACAATTATGTTACTACCACTGACATG GCTTTCCAAAAAACACATGATTTGGATCAACACAACCACCCACAGCCTAATT ATTAGCATCATCCCTCTACTATTTTTTAACCAAATCAACAACAACCTATTTAG CTGTTCCCCAACCTTTTCCTCCGACCCCCTAACAACCCCCCTCCTAATGCTAA CTACCTGGCTCCTACCCCTCACAATCATGGCAAGCCAACGCCACTTATCCAGT GAACCACTATCACGAAAAAAACTCTACCTCTCTATGCTAATCTCCCTACAAA TCTCCTTAATTATGACATTCACAGCCACAGAACTAATCATGTTTTATATCTTC TTCGAAACCACACTTATCCCCACCTTGGCTATCATCACCCGATGGGGCAACC AGCCAGAACGCCTGAACGCAGGCACATACTTCCTATTCTACACCCTAGTAGG CTCCCTTCCCCTACTCATCGCACTAATTTACACTCACAACACCCTAGGCTCAC TAAACATTCTACTACTCACTCTCACTGCCCAAGAACTATCAAACTCCTGGGCC AACAACTTAATGTGGCTAGCTTACACAATGGCTTTTATGGTAAAGATGCCTCT |
| | | TTACGGACTCCACTTATGGCTCCCTAAAGCCCATGTCGAAGCCCCCATCGCTG GGTCAATGGTACTTGCCGCAGTACTCTTAAAACTAGGCGGCTATGGTATGAT GCGCCTCACACTCATTCTCAACCCCCTGACAAAACACATGGCCTACCCCTTCC TTGTACTATCCCTATGGGGCATGATTATGACAAGCTCCATCTGCCTACGACAA ACAGACCTAAAATCGCTCATTGCATACTCTTCAATCAGCCACATGGCCCTCGT AGTAACAGCCATTCTCATCCAAACCCCCTGGAGCTTCACCGGCGCAGTCATT CTCATGATCGCCCACGGGCTTACATCCTCATTACTATTCTGCCTAGCAAACTC AAACTACGAACGCACTCACAGTCGCATCATGATCCTCTCTCAAGGACTTCAA ACTCTACTCCCACTAATGGCTTTTTGGTGGCTTCTAGCAAGCCTCGCTAACCT CGCCTTACCCCCCACTATTAACCTACTGGGAGAACTCTCTGTGCTAGTAACCA CGTTCTCCTGGTCAAATATCACTCTCCTACTTACAGGACTCAACATGCTAGTC ACAGCCCTATACTCCCTCTACATGTTTACCACAACACAATGGGGCTCACTCAC CCACCACATTAACAACATGAAACCCTCATTCACACGAGAAAACACCCTCATG TTCATGCACCTATCCCCCATTCTCCTCCTATCCCTCAACCCCGACATCATTAC CGGGTTTTCCTCTTAAGAGCACTGGGACGCCCACCGCCCCTTTCCCTCCGCTG CCAGGCGAGCATGTTGTGGTAATTCTGGAACACAAGAAGAGAAATTGCTGG GTTTAGAACAAGATTATAAACGAATTCGGTGCTCAGTGATCACTTGACAGTT TTTTTTTTTTTTAAATATTACCCAAAATGCTCCCCAAATAAGAAATGCATCAG CTCAGTCAGTGAATACAAAAAAGGAATTATTTTTCCCTTTGAGGGTCTTTTAT ACATCTCTCCTCCAACCCCACCCTCTATTCTGTTTCTTCCTCCTCACATGGGGG TACACATACACAGCTTCCTCTTTTGGTTCCATCCTTACCACCACACCACACGC ACACTCCACATGCCCAGCAGAGTGGCACTTGGTGGCCAGAAAGTGTGAGCCT CATGATCTGCTGTCTGTAGTTCTGTGAGCTCAGGTCCCTCAAAGGCCTCGGAG CACCCCCTTCCTTGTGACTGAGCCAGGGCCTGCATTTTTGGTTTTCCCCACCC CACACATTCTCAACCATAGTCCTTCTAACAATACCAATAGCTAGGACCCGGC TGCTGTGCACTGGGACTGGGGATTCCACATGTTTGCCTTGGGAGTCTCAAGCT GGACTGCCAGCCCCTGTCCTCCCTTCACCCCCATTGCGTATGAGCATTTCAGA ACTCCAAGGAGTCACAGGCATCTTTATAGTTCACGTTAACATATAGACACTG TTGGAAGCAGTTCCTTCTAAAAGGGTAGCCCTGGACTTAATACCAGCCGGAT ACCTCTGGCCCCCACCCCATTACTGTACCTCTGGAGTCACTACTGTGGGTCGC CACTCCTCTGCTACACAGCACGGCTTTTTCAAGGCTGTATTGAGAAGGGAAG TTAGGAAGAAGGGTGTGCTGGGCTAACCAGCCCACAGAGCTCACATTCCTGT CCCTTGGGTGAAAAATACATGTCCATCCTGATATCTCCTGAATTCAGAAATTA GCCTCCACATGTGCAATGGCTTTAAGAGCCAGAAGCAGGGTTCTGGGAATTT TGCAAGTTACCTGTGGCCAGGTGTGGTCTCGGTTACCAAATACGGTTACCTG CAGCTTTTTAGTCCTTTGTGCTCCCACGGGTCTACAGAGTCCCATCTGCCCAA AGGTCTTGAAGCTTGACAGGATGTTTTCGATTACTCAGTCTCCCAGGGCACTA CTGGTCCGTAGGATTCGATTGGTCGGGGTAGGAGAGTTAAACAACATTTAAA CAGAGTTCTCTCAAAAATGTCTAAAGGGATTGTAGGTAGATAACATCCAATC ACTGTTTGCACTTATCTGAAATCTTCCCTCTTGGCTGCCCCCAGGTATTTACT |
| | | GTGGAGAACATTGCATAGGAATGTCTGGAAAAAGCTTCTACAACTTGTTACA GCCTTCACATTTGTAGAAGCTTT |
| 72 | OPAl-ND4-3' UTR* | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGCTAAAACTAATCGTCCCAACAATTATGTTACTACCACTGACATG GCTTTCCAAAAAACACATGATTTGGATCAACACAACCACCCACAGCCTAATT ATTAGCATCATCCCTCTACTATTTTTTAACCAAATCAACAACAACCTATTTAG CTGTTCCCCAACCTTTTCCTCCGACCCCCTAACAACCCCCCTCCTAATGCTAA CTACCTGGCTCCTACCCCTCACAATCATGGCAAGCCAACGCCACTTATCCAGT GAACCACTATCACGAAAAAAACTCTACCTCTCTATGCTAATCTCCCTACAAA TCTCCTTAATTATGACATTCACAGCCACAGAACTAATCATGTTTTATATCTTC TTCGAAACCACACTTATCCCCACCTTGGCTATCATCACCCGATGGGGCAACC AGCCAGAACGCCTGAACGCAGGCACATACTTCCTATTCTACACCCTAGTAGG CTCCCTTCCCCTACTCATCGCACTAATTTACACTCACAACACCCTAGGCTCAC TAAACATTCTACTACTCACTCTCACTGCCCAAGAACTATCAAACTCCTGGGCC AACAACTTAATGTGGCTAGCTTACACAATGGCTTTTATGGTAAAGATGCCTCT TTACGGACTCCACTTATGGCTCCCTAAAGCCCATGTCGAAGCCCCCATCGCTG GGTCAATGGTACTTGCCGCAGTACTCTTAAAACTAGGCGGCTATGGTATGAT GCGCCTCACACTCATTCTCAACCCCCTGACAAAACACATGGCCTACCCCTTCC TTGTACTATCCCTATGGGGCATGATTATGACAAGCTCCATCTGCCTACGACAA ACAGACCTAAAATCGCTCATTGCATACTCTTCAATCAGCCACATGGCCCTCGT AGTAACAGCCATTCTCATCCAAACCCCCTGGAGCTTCACCGGCGCAGTCATT CTCATGATCGCCCACGGGCTTACATCCTCATTACTATTCTGCCTAGCAAACTC AAACTACGAACGCACTCACAGTCGCATCATGATCCTCTCTCAAGGACTTCAA ACTCTACTCCCACTAATGGCTTTTTGGTGGCTTCTAGCAAGCCTCGCTAACCT CGCCTTACCCCCCACTATTAACCTACTGGGAGAACTCTCTGTGCTAGTAACCA CGTTCTCCTGGTCAAATATCACTCTCCTACTTACAGGACTCAACATGCTAGTC ACAGCCCTATACTCCCTCTACATGTTTACCACAACACAATGGGGCTCACTCAC CCACCACATTAACAACATGAAACCCTCATTCACACGAGAAAACACCCTCATG TTCATGCACCTATCCCCCATTCTCCTCCTATCCCTCAACCCCGACATCATTAC CGGGTTTTCCTCTTAAGAGCACTGGGACGCCCACCGCCCCTTTCCCTCCGCTG CCAGGCGAGCATGTTGTGGTAATTCTGGAACACAAGAAGAGAAATTGCTGG GTTTAGAACAAGATTATAAACGAATTCGGTGCTCAGTGATCACTTGACAGTT TTTTTTTTTTTTAAATATTACCCAAAATGCTCCCCAAATAAGAAATGCATCAG CTCAGTCAGTGAATACAAAAAAGGAATTATTTTTCCCTTTGAGGGTCTTTTAT ACATCTCTCCTCCAACCCCACCCTCTATTCTGTTTCTTCCTCCTCACATGGGGG TACACATACACAGCTTCCTCTTTTGGTTCCATCCTTACCACCACACCACACGC |
| | | ACACTCCACATGCCCAGCAGAGTGGCACTTGGTGGCCAGAAAGTGTGAGCCT CATGATCTGCTGTCTGTAGTTCTGTGAGCTCAGGTCCCTCAAAGGCCTCGGAG CACCCCCTTCCTTGTGACTGAGCCAGGGCCTGCATTTTTGGTTTTCCCCACCC CACACATTCTCAACCATAGTCCTTCTAACAATACCAATAGCTAGGACCCGGC TGCTGTGCACTGGGACTGGGGATTCCACATGTTTGCCTTGGGAGTCTCAAGCT GGACTGCCA |
| 73 | OPA1-opt_ND 4-3'UTR | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGCTGAAGCTGATCGTGCCCACCATCATGCTGCTGCCTCTGACCTGG CTGAGCAAGAAACACATGATCTGGATCAACACCACCACGCACAGCCTGATCA TCAGCATCATCCCTCTGCTGTTCTTCAACCAGATCAACAACAACCTGTTCAGC TGCAGCCCCACCTTCAGCAGCGACCCTCTGACAACACCTCTGCTGATGCTGA CCACCTGGCTGCTGCCCCTCACAATCATGGCCTCTCAGAGACACCTGAGCAG CGAGCCCCTGAGCCGGAAGAAACTGTACCTGAGCATGCTGATCTCCCTGCAG ATCTCTCTGATCATGACCTTCACCGCCACCGAGCTGATCATGTTCTACATCTT TTTCGAGACAACGCTGATCCCCACACTGGCCATCATCACCAGATGGGGCAAC CAGCCTGAGAGACTGAACGCCGGCACCTACTTTCTGTTCTACACCCTCGTGG GCAGCCTGCCACTGCTGATTGCCCTGATCTACACCCACAACACCCTGGGCTC CCTGAACATCCTGCTGCTGACACTGACAGCCCAAGAGCTGAGCAACAGCTGG GCCAACAATCTGATGTGGCTGGCCTACACAATGGCCTTCATGGTCAAGATGC CCCTGTACGGCCTGCACCTGTGGCTGCCTAAAGCTCATGTGGAAGCCCCTAT CGCCGGCTCTATGGTGCTGGCTGCAGTGCTGCTGAAACTCGGCGGCTACGGC ATGATGCGGCTGACCCTGATTCTGAATCCCCTGACCAAGCACATGGCCTATC CATTTCTGGTGCTGAGCCTGTGGGGCATGATTATGACCAGCAGCATCTGCCT GCGGCAGACCGATCTGAAGTCCCTGATCGCCTACAGCTCCATCAGCCACATG GCCCTGGTGGTCACCGCCATCCTGATTCAGACCCCTTGGAGCTTTACAGGCG CCGTGATCCTGATGATTGCCCACGGCCTGACAAGCAGCCTGCTGTTTTGTCTG GCCAACAGCAACTACGAGCGGACCCACAGCAGAATCATGATCCTGTCTCAGG GCCTGCAGACCCTCCTGCCTCTTATGGCTTTTTGGTGGCTGCTGGCCTCTCTG GCCAATCTGGCACTGCCTCCTACCATCAATCTGCTGGGCGAGCTGAGCGTGC TGGTCACCACATTCAGCTGGTCCAATATCACCCTGCTGCTCACCGGCCTGAAC ATGCTGGTTACAGCCCTGTACTCCCTGTACATGTTCACCACCACACAGTGGG GAAGCCTGACACACCACATCAACAATATGAAGCCCAGCTTCACCCGCGAGA ACACCCTGATGTTCATGCATCTGAGCCCCATTCTGCTGCTGTCCCTGAATCCT GATATCATCACCGGCTTCTCCAGCTGAGAGCACTGGGACGCCCACCGCCCCT TTCCCTCCGCTGCCAGGCGAGCATGTTGTGGTAATTCTGGAACACAAGAAGA GAAATTGCTGGGTTTAGAACAAGATTATAAACGAATTCGGTGCTCAGTGATC |
| | | ACTTGACAGTTTTTTTTTTTTTTAAATATTACCCAAAATGCTCCCCAAATAAG AAATGCATCAGCTCAGTCAGTGAATACAAAAAAGGAATTATTTTTCCCTTTG AGGGTCTTTTATACATCTCTCCTCCAACCCCACCCTCTATTCTGTTTCTTCCTC CTCACATGGGGGTACACATACACAGCTTCCTCTTTTGGTTCCATCCTTACCAC CACACCACACGCACACTCCACATGCCCAGCAGAGTGGCACTTGGTGGCCAGA AAGTGTGAGCCTCATGATCTGCTGTCTGTAGTTCTGTGAGCTCAGGTCCCTCA AAGGCCTCGGAGCACCCCCTTCCTTGTGACTGAGCCAGGGCCTGCATTTTTG GTTTTCCCCACCCCACACATTCTCAACCATAGTCCTTCTAACAATACCAATAG CTAGGACCCGGCTGCTGTGCACTGGGACTGGGGATTCCACATGTTTGCCTTG GGAGTCTCAAGCTGGACTGCCAGCCCCTGTCCTCCCTTCACCCCCATTGCGTA TGAGCATTTCAGAACTCCAAGGAGTCACAGGCATCTTTATAGTTCACGTTAA CATATAGACACTGTTGGAAGCAGTTCCTTCTAAAAGGGTAGCCCTGGACTTA ATACCAGCCGGATACCTCTGGCCCCCACCCCATTACTGTACCTCTGGAGTCAC TACTGTGGGTCGCCACTCCTCTGCTACACAGCACGGCTTTTTCAAGGCTGTAT TGAGAAGGGAAGTTAGGAAGAAGGGTGTGCTGGGCTAACCAGCCCACAGAG CTCACATTCCTGTCCCTTGGGTGAAAAATACATGTCCATCCTGATATCTCCTG AATTCAGAAATTAGCCTCCACATGTGCAATGGCTTTAAGAGCCAGAAGCAGG GTTCTGGGAATTTTGCAAGTTACCTGTGGCCAGGTGTGGTCTCGGTTACCAAA TACGGTTACCTGCAGCTTTTTAGTCCTTTGTGCTCCCACGGGTCTACAGAGTC CCATCTGCCCAAAGGTCTTGAAGCTTGACAGGATGTTTTCGATTACTCAGTCT CCCAGGGCACTACTGGTCCGTAGGATTCGATTGGTCGGGGTAGGAGAGTTAA ACAACATTTAAACAGAGTTCTCTCAAAAATGTCTAAAGGGATTGTAGGTAGA TAACATCCAATCACTGTTTGCACTTATCTGAAATCTTCCCTCTTGGCTGCCCC CAGGTATTTACTGTGGAGAACATTGCATAGGAATGTCTGGAAAAAGCTTCTA CAACTTGTTACAGCCTTCACATTTGTAGAAGCTTT |
| 74 | OPA1-opt_ND 4-3'UTR* | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGCTGAAGCTGATCGTGCCCACCATCATGCTGCTGCCTCTGACCTGG CTGAGCAAGAAACACATGATCTGGATCAACACCACCACGCACAGCCTGATCA TCAGCATCATCCCTCTGCTGTTCTTCAACCAGATCAACAACAACCTGTTCAGC TGCAGCCCCACCTTCAGCAGCGACCCTCTGACAACACCTCTGCTGATGCTGA CCACCTGGCTGCTGCCCCTCACAATCATGGCCTCTCAGAGACACCTGAGCAG CGAGCCCCTGAGCCGGAAGAAACTGTACCTGAGCATGCTGATCTCCCTGCAG ATCTCTCTGATCATGACCTTCACCGCCACCGAGCTGATCATGTTCTACATCTT TTTCGAGACAACGCTGATCCCCACACTGGCCATCATCACCAGATGGGGCAAC CAGCCTGAGAGACTGAACGCCGGCACCTACTTTCTGTTCTACACCCTCGTGG GCAGCCTGCCACTGCTGATTGCCCTGATCTACACCCACAACACCCTGGGCTC |
| | | CCTGAACATCCTGCTGCTGACACTGACAGCCCAAGAGCTGAGCAACAGCTGG GCCAACAATCTGATGTGGCTGGCCTACACAATGGCCTTCATGGTCAAGATGC CCCTGTACGGCCTGCACCTGTGGCTGCCTAAAGCTCATGTGGAAGCCCCTAT CGCCGGCTCTATGGTGCTGGCTGCAGTGCTGCTGAAACTCGGCGGCTACGGC ATGATGCGGCTGACCCTGATTCTGAATCCCCTGACCAAGCACATGGCCTATC CATTTCTGGTGCTGAGCCTGTGGGGCATGATTATGACCAGCAGCATCTGCCT GCGGCAGACCGATCTGAAGTCCCTGATCGCCTACAGCTCCATCAGCCACATG GCCCTGGTGGTCACCGCCATCCTGATTCAGACCCCTTGGAGCTTTACAGGCG CCGTGATCCTGATGATTGCCCACGGCCTGACAAGCAGCCTGCTGTTTTGTCTG GCCAACAGCAACTACGAGCGGACCCACAGCAGAATCATGATCCTGTCTCAGG GCCTGCAGACCCTCCTGCCTCTTATGGCTTTTTGGTGGCTGCTGGCCTCTCTG GCCAATCTGGCACTGCCTCCTACCATCAATCTGCTGGGCGAGCTGAGCGTGC TGGTCACCACATTCAGCTGGTCCAATATCACCCTGCTGCTCACCGGCCTGAAC ATGCTGGTTACAGCCCTGTACTCCCTGTACATGTTCACCACCACACAGTGGG GAAGCCTGACACACCACATCAACAATATGAAGCCCAGCTTCACCCGCGAGA ACACCCTGATGTTCATGCATCTGAGCCCCATTCTGCTGCTGTCCCTGAATCCT GATATCATCACCGGCTTCTCCAGCTGAGAGCACTGGGACGCCCACCGCCCCT TTCCCTCCGCTGCCAGGCGAGCATGTTGTGGTAATTCTGGAACACAAGAAGA GAAATTGCTGGGTTTAGAACAAGATTATAAACGAATTCGGTGCTCAGTGATC ACTTGACAGTTTTTTTTTTTTTTAAATATTACCCAAAATGCTCCCCAAATAAG AAATGCATCAGCTCAGTCAGTGAATACAAAAAAGGAATTATTTTTCCCTTTG AGGGTCTTTTATACATCTCTCCTCCAACCCCACCCTCTATTCTGTTTCTTCCTC CTCACATGGGGGTACACATACACAGCTTCCTCTTTTGGTTCCATCCTTACCAC CACACCACACGCACACTCCACATGCCCAGCAGAGTGGCACTTGGTGGCCAGA AAGTGTGAGCCTCATGATCTGCTGTCTGTAGTTCTGTGAGCTCAGGTCCCTCA AAGGCCTCGGAGCACCCCCTTCCTTGTGACTGAGCCAGGGCCTGCATTTTTG GTTTTCCCCACCCCACACATTCTCAACCATAGTCCTTCTAACAATACCAATAG CTAGGACCCGGCTGCTGTGCACTGGGACTGGGGATTCCACATGTTTGCCTTG GGAGTCTCAAGCTGGACTGCCA |
| 75 | OPA1-opt_ND 4*-3'UTR | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGCTGAAGCTGATCGTGCCCACCATCATGCTGCTGCCCCTGACCTG GCTGAGCAAGAAGCACATGATCTGGATCAACACCACCACCCACAGCCTGATC ATCAGCATCATCCCCCTGCTGTTCTTCAACCAGATCAACAACAACCTGTTCAG CTGCAGCCCCACCTTCAGCAGCGACCCCCTGACCACCCCCCTGCTGATGCTG ACCACCTGGCTGCTGCCCCTGACCATCATGGCCAGCCAGCGCCACCTGAGCA GCGAGCCCCTGAGCCGCAAGAAGCTGTACCTGAGCATGCTGATCAGCCTGCA |
| | | GATCAGCCTGATCATGACCTTCACCGCCACCGAGCTGATCATGTTCTACATCT TCTTCGAGACCACCCTGATCCCCACCCTGGCCATCATCACCCGCTGGGGCAA CCAGCCCGAGCGCCTGAACGCCGGCACCTACTTCCTGTTCTACACCCTGGTG GGCAGCCTGCCCCTGCTGATCGCCCTGATCTACACCCACAACACCCTGGGCA GCCTGAACATCCTGCTGCTGACCCTGACCGCCCAGGAGCTGAGCAACAGCTG GGCCAACAACCTGATGTGGCTGGCCTACACCATGGCCTTCATGGTGAAGATG CCCCTGTACGGCCTGCACCTGTGGCTGCCCAAGGCCCACGTGGAGGCCCCCA TCGCCGGCAGCATGGTGCTGGCCGCCGTGCTGCTGAAGCTGGGCGGCTACGG CATGATGCGCCTGACCCTGATCCTGAACCCCCTGACCAAGCACATGGCCTAC CCCTTCCTGGTGCTGAGCCTGTGGGGCATGATCATGACCAGCAGCATCTGCC TGCGCCAGACCGACCTGAAGAGCCTGATCGCCTACAGCAGCATCAGCCACAT GGCCCTGGTGGTGACCGCCATCCTGATCCAGACCCCCTGGAGCTTCACCGGC GCCGTGATCCTGATGATCGCCCACGGCCTGACCAGCAGCCTGCTGTTCTGCCT GGCCAACAGCAACTACGAGCGCACCCACAGCCGCATCATGATCCTGAGCCA GGGCCTGCAGACCCTGCTGCCCCTGATGGCCTTCTGGTGGCTGCTGGCCAGC CTGGCCAACCTGGCCCTGCCCCCCACCATCAACCTGCTGGGCGAGCTGAGCG TGCTGGTGACCACCTTCAGCTGGAGCAACATCACCCTGCTGCTGACCGGCCT GAACATGCTGGTGACCGCCCTGTACAGCCTGTACATGTTCACCACCACCCAG TGGGGCAGCCTGACCCACCACATCAACAACATGAAGCCCAGCTTCACCCGCG AGAACACCCTGATGTTCATGCACCTGAGCCCCATCCTGCTGCTGAGCCTGAA CCCCGACATCATCACCGGCTTCAGCAGCTAAGAGCACTGGGACGCCCACCGC CCCTTTCCCTCCGCTGCCAGGCGAGCATGTTGTGGTAATTCTGGAACACAAG AAGAGAAATTGCTGGGTTTAGAACAAGATTATAAACGAATTCGGTGCTCAGT GATCACTTGACAGTTTTTTTTTTTTTTAAATATTACCCAAAATGCTCCCCAAAT AAGAAATGCATCAGCTCAGTCAGTGAATACAAAAAAGGAATTATTTTTCCCT TTGAGGGTCTTTTATACATCTCTCCTCCAACCCCACCCTCTATTCTGTTTCTTC CTCCTCACATGGGGGTACACATACACAGCTTCCTCTTTTGGTTCCATCCTTAC CACCACACCACACGCACACTCCACATGCCCAGCAGAGTGGCACTTGGTGGCC AGAAAGTGTGAGCCTCATGATCTGCTGTCTGTAGTTCTGTGAGCTCAGGTCCC TCAAAGGCCTCGGAGCACCCCCTTCCTTGTGACTGAGCCAGGGCCTGCATTTT TGGTTTTCCCCACCCCACACATTCTCAACCATAGTCCTTCTAACAATACCAAT AGCTAGGACCCGGCTGCTGTGCACTGGGACTGGGGATTCCACATGTTTGCCT TGGGAGTCTCAAGCTGGACTGCCAGCCCCTGTCCTCCCTTCACCCCCATTGCG TATGAGCATTTCAGAACTCCAAGGAGTCACAGGCATCTTTATAGTTCACGTT AACATATAGACACTGTTGGAAGCAGTTCCTTCTAAAAGGGTAGCCCTGGACT TAATACCAGCCGGATACCTCTGGCCCCCACCCCATTACTGTACCTCTGGAGTC ACTACTGTGGGTCGCCACTCCTCTGCTACACAGCACGGCTTTTTCAAGGCTGT ATTGAGAAGGGAAGTTAGGAAGAAGGGTGTGCTGGGCTAACCAGCCCACAG AGCTCACATTCCTGTCCCTTGGGTGAAAAATACATGTCCATCCTGATATCTCC TGAATTCAGAAATTAGCCTCCACATGTGCAATGGCTTTAAGAGCCAGAAGCA |
| | | GGGTTCTGGGAATTTTGCAAGTTACCTGTGGCCAGGTGTGGTCTCGGTTACCA AATACGGTTACCTGCAGCTTTTTAGTCCTTTGTGCTCCCACGGGTCTACAGAG TCCCATCTGCCCAAAGGTCTTGAAGCTTGACAGGATGTTTTCGATTACTCAGT CTCCCAGGGCACTACTGGTCCGTAGGATTCGATTGGTCGGGGTAGGAGAGTT AAACAACATTTAAACAGAGTTCTCTCAAAAATGTCTAAAGGGATTGTAGGTA GATAACATCCAATCACTGTTTGCACTTATCTGAAATCTTCCCTCTTGGCTGCC CCCAGGTATTTACTGTGGAGAACATTGCATAGGAATGTCTGGAAAAAGCTTC TACAACTTGTTACAGCCTTCACATTTGTAGAAGCTTT |
| 76 | OPA1-opt_ND 4*-3'UTR* | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGCTGAAGCTGATCGTGCCCACCATCATGCTGCTGCCCCTGACCTG GCTGAGCAAGAAGCACATGATCTGGATCAACACCACCACCCACAGCCTGATC ATCAGCATCATCCCCCTGCTGTTCTTCAACCAGATCAACAACAACCTGTTCAG CTGCAGCCCCACCTTCAGCAGCGACCCCCTGACCACCCCCCTGCTGATGCTG ACCACCTGGCTGCTGCCCCTGACCATCATGGCCAGCCAGCGCCACCTGAGCA GCGAGCCCCTGAGCCGCAAGAAGCTGTACCTGAGCATGCTGATCAGCCTGCA GATCAGCCTGATCATGACCTTCACCGCCACCGAGCTGATCATGTTCTACATCT TCTTCGAGACCACCCTGATCCCCACCCTGGCCATCATCACCCGCTGGGGCAA CCAGCCCGAGCGCCTGAACGCCGGCACCTACTTCCTGTTCTACACCCTGGTG GGCAGCCTGCCCCTGCTGATCGCCCTGATCTACACCCACAACACCCTGGGCA GCCTGAACATCCTGCTGCTGACCCTGACCGCCCAGGAGCTGAGCAACAGCTG GGCCAACAACCTGATGTGGCTGGCCTACACCATGGCCTTCATGGTGAAGATG CCCCTGTACGGCCTGCACCTGTGGCTGCCCAAGGCCCACGTGGAGGCCCCCA TCGCCGGCAGCATGGTGCTGGCCGCCGTGCTGCTGAAGCTGGGCGGCTACGG CATGATGCGCCTGACCCTGATCCTGAACCCCCTGACCAAGCACATGGCCTAC CCCTTCCTGGTGCTGAGCCTGTGGGGCATGATCATGACCAGCAGCATCTGCC TGCGCCAGACCGACCTGAAGAGCCTGATCGCCTACAGCAGCATCAGCCACAT GGCCCTGGTGGTGACCGCCATCCTGATCCAGACCCCCTGGAGCTTCACCGGC GCCGTGATCCTGATGATCGCCCACGGCCTGACCAGCAGCCTGCTGTTCTGCCT GGCCAACAGCAACTACGAGCGCACCCACAGCCGCATCATGATCCTGAGCCA GGGCCTGCAGACCCTGCTGCCCCTGATGGCCTTCTGGTGGCTGCTGGCCAGC CTGGCCAACCTGGCCCTGCCCCCCACCATCAACCTGCTGGGCGAGCTGAGCG TGCTGGTGACCACCTTCAGCTGGAGCAACATCACCCTGCTGCTGACCGGCCT GAACATGCTGGTGACCGCCCTGTACAGCCTGTACATGTTCACCACCACCCAG TGGGGCAGCCTGACCCACCACATCAACAACATGAAGCCCAGCTTCACCCGCG AGAACACCCTGATGTTCATGCACCTGAGCCCCATCCTGCTGCTGAGCCTGAA CCCCGACATCATCACCGGCTTCAGCAGCTAAGAGCACTGGGACGCCCACCGC |
| | | CCCTTTCCCTCCGCTGCCAGGCGAGCATGTTGTGGTAATTCTGGAACACAAG AAGAGAAATTGCTGGGTTTAGAACAAGATTATAAACGAATTCGGTGCTCAGT GATCACTTGACAGTTTTTTTTTTTTTTAAATATTACCCAAAATGCTCCCCAAAT AAGAAATGCATCAGCTCAGTCAGTGAATACAAAAAAGGAATTATTTTTCCCT TTGAGGGTCTTTTATACATCTCTCCTCCAACCCCACCCTCTATTCTGTTTCTTC CTCCTCACATGGGGGTACACATACACAGCTTCCTCTTTTGGTTCCATCCTTAC CACCACACCACACGCACACTCCACATGCCCAGCAGAGTGGCACTTGGTGGCC AGAAAGTGTGAGCCTCATGATCTGCTGTCTGTAGTTCTGTGAGCTCAGGTCCC TCAAAGGCCTCGGAGCACCCCCTTCCTTGTGACTGAGCCAGGGCCTGCATTTT TGGTTTTCCCCACCCCACACATTCTCAACCATAGTCCTTCTAACAATACCAAT AGCTAGGACCCGGCTGCTGTGCACTGGGACTGGGGATTCCACATGTTTGCCT TGGGAGTCTCAAGCTGGACTGCCA |
| 77 | OPA1-ND6-3' UTR | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGATGTATGCTTTGTTTCTGTTGAGTGTGGGTTTAGTAATGGGGTTT GTGGGGTTTTCTTCTAAGCCTTCTCCTATTTATGGGGGTTTAGTATTGATTGTT AGCGGTGTGGTCGGGTGTGTTATTATTCTGAATTTTGGGGGAGGTTATATGGG TTTAATGGTTTTTTTAATTTATTTAGGGGGAATGATGGTTGTCTTTGGATATAC TACAGCGATGGCTATTGAGGAGTATCCTGAGGCATGGGGGTCAGGGGTTGAG GTCTTGGTGAGTGTTTTAGTGGGGTTAGCGATGGAGGTAGGATTGGTGCTGT GGGTGAAAGAGTATGATGGGGTGGTGGTTGTGGTAAACTTTAATAGTGTAGG AAGCTGGATGATTTATGAAGGAGAGGGGTCAGGGTTGATTCGGGAGGATCCT ATTGGTGCGGGGGCTTTGTATGATTATGGGCGTTGGTTAGTAGTAGTTACTGG TTGGACATTGTTTGTTGGTGTATATATTGTAATTGAGATTGCTCGGGGGAATT AGGAGCACTGGGACGCCCACCGCCCCTTTCCCTCCGCTGCCAGGCGAGCATG TTGTGGTAATTCTGGAACACAAGAAGAGAAATTGCTGGGTTTAGAACAAGAT TATAAACGAATTCGGTGCTCAGTGATCACTTGACAGTTTTTTTTTTTTTTAAAT ATTACCCAAAATGCTCCCCAAATAAGAAATGCATCAGCTCAGTCAGTGAATA CAAAAAAGGAATTATTTTTCCCTTTGAGGGTCTTTTATACATCTCTCCTCCAA CCCCACCCTCTATTCTGTTTCTTCCTCCTCACATGGGGGTACACATACACAGC TTCCTCTTTTGGTTCCATCCTTACCACCACACCACACGCACACTCCACATGCC CAGCAGAGTGGCACTTGGTGGCCAGAAAGTGTGAGCCTCATGATCTGCTGTC TGTAGTTCTGTGAGCTCAGGTCCCTCAAAGGCCTCGGAGCACCCCCTTCCTTG TGACTGAGCCAGGGCCTGCATTTTTGGTTTTCCCCACCCCACACATTCTCAAC CATAGTCCTTCTAACAATACCAATAGCTAGGACCCGGCTGCTGTGCACTGGG ACTGGGGATTCCACATGTTTGCCTTGGGAGTCTCAAGCTGGACTGCCAGCCC CTGTCCTCCCTTCACCCCCATTGCGTATGAGCATTTCAGAACTCCAAGGAGTC |
| | | ACAGGCATCTTTATAGTTCACGTTAACATATAGACACTGTTGGAAGCAGTTC CTTCTAAAAGGGTAGCCCTGGACTTAATACCAGCCGGATACCTCTGGCCCCC ACCCCATTACTGTACCTCTGGAGTCACTACTGTGGGTCGCCACTCCTCTGCTA CACAGCACGGCTTTTTCAAGGCTGTATTGAGAAGGGAAGTTAGGAAGAAGG GTGTGCTGGGCTAACCAGCCCACAGAGCTCACATTCCTGTCCCTTGGGTGAA AAATACATGTCCATCCTGATATCTCCTGAATTCAGAAATTAGCCTCCACATGT GCAATGGCTTTAAGAGCCAGAAGCAGGGTTCTGGGAATTTTGCAAGTTACCT GTGGCCAGGTGTGGTCTCGGTTACCAAATACGGTTACCTGCAGCTTTTTAGTC CTTTGTGCTCCCACGGGTCTACAGAGTCCCATCTGCCCAAAGGTCTTGAAGCT TGACAGGATGTTTTCGATTACTCAGTCTCCCAGGGCACTACTGGTCCGTAGG ATTCGATTGGTCGGGGTAGGAGAGTTAAACAACATTTAAACAGAGTTCTCTC AAAAATGTCTAAAGGGATTGTAGGTAGATAACATCCAATCACTGTTTGCACT TATCTGAAATCTTCCCTCTTGGCTGCCCCCAGGTATTTACTGTGGAGAACATT GCATAGGAATGTCTGGAAAAAGCTTCTACAACTTGTTACAGCCTTCACATTT GTAGAAGCTTT |
| 78 | OPA1-ND6-3' UTR* | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGATGTATGCTTTGTTTCTGTTGAGTGTGGGTTTAGTAATGGGGTTT GTGGGGTTTTCTTCTAAGCCTTCTCCTATTTATGGGGGTTTAGTATTGATTGTT AGCGGTGTGGTCGGGTGTGTTATTATTCTGAATTTTGGGGGAGGTTATATGGG TTTAATGGTTTTTTTAATTTATTTAGGGGGAATGATGGTTGTCTTTGGATATAC TACAGCGATGGCTATTGAGGAGTATCCTGAGGCATGGGGGTCAGGGGTTGAG GTCTTGGTGAGTGTTTTAGTGGGGTTAGCGATGGAGGTAGGATTGGTGCTGT GGGTGAAAGAGTATGATGGGGTGGTGGTTGTGGTAAACTTTAATAGTGTAGG AAGCTGGATGATTTATGAAGGAGAGGGGTCAGGGTTGATTCGGGAGGATCCT ATTGGTGCGGGGGCTTTGTATGATTATGGGCGTTGGTTAGTAGTAGTTACTGG TTGGACATTGTTTGTTGGTGTATATATTGTAATTGAGATTGCTCGGGGGAATT AGGAGCACTGGGACGCCCACCGCCCCTTTCCCTCCGCTGCCAGGCGAGCATG TTGTGGTAATTCTGGAACACAAGAAGAGAAATTGCTGGGTTTAGAACAAGAT TATAAACGAATTCGGTGCTCAGTGATCACTTGACAGTTTTTTTTTTTTTTAAAT ATTACCCAAAATGCTCCCCAAATAAGAAATGCATCAGCTCAGTCAGTGAATA CAAAAAAGGAATTATTTTTCCCTTTGAGGGTCTTTTATACATCTCTCCTCCAA CCCCACCCTCTATTCTGTTTCTTCCTCCTCACATGGGGGTACACATACACAGC TTCCTCTTTTGGTTCCATCCTTACCACCACACCACACGCACACTCCACATGCC CAGCAGAGTGGCACTTGGTGGCCAGAAAGTGTGAGCCTCATGATCTGCTGTC TGTAGTTCTGTGAGCTCAGGTCCCTCAAAGGCCTCGGAGCACCCCCTTCCTTG TGACTGAGCCAGGGCCTGCATTTTTGGTTTTCCCCACCCCACACATTCTCAAC |
| | | CATAGTCCTTCTAACAATACCAATAGCTAGGACCCGGCTGCTGTGCACTGGG ACTGGGGATTCCACATGTTTGCCTTGGGAGTCTCAAGCTGGACTGCCA |
| 79 | OPA1-opt_ND 6-3'UTR | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGATGTACGCCCTGTTCCTGCTGAGCGTGGGCCTGGTGATGGGCTTC GTGGGCTTCAGCAGCAAGCCCAGCCCCATCTACGGCGGCCTGGTGCTGATCG TGAGCGGCGTGGTGGGCTGCGTGATCATCCTGAACTTCGGCGGCGGCTACAT GGGCCTGATGGTGTTCCTGATCTACCTGGGCGGCATGATGGTGGTGTTCGGC TACACCACCGCCATGGCCATCGAGGAGTACCCCGAGGCCTGGGGCAGCGGC GTGGAGGTGCTGGTGAGCGTGCTGGTGGGCCTGGCCATGGAGGTGGGCCTGG TGCTGTGGGTGAAGGAGTACGACGGCGTGGTGGTGGTGAACTTCAACAG CGTGGGCAGCTGGATGATCTACGAGGGCGAGGGCAGCGGCCTGATCCGCGA GGACCCCATCGGCGCCGGCGCCCTGTACGACTACGGCCGCTGGCTGGTGGTG GTGACCGGCTGGACCCTGTTCGTGGGCGTGTACATCGTGATCGAGATCGCCC GCGGCAACTAAGAGCACTGGGACGCCCACCGCCCCTTTCCCTCCGCTGCCAG GCGAGCATGTTGTGGTAATTCTGGAACACAAGAAGAGAAATTGCTGGGTTTA GAACAAGATTATAAACGAATTCGGTGCTCAGTGATCACTTGACAGTTTTTTT TTTTTAAATATTACCCAAAATGCTCCCCAAATAAGAAATGCATCAGCTCAGT CAGTGAATACAAAAAAGGAATTATTTTCCCTTTGAGGGTCTTTTATACATCT CTCCTCCAACCCCACCCTCTATTCTGTTTCTTCCTCCTCACATGGGGGTACAC ATACACAGCTTCCTCTTTTGGTTCCATCCTTACCACCACACCACACGCACACT CCACATGCCCAGCAGAGTGGCACTTGGTGGCCAGAAAGTGTGAGCCTCATGA TCTGCTGTCTGTAGTTCTGTGAGCTCAGGTCCCTCAAAGGCCTCGGAGCACCC CCTTCCTTGTGACTGAGCCAGGGCCTGCATTTTTGGTTTTCCCCACCCCACAC ATTCTCAACCATAGTCCTTCTAACAATACCAATAGCTAGGACCCGGCTGCTGT GCACTGGGACTGGGGATTCCACATGTTTGCCTTGGGAGTCTCAAGCTGGACT GCCAGCCCCTGTCCTCCCTTCACCCCCATTGCGTATGAGCATTTCAGAACTCC AAGGAGTCACAGGCATCTTTATAGTTCACGTTAACATATAGACACTGTTGGA AGCAGTTCCTTCTAAAAGGGTAGCCCTGGACTTAATACCAGCCGGATACCTC TGGCCCCCACCCCATTACTGTACCTCTGGAGTCACTACTGTGGGTCGCCACTC CTCTGCTACACAGCACGGCTTTTTCAAGGCTGTATTGAGAAGGGAAGTTAGG AAGAAGGGTGTGCTGGGCTAACCAGCCCACAGAGCTCACATTCCTGTCCCTT GGGTGAAAAATACATGTCCATCCTGATATCTCCTGAATTCAGAAATTAGCCT CCACATGTGCAATGGCTTTAAGAGCCAGAAGCAGGGTTCTGGGAATTTTGCA AGTTACCTGTGGCCAGGTGTGGTCTCGGTTACCAAATACGGTTACCTGCAGC TTTTTAGTCCTTTGTGCTCCCACGGGTCTACAGAGTCCCATCTGCCCAAAGGT CTTGAAGCTTGACAGGATGTTTTCGATTACTCAGTCTCCCAGGGCACTACTGG |
| | | TCCGTAGGATTCGATTGGTCGGGGTAGGAGAGTTAAACAACATTTAAACAGA GTTCTCTCAAAAATGTCTAAAGGGATTGTAGGTAGATAACATCCAATCACTG TTTGCACTTATCTGAAATCTTCCCTCTTGGCTGCCCCCAGGTATTTACTGTGG AGAACATTGCATAGGAATGTCTGGAAAAAGCTTCTACAACTTGTTACAGCCT TCACATTTGTAGAAGCTTT |
| 80 | OPA1-opt_ND 6-3'UTR* | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGATGTACGCCCTGTTCCTGCTGAGCGTGGGCCTGGTGATGGGCTTC GTGGGCTTCAGCAGCAAGCCCAGCCCCATCTACGGCGGCCTGGTGCTGATCG TGAGCGGCGTGGTGGGCTGCGTGATCATCCTGAACTTCGGCGGCGGCTACAT GGGCCTGATGGTGTTCCTGATCTACCTGGGCGGCATGATGGTGGTGTTCGGC TACACCACCGCCATGGCCATCGAGGAGTACCCCGAGGCCTGGGGCAGCGGC GTGGAGGTGCTGGTGAGCGTGCTGGTGGGCCTGGCCATGGAGGTGGGCCTGG TGCTGTGGGTGAAGGAGTACGACGGCGTGGTGGTGGTGAACTTCAACAG CGTGGGCAGCTGGATGATCTACGAGGGCGAGGGCAGCGGCCTGATCCGCGA GGACCCCATCGGCGCCGGCGCCCTGTACGACTACGGCCGCTGGCTGGTGGTG GTGACCGGCTGGACCCTGTTCGTGGGCGTGTACATCGTGATCGAGATCGCCC GCGGCAACTAAGAGCACTGGGACGCCCACCGCCCCTTTCCCTCCGCTGCCAG GCGAGCATGTTGTGGTAATTCTGGAACACAAGAAGAGAAATTGCTGGGTTTA GAACAAGATTATAAACGAATTCGGTGCTCAGTGATCACTTGACAGTTTTTTTTT TTTTTAAATATTACCCAAAATGCTCCCCAAATAAGAAATGCATCAGCTCAGT CAGTGAATACAAAAAAGGAATTATTTTTCCCTTTGAGGGTCTTTTATACATCT CTCCTCCAACCCCACCCTCTATTCTGTTTCTTCCTCCTCACATGGGGGTACAC ATACACAGCTTCCTCTTTTGGTTCCATCCTTACCACCACACCACACGCACACT CCACATGCCCAGCAGAGTGGCACTTGGTGGCCAGAAAGTGTGAGCCTCATGA TCTGCTGTCTGTAGTTCTGTGAGCTCAGGTCCCTCAAAGGCCTCGGAGCACCC CCTTCCTTGTGACTGAGCCAGGGCCTGCATTTTTGGTTTTCCCCACCCCACAC ATTCTCAACCATAGTCCTTCTAACAATACCAATAGCTAGGACCCGGCTGCTGT GCACTGGGACTGGGGATTCCACATGTTTGCCTTGGGAGTCTCAAGCTGGACT GCCA |
| 81 | OPA1-ND1-3' UTR | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGGCCAACCTCCTACTCCTCATTGTACCCATTCTAATCGCAATGGCA |
| | | TTCCTAATGCTTACCGAACGAAAAATTCTAGGCTATATGCAACTACGCAAAG GCCCCAACGTTGTAGGCCCCTACGGGCTACTACAACCCTTCGCTGACGCCAT AAAACTCTTCACCAAAGAGCCCCTAAAACCCGCCACATCTACCATCACCCTC TACATCACCGCCCCGACCTTAGCTCTCACCATCGCTCTTCTACTATGGACCCC CCTCCCCATGCCCAACCCCCTGGTCAACCTCAACCTAGGCCTCCTATTTATTC TAGCCACCTCTAGCCTAGCCGTTTACTCAATCCTCTGGTCAGGGTGGGCATCA AACTCAAACTACGCCCTGATCGGCGCACTGCGAGCAGTAGCCCAAACAATCT CATATGAAGTCACCCTAGCCATCATTCTACTATCAACATTACTAATGAGTGGC TCCTTTAACCTCTCCACCCTTATCACAACACAAGAACACCTCTGGTTACTCCT GCCATCATGGCCCTTGGCCATGATGTGGTTTATCTCCACACTAGCAGAGACC AACCGAACCCCCTTCGACCTTGCCGAAGGGGAGTCCGAACTAGTCTCAGGCT TCAACATCGAATACGCCGCAGGCCCCTTCGCCCTATTCTTCATGGCCGAATAC ACAAACATTATTATGATGAACACCCTCACCACTACAATCTTCCTAGGAACAA CATATGACGCACTCTCCCCTGAACTCTACACAACATATTTTGTCACCAAGACC CTACTTCTAACCTCCCTGTTCTTATGGATTCGAACAGCATACCCCCGATTCCG CTACGACCAACTCATGCACCTCCTATGGAAAAACTTCCTACCACTCACCCTA GCATTACTTATGTGGTATGTCTCCATGCCCATTACAATCTCCAGCATTCCCCC TCAAACCTAAGAGCACTGGGACGCCCACCGCCCCTTTCCCTCCGCTGCCAGG CGAGCATGTTGTGGTAATTCTGGAACACAAGAAGAGAAATTGCTGGGTTTAG AACAAGATTATAAACGAATTCGGTGCTCAGTGATCACTTGACAGTTTTTTTT TTTTTTAAATATTACCCAAAATGCTCCCCAAATAAGAAATGCATCAGCTCAGT CAGTGAATACAAAAAAGGAATTATTTTTCCCTTTGAGGGTCTTTTATACATCT CTCCTCCAACCCCACCCTCTATTCTGTTTCTTCCTCCTCACATGGGGGTACAC ATACACAGCTTCCTCTTTTGGTTCCATCCTTACCACCACACCACACGCACACT CCACATGCCCAGCAGAGTGGCACTTGGTGGCCAGAAAGTGTGAGCCTCATGA TCTGCTGTCTGTAGTTCTGTGAGCTCAGGTCCCTCAAAGGCCTCGGAGCACCC CCTTCCTTGTGACTGAGCCAGGGCCTGCATTTTTGGTTTTCCCCACCCCACAC ATTCTCAACCATAGTCCTTCTAACAATACCAATAGCTAGGACCCGGCTGCTGT GCACTGGGACTGGGGATTCCACATGTTTGCCTTGGGAGTCTCAAGCTGGACT GCCAGCCCCTGTCCTCCCTTCACCCCCATTGCGTATGAGCATTTCAGAACTCC AAGGAGTCACAGGCATCTTTATAGTTCACGTTAACATATAGACACTGTTGGA AGCAGTTCCTTCTAAAAGGGTAGCCCTGGACTTAATACCAGCCGGATACCTC TGGCCCCCACCCCATTACTGTACCTCTGGAGTCACTACTGTGGGTCGCCACTC CTCTGCTACACAGCACGGCTTTTTCAAGGCTGTATTGAGAAGGGAAGTTAGG AAGAAGGGTGTGCTGGGCTAACCAGCCCACAGAGCTCACATTCCTGTCCCTT GGGTGAAAAATACATGTCCATCCTGATATCTCCTGAATTCAGAAATTAGCCT CCACATGTGCAATGGCTTTAAGAGCCAGAAGCAGGGTTCTGGGAATTTTGCA AGTTACCTGTGGCCAGGTGTGGTCTCGGTTACCAAATACGGTTACCTGCAGC TTTTTAGTCCTTTGTGCTCCCACGGGTCTACAGAGTCCCATCTGCCCAAAGGT CTTGAAGCTTGACAGGATGTTTTCGATTACTCAGTCTCCCAGGGCACTACTGG |
| | | TCCGTAGGATTCGATTGGTCGGGGTAGGAGAGTTAAACAACATTTAAACAGA GTTCTCTCAAAAATGTCTAAAGGGATTGTAGGTAGATAACATCCAATCACTG TTTGCACTTATCTGAAATCTTCCCTCTTGGCTGCCCCCAGGTATTTACTGTGG AGAACATTGCATAGGAATGTCTGGAAAAAGCTTCTACAACTTGTTACAGCCT TCACATTTGTAGAAGCTTT |
| 82 | OPA1-ND1-3' UTR* | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGGCCAACCTCCTACTCCTCATTGTACCCATTCTAATCGCAATGGCA TTCCTAATGCTTACCGAACGAAAAATTCTAGGCTATATGCAACTACGCAAAG GCCCCAACGTTGTAGGCCCCTACGGGCTACTACAACCCTTCGCTGACGCCAT AAAACTCTTCACCAAAGAGCCCCTAAAACCCGCCACATCTACCATCACCCTC TACATCACCGCCCCGACCTTAGCTCTCACCATCGCTCTTCTACTATGGACCCC CCTCCCCATGCCCAACCCCCTGGTCAACCTCAACCTAGGCCTCCTATTTATTC TAGCCACCTCTAGCCTAGCCGTTTACTCAATCCTCTGGTCAGGGTGGGCATCA AACTCAAACTACGCCCTGATCGGCGCACTGCGAGCAGTAGCCCAAACAATCT CATATGAAGTCACCCTAGCCATCATTCTACTATCAACATTACTAATGAGTGGC TCCTTTAACCTCTCCACCCTTATCACAACACAAGAACACCTCTGGTTACTCCT GCCATCATGGCCCTTGGCCATGATGTGGTTTATCTCCACACTAGCAGAGACC AACCGAACCCCCTTCGACCTTGCCGAAGGGGAGTCCGAACTAGTCTCAGGCT TCAACATCGAATACGCCGCAGGCCCCTTCGCCCTATTCTTCATGGCCGAATAC ACAAACATTATTATGATGAACACCCTCACCACTACAATCTTCCTAGGAACAA CATATGACGCACTCTCCCCTGAACTCTACACAACATATTTTGTCACCAAGACC CTACTTCTAACCTCCCTGTTCTTATGGATTCGAACAGCATACCCCCGATTCCG CTACGACCAACTCATGCACCTCCTATGGAAAAACTTCCTACCACTCACCCTA GCATTACTTATGTGGTATGTCTCCATGCCCATTACAATCTCCAGCATTCCCCC TCAAACCTAAGAGCACTGGGACGCCCACCGCCCCTTTCCCTCCGCTGCCAGG CGAGCATGTTGTGGTAATTCTGGAACACAAGAAGAGAAATTGCTGGGTTTAG AACAAGATTATAAACGAATTCGGTGCTCAGTGATCACTTGACAGTTTTTTTT TTTTTTAAATATTACCCAAAATGCTCCCCAAATAAGAAATGCATCAGCTCAGT CAGTGAATACAAAAAAGGAATTATTTTTCCCTTTGAGGGTCTTTTATACATCT CTCCTCCAACCCCACCCTCTATTCTGTTTCTTCCTCCTCACATGGGGGTACAC ATACACAGCTTCCTCTTTTGGTTCCATCCTTACCACCACACCACACGCACACT CCACATGCCCAGCAGAGTGGCACTTGGTGGCCAGAAAGTGTGAGCCTCATGA TCTGCTGTCTGTAGTTCTGTGAGCTCAGGTCCCTCAAAGGCCTCGGAGCACCC CCTTCCTTGTGACTGAGCCAGGGCCTGCATTTTTGGTTTTCCCCACCCCACAC ATTCTCAACCATAGTCCTTCTAACAATACCAATAGCTAGGACCCGGCTGCTGT GCACTGGGACTGGGGATTCCACATGTTTGCCTTGGGAGTCTCAAGCTGGACT |
| | | GCCA |
| 83 | OPA1-opt_ND 1-3'UTR | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGGCCAACCTGCTGCTGCTGATCGTGCCCATCCTGATCGCCATGGCC TTCCTGATGCTGACCGAGCGCAAGATCCTGGGCTACATGCAGCTGCGCAAGG GCCCCAACGTGGTGGGCCCCTACGGCCTGCTGCAGCCCTTCGCCGACGCCAT CAAGCTGTTCACCAAGGAGCCCCTGAAGCCCGCCACCAGCACCATCACCCTG TACATCACCGCCCCCACCCTGGCCCTGACCATCGCCCTGCTGCTGTGGACCCC CCTGCCCATGCCCAACCCCCTGGTGAACCTGAACCTGGGCCTGCTGTTCATCC TGGCCACCAGCAGCCTGGCCGTGTACAGCATCCTGTGGAGCGGCTGGGCCAG CAACAGCAACTACGCCCTGATCGGCGCCCTGCGCGCCGTGGCCCAGACCATC AGCTACGAGGTGACCCTGGCCATCATCCTGCTGAGCACCCTGCTGATGAGCG GCAGCTTCAACCTGAGCACCCTGATCACCACCCAGGAGCACCTGTGGCTGCT GCTGCCCAGCTGGCCCCTGGCCATGATGTGGTTCATCAGCACCCTGGCCGAG ACCAACCGCACCCCCTTCGACCTGGCCGAGGGCGAGAGCGAGCTGGTGAGC GGCTTCAACATCGAGTACGCCGCCGGCCCCTTCGCCCTGTTCTTCATGGCCGA GTACACCAACATCATCATGATGAACACCCTGACCACCACCATCTTCCTGGGC ACCACCTACGACGCCCTGAGCCCCGAGCTGTACACCACCTACTTCGTGACCA AGACCCTGCTGCTGACCAGCCTGTTCCTGTGGATCCGCACCGCCTACCCCCGC TTCCGCTACGACCAGCTGATGCACCTGCTGTGGAAGAACTTCCTGCCCCTGA CCCTGGCCCTGCTGATGTGGTACGTGAGCATGCCCATCACCATCAGCAGCAT CCCCCCCCAGACCTAAGAGCACTGGGACGCCCACCGCCCCTTTCCCTCCGCT GCCAGGCGAGCATGTTGTGGTAATTCTGGAACACAAGAAGAGAAATTGCTG GGTTTAGAACAAGATTATAAACGAATTCGGTGCTCAGTGATCACTTGACAGT TTTTTTTTTTTTTAAATATTACCCAAAATGCTCCCCAAATAAGAAATGCATCA GCTCAGTCAGTGAATACAAAAAAGGAATTATTTTTCCCTTTGAGGGTCTTTTA TACATCTCTCCTCCAACCCCACCCTCTATTCTGTTTCTTCCTCCTCACATGGGG GTACACATACACAGCTTCCTCTTTTGGTTCCATCCTTACCACCACACCACACG CACACTCCACATGCCCAGCAGAGTGGCACTTGGTGGCCAGAAAGTGTGAGCC TCATGATCTGCTGTCTGTAGTTCTGTGAGCTCAGGTCCCTCAAAGGCCTCGGA GCACCCCCTTCCTTGTGACTGAGCCAGGGCCTGCATTTTTGGTTTTCCCCACC CCACACATTCTCAACCATAGTCCTTCTAACAATACCAATAGCTAGGACCCGG CTGCTGTGCACTGGGACTGGGGATTCCACATGTTTGCCTTGGGAGTCTCAAG CTGGACTGCCAGCCCCTGTCCTCCCTTCACCCCCATTGCGTATGAGCATTTCA GAACTCCAAGGAGTCACAGGCATCTTTATAGTTCACGTTAACATATAGACAC TGTTGGAAGCAGTTCCTTCTAAAAGGGTAGCCCTGGACTTAATACCAGCCGG ATACCTCTGGCCCCCACCCCATTACTGTACCTCTGGAGTCACTACTGTGGGTC |
| | | GCCACTCCTCTGCTACACAGCACGGCTTTTTCAAGGCTGTATTGAGAAGGGA AGTTAGGAAGAAGGGTGTGCTGGGCTAACCAGCCCACAGAGCTCACATTCCT GTCCCTTGGGTGAAAAATACATGTCCATCCTGATATCTCCTGAATTCAGAAAT TAGCCTCCACATGTGCAATGGCTTTAAGAGCCAGAAGCAGGGTTCTGGGAAT TTTGCAAGTTACCTGTGGCCAGGTGTGGTCTCGGTTACCAAATACGGTTACCT GCAGCTTTTTAGTCCTTTGTGCTCCCACGGGTCTACAGAGTCCCATCTGCCCA AAGGTCTTGAAGCTTGACAGGATGTTTTCGATTACTCAGTCTCCCAGGGCACT ACTGGTCCGTAGGATTCGATTGGTCGGGGTAGGAGAGTTAAACAACATTTAA ACAGAGTTCTCTCAAAAATGTCTAAAGGGATTGTAGGTAGATAACATCCAAT CACTGTTTGCACTTATCTGAAATCTTCCCTCTTGGCTGCCCCCAGGTATTTACT GTGGAGAACATTGCATAGGAATGTCTGGAAAAAGCTTCTACAACTTGTTACA GCCTTCACATTTGTAGAAGCTTT |
| 84 | OPA1-opt_ND 1-3'UTR* | GTGCTGCCCGCCTAGAAAGGGTGAAGTGGTTGTTTCCGTGACGGACTGAGTA CGGGTGCCTGTCAGGCTCTTGCGGAAGTCCATGCGCCATTGGGAGGGCCTCG GCCGCGGCTCTGTGCCCTTGCTGCTGAGGGCCACTTCCTGGGTCATTCCTGGA CCGGGAGCCGGGCTGGGGCTCACACGGGGGCTCCCGCGTGGCCGTCTCGGCG CCTGCGTGACCTCCCCGCCGGCGGGATGTGGCGACTACGTCGGGCCGCTGTG GCCTGATGGCCAACCTGCTGCTGCTGATCGTGCCCATCCTGATCGCCATGGCC TTCCTGATGCTGACCGAGCGCAAGATCCTGGGCTACATGCAGCTGCGCAAGG GCCCCAACGTGGTGGGCCCCTACGGCCTGCTGCAGCCCTTCGCCGACGCCAT CAAGCTGTTCACCAAGGAGCCCCTGAAGCCCGCCACCAGCACCATCACCCTG TACATCACCGCCCCCACCCTGGCCCTGACCATCGCCCTGCTGCTGTGGACCCC CCTGCCCATGCCCAACCCCCTGGTGAACCTGAACCTGGGCCTGCTGTTCATCC TGGCCACCAGCAGCCTGGCCGTGTACAGCATCCTGTGGAGCGGCTGGGCCAG CAACAGCAACTACGCCCTGATCGGCGCCCTGCGCGCCGTGGCCCAGACCATC AGCTACGAGGTGACCCTGGCCATCATCCTGCTGAGCACCCTGCTGATGAGCG GCAGCTTCAACCTGAGCACCCTGATCACCACCCAGGAGCACCTGTGGCTGCT GCTGCCCAGCTGGCCCCTGGCCATGATGTGGTTCATCAGCACCCTGGCCGAG ACCAACCGCACCCCCTTCGACCTGGCCGAGGGCGAGAGCGAGCTGGTGAGC GGCTTCAACATCGAGTACGCCGCCGGCCCCTTCGCCCTGTTCTTCATGGCCGA GTACACCAACATCATCATGATGAACACCCTGACCACCACCATCTTCCTGGGC ACCACCTACGACGCCCTGAGCCCCGAGCTGTACACCACCTACTTCGTGACCA AGACCCTGCTGCTGACCAGCCTGTTCCTGTGGATCCGCACCGCCTACCCCCGC TTCCGCTACGACCAGCTGATGCACCTGCTGTGGAAGAACTTCCTGCCCCTGA CCCTGGCCCTGCTGATGTGGTACGTGAGCATGCCCATCACCATCAGCAGCAT CCCCCCCCAGACCTAAGAGCACTGGGACGCCCACCGCCCCTTTCCCTCCGCT GCCAGGCGAGCATGTTGTGGTAATTCTGGAACACAAGAAGAGAAATTGCTG GGTTTAGAACAAGATTATAAACGAATTCGGTGCTCAGTGATCACTTGACAGT TTTTTTTTTTTTTAAATATTACCCAAAATGCTCCCCAAATAAGAAATGCATCA GCTCAGTCAGTGAATACAAAAAAGGAATTATTTTTCCCTTTGAGGGTCTTTTA |
| | | TACATCTCTCCTCCAACCCCACCCTCTATTCTGTTTCTTCCTCCTCACATGGGG GTACACATACACAGCTTCCTCTTTTGGTTCCATCCTTACCACCACACCACACG CACACTCCACATGCCCAGCAGAGTGGCACTTGGTGGCCAGAAAGTGTGAGCC TCATGATCTGCTGTCTGTAGTTCTGTGAGCTCAGGTCCCTCAAAGGCCTCGGA GCACCCCCTTCCTTGTGACTGAGCCAGGGCCTGCATTTTTGGTTTTCCCCACC CCACACATTCTCAACCATAGTCCTTCTAACAATACCAATAGCTAGGACCCGG CTGCTGTGCACTGGGACTGGGGATTCCACATGTTTGCCTTGGGAGTCTCAAG CTGGACTGCCA |
| 85 | β-actin-S primer | CGAGATCGTGCGGGACAT |
| 86 | β-actin-A primer | CAGGAAGGAGGGCTGGAAC |
| 87 | ND4-S primer | CTGCCTACGACAAACAGAC |
| 88 | ND4-A primer | AGTGCGTTCGTAGTTTGAG |
| 89 | ND6-F primer | ATGATGTATGCTTTGTTTCTG |
| 90 | ND6-R primer | CTAATTCCCCCGAGCAATCTC |
| 91 | ND6-S primer | AGTGTGGGTTTAGTAATG |
| 92 | ND6-A primer | TGCCTCAGGATACTCCTC |
| 93 | β-actin-F primer | CTCCATCCTGGCCTCGCTGT |
| 94 | β-actin-R primer | GCTGTCACCTTCACCGTTCC |
| 95 | ND6-F primer | GGGTTTTCTTCTAAGCCTTCTCC |
| 96 | ND6-R primer | CCATCATACTCTTTCACCCACAG |
| 97 | opt_ND6-F primer | CGCCTGCTGACCGGCTGCGT |
| 98 | opt_ND6-R | CCAGGCCTCGGGGTACTCCT |
| 99 | ND1-F primer | ATGGCCGCATCTCCGCACACT |
| 100 | ND1-R primer | TTAGGTTTGAGGGGGAATGCT |
| 101 | ND1-F primer | AACCTCAACCTAGGCCTCCTA |
| 102 | ND1-R primer | TGGCAGGAGTAACCAGAGGTG |
| 103 | ND1-F primer | AGGAGGCTCTGTCTGGTATCTTG |
| 104 | ND1-R primer | TTTTAGGGGCTCTTTGGTGAA |
| 105 | opt-ND1-F primer | GCCGCCTGCTGACCGGCTGCGT |
| 106 | opt-ND1-R | TGATGTACAGGGTGATGGTGCTGG |
| | primer | |
| 107 | ND4-S primer | GCCAACAGCAACTACGAGC |
| 108 | ND4-A primer | TGATGTTGCTCCAGCTGAAG |
| 109 | opt-ND4-S primer | GCCTGACCCTGATCCTGAAC |
| 110 | opt-ND4-A primer | GTGCGCTCGTAGTTGCTGTT |
| 111 | hsACO2 | GGGCAGTGCCTCCCCGCCCCGCCGCTGGCGTCAAGTTCAGCTCCACGTGTGC CATCAGTGGATCCGATCCGTCCAGCCATGGCTTCCTATTCCAAGATGGTGTG ACCAGACATGCTTCCTGCTCCCCGCTTAGCCCACGGAGTGACTGTGGTTGTG GTGGGGGGGTTCTTAAAATAACTTTTTAGCCCCCGTCTTCCTATTTTGAGTTT GGTTCAGATCTTAAGCAGCTCCATGCAACTGTATTTATTTTTGATGACAAGAC TCCCATCTAAAGTTTTTCTCCTGCCTGATCATTTCATTGGTGGCTGAAGGATT CTAGAGAACCTTTTGTTCTTGCAAGGAAAACAAGAATCCAAAACCAGTGACT GTTCTGTGA |
| 112 | hsATP5B | GGGGTCTTTGTCCTCTGTACTGTCTCTCTCCTTGCCCCTAACCCAAAAAGCTT CATTTTTCTGTGTAGGCTGCACAAGAGCCTTGATTGAAGATATATTCTTTCTG AACAGTATTTAAGGTTTCCAATAAAATGTACACCCCTCAG |
| 113 | hsAK2 | TGTTGGGTCCAAGAAGGAATTTCTTTCCATCCCTGTGAGGCAATGGGTGGGA ATGATAGGACAGGCAAAGAGAAGCTTCCTCAGGCTAGCAAAAATATCATTTG ATGTATTGATTAAAAAAGCACTTGCTTGATGTATCTTTGGCGTGTGTGCTACT CTCATCTGTGTGTATGTGTGTTGTGTGTGTGTGTGTGTGCATGCACATATGTG TTCACTCTGCTACTTTGTAAGTTTTAGGCTAGGTTGCTTTACCAGCTGTTTACT TCTTTTTTGTTGTTGTTTTGAGACAAGGTTTCGCTCTGCCACCCTGGCTGGAGT GCAGTGGCGTGATCTTGGCTCACGGCAACCTCTGCCTCCTGGGGCTCAAGCA ATTATCCCACCTCAGCCTCCTGAGCAGCTGGGACTACAGGTGCATGCCACAA CACCTGGCTGATATTTGTATTTTTTGTAGAGACAGGATTTTGCCAAGTTGCCC AGGCTGGTCTTGAACTCCTAGGCTTAAGCAATCCACCCACCTTGGCCTCCTGA AGTGCCAGGATCACAGACGTGAGCCACTACACCCAGCCCAGCTGTTTACTTC TTTAACCATACTTTTGATTTTATTTTTTGACCAAAATGAACTAACCCAGGTAA TCTTCCAGGGACCGCAATTCCAGAACCTCATAGTATTTCTTCCATTTCCAGCA GCTGATTAGAAGTCCAGGATCATGTGAAGTCAGGCAGGGTCACAGTTCCTGA TGGCACATTATGGACAGAGAATTCCATTTTGTTTTCTAACCCATGATGAAAAC CCACGTGAGTCAGTGTGTGAACAGGGATCATTAATTTTTTCCCCCTAGGTGG AAGGAAAAAGGCACTTACTTTGCAGGTTACAGAAATTACTGGGAGAGGATA TCGTCATAAAAAGAGCCAGGCCAAATTGGAATATTTTTGTGATCTGCATCAT GATGCTGAAAATAGCAATTATTTGGGAATTGGGTTTGAAAACTGAATTGTTG CCAGAGAATTAAACCAGGTGAAAGGTCCTTTTGAATTCAGATTGTCTTCTGA ACATCCAGGCTGATCATCTGAGAGCAGTCAAATCTACTTCCCCAAAAAGAGA |
| | | CCAGGGTAGGTTTATTTGCTTTTATTTTTAATGTTTGCCTGTGTTTCCAAGTGT GAACAAAACAGTGTGTGATCTATTCTTGGATTCATTTTGATCAGTATTTATTC AAACCCAGTCTCTCTCCAGGACATAAAACTGAAATCAGATATGTTCTTTTTAA GCCCAAACCCTCTCCTTTCTAGATCCAACCCTTCACCCCTAATTTTATGATGG CTATAGCCATGGACTTCCCCAAGAAAAGATCACCCAGAAATAAGACCACCTG TGACAGTTACCAGCTTTTATTCATAACCTTAGCTTCCCAACTATTGAGCATTT TCTAAGGTCCCTGCTGTCTTTTGGTCTCTGGTTTGATTTGTGGCAAACAGATG AAGTAACAGACTGCTATGAAGGACCACAAAAACGGCAGCCTCTGGAAAAAC CATTAGAAAGTCAGTGGCAGATCCAGTAAATAATATCGCCAGCCTCAGCATA ATCTGCTGCTGACTCGATTCAGTGGACTCTAAAGTGCCCAGCCTCCTGACCTG AGCTCTCCTGCCATCTGTGAGACTACCAGAGGTCTTATCTGCTGTCCACATGG CAACTGGGCATGAGTACCTGGCCACCTTGCTTCCCTCTTTGCCTGGTCCAAGT GAGTGTCTGCTGCCTCTGTCCTGCCTTGTTTTCCTGGCTCTAAACCAACTCCA CCCACTCTTAATGGAAACTCAGTCTGGCTTTGTGTGTTTCTGGGAAGCACATG ACTTCTGGGAATGGGCAAGGAAGAGGAGTGAAACAAAAACTGTCAGCTATG TGTGCCTGGTCTGGGATCCTTCTCTGGGTGACAGTGGCATCATGAATCTTAGA ATCAGCTCCCC |
| 114 | hsALDH2 | GAATCATGCAAGCTTCCTCCCTCAGCCATTGATGGAAAGTTCAGCAAGATCA GCAACAAAACCAAGAAAAATGATCCTTGCGTGCTGAATATCTGAAAAGAGA AATTTTTCCTACAAAATCTCTTGGGTCAAGAAAGTTCTAGAATTTGAATTGAT AAACATGGTGGGTTGGCTGAGGGTAAGAGTATATGAGGAACCTTTTAAACGA CAACAATACTGCTAGCTTTCAGGATGATTTTTAAAAAATAGATTCAAATGTGT TATCCTCTCTCTGAAACGCTTCCTATAACTCGAGTTTATAGGGGAAGAAAAA GCTATTGTTTACAATTATATCACCATTAAGGCAACTGCTACACCCTGCTTTGT ATTCTGGGCTAAGATTCATTAAAAACTAGCTGCTCTTAACTTACA |
| 115 | hsCOX10 | GAGCACTGGGACGCCCACCGCCCCTTTCCCTCCGCTGCCAGGCGAGCATGTT GTGGTAATTCTGGAACACAAGAAGAGAAATTGCTGGGTTTAGAACAAGATTA TAAACGAATTCGGTGCTCAGTGATCACTTGACAGTTTTTTTTTTTTTTAAATAT TACCCAAAATGCTCCCCAAATAAGAAATGCATCAGCTCAGTCAGTGAATACA AAAAAGGAATTATTTTTCCCTTTGAGGGTCTTTATACATCTCTCCTCCAACCC CACCCTCTATTCTGTTTCTTCCTCCTCACATGGGGGTACACATACACAGCTTC CTCTTTTGGTTCCATCCTTACCACCACACCACACGCACACTCCACATGCCCAG CAGAGTGGCACTTGGTGGCCAGAAAGTGTGAGCCTCATGATCTGCTGTCTGT AGTTCTGTGAGCTCAGGTCCCTCAAAGGCCTCGGAGCACCCCCTTCCTGGTG ACTGAGCCAGGGCCTGCATTTTTGGTTTTCCCCACCCCACACATTCTCAACCA TAGTCCTTCTAACAATACCAATAGCTAGGACCCGGCTGCTGTGCACTGGGAC TGGGGATTCCACATGTTTGCCTTGGGAGTCTCAAGCTGGACTGCCAGCCCCT GTCCTCCCTTCACCCCCATTGCGTATGAGCATTTCAGAACTCCAAGGAGTCAC AGGCATCTTTATAGTTCACGTTAACATATAGACACTGTTGGAAGCAGTTCCTT |
| | | CTAAAAGGGTAGCCCTGGACTTAATACCAGCCGGATACCTCTGGCCCCCACC CCATTACTGTACCTCTGGAGTCACTACTGTGGGTCGCCACTCCTCTGCTACAC AGCACGGCTTTTTCAAGGCTGTATTGAGAAGGGAAGTTAGGAAGAAGGGTGT GCTGGGCTAACCAGCCCACAGAGCTCACATTCCTGTCCCTTGGGTGAAAAAT ACATGTCCATCCTGATATCTCCTGAATTCAGAAATTAGCCTCCACATGTGCAA TGGCTTTAAGAGCCAGAAGCAGGGTTCTGGGAATTTTGCAAGTTATCCTGTG GCCAGGTGTGGTCTCGGTTACCAAATACGGTTACCTGCAGCTTTTTAGTCCTT TGTGCTCCCACGGGTCTGCAGAGTCCCATCTGCCCAAAGGTCTTGAAGCTTG ACAGGATGTTTTCATTACTCAGTCTCCCAGGGCACTGCTGGTCCGTAGGGATT CATTGGTCGGGGTGGGAGAGTTAAACAACATTTAAACAGAGTTCTCTCAAAA ATGTCTAAAGGGATTGTAGGTAGATAACATCCAATCACTGTTTGCACTTATCT GAAATCTTCCCTCTTGGCTGCCCCCAGGTATTTACTGTGGAGAACATTGCATA GGAATGTCTGGAAAAAGCCTCTACAACTTGTTACAGCCTTCACATTTGTACA ATTCATTGATTCTCTTTTCCTTCCACAATAAAATGGTATACAAGAAC |
| 116 | hsUQCRFS1 | GAGACTTGGACTCAAGTCATAGGCTTCTTTCAGTCTTTATGTCACCTCAGGAG ACTTATTTGAGAGGAAGCCTTCTGTACTTGAAGTTGATTTGAAATATGTAAGA ATTGATGATGTATTTGCAAACATTAATGTGAAATAAATTGAATTTAATGTTGA ATACTTTCAGGCATTCACTTAATAAAGACACTGTTAAGCACTGTTATGCTCAG TCATACACGCGAAAGGTACAATGTCTTTTAGCTAATTCTAATTAAAAATTAC AGACTGGTGTACAAGATACTTGTG |
| 117 | hsNDUFV1 | CCCACCACCCTGGCCTGCTGTCCTGCGTCTATCCATGTGGAATGCTGGACAAT AAAGCGAGTGCTGCCCACCCTCCAGCTGCC |
| 118 | hsNDUFV2 | TTTATATTGAACTGTAAATATGTCACTAGAGAAATAAAATATGGACTTCCAA TCTACGTAAACTTA |
| 119 | hsSOD2 | ACCACGATCGTTATGCTGAGTATGTTAAGCTCTTTATGACTGTTTTTGTAGTG GTATAGAGTACTGCAGAATACAGTAAGCTGCTCTATTGTAGCATTTCTTGATG TTGCTTAGTCACTTATTTCATAAACAACTTAATGTTCTGAATAATTTCTTACTA AACATTTTGTTATTGGGCAAGTGATTGAAAATAGTAAATGCTTTGTGTGATTG A |
| 120 | hsCOX6c | TCTTGGAATATAAAGAATTTCTTCAGGTTGAATTACCTAGAAGTTTGTCACTG ACTTGTGTTCCTGAACTATGACACATGAATATGTGGGCTAAGAAATAGTTCC TCTTGATAAATAAACAATTAACAAATACTTTGGACAGTAAGTCTTTCTCAGTT CTTAATGATAATGCAGGGCACTTACTAGCATAAGAATTGGTTTGGGATTTAA CTGTTTATGAAGCTAACTTGATTTCCGTGTTTTGTTAAAATTTCATTGTTCTAG CACATCTTTAACTGTGATAGTT |
| 121 | hsIRP1 | GAGACGTGCACTTGGTCGTGCGCCCAGGGAGGAAGCCGCACCACCAGCCAG CGCAGGCCCTGGTGGAGAGGCCTCCCTGGCTGCCTCTGGGAGGGGTGCTGCC TTGTAGATGGAGCAAGTGAGCACTGAGGGTCTGGTGCCAATCCTGTAGGCAC AAAACCAGAAGTTTCTACATTCTCTATTTTTGTTAATCATCTTCTCTTTTTCCA |
| | | GAATTTGGAAGCTAGAATGGTGGGAATGTCAGTAGTGCCAGAAAGAGAGAA CCAAGCTTGTCTTTAAAGTTACTGATCACAGGACGTTGCTTTTTCACTGTTTC CTATTAATCTTCAGCTGAACACAAGCAAACCTTCTCAGGAGGTGTCTCCTACC CTCTTATTGTTCCTCTTACGCTCTGCTCAATGAAACCTTCCTCTTGAGGGTCAT TTTCCTTTCTGTATTAATTATACCAGTGTTAAGTGACATAGATAAGAACTTTG CACACTTCAAATCAGAGCAGTGATTCTCTCTTCTCTCCCCTTTTCCTTCAGAG TGAATCATCCAGACTCCTCATGGATAGGTCGGGTGTTAAAGTTGTTTTGATTA TGTACCTTTTGATAGATCCACATAAAAAGAAATGTGAAGTTTTCTTTTACTAT CTTTTCATTTATCAAGCAGAGACCTTTGTTGGGAGGCGGTTTGGGAGAACAC ATTTCTAATTTGAATGAAATGAAATCTATTTTCAGTG |
| 122 | hsMRPS12 | CAGAAGAAGTGACGGCTGGGGGCACAGTGGGCTGGGCGCCCCTGCAGAACA TGAACCTTCCGCTCCTGGCTGCCACAGGGTCCTCCGATGCTGGCCTTTGCGCC TCTAGAGGCAGCCACTCATGGATTCAAGTCCTGGCTCCGCCTCTTCCATCAGG ACCACT |
| 123 | hsATP5J2 | AGAGGACACACTCTGCACCCCCCCACCCCACGACCTTGGCCCGAGCCCCTCC GTGAGGAA |
| 124 | rnSOD2 | AGCCCTTCCGCCAGGCTGTGTGTCAGGCCCGTGGTGGGTGTTTTGTAGTAGTG TAGAGCATTGCA |
| 125 | hsOXA1L | CTTATGTTCTGTGCGCATTCTGGCAGGAATTCTGTCTCTTCAGAGACTCATCC TCAAAACAAGACTTGACACTGTGTCCTTGCCCCAGTCCTAGGAACTGTGGCA CACAGAGATGTTCATTTTAAAAACGGATTTCATGAAACACTCTTGTACTTATG TTTATAAGAGAGCACTGGGTAGCCAAGTGATCTTCCCATTCACAGAGTTAGT AAACCTCTGTACTACATGCTG |
| 126 | MTS-COX10 | MAASPHTLSSRLLTGCVGGSVWYLERRT |
| 127 | MTS-COX8 | MSVLTRLLLRGLTRLGSAAPVRRARIHSL |
| 128 | MTS-OPA1 | MWRLRRAAVA |
| 129 | hsCOX10 | MAASPHTLSSRLLTGCVGGSVWYLERRT |
| 130 | scRPM2 | MAFKSFIYSKGYHRSAAQKKTATSFFDSSYQYLRQNQGLVNSDPVLHASHLHPH PVVVANVNYNNVDDILHPHDLDSSINNTNNPLTHEELLYNQNVSLRSLKQQQST NYVNNNNNNQHRYY |
| 131 | lcSirt5 | MRKRSLRCHLWSANASLSPRKDEVTSRKESENLVKGKKNKKSHLHLLLFTASKI GTDSVFDVQKSKECCKELGLLFTSLIHSIGSFPFDEEPKAAAVFLPGSLPQLTVLV LAPGSGSCPTGKSTPHLAASGRNAELLRPQNSMIVRQFTCRGTISSHLCAHLRKP HDSRNMARP |
| 132 | tbNDUS7 | MLRRTSFNFTGRAMISRGSPEWSHRLDLKKGKKTTMMHKLGTSKPNNALQYAQ MTL |
| 133 | ncQCR2 | MISRSALSRGSQLALRRPAAAKTAQRGFAAAAASPAASYEPTTIAG |
| 134 | hsATP5G2 | MPELILYVAITLSVAERLVGPGHACAEPSFRSSRCSAPLCLLCSGSSSPATAPHPL |
| | | KMFACSKFVSTPSLVKSTSQLLSRPLSAVVLKRPEILTDESLSSLAVSCPLTSLVSS RSFQTSAISRDIDTA |
| 135 | hsLACTB | MYRLMSAVTARAAAPGGLASSCGRRGVHQRAGLPPLGHGWVGGLGLGLGLAL GVKLAGGLRGAAPAQSPAAPDPEASPLAEPPQEQSLAPWSPQTPAPPCSRCFARA IESSRDLL |
| 136 | spilv1 | MTVLAPLRRLHTRAAFSSYGREIALQKRFLNLNSCSAVRRYGTGFSNNLRIKKLK NAFGVVRANSTKSTSTVTTASPIKYDSSFVGKTGGEIFHDMMLKHNVKHVFGYP GGAILPVFDAIYRSPHFEFILPRHEQAAGHA |
| 137 | gmCOX2 | MILCPLEAFIVQHILTISVMGLLSCFRSTVLRKCSKGSSGMSRFLYTNNFQRNLISS GGNESYYGYFNRRSYTSLYMGTGTVGGITSARIRVPNVGCEGFMCSSHLSITQRN SRLIHSTSKIVPN |
| 138 | crATP6 | MALQQAAPRVFGLLGRAPVALGQSGILTGSSGFKNQGFNGSLQSVENHVYAQA FSTSSQEEQAAPSIQGASGMKLPGMAGSMLLGKSRSGLRTGSMVPFAAQQAMN M |
| 139 | hsOPA1 | MWRLRRAAVACEVCQSLVKHSSGIKGSLPLQKLHLVSRSIYHSHHPTLKLQRPQ LRTSFQQFSSLTNLPLRKLKFSPIKYGYQPRRN |
| 140 | hsSDHD | MAVLWRLSAVCGALGGRALLLRTPVVRPAHISAFLQDRPIPEWCGVQHIHLSPS HH |
| 141 | hsADCK3 | MAAILGDTIMVAKGLVKLTQAAVETHLQHLGIGGELIMAARALQSTAVEQIGMF LGKVQGQDKHEEYFAENFGGPEGEFHFSVPHAAGASTDFSSASAPDQSAPPSLG HAHSEGPAPAYVASGPFREAGFPGQASSPLGRANGRLFANPRDSFSAMGFQRRF |
| 142 | osP0644B06.2 4-2 | MALLLRHSPKLRRAHAILGCERGTVVRHFSSSTCSSLVKEDTVSSSNLHPEYAKK IGGSDFSHDRQSGKELQNFKVSPQEASRASNFMRASKYGMPITANGVHSLFSCG QVVPSRCF |
| 143 | Neurospora crassa ATP9 (ncATP9) | MASTRVLASRLASQMAASAKVARPAVRVAQVSKRTIQTGSPLQTLKRTQMTSIV NATTRQAFQKRA |
| 144 | hsGHITM | MLAARLVCLRTLPSRVFHPAFTKASPVVKNSITKNQWLLTPSRE |
| 145 | hsNDUFAB1 | MASRVLSAYVSRLPAAFAPLPRVRMLAVARPLSTALCSAGTQTRLGTLQPALVL AQVPGRVTQLCRQY |
| 146 | hsATP5G3 | MFACAKLACTPSLIRAGSRVAYRPISASVLSRPEASRTGEGSTVFNGAQNGVSQL IQREFQTSAISR |
| 147 | crATP6 _hsADCK3 | MALQQAAPRVFGLLGRAPVALGQSGILTGSSGFKNQGFNGSLQSVENHVYAQA FSTSSQEEQAAPSIQGASGMKLPGMAGSMLLGKSRSGLRTGSMVPFAAQQAMN MGGMAAILGDTIMVAKGLVKLTQAAVETHLQHLGIGGELIMAARALQSTAVEQ IGMFLGKVQGQDKHEEYFAENFGGPEGEFHFSVPHAAGASTDFSSASAPDQSAP PSLGHAHSEGPAPAYVASGPFREAGFPGQASSPLGRANGRLFANPRDSFSAMGF QRRFGG |
| 148 | ncATP9_ncAT P9 | MASTRVLASRLASQMAASAKVARPAVRVAQVSKRTIQTGSPLQTLKRTQMTSIV NATTRQAFQKRAMASTRVLASRLASQMAASAKVARPAVRVAQVSKRTIQTGSP LQTLKRTQMTSIVNATTRQAFQKRA |
| 149 | zmLOC 100282 174 | MALLRAAVSELRRRGRGALTPLPALSSLLSSLSPRSPASTRPEPNNPHADRRHVIA LRRCPPLPASAVLAPELLHARGLLPRHWSHASPLSTSSSSSRPADKAQLTWVDK WIPEAARPY |
| 150 | ncATP9_zmL OC 100282174 _spilv1_ncATP 9 | MASTRVLASRLASQMAASAKVARPAVRVAQVSKRTIQTGSPLQTLKRTQMTSIV NATTRQAFQKRAMALLRAAVSELRRRGRGALTPLPALSSLLSSLSPRSPASTRPE PNNPHADRRHVIALRRCPPLPASAVLAPELLHARGLLPRHWSHASPLSTSSSSSRP ADKAQLTWVDKWIPEAARPYMTVLAPLRRLHTRAAFSSYGREIALQKRFLNLNS CSAVRRYGTGFSNNLRIKKLKNAFGVVRANSTKSTSTVTTASPIKYDSSFVGKTG GEIFHDMMLKHNVKHVFGYPGGAILPVFDAIYRSPHFEFILPRHEQAAGHAMAS TRVLASRLASQMAASAKVARPAVRVAQVSKRTIQTGSPLQTLKRTQMTSIVNAT TRQAFQKRA |
| 151 | zmLOC 100282 174_hsADCK3 _crATP6 _hsATP5G3 | MALLRAAVSELRRRGRGALTPLPALSSLLSSLSPRSPASTRPEPNNPHADRRHVIA LRRCPPLPASAVLAPELLHARGLLPRHWSHASPLSTSSSSSRPADKAQLTWVDK WIPEAARPYMAAILGDTIMVAKGLVKLTQAAVETHLQHLGIGGELIMAARALQS TAVEQIGMFLGKVQGQDKHEEYFAENFGGPEGEFHFSVPHAAGASTDFSSASAP DQSAPPSLGHAHSEGPAPAYVASGPFREAGFPGQASSPLGRANGRLFANPRDSFS AMGFQRRFMALQQAAPRVFGLLGRAPVALGQSGILTGSSGFKNQGFNGSLQSVE NHVYAQAFSTSSQEEQAAPSIQGASGMKLPGMAGSMLLGKSRSGLRTGSMVPF AAQQAMNMMFACAKLACTPSLIRAGSRVAYRPISASVLSRPEASRTGEGSTVFN GAQNGVSQLIQREFQTSAISR |
| 152 | zmLOC 100282 174_hsADCK3 _hsATP5G3 | MALLRAAVSELRRRGRGALTPLPALSSLLSSLSPRSPASTRPEPNNPHADRRHVIA LRRCPPLPASAVLAPELLHARGLLPRHWSHASPLSTSSSSSRPADKAQLTWVDK WIPEAARPYMAAILGDTIMVAKGLVKLTQAAVETHLQHLGIGGELIMAARALQS TAVEQIGMFLGKVQGQDKHEEYFAENFGGPEGEFHFSVPHAAGASTDFSSASAP DQSAPPSLGHAHSEGPAPAYVASGPFREAGFPGQASSPLGRANGRLFANPRDSFS AMGFQRRFMFACAKLACTPSLIRAGSRVAYRPISASVLSRPEASRTGEGSTVFNG AQNGVSQLIQREFQTSAISR |
| 153 | ncATP9_zmL OC100282174 | MASTRVLASRLASQMAASAKVARPAVRVAQVSKRTIQTGSPLQTLKRTQMTSIV NATTRQAFQKRAMALLRAAVSELRRRGRGALTPLPALSSLLSSLSPRSPASTRPE PNNPHADRRHVIALRRCPPLPASAVLAPELLHARGLLPRHWSHASPLSTSSSSSRP ADKAQLTWVDKWIPEAARPY |
| 154 | hsADCK3_zm LOC 10028217 4_crATP6 _hsATP5G3 | MAAILGDTIMVAKGLVKLTQAAVETHLQHLGIGGELIMAARALQSTAVEQIGMF LGKVQGQDKHEEYFAENFGGPEGEFHFSVPHAAGASTDFSSASAPDQSAPPSLG HAHSEGPAPAYVASGPFREAGFPGQASSPLGRANGRLFANPRDSFSAMGFQRRF MALLRAAVSELRRRGRGALTPLPALSSLLSSLSPRSPASTRPEPNNPHADRRHVIA LRRCPPLPASAVLAPELLHARGLLPRHWSHASPLSTSSSSSRPADKAQLTWVDK |
| | | WIPEAARPYMALQQAAPRVFGLLGRAPVALGQSGILTGSSGFKNQGFNGSLQSV ENHVYAQAFSTSSQEEQAAPSIQGASGMKLPGMAGSMLLGKSRSGLRTGSMVPF AAQQAMNMMFACAKLACTPSLIRAGSRVAYRPISASVLSRPEASRTGEGSTVFN GAQNGVSQLIQREFQTSAISR |
| 155 | crATP6 _hsADCK3_z mLOC 1002821 74_hsATP5G3 | MALQQAAPRVFGLLGRAPVALGQSGILTGSSGFKNQGFNGSLQSVENHVYAQA FSTSSQEEQAAPSIQGASGMKLPGMAGSMLLGKSRSGLRTGSMVPFAAQQAMN MMAAILGDTIMVAKGLVKLTQAAVETHLQHLGIGGELIMAARALQSTAVEQIG MFLGKVQGQDKHEEYFAENFGGPEGEFHFSVPHAAGASTDFSSASAPDQSAPPS LGHAHSEGPAPAYVASGPFREAGFPGQASSPLGRANGRLFANPRDSFSAMGFQR RFMALLRAAVSELRRRGRGALTPLPALSSLLSSLSPRSPASTRPEPNNPHADRRH VIALRRCPPLPASAVLAPELLHARGLLPRHWSHASPLSTSSSSSRPADKAQLTWV DKWIPEAARPYMFACAKLACTPSLIRAGSRVAYRPISASVLSRPEASRTGEGSTV FNGAQNGVSQLIQREFQTSAISR |
| 156 | hsADCK3_zm LOC 10028217 4 | MAAILGDTIMVAKGLVKLTQAAVETHLQHLGIGGELIMAARALQSTAVEQIGMF LGKVQGQDKHEEYFAENFGGPEGEFHFSVPHAAGASTDFSSASAPDQSAPPSLG HAHSEGPAPAYVASGPFREAGFPGQASSPLGRANGRLFANPRDSFSAMGFQRRF GGMALLRAAVSELRRRGRGALTPLPALSSLLSSLSPRSPASTRPEPNNPHADRRH VIALRRCPPLPASAVLAPELLHARGLLPRHWSHASPLSTSSSSSRPADKAQLTWV DKWIPEAARPYGG |
| 157 | hsADCK3_zm LOC 10028217 4_crATP6 | MAAILGDTIMVAKGLVKLTQAAVETHLQHLGIGGELIMAARALQSTAVEQIGMF LGKVQGQDKHEEYFAENFGGPEGEFHFSVPHAAGASTDFSSASAPDQSAPPSLG HAHSEGPAPAYVASGPFREAGFPGQASSPLGRANGRLFANPRDSFSAMGFQRRF GGMALLRAAVSELRRRGRGALTPLPALSSLLSSLSPRSPASTRPEPNNPHADRRH VIALRRCPPLPASAVLAPELLHARGLLPRHWSHASPLSTSSSSSRPADKAQLTWV DKWIPEAARPYGGMALQQAAPRVFGLLGRAPVALGQSGILTGSSGFKNQGFNGS LQSVENHVYAQAFSTSSQEEQAAPSIQGASGMKLPGMAGSMLLGKSRSGLRTGS MVPFAAQQAMNMGG |
| 158 | ncATP9_zmL OC100282174 _spilv1_GNFP _ncATP9 | MASTRVLASRLASQMAASAKVARPAVRVAQVSKRTIQTGSPLQTLKRTQMTSIV NATTRQAFQKRAMALLRAAVSELRRRGRGALTPLPALSSSLLSSLSPRSPASTRPE PNNPHADRRHVIALRRCPPLPASAVLAPELLHARGLLPRHWSHASPLSTSSSSSRP ADKAQLTWVDKWIPEAARPYMTVLAPLRRLHTRAAFSSYGREIALQKRFLNLNS CSAVRRYGTGFSNNLRIKKLKNAFGVVRANSTKSTSTVTTASPIKYDSSFVGKTG GEIFHDMMLKHNVKHVFGYPGGAILPVFDAIYRSPHFEFILPRHEQAAGHAVSGE GDATYGKLTLKFICTTGKLPVPWPTLVTTLTYGVQCFSRYPDHMKQHDFFKSA MPEGYVQERTIFFKDDGNYKTRAEVKFEGDTLVNRIELKGIDFKEDGNILGHKLE YNYNSHNVYIMADKQKNGIKVNFKIRHNIEDGSVQLADHYQQNTPIGDGPVLLP DNHYLSTQSALSKDPNEMASTRVLASRLASQMAASAKVARPAVRVAQVSKRTI QTGSPLQTLKRTQMTSIVNATTRQAFQKRA |
| 159 | ncATP9_zmL | MASTRVLASRLASQMAASAKVARPAVRVAQVSKRTIQTGSPLQTLKRTQMTSIV |
| | OC 100282174 _spilv1_lcSirt5 _osP0644B06. 24-2_hsATP5 G2_ncATP9 | NATTRQAFQKRAMALLRAAVSELRRRGRGALTPLPALSSLLSSLSPRSPASTRPE PNNPHADRRHVIALRRCPPLPASAVLAPELLHARGLLPRHWSHASPLSTSSSSSRP ADKAQLTWVDKWIPEAARPYMTVLAPLRRLHTRAAFSSYGREIALQKRFLNLNS CSAVRRYGTGFSNNLRIKKLKNAFGVVRANSTKSTSTVTTASPIKYDSSFVGKTG GEIFHDMMLKHNVKHVFGYPGGAILPVFDAIYRSPHFEFILPRHEQAAGHAMRK RSLRCHLWSANASLSPRKDEVTSRKESENLVKGKKNKKSHLHLLLFTASKIGTD SVFDVQKSKECCKELGLLFTSLIHSIGSFPFDEEPKAAAVFLPGSLPQLTVLVLAP GSGSCPTGKSTPHLAASGRNAELLRPQNSMIVRQFTCRGTISSHLCAHLRKPHDS RNMARPMALLLRHSPKLRRAHAILGCERGTVVRHFSSSTCSSLVKEDTVSSSNLH PEYAKKIGGSDFSHDRQSGKELQNFKVSPQEASRASNFMRASKYGMPITANGVH SLFSCGQVVPSRCFMPELILYVAITLSVAERLVGPGHACAEPSFRSSRCSAPLCLL CSGSSSPATAPHPLKMFACSKFVSTPSLVKSTSQLLSRPLSAVVLKRPEILTDESLS SLAVSCPLTSLVSSRSFQTSAISRDIDTAMASTRVLASRLASQMAASAKVARPAV RVAQVSKRTIQTGSPLQTLKRTQMTSIVNATTRQAFQKRA |
| 160 | ND4 | MLKLIVPTIMLLPLTWLSKKHMIWINTTTHSLIISIIPLLFFNQINNNLFSCSPTFSSD PLTTPLLMLTTWLLPLTIMASQRHLSSEPLSRKKLYLSMLISLQISLIMTFTATELI MFYIFFETTLIPTLAIITRWGNQPERLNAGTYFLFYTLVGSLPLLIALIYTHNTLGSL NILLLTLTAQELSNSWANNLMWLAYTMAFMVKMPLYGLHLWLPKAHVEAPIA GSMVLAAVLLKLGGYGMMRLTLILNPLTKHMAYPFLVLSLWGMIMTSSICLRQ TDLKSLIAYSSISHMALVVTAILIQTPWSFTGAVILMIAHGLTSSLLFCLANSNYER THSRIMILSQGLQTLLPLMAFWWLLASLANLALPPTINLLGELSVLVTTFSWSNIT LLLTGLNMLVTALYSLYMFTTTQWGSLTHHINNMKPSFTRENTLMFMHLSPILL LSLNPDIITGFSS |
| 161 | ND6 | MMYALFLLSVGLVMGFVGFSSKPSPIYGGLVLIVSGVVGCVIILNFGGGYMGLM VFLIYLGGMMVVFGYTTAMAIEEYPEAWGSGVEVLVSVLVGLAMEVGLVLWV KEYDGVVVVVNFNSVGSWMIYEGEGSGLIREDPIGAGALYDYGRWLVVVTGW TLFVGVYIVIEIARGN |
| 162 | ND1 | MANLLLLIVPILIAMAFLMLTERKILGYMQLRKGPNVVGPYGLLQPFADAIKLFT KEPLKPATSTITLYITAPTLALTIALLLWTPLPMPNPLVNLNLGLLFILATSSLAVY SILWSGWASNSNYALIGALRAVAQTISYEVTLAIILLSTLLMSGSFNLSTLITTQEH LWLLLPSWPLAMMWFISTLAETNRTPFDLAEGESELVSGFNIEYAAGPFALFFMA EYTNIIMMNTLTTTIFLGTTYDALSPELYTTYFVTKTLLLTSLFLWIRTAYPRFRY DQLMHLLWKNFLPLTLALLMWYVSMPITISSIPPQT |
| 163 | GFP-F | ACAAGTTCAGCGTGTCCG |
| 164 | GFP-R | CTCGTTGGGGTCTTTGCT |
| 165 | ND4-F | ATCTCCGCACACTCTCTCCTCA |
| 166 | ND4-R | TAGGTTGTTGTTGATTTGGTT |
| 167 | B-actin-F2 | CCTAGAAGCATTTGCGGT |
| 168 | B-actin-R2 | GAGCTACGAGCTGCCTGA |
| 169 | CMV promoter | CGTTACATAACTTACGGTAAATGGCCCGCCTGGCTGACCGCCCAACGACCCC CGCCCATTGACGTCAATAATGACGTATGTTCCCATAGTAACGCCAATAGGGA CTTTCCATTGACGTCAATGGGTGGAGTATTTACGGTAAACTGCCCACTTGGCA GTACATCAAGTGTATCATATGCCAAGTACGCCCCCTATTGACGTCAATGACG GTAAATGGCCCGCCTGGCATTATGCCCAGTACATGACCTTATGGGACTTTCCT ACTTGGCAGTACATCTACGTATTAGTCATCGCTATTACCATGGTGATGCGGTT TTGGCAGTACATCAATGGGCGTGGATAGCGGTTTGACTCACGGGGATTTCCA AGTCTCCACCCCATTGACGTCAATGGGAGTTTGTTTTGCACCAAAATCAACG GGACTTTCCAAAATGTCGTAACAACTCCGCCCCATTGACGCAAATGGGCGGT AGGCGTGTACGGTGGGAGGTCTATATAAGCAGAGCT |
| 170 | Chimeric intron | GTAAGTATCAAGGTTACAAGACAGGTTTAAGGAGACCAATAGAAACTGGGC TTGTCGAGACAGAGAAGACTCTTGCGTTTCTGATAGGCACCTATTGGTCTTAC TGACATCCACTTTGCCTTTCTCTCCACAG |
| 171 | Kozak | GCCACC |
| 172 | bGH polyA tail | CTGTGCCTTCTAGTTGCCAGCCATCTGTTGTTTGCCCCTCCCCCGTGCCTTCCT TGACCCTGGAAGGTGCCACTCCCACTGTCCTTTCCTAATAAAATGAGGAAAT TGCATCGCATTGTCTGAGTAGGTGTCATTCTATTCTGGGGGGTGGGGTGGGG CAGGACAGCAAGGGGGAGGATTGGGAAGAGAATAGCAGGCATGCTGGGGA |
| 173 | SV40 polyA tail | TAAGATACATTGATGAGTTTGGACAAACCACAACTAGAATGCAGTGAAAAA AATGCTTTATTTGTGAAATTTGTGATGCTATTGCTTTATTTGTAACCATTATAA GCTGCAATAAACAAGTT |
| 174 | CMV-Kozak-MTS-ND4-3'U TR (no polyA tail) | CGTTACATAACTTACGGTAAATGGCCCGCCTGGCTGACCGCCCAACGACCCC CGCCCATTGACGTCAATAATGACGTATGTTCCCATAGTAACGCCAATAGGGA CTTTCCATTGACGTCAATGGGTGGAGTATTTACGGTAAACTGCCCACTTGGCA GTACATCAAGTGTATCATATGCCAAGTACGCCCCCTATTGACGTCAATGACG GTAAATGGCCCGCCTGGCATTATGCCCAGTACATGACCTTATGGGACTTTCCT ACTTGGCAGTACATCTACGTATTAGTCATCGCTATTACCATGGTGATGCGGTT TTGGCAGTACATCAATGGGCGTGGATAGCGGTTTGACTCACGGGGATTTCCA AGTCTCCACCCCATTGACGTCAATGGGAGTTTGTTTTGCACCAAAATCAACG GGACTTTCCAAAATGTCGTAACAACTCCGCCCCATTGACGCAAATGGGCGGT AGGCGTGTACGGTGGGAGGTCTATATAAGCAGAGCTCGTTTAGTGAACCGTC AGATCACTAGAAGCTTTATTGCGGTAGTTTATCACAGTTAAATTGCTAACGC AGTCAGTGCTTCTGACACAACAGTCTCGAACTTAAGCTGCAGAAGTTGGTCG TGAGGCACTGGGCAGGTAAGTATCAAGGTTACAAGACAGGTTTAAGGAGAC CAATAGAAACTGGGCTTGTCGAGACAGAGAAGACTCTTGCGTTTCTGATAGG CACCTATTGGTCTTACTGACATCCACTTTGCCTTTCTCTCCACAGGTGTCCACT CCCAGTTCAATTACAGCTCTTAAGGCTAGAGTACTTAATACGACTCACTATA GGCTAGCCTCGAGAATTCACGCGTGGTACCGCCACCGGATCCGCCGCCACCA |
| | | TGGCCGCATCTCCGCACACTCTCTCCTCACGCCTCCTGACAGGTTGCGTAGGA GGCTCTGTCTGGTATCTTGAAAGAAGAACTATGCTAAAACTAATCGTCCCAA CAATTATGTTACTACCACTGACATGGCTTTCCAAAAAACACATGATTTGGATC AACACAACCACCCACAGCCTAATTATTAGCATCATCCCTCTACTATTTTTTAA CCAAATCAACAACAACCTATTTAGCTGTTCCCCAACCTTTTCCTCCGACCCCC TAACAACCCCCCTCCTAATGCTAACTACCTGGCTCCTACCCCTCACAATCATG GCAAGCCAACGCCACTTATCCAGTGAACCACTATCACGAAAAAAACTCTACC TCTCTATGCTAATCTCCCTACAAATCTCCTTAATTATGACATTCACAGCCACA GAACTAATCATGTTTTATATCTTCTTCGAAACCACACTTATCCCCACCTTGGC TATCATCACCCGATGGGGCAACCAGCCAGAACGCCTGAACGCAGGCACATA CTTCCTATTCTACACCCTAGTAGGCTCCCTTCCCCTACTCATCGCACTAATTTA CACTCACAACACCCTAGGCTCACTAAACATTCTACTACTCACTCTCACTGCCC AAGAACTATCAAACTCCTGGGCCAACAACTTAATGTGGCTAGCTTACACAAT GGCTTTTATGGTAAAGATGCCTCTTTACGGACTCCACTTATGGCTCCCTAAAG CCCATGTCGAAGCCCCCATCGCTGGGTCAATGGTACTTGCCGCAGTACTCTTA AAACTAGGCGGCTATGGTATGATGCGCCTCACACTCATTCTCAACCCCCTGA CAAAACACATGGCCTACCCCTTCCTTGTACTATCCCTATGGGGCATGATTATG ACAAGCTCCATCTGCCTACGACAAACAGACCTAAAATCGCTCATTGCATACT CTTCAATCAGCCACATGGCCCTCGTAGTAACAGCCATTCTCATCCAAACCCCC TGGAGCTTCACCGGCGCAGTCATTCTCATGATCGCCCACGGGCTTACATCCTC ATTACTATTCTGCCTAGCAAACTCAAACTACGAACGCACTCACAGTCGCATC ATGATCCTCTCTCAAGGACTTCAAACTCTACTCCCACTAATGGCTTTTTGGTG GCTTCTAGCAAGCCTCGCTAACCTCGCCTTACCCCCCACTATTAACCTACTGG GAGAACTCTCTGTGCTAGTAACCACGTTCTCCTGGTCAAATATCACTCTCCTA CTTACAGGACTCAACATGCTAGTCACAGCCCTATACTCCCTCTACATGTTTAC CACAACACAATGGGGCTCACTCACCCACCACATTAACAACATGAAACCCTCA TTCACACGAGAAAACACCCTCATGTTCATGCACCTATCCCCCATTCTCCTCCT ATCCCTCAACCCCGACATCATTACCGGGTTTTCCTCTTAAGAGCACTGGGACG CCCACCGCCCCTTTCCCTCCGCTGCCAGGCGAGCATGTTGTGGTAATTCTGGA ACACAAGAAGAGAAATTGCTGGGTTTAGAACAAGATTATAAACGAATTCGG TGCTCAGTGATCACTTGACAGTTTTTTTTTTTTTTAAATATTACCCAAAATGCT CCCCAAATAAGAAATGCATCAGCTCAGTCAGTGAATACAAAAAAGGAATTA TTTTTCCCTTTGAGGGTCTTTTATACATCTCTCCTCCAACCCCACCCTCTATTC TGTTTCTTCCTCCTCACATGGGGGTACACATACACAGCTTCCTCTTTTGGTTCC ATCCTTACCACCACACCACACGCACACTCCACATGCCCAGCAGAGTGGCACT TGGTGGCCAGAAAGTGTGAGCCTCATGATCTGCTGTCTGTAGTTCTGTGAGCT CAGGTCCCTCAAAGGCCTCGGAGCACCCCCTTCCTTGTGACTGAGCCAGGGC CTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCAGCCCCTGTCCTCCCTTCACC |
| | | CCCATTGCGTATGAGCATTTCAGAACTCCAAGGAGTCACAGGCATCTTTATA GTTCACGTTAACATATAGACACTGTTGGAAGCAGTTCCTTCTAAAAGGGTAG CCCTGGACTTAATACCAGCCGGATACCTCTGGCCCCCACCCCATTACTGTACC TCTGGAGTCACTACTGTGGGTCGCCACTCCTCTGCTACACAGCACGGCTTTTT CAAGGCTGTATTGAGAAGGGAAGTTAGGAAGAAGGGTGTGCTGGGCTAACC AGCCCACAGAGCTCACATTCCTGTCCCTTGGGTGAAAAATACATGTCCATCC TGATATCTCCTGAATTCAGAAATTAGCCTCCACATGTGCAATGGCTTTAAGA GCCAGAAGCAGGGTTCTGGGAATTTTGCAAGTTACCTGTGGCCAGGTGTGGT CTCGGTTACCAAATACGGTTACCTGCAGCTTTTTAGTCCTTTGTGCTCCCACG GGTCTACAGAGTCCCATCTGCCCAAAGGTCTTGAAGCTTGACAGGATGTTTT CGATTACTCAGTCTCCCAGGGCACTACTGGTCCGTAGGATTCGATTGGTCGG GGTAGGAGAGTTAAACAACATTTAAACAGAGTTCTCTCAAAAATGTCTAAAG GGATTGTAGGTAGATAACATCCAATCACTGTTTGCACTTATCTGAAATCTTCC CTCTTGGCTGCCCCCAGGTATTTACTGTGGAGAACATTGCATAGGAATGTCTG GAAAAAGCTTCTACAACTTGTTACAGCCTTCACATTTGTAGAAGCTTT |
| 175 | CMV-Kozak-MTS-ND4-3'U TR-bGH | CGTTACATAACTTACGGTAAATGGCCCGCCTGGCTGACCGCCCAACGACCCC CGCCCATTGACGTCAATAATGACGTATGTTCCCATAGTAACGCCAATAGGGA CTTTCCATTGACGTCAATGGGTGGAGTATTTACGGTAAACTGCCCACTTGGCA GTACATCAAGTGTATCATATGCCAAGTACGCCCCCTATTGACGTCAATGACG GTAAATGGCCCGCCTGGCATTATGCCCAGTACATGACCTTATGGGACTTTCCT ACTTGGCAGTACATCTACGTATTAGTCATCGCTATTACCATGGTGATGCGGTT TTGGCAGTACATCAATGGGCGTGGATAGCGGTTTGACTCACGGGGATTTCCA AGTCTCCACCCCATTGACGTCAATGGGAGTTTGTTTTGCACCAAAATCAACG GGACTTTCCAAAATGTCGTAACAACTCCGCCCCATTGACGCAAATGGGCGGT AGGCGTGTACGGTGGGAGGTCTATATAAGCAGAGCTCGTTTAGTGAACCGTC AGATCACTAGAAGCTTTATTGCGGTAGTTTATCACAGTTAAATTGCTAACGC AGTCAGTGCTTCTGACACAACAGTCTCGAACTTAAGCTGCAGAAGTTGGTCG TGAGGCACTGGGCAGGTAAGTATCAAGGTTACAAGACAGGTTTAAGGAGAC CAATAGAAACTGGGCTTGTCGAGACAGAGAAGACTCTTGCGTTTCTGATAGG CACCTATTGGTCTTACTGACATCCACTTTGCCTTTCTCTCCACAGGTGTCCACT CCCAGTTCAATTACAGCTCTTAAGGCTAGAGTACTTAATACGACTCACTATA GGCTAGCCTCGAGAATTCACGCGTGGTACCGCCACCGGATCCGCCGCCACCA TGGCCGCATCTCCGCACACTCTCTCCTCACGCCTCCTGACAGGTTGCGTAGGA GGCTCTGTCTGGTATCTTGAAAGAAGAACTATGCTAAAACTAATCGTCCCAA CAATTATGTTACTACCACTGACATGGCTTTCCAAAAAACACATGATTTGGATC AACACAACCACCCACAGCCTAATTATTAGCATCATCCCTCTACTATTTTTTAA CCAAATCAACAACAACCTATTTAGCTGTTCCCCAACCTTTTCCTCCGACCCCC TAACAACCCCCCTCCTAATGCTAACTACCTGGCTCCTACCCCTCACAATCATG GCAAGCCAACGCCACTTATCCAGTGAACCACTATCACGAAAAAAACTCTACC TCTCTATGCTAATCTCCCTACAAATCTCCTTAATTATGACATTCACAGCCACA |
| | | GAACTAATCATGTTTTATATCTTCTTCGAAACCACACTTATCCCCACCTTGGC TATCATCACCCGATGGGGCAACCAGCCAGAACGCCTGAACGCAGGCACATA CTTCCTATTCTACACCCTAGTAGGCTCCCTTCCCCTACTCATCGCACTAATTTA CACTCACAACACCCTAGGCTCACTAAACATTCTACTACTCACTCTCACTGCCC AAGAACTATCAAACTCCTGGGCCAACAACTTAATGTGGCTAGCTTACACAAT GGCTTTTATGGTAAAGATGCCTCTTTACGGACTCCACTTATGGCTCCCTAAAG CCCATGTCGAAGCCCCCATCGCTGGGTCAATGGTACTTGCCGCAGTACTCTTA AAACTAGGCGGCTATGGTATGATGCGCCTCACACTCATTCTCAACCCCCTGA CAAAACACATGGCCTACCCCTTCCTTGTACTATCCCTATGGGGCATGATTATG ACAAGCTCCATCTGCCTACGACAAACAGACCTAAAATCGCTCATTGCATACT CTTCAATCAGCCACATGGCCCTCGTAGTAACAGCCATTCTCATCCAAACCCCC TGGAGCTTCACCGGCGCAGTCATTCTCATGATCGCCCACGGGCTTACATCCTC ATTACTATTCTGCCTAGCAAACTCAAACTACGAACGCACTCACAGTCGCATC ATGATCCTCTCTCAAGGACTTCAAACTCTACTCCCACTAATGGCTTTTTGGTG GCTTCTAGCAAGCCTCGCTAACCTCGCCTTACCCCCCACTATTAACCTACTGG GAGAACTCTCTGTGCTAGTAACCACGTTCTCCTGGTCAAATATCACTCTCCTA CTTACAGGACTCAACATGCTAGTCACAGCCCTATACTCCCTCTACATGTTTAC CACAACACAATGGGGCTCACTCACCCACCACATTAACAACATGAAACCCTCA TTCACACGAGAAAACACCCTCATGTTCATGCACCTATCCCCCATTCTCCTCCT ATCCCTCAACCCCGACATCATTACCGGGTTTTCCTCTTAAGAGCACTGGGACG CCCACCGCCCCTTTCCCTCCGCTGCCAGGCGAGCATGTTGTGGTAATTCTGGA ACACAAGAAGAGAAATTGCTGGGTTTAGAACAAGATTATAAACGAATTCGG TGCTCAGTGATCACTTGACAGTTTTTTTTTTTTTTAAATATTACCCAAAATGCT CCCCAAATAAGAAATGCATCAGCTCAGTCAGTGAATACAAAAAAGGAATTA TTTTTCCCTTTGAGGGTCTTTTATACATCTCTCCTCCAACCCCACCCTCTATTC TGTTTCTTCCTCCTCACATGGGGGTACACATACACAGCTTCCTCTTTTGGTTCC ATCCTTACCACCACACCACACGCACACTCCACATGCCCAGCAGAGTGGCACT TGGTGGCCAGAAAGTGTGAGCCTCATGATCTGCTGTCTGTAGTTCTGTGAGCT CAGGTCCCTCAAAGGCCTCGGAGCACCCCCTTCCTTGTGACTGAGCCAGGGC CTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCAGCCCCTGTCCTCCCTTCACC CCCATTGCGTATGAGCATTTCAGAACTCCAAGGAGTCACAGGCATCTTTATA GTTCACGTTAACATATAGACACTGTTGGAAGCAGTTCCTTCTAAAAGGGTAG CCCTGGACTTAATACCAGCCGGATACCTCTGGCCCCCACCCCATTACTGTACC TCTGGAGTCACTACTGTGGGTCGCCACTCCTCTGCTACACAGCACGGCTTTTT CAAGGCTGTATTGAGAAGGGAAGTTAGGAAGAAGGGTGTGCTGGGCTAACC AGCCCACAGAGCTCACATTCCTGTCCCTTGGGTGAAAAATACATGTCCATCC TGATATCTCCTGAATTCAGAAATTAGCCTCCACATGTGCAATGGCTTTAAGA GCCAGAAGCAGGGTTCTGGGAATTTTGCAAGTTACCTGTGGCCAGGTGTGGT |
| | | CTCGGTTACCAAATACGGTTACCTGCAGCTTTTTAGTCCTTTGTGCTCCCACG GGTCTACAGAGTCCCATCTGCCCAAAGGTCTTGAAGCTTGACAGGATGTTTT CGATTACTCAGTCTCCCAGGGCACTACTGGTCCGTAGGATTCGATTGGTCGG GGTAGGAGAGTTAAACAACATTTAAACAGAGTTCTCTCAAAAATGTCTAAAG GGATTGTAGGTAGATAACATCCAATCACTGTTTGCACTTATCTGAAATCTTCC CTCTTGGCTGCCCCCAGGTATTTACTGTGGAGAACATTGCATAGGAATGTCTG GAAAAAGCTTCTACAACTTGTTACAGCCTTCACATTTGTAGAAGCTTTGCGGC CGCTTCGAGCAGACATGACTGTGCCTTCTAGTTGCCAGCCATCTGTTGTTTGC CCCTCCCCCGTGCCTTCCTTGACCCTGGAAGGTGCCACTCCCACTGTCCTTTC CTAATAAAATGAGGAAATTGCATCGCATTGTCTGAGTAGGTGTCATTCTATTC TGGGGGGTGGGGTGGGGCAGGACAGCAAGGGGGAGGATTGGGAAGAGAAT AGCAGGCATGCTGGGGA |
| 176 | CMV-Kozak-MTS-ND4-3'U TR-SV40 | CGTTACATAACTTACGGTAAATGGCCCGCCTGGCTGACCGCCCAACGACCCC CGCCCATTGACGTCAATAATGACGTATGTTCCCATAGTAACGCCAATAGGGA CTTTCCATTGACGTCAATGGGTGGAGTATTTACGGTAAACTGCCCACTTGGCA GTACATCAAGTGTATCATATGCCAAGTACGCCCCCTATTGACGTCAATGACG GTAAATGGCCCGCCTGGCATTATGCCCAGTACATGACCTTATGGGACTTTCCT ACTTGGCAGTACATCTACGTATTAGTCATCGCTATTACCATGGTGATGCGGTT TTGGCAGTACATCAATGGGCGTGGATAGCGGTTTGACTCACGGGGATTTCCA AGTCTCCACCCCATTGACGTCAATGGGAGTTTGTTTTGCACCAAAATCAACG GGACTTTCCAAAATGTCGTAACAACTCCGCCCCATTGACGCAAATGGGCGGT AGGCGTGTACGGTGGGAGGTCTATATAAGCAGAGCTCGTTTAGTGAACCGTC AGATCACTAGAAGCTTTATTGCGGTAGTTTATCACAGTTAAATTGCTAACGC AGTCAGTGCTTCTGACACAACAGTCTCGAACTTAAGCTGCAGAAGTTGGTCG TGAGGCACTGGGCAGGTAAGTATCAAGGTTACAAGACAGGTTTAAGGAGAC CAATAGAAACTGGGCTTGTCGAGACAGAGAAGACTCTTGCGTTTCTGATAGG CACCTATTGGTCTTACTGACATCCACTTTGCCTTTCTCTCCACAGGTGTCCACT CCCAGTTCAATTACAGCTCTTAAGGCTAGAGTACTTAATACGACTCACTATA GGCTAGCCTCGAGAATTCACGCGTGGTACCGCCACCGGATCCGCCGCCACCA TGGCCGCATCTCCGCACACTCTCTCCTCACGCCTCCTGACAGGTTGCGTAGGA GGCTCTGTCTGGTATCTTGAAAGAAGAACTATGCTAAAACTAATCGTCCCAA CAATTATGTTACTACCACTGACATGGCTTTCCAAAAAACACATGATTTGGATC AACACAACCACCCACAGCCTAATTATTAGCATCATCCCTCTACTATTTTTTAA CCAAATCAACAACAACCTATTTAGCTGTTCCCCAACCTTTTCCTCCGACCCCC TAACAACCCCCCTCCTAATGCTAACTACCTGGCTCCTACCCCTCACAATCATG GCAAGCCAACGCCACTTATCCAGTGAACCACTATCACGAAAAAAACTCTACC TCTCTATGCTAATCTCCCTACAAATCTCCTTAATTATGACATTCACAGCCACA GAACTAATCATGTTTTATATCTTCTTCGAAACCACACTTATCCCCACCTTGGC TATCATCACCCGATGGGGCAACCAGCCAGAACGCCTGAACGCAGGCACATA CTTCCTATTCTACACCCTAGTAGGCTCCCTTCCCCTACTCATCGCACTAATTTA |
| | | CACTCACAACACCCTAGGCTCACTAAACATTCTACTACTCACTCTCACTGCCC AAGAACTATCAAACTCCTGGGCCAACAACTTAATGTGGCTAGCTTACACAAT GGCTTTTATGGTAAAGATGCCTCTTTACGGACTCCACTTATGGCTCCCTAAAG CCCATGTCGAAGCCCCCATCGCTGGGTCAATGGTACTTGCCGCAGTACTCTTA AAACTAGGCGGCTATGGTATGATGCGCCTCACACTCATTCTCAACCCCCTGA CAAAACACATGGCCTACCCCTTCCTTGTACTATCCCTATGGGGCATGATTATG ACAAGCTCCATCTGCCTACGACAAACAGACCTAAAATCGCTCATTGCATACT CTTCAATCAGCCACATGGCCCTCGTAGTAACAGCCATTCTCATCCAAACCCCC TGGAGCTTCACCGGCGCAGTCATTCTCATGATCGCCCACGGGCTTACATCCTC ATTACTATTCTGCCTAGCAAACTCAAACTACGAACGCACTCACAGTCGCATC ATGATCCTCTCTCAAGGACTTCAAACTCTACTCCCACTAATGGCTTTTTGGTG GCTTCTAGCAAGCCTCGCTAACCTCGCCTTACCCCCCACTATTAACCTACTGG GAGAACTCTCTGTGCTAGTAACCACGTTCTCCTGGTCAAATATCACTCTCCTA CTTACAGGACTCAACATGCTAGTCACAGCCCTATACTCCCTCTACATGTTTAC CACAACACAATGGGGCTCACTCACCCACCACATTAACAACATGAAACCCTCA TTCACACGAGAAAACACCCTCATGTTCATGCACCTATCCCCCATTCTCCTCCT ATCCCTCAACCCCGACATCATTACCGGGTTTTCCTCTTAAGAGCACTGGGACG CCCACCGCCCCTTTCCCTCCGCTGCCAGGCGAGCATGTTGTGGTAATTCTGGA ACACAAGAAGAGAAATTGCTGGGTTTAGAACAAGATTATAAACGAATTCGG TGCTCAGTGATCACTTGACAGTTTTTTTTTTTTTTAAATATTACCCAAAATGCT CCCCAAATAAGAAATGCATCAGCTCAGTCAGTGAATACAAAAAAGGAATTA TTTTTCCCTTTGAGGGTCTTTTATACATCTCTCCTCCAACCCCACCCTCTATTC TGTTTCTTCCTCCTCACATGGGGGTACACATACACAGCTTCCTCTTTTGGTTCC ATCCTTACCACCACACCACACGCACACTCCACATGCCCAGCAGAGTGGCACT TGGTGGCCAGAAAGTGTGAGCCTCATGATCTGCTGTCTGTAGTTCTGTGAGCT CAGGTCCCTCAAAGGCCTCGGAGCACCCCCTTCCTTGTGACTGAGCCAGGGC CTGCATTTTTGGTTTTCCCCACCCCACACATTCTCAACCATAGTCCTTCTAAC AATACCAATAGCTAGGACCCGGCTGCTGTGCACTGGGACTGGGGATTCCACA TGTTTGCCTTGGGAGTCTCAAGCTGGACTGCCAGCCCCTGTCCTCCCTTCACC CCCATTGCGTATGAGCATTTCAGAACTCCAAGGAGTCACAGGCATCTTTATA GTTCACGTTAACATATAGACACTGTTGGAAGCAGTTCCTTCTAAAAGGGTAG CCCTGGACTTAATACCAGCCGGATACCTCTGGCCCCCACCCCATTACTGTACC TCTGGAGTCACTACTGTGGGTCGCCACTCCTCTGCTACACAGCACGGCTTTTT CAAGGCTGTATTGAGAAGGGAAGTTAGGAAGAAGGGTGTGCTGGGCTAACC AGCCCACAGAGCTCACATTCCTGTCCCTTGGGTGAAAAATACATGTCCATCC TGATATCTCCTGAATTCAGAAATTAGCCTCCACATGTGCAATGGCTTTAAGA GCCAGAAGCAGGGTTCTGGGAATTTTGCAAGTTACCTGTGGCCAGGTGTGGT CTCGGTTACCAAATACGGTTACCTGCAGCTTTTTAGTCCTTTGTGCTCCCACG GGTCTACAGAGTCCCATCTGCCCAAAGGTCTTGAAGCTTGACAGGATGTTTT CGATTACTCAGTCTCCCAGGGCACTACTGGTCCGTAGGATTCGATTGGTCGG |
| | | GGTAGGAGAGTTAAACAACATTTAAACAGAGTTCTCTCAAAAATGTCTAAAG GGATTGTAGGTAGATAACATCCAATCACTGTTTGCACTTATCTGAAATCTTCC CTCTTGGCTGCCCCCAGGTATTTACTGTGGAGAACATTGCATAGGAATGTCTG GAAAAAGCTTCTACAACTTGTTACAGCCTTCACATTTGTAGAAGCTTTGCGGC CGCTTCGAGCAGACATGATAAGATACATTGATGAGTTTGGACAAACCACAAC TAGAATGCAGTGAAAAAAATGCTTTATTTGTGAAATTTGTGATGCTATTGCTT TATTTGTAACCATTATAAGCTGCAATAAACAAGTT |
| 177 | Kozak sequence (including a translation initiation sequence) | GCCACCATGG |
| 178 | First ITR sequence | CCTGCAGGCAGCTGCGCGCTCGCTCGCTCACTGAGGCCGCCCGGGCGTCGGG CGACCTTTGGTCGCCCGACGCCCGGGCGGCCTCAGTGAGCGAGCGAGCGCGC AGAGAGGGAGTGGCCAACTCCATCACTAGGGGTTCCT |
| 179 | Second ITR sequence | AGGAACCCCTAGTGATGGAGTTGGCCACTCCCTCTCTGCGCGCTCGCTCGCTC ACTGAGGCCGGGCGACCAAAGGTCGCCCGACGCCCGGGCTTTGCCCGGGCG GCCTCAGTGAGCGAGCGAGCGCGCAGCTGCCTGCAGG |
| 180 | ITR-CMV-Koz ak-MTS-ND4-3'UTR-SV40-I TR | CCTGCAGGCAGCTGCGCGCTCGCTCGCTCACTGAGGCCGCCCGGGCGTCGGG CGACCTTTGGTCGCCCGACGCCCGGGCGGCCTCAGTGAGCGAGCGAGCGCGC AGAGAGGGAGTGGCCAACTCCATCACTAGGGGTTCCTGCGGCCGCACGCGTC TAGTTATTAATAGTAATCAATTACGGGGTCATTAGTTCATAGCCCATATATGG AGTTCCGCGTTACATAACTTACGGTAAATGGCCCGCCTGGCTGACCGCCCAA CGACCCCCGCCCATTGACGTCAATAATGACGTATGTTCCCATAGTAACGCCA ATAGGGACTTTCCATTGACGTCAATGGGTGGAGTATTTACGGTAAACTGCCC ACTTGGCAGTACATCAAGTGTATCATATGCCAAGTACGCCCCCTATTGACGT CAATGACGGTAAATGGCCCGCCTGGCATTATGCCCAGTACATGACCTTATGG GACTTTCCTACTTGGCAGTACATCTACGTATTAGTCATCGCTATTACCATGGT GATGCGGTTTTGGCAGTACATCAATGGGCGTGGATAGCGGTTTGACTCACGG GGATTTCCAAGTCTCCACCCCATTGACGTCAATGGGAGTTTGTTTTGCACCAA AATCAACGGGACTTTCCAAAATGTCGTAACAACTCCGCCCCATTGACGCAAA TGGGCGGTAGGCGTGTACGGTGGGAGGTCTATATAAGCAGAGCTCGTTTAGT GAACCGTCAGATCACTAGAAGCTTTATTGCGGTAGTTTATCACAGTTAAATT GCTAACGCAGTCAGTGCTTCTGACACAACAGTCTCGAACTTAAGCTGCAGAA GTTGGTCGTGAGGCACTGGGCAGGTAAGTATCAAGGTTACAAGACAGGTTTA AGGAGACCAATAGAAACTGGGCTTGTCGAGACAGAGAAGACTCTTGCGTTTC TGATAGGCACCTATTGGTCTTACTGACATCCACTTTGCCTTTCTCTCCACAGG TGTCCACTCCCAGTTCAATTACAGCTCTTAAGGCTAGAGTACTTAATACGACT |
| | | CACTATAGGCTAGCCTCGAGAATTCACGCGTGGTACCGCCACCGGATCCGCC GCCACCATGGCCGCATCTCCGCACACTCTCTCCTCACGCCTCCTGACAGGTTG CGTAGGAGGCTCTGTCTGGTATCTTGAAAGAAGAACTATGCTAAAACTAATC GTCCCAACAATTATGTTACTACCACTGACATGGCTTTCCAAAAAACACATGA TTTGGATCAACACAACCACCCACAGCCTAATTATTAGCATCATCCCTCTACTA TTTTTTAACCAAATCAACAACAACCTATTTAGCTGTTCCCCAACCTTTTCCTC CGACCCCCTAACAACCCCCCTCCTAATGCTAACTACCTGGCTCCTACCCCTCA CAATCATGGCAAGCCAACGCCACTTATCCAGTGAACCACTATCACGAAAAAA ACTCTACCTCTCTATGCTAATCTCCCTACAAATCTCCTTAATTATGACATTCA CAGCCACAGAACTAATCATGTTTTATATCTTCTTCGAAACCACACTTATCCCC ACCTTGGCTATCATCACCCGATGGGGCAACCAGCCAGAACGCCTGAACGCAG GCACATACTTCCTATTCTACACCCTAGTAGGCTCCCTTCCCCTACTCATCGCA CTAATTTACACTCACAACACCCTAGGCTCACTAAACATTCTACTACTCACTCT CACTGCCCAAGAACTATCAAACTCCTGGGCCAACAACTTAATGTGGCTAGCT TACACAATGGCTTTTATGGTAAAGATGCCTCTTTACGGACTCCACTTATGGCT CCCTAAAGCCCATGTCGAAGCCCCCATCGCTGGGTCAATGGTACTTGCCGCA GTACTCTTAAAACTAGGCGGCTATGGTATGATGCGCCTCACACTCATTCTCAA CCCCCTGACAAAACACATGGCCTACCCCTTCCTTGTACTATCCCTATGGGGCA TGATTATGACAAGCTCCATCTGCCTACGACAAACAGACCTAAAATCGCTCAT TGCATACTCTTCAATCAGCCACATGGCCCTCGTAGTAACAGCCATTCTCATCC AAACCCCCTGGAGCTTCACCGGCGCAGTCATTCTCATGATCGCCCACGGGCT TACATCCTCATTACTATTCTGCCTAGCAAACTCAAACTACGAACGCACTCACA GTCGCATCATGATCCTCTCTCAAGGACTTCAAACTCTACTCCCACTAATGGCT TTTTGGTGGCTTCTAGCAAGCCTCGCTAACCTCGCCTTACCCCCCACTATTAA CCTACTGGGAGAACTCTCTGTGCTAGTAACCACGTTCTCCTGGTCAAATATCA CTCTCCTACTTACAGGACTCAACATGCTAGTCACAGCCCTATACTCCCTCTAC ATGTTTACCACAACACAATGGGGCTCACTCACCCACCACATTAACAACATGA AACCCTCATTCACACGAGAAAACACCCTCATGTTCATGCACCTATCCCCCATT CTCCTCCTATCCCTCAACCCCGACATCATTACCGGGTTTTCCTCTTAAGAGCA CTGGGACGCCCACCGCCCCTTTCCCTCCGCTGCCAGGCGAGCATGTTGTGGT AATTCTGGAACACAAGAAGAGAAATTGCTGGGTTTAGAACAAGATTATAAA CGAATTCGGTGCTCAGTGATCACTTGACAGTTTTTTTTTTTTTTAAATATTACC CAAAATGCTCCCCAAATAAGAAATGCATCAGCTCAGTCAGTGAATACAAAA AAGGAATTATTTTTCCCTTTGAGGGTCTTTTATACATCTCTCCTCCAACCCCA CCCTCTATTCTGTTTCTTCCTCCTCACATGGGGGTACACATACACAGCTTCCT CTTTTGGTTCCATCCTTACCACCACACCACACGCACACTCCACATGCCCAGCA GAGTGGCACTTGGTGGCCAGAAAGTGTGAGCCTCATGATCTGCTGTCTGTAG TTCTGTGAGCTCAGGTCCCTCAAAGGCCTCGGAGCACCCCCTTCCTTGTGACT GAGCCAGGGCCTGCATTTTTGGTTTTCCCCACCCCACACATTCTCAACCATAG TCCTTCTAACAATACCAATAGCTAGGACCCGGCTGCTGTGCACTGGGACTGG |
| | | GGATTCCACATGTTTGCCTTGGGAGTCTCAAGCTGGACTGCCAGCCCCTGTCC TCCCTTCACCCCCATTGCGTATGAGCATTTCAGAACTCCAAGGAGTCACAGG CATCTTTATAGTTCACGTTAACATATAGACACTGTTGGAAGCAGTTCCTTCTA AAAGGGTAGCCCTGGACTTAATACCAGCCGGATACCTCTGGCCCCCACCCCA TTACTGTACCTCTGGAGTCACTACTGTGGGTCGCCACTCCTCTGCTACACAGC ACGGCTTTTTCAAGGCTGTATTGAGAAGGGAAGTTAGGAAGAAGGGTGTGCT GGGCTAACCAGCCCACAGAGCTCACATTCCTGTCCCTTGGGTGAAAAATACA TGTCCATCCTGATATCTCCTGAATTCAGAAATTAGCCTCCACATGTGCAATGG CTTTAAGAGCCAGAAGCAGGGTTCTGGGAATTTTGCAAGTTACCTGTGGCCA GGTGTGGTCTCGGTTACCAAATACGGTTACCTGCAGCTTTTTAGTCCTTTGTG CTCCCACGGGTCTACAGAGTCCCATCTGCCCAAAGGTCTTGAAGCTTGACAG GATGTTTTCGATTACTCAGTCTCCCAGGGCACTACTGGTCCGTAGGATTCGAT TGGTCGGGGTAGGAGAGTTAAACAACATTTAAACAGAGTTCTCTCAAAAATG TCTAAAGGGATTGTAGGTAGATAACATCCAATCACTGTTTGCACTTATCTGA AATCTTCCCTCTTGGCTGCCCCCAGGTATTTACTGTGGAGAACATTGCATAGG AATGTCTGGAAAAAGCTTCTACAACTTGTTACAGCCTTCACATTTGTAGAAG CTTTGCGGCCGCTTCGAGCAGACATGATAAGATACATTGATGAGTTTGGACA AACCACAACTAGAATGCAGTGAAAAAAATGCTTTATTTGTGAAATTTGTGAT GCTATTGCTTTATTTGTAACCATTATAAGCTGCAATAAACAAGTTAACAACA ACACTCGAGTTAAGGGCGAATTCCCGATAAGGATCTTCCTAGAGCATGGCTA CGTAGATAAGTAGCATGGCGGGTTAATCATTAACTACAAGGAACCCCTAGTG ATGGAGTTGGCCACTCCCTCTCTGCGCGCTCGCTCGCTCACTGAGGCCGGGC GACCAAAGGTCGCCCGACGCCCGGGCTTTGCCCGGGCGGCCTCAGTGAGCGA GCGAGCGCGCAGCTGCCTGCAGG |
| 181 | flag amplification forward primer | AGACCATGACGGTGAT |
| 182 | flag amplification reverse primer | CTTGTCATCGTCATCCT |
| 183 | actin amplification forward primer | GGACTTCGAGCAAGAGATGG |
| 184 | actin amplification reverse primer | AGGAAGGAAGGCTGGAAGAG |
| 185 | Spacer sequence | GCGGCCGCTTCGAGCAGACATGA |

### Adeno-associated virus (AAV)

Adeno-associated virus (AAV) is a small virus that infects humans and some other primate species. The composition disclosed herein first comprises an adeno-associated virus (AAV) genome or a derivative thereof.

The AAV genome is a polynucleotide sequence encoding functions required for production of AAV virus particles. These functions include functions of the AAV operating in the replication and packaging cycles of host cells, including encapsidation of the AAV genome into the AAV virus particles. Naturally occurring AAV viruses are replication-deficient and rely on a trans-providing helper function to complete the replication and packaging cycles. Thus, AAV genomes of vectors of the present invention are generally replication-deficient.

The AAV genomes may be in a positive sense or negative sense single-strand form, or in a double-strand form. The use of the double-strand form allows bypassing the step of DNA replication in target cells, and thus can accelerate transgene expression.

The AAV genome may be derived from any naturally derived serotype or isolate or Clade of the AAV. Thus, the AAV genome may be a whole genome of the naturally occurring AAV viruses. As known to the skilled person, AAV viruses occurring in the nature can be classified according to various biological systems.

Typically, AAV viruses are referred to in terms of their serotype. Serotypes correspond to variant subspecies of the AAV, the variant subspecies has unique reactivity due to an expression profile of their capsid surface antigens, which can be used for distinguishing it from other variant subspecies. Typically, viruses having a particular AAV serotype do not efficiently cross-react with neutralizing antibodies specific for any other AAV serotype. AAV serotypes include AAV1, AAV2, AAV3, AAV4, AAV5, AAV6, AAV7, AAV8, AAV9, AAV10, AAV11, AAV12, AAV13, AAV14, AAV15 and AAV16, as well as recombinant serotypes, such as Rec2 and Rec3, recently identified from primate brains.

A preferred AAV serotype for use in the present invention is AAV2. Other serotypes of particular interest for use in the present invention include AAV4, AAV5 and AAV8, which efficiently transduce tissues in eyes, such as retinal pigment epitheliums. A serotype of AAV used may be an AAV serotype other than AAV4. Reviews of the AAV serotypes can be found in Choi et al. (Curr Gene Ther. 2005; 5(3); 299-310) and Wu et al. (Molecular Therapy. 2006; 14(3), 316-327). Sequences of the AAV genomes or elements of the AAV genomes used in the present invention (including ITR sequences, rep or cap genes) may be derived from the following accession numbers for AAV whole genome sequences: adeno-associated virus 1 NC_002077, AF063497; adeno-associated virus 2 NC_001401; adeno-associated virus 3 NC_001729; adeno-associated virus 3B NC_001863; adeno-associated virus 4 NC_001829; adeno-associated virus 5 Y18065, AF085716; adeno-associated virus 6 NC_001862; Avian AAV ATCC VR-865 AY186198, AY629583, NC_004828; Avian AAV strain DA-1 NC_006263, AY629583; Bovine AAV NC_005889, AY388617.

AAV viruses may also be referred to in terms of clades or clones. This refers to a phylogenetic relationship of naturally derived AAV viruses, and typically refers to a phylogenetic group of AAV viruses, which can be traced back to a common ancestor, and includes all descendants thereof. Additionally, AAV viruses may be referred to in terms of a specific isolate, i.e. a genetic isolate of a specific AAV virus found in the nature. The term genetic isolate describes a population of AAV viruses that has undergone limited genetic mixing with other naturally occurring AAV viruses, thereby defining identifiable distinct populations at a genetic level.

Examples of clades and isolates of AAV that may be used in the present invention include: Clade A: AAV1 NC_002077, AF063497, AAV6 NC_001862, Hu. 48 AY530611, Hu 43 AY530606, Hu 44 AY530607, Hu 46 AY530609; Clade B: Hu. 19 AY530584, Hu. 20 AY530586, Hu 23 AY530589, Hu22 AY530588, Hu24 AY530590, Hu21 AY530587, Hu27 AY530592, Hu28 AY530593, Hu 29 AY530594, Hu63 AY530624, Hu64 AY530625, Hu13 AY530578, Hu56 AY530618, Hu57 AY530619, Hu49 AY530612, Hu58 AY530620, Hu34 AY530598, Hu35 AY530599, AAV2 NC_001401, Hu45 AY530608, Hu47 AY530610, Hu51 AY530613, Hu52 AY530614, Hu T41 AY695378, Hu S17 AY695376, Hu T88 AY695375, Hu T71 AY695374, Hu T70 AY695373, Hu T40 AY695372, Hu T32 AY695371, Hu T17 AY695370, Hu LG15 AY695377; Clade C: Hu9 AY530629, Hu10 AY530576, Hu11 AY530577, Hu53 AY530615, Hu55 AY530617, Hu54 AY530616, Hu7 AY530628, Hu18 AY530583, Hu15 AY530580, Hu16 AY530581, Hu25 AY530591, Hu60 AY530622, Ch5 AY243021, Hu3 AY530595, Hu1 AY530575, Hu4 AY530602 Hu2, AY530585, Hu61 AY530623; Clade D: Rh62 AY530573, Rh48 AY530561, Rh54 AY530567, Rh55 AY530568, Cy2 AY243020, AAV7 AF513851, Rh35 AY243000, Rh37 AY242998, Rh36 AY242999, Cy6 AY243016, Cy4 AY243018, Cy3 AY243019, Cy5 AY243017, Rh13 AY243013; Clade E: Rh38 AY530558, Hu66 AY530626, Hu42 AY530605, Hu67 AY530627, Hu40 AY530603, Hu41 AY530604, Hu37 AY530600, Rh40 AY530559, Rh2 AY243007, Bb1 AY243023, Bb2 AY243022, Rh10 AY243015, Hu17 AY530582, Hu6 AY530621, Rh25 AY530557, Pi2 AY530554, Pi1 AY530553, Pi3 AY530555, Rh57 AY530569, Rh50 AY530563, Rh49 AY530562, Hu39 AY530601, Rh58 AY530570, Rh61 AY530572, Rh52 AY530565, Rh53 AY530566, Rh51 AY530564, Rh64 AY530574, Rh43 AY530560, AAV8 AF513852, Rh8 AY242997, Rh1 AY530556; Clade F: Hu14 (AAV9) AY530579, Hu31 AY530596, Hu32 AY530597, Clonal Isolate AAV5 Y18065, AF085716, AAV 3 NC_001729, AAV 3B NC_001863, AAV4 NC_001829, Rh34 AY243001, Rh33 AY243002, Rh32 AY243003.

The skilled person can select appropriate serotypes, clades, clones or isolates of AAV for use in the present invention based on their common general knowledge. For example, an AAV5 capsid has been shown to efficiently transduce primate cone photoreceptors, as evidenced by successful correction of inherited color vision defects (Mancuso et al., Nature 2009, 461:784-7).

It should be understood however that the present invention also encompasses use of AAV genomes of other serotypes that may not yet have been identified or characterized. The AAV serotype determines tissue specificity (or tropism) of AAV virus infection. Thus, a preferred AAV serotype of AAV viruses used for administration to patients according to the present invention is that which has natural tropism for or a high efficiency of infection of target cells in eyes in LHON. Thus, the AAV serotype of the AAV viruses used for administration to the patients may be a serotype that infects neurosensory retinas and retinal pigment epitheliums.

Typically, an AAV genome of naturally derived serotypes or isolates or clades of AAV includes at least one inverted terminal repeat (ITR) sequence. The ITR sequences act in cis to provide a functional origin of replication and allow for integration and excision of a vector from a genome of cells. Selectable ITR sequences may be derived from any of AAV serotypes, such as AAV1, AAV2, AAV3, AAV4, AAV5, AAV6, AAV7, AAV8, AAV9, AAV10, AAV11, AAV12, AAV13, AAV14, AAV15 and AAV16, as well as recombinant serotypes, such as Rec2 and Rec3, recently identified from primate brains, non-primate AAV, avian AAV, bovine AAV, canine AAV, equine AAV, ovine AAV, etc. Preferred ITR sequences are those of AAV2 and variants thereof.

In preferred embodiments, one or more ITR sequences are flanked by a polynucleotide sequence encoding ND4 or variants thereof.

The AAV genome also typically includes packaging genes, such as rep and/or cap genes, that encode packaging functions for AAV viral particles. The rep gene encodes one or more of proteins Rep78, Rep68, Rep52 and Rep40 or variants thereof. The cap gene encodes one or more capsid proteins such as VP1, VP2 and VP3 or variants thereof. These proteins make up a capsid of the AAV viral particles. Capsid variants are discussed below.

A promoter will be operably linked to each of the packaging genes. Specific examples of such promoters include p5, p19 and p40 promoters (Laughlin et al., 1979, PNAS, 76:5567-5571). For example, the p5 and p19 promoters are typically used for expressing the rep gene, while the p40 promoter is typically used for expressing the cap gene.

As discussed above, the AAV genome used for vectors of the present invention may thus be the whole genome of the naturally occurring AAV viruses. For example, vectors including the whole AAV genome can be used for preparing AAV viruses in vitro. However, while such a vector may in principle be administered to the patients, this will be done rarely in practice. Preferably, the AAV genome will be derivatized for the purpose of administration to the patients. Such derivatization is standard in the art, and the present invention encompasses the use of any known derivatives of the AAV genome as well as derivatives that can be produced by applying techniques known in the art. Derivatization of the AAV genome and of the AAV capsid are reviewed in the references cited above by Coura and Nardi (Virology Journal, 2007, 4:99), and by Choi et al. and Wu et al.

Derivatives of the AAV genome include any truncated or modified form of the AAV genome that allows for in-vivo expression of an ND4 transgene from the vectors of the present invention. Typically, it is possible to truncate the AAV genome significantly to include a minimal viral sequence, but retain the above functions. This is preferred for safety reasons to reduce the risk of recombination of the vectors with wild-type viruses, and also to avoid triggering cellular immune responses by the presence of viral gene proteins in target cells.

Typically, a derivative will include at least one inverted terminal repeat (ITR) sequence, preferably more than one ITR, such as two or more ITRs. One or more ITRs may be derived from AAV genomes with different serotypes, or may be chimeric or mutant ITRs. A preferred mutant ITR is one having a deletion of trs (terminal resolution site). This deletion allows for continued replication of the genome to generate a single-stranded genome which contains both coding and complementary sequences i.e., a self-complementary AAV genome. This allows for bypassing of DNA replication in the target cells, and so enables to accelerate transgene expression.

The one or more ITRs will preferably be flanked by a polynucleotide sequence encoding ND4, ND6, ND1, or a variant thereof at either end. The inclusion of one or more ITRs is preferred to aid concatemer formation of the vector of the present invention in a nucleus of a host cell, for example following the conversion of single-stranded vector DNA into double-stranded DNA by the action of host cell DNA polymerases. The formation of such episomal concatemers protects vector constructs during the life of the host cell, thereby allowing for prolonged expression of the transgene in vivo.

In preferred embodiments, ITR elements will be only sequences retained from an original AAV genome in the derivatives. Thus, derivatives will preferably not include the rep and/or cap genes of the original genome and any other sequences of the original genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the transgene.

Therefore, with reference to an AAV2 genome, the following portions could be removed in a derivative of the present invention: one inverted terminal repeat (ITR) sequence, replication (rep) and capsid (cap) genes (NB: the rep gene in the wild-type AAV genome should not to be confused with human gene ND4 affected in LHON). However, in some embodiments, including in vitro embodiments, derivatives may additionally include one or more rep and/or cap genes or other viral sequences of an AAV genome. Naturally occurring AAV viruses integrate with a high frequency at a specific site on human chromosome 19, and show a negligible frequency of random integration, such that retention of an integrative capacity in the vector may be tolerated in a therapeutic setting.

Where a derivative genome includes genes encoding capsid proteins i.e., VP1, VP2 and/or VP3, the derivative may be a chimeric, shuffled or capsid-modified derivative of one or more naturally occurring AAV viruses. In particular, the present invention encompasses the provision of capsid protein sequences from different serotypes, clades, clones, or isolates of AAV within the same vector, i.e., pseudotyping.

Chimeric, shuffled or capsid-modified derivatives will be typically selected to provide one or more desired functionalities for the viral vector. Thus, these derivatives may display increased efficiency of gene delivery, decreased immunogenicity (humoral or cellular), an altered tropism range and/or improved targeting of a particular cell type compared to an AAV viral vector including a naturally occurring AAV genome, such as that of an AAV2 genome. Increased efficiency of gene delivery may be effected by improved receptor or co-receptor binding at the cell surface, improved internalisation, improved transfer within cells and into nucleuses, improved uncoating of viral particles and improved conversion of a single-stranded genome to a double-stranded form. Increased efficiency may also relate to an altered tropism range or targeting of a specific cell population, such that a vector dose will not be diluted by administration to tissues where it is not needed.

Chimeric capsid proteins include those generated by recombination between two or more capsid coding sequences of naturally occurring AAV serotypes. This may be performed for example by a marker rescue approach in which non-infectious capsid sequences of one serotype are cotransfected with capsid sequences of a different serotype, and directed selection is used for selecting for capsid sequences having desired properties. The capsid sequences of the different serotypes can be altered by homologous recombination within the cells to produce novel chimeric capsid proteins.

Chimeric capsid proteins also include those generated by engineering of capsid protein sequences to transfer specific capsid protein domains, surface loops or specific amino acid residues between two or more capsid proteins (for example, between two or more capsid proteins of different serotypes).

Shuffled or chimeric capsid proteins may also be generated by DNA shuffling or by error-prone PCR. Hybrid AAV capsid genes can be created by randomly fragmenting sequences of related AAV genes (e.g., those encoding capsid proteins of multiple different serotypes), and then subsequently reassembling fragments in a self-priming polymerase reaction, which may also cause crossovers in regions of sequence homology. A library of hybrid AAV genes created in this way by shuffling the capsid genes of several serotypes can be screened to identify viral clones having a desired functionality. Similarly, error-prone PCR may be used for randomly mutating AAV capsid genes to create a diverse library of variants which may then be selected for a desired property.

The sequences of the capsid genes may also be genetically modified to introduce specific deletions, substitutions or insertions with respect to the native wild-type sequence. In particular, capsid genes may be modified by the insertion of a sequence of an unrelated protein or peptide within an open reading frame of a capsid coding sequence, or at the N- and/or C-terminus of a capsid coding sequence.

The unrelated protein or peptide may advantageously be one which acts as a ligand for a particular cell type, thereby conferring improved binding to a target cell or improving the specificity of targeting of the vector to a particular cell population. An example might include the use of RGD peptide to block uptake in the retinal pigment epitheliums and thereby enhance transduction of surrounding retinal tissues (Cronin et al., 2008 ARVO Abstract: D1048). The unrelated protein may also be one which assists purification of the viral particles as part of the production process, i.e., an epitope or affinity tag. The site of insertion will typically be selected so as not to interfere with other functions of the viral particles, e.g., internalisation, transferring of the viral particles. The skilled person can identify suitable sites for insertion based on their common general knowledge. Particular sites are disclosed by Choi et al., referenced above.

The present invention additionally encompasses the provision of sequences of an AAV genome in a different order and configuration to that of a native AAV genome. The present invention also encompasses the replacement of one or more AAV sequences or genes with sequences from another virus or with chimeric genes composed of sequences from more than one viruses. Such chimeric genes may be composed of sequences from two or more related viral proteins of different viral species.

The vector of the present invention takes the form of a polynucleotide sequence including an AAV genome or derivative thereof and a sequence encoding ND4, ND6, ND1 or a variant thereof.

For the avoidance of doubt, the present invention also provides an AAV viral particle including a vector of the present invention. The AAV particles of the present invention include transcapsidated forms wherein an AAV genome or derivative having an ITR of one serotype is packaged in the capsid of a different serotype. The AAV particles of the present invention also include mosaic forms wherein a mixture of unmodified capsid proteins from two or more different serotypes makes up a viral envelope. The AAV particle also includes chemically modified forms bearing ligands adsorbed to the capsid surface. For example, such ligands may include antibodies used for targeting a particular cell surface receptor.

The present invention additionally provides a host cell including a vector or AAV viral particle of the present invention.

### Recombinant nucleic acid sequences

Also disclosed herein are recombinant nucleic acid sequences including a polynucleotide sequence encoding an NADH dehydrogenase subunit-4 (ND4), NADH dehydrogenase subunit-1 (ND1) and NADH dehydrogenase subunit-6 (ND6) polypeptide or a variant thereof.

The polynucleotide sequence for ND4 is shown in SEQ ID NO: 6 and encodes a protein shown in SEQ ID NO: 160. Further nucleic acid sequences for ND4 are SEQ ID NOs: 7 and 8.

A variant of SEQ ID NO: 160 may include truncations, mutants or homologues thereof, and any transcript variants thereof which encode a functional ND4 polypeptide. Any homologues mentioned herein typically have at least 70% identity to a relevant region of ND4, and can functionally compensate for polypeptide deficiency.

Homology/identity can be measured using known methods. For example, the UWGCG Package provides a BESTFIT program which can be used for calculating homology (for example used on its default settings) (Devereux et al. (1984) Nucleic Acids Research 12, 387-395). PILEUP and BLAST algorithms can be used for calculating homology or line up sequences (typically on their default settings), for example as described in Altschul S. F. (1993) J Mol Evol 36:290-300; Altschul, S, F et al. (1990) J Mol Biol 215:403-10. Software for performing BLAST analyses is publicly available through the National Center for Biotechnology Information (http://www.ncbi.nlm.nih.gov/).

In preferred embodiments, a recombinant nucleic acid sequence may encode a polypeptide having least 55%, 65%, 70%, 75%, 80%, 85%, 90% and more preferably at least 95%, 97%, 99%, 99.5%, or 100% identity to a relevant region of an ND4 protein (SEQ ID NO: 160) over at least 20 (preferably at least 30, for instance at least 40, 60, 100, 200, 300, 400 or more contiguous amino acids), or even over the entire sequence of the recombinant nucleic acid. The relevant region will be one which provides for functional activity of ND4.

Alternatively, and preferably the recombinant nucleic acid sequence may encode a polypeptide having at least 70%, 75%, 80%, 85%, 90% and more preferably at least 95%, 97%, 99%, 99.5%, or 100% identity to full-length ND4 (SEQ ID NO: 160) over its entire sequence. Typically the recombinant nucleic acid sequence differs from the relevant region of ND4 (SEQ ID NO: 160) by at least, or less than, 2, 5, 10, 20, 40, 50 or 60 mutations (each of which may be substitutions, insertions or deletions).

A recombinant nucleic acid ND4 polypeptide may have a certain percent identity with a particular region of SEQ ID NO: 160 which is the same as any of specific percent identity values (i.e., it may have at least 70%, 80% or 90% and more preferably at least 95%, 97%, 99% identity) across any of the lengths of sequence mentioned above.

Variants of ND4 (SEQ ID NO: 160) also include truncations. Any truncation may be used so long as the variant is still functional. Truncations will typically be made to remove sequences that are non-essential for protein activity and/or do not affect conformation of a folded protein, in particular folding of an active site. Appropriate truncations can routinely be identified by systematic truncation of sequences of varying length from the N- or C-terminus. Preferred truncations are N-terminal and may remove all other sequences except for the catalytic domain.

Variants of ND4 (SEQ ID NO: 160) further include mutants which have one or more (e.g., 2, 3, 4, 5 to 10, 10 to 20, 20 to 40, or more amino acid insertions, substitutions, or deletions) with respect to a particular region of ND4 (SEQ ID NO: 160). Deletions and insertions are made preferably outside the catalytic domain as described below. Substitutions are also typically made in regions that are non-essential for protease activity and/or do not affect the conformation of the folded protein.

Substitutions preferably introduce one or more conservative changes, which replace amino acids with another amino acids of similar chemical structure, similar chemical properties, or similar side-chain volume. The amino acids introduced may have similar polarity, hydrophilicity, hydrophobicity, basicity, acidity, neutrality, or charge to the amino acids they replace. Alternatively, the conservative change may introduce another aromatic or aliphatic amino acid in place of a pre-existing aromatic or aliphatic amino acid. Conservative amino acid changes are well known in the art and may be selected in accordance with the properties of the amino acids.

Similarly, preferred variants of the polynucleotide sequence of ND4 (SEQ ID NO: 6) include polynucleotides having at least 70%, 75%, 80%, 85%, 90% and more preferably at least 95%, 96%, 97%, 98%, 99%, or 99.5% identity to a relevant region of ND4 (SEQ ID NO: 6). Preferably the variant displays these levels of identity to full-length ND4 (SEQ ID NO: 6) over its entire sequence.

Mitochondrial targeting sequences (MTS) and 3' untranslated regions (3' UTRs) can be used to target proteins or mRNA to the mitochondria. The charge, length, and structure of MTS can be important for protein import into the mitochondria. Particular 3' UTRs may drive mRNA localization to the mitochondrial surface and thus facilitate cotranslational protein import into the mitochondria.

The polynucleotide sequence for a mitochondrial targeting sequence may encode a polypeptide selected from the group consisting of hsCOX10, hsCOX8, scRPM2, lcSirt5, tbNDUS7, ncQCR2, hsATP5G2, hsLACTB, spilv1, gmCOX2, crATP6, hsOPA1, hsSDHD, hsADCK3, osP0644B06.24-2, *Neurospora crassa* ATP9 (ncATP9), hsGHITM, hsNDUFAB1, hsATP5G3, crATP6_hsADCK3, ncATP9_ncATP9, zmLOC100282174, ncATP9_zmLOC100282174_spilv1_ncATP9, zmLOC 100282174_hsADCK3_crATP6_hsATP5G3, zmLOC100282174_hsADCK3_hsATP5G3, ncATP9_zmLOC100282174, hsADCK3_zmLOC100282174_crATP6_hsATP5G3, crATP6_hsADCK3_zmLOC100282174_hsATP5G3, hsADCK3_zmLOC 100282174, hsADCK3_zmLOC 100282174_crATP6, ncATP9_zmLOC100282174_spilv1_GNFP_ncATP9, and ncATP9_zmLOC100282174_spilv_lcSirt5_osP0644B06.24-2_hsATP5G2_ncATP9 (See Table 1 for SEQ ID NO). In one example, the polynucleotide sequence, COX10 (SEQ ID NO: 1, 2, or 3) may encode the mitochondrial targeting sequence, MTS-COX10 (SEQ ID NO: 126). In another example, the polynucleotide sequence, COX8 (SEQ ID NO: 4) may encode the mitochondrial targeting sequence, MTS-COX8 (SEQ ID NO: 127). In another example, the polynucleotide sequence, OPA1 (SEQ ID NO: 5) may encode the mitochondrial targeting sequence, MTS-OPA1 (SEQ ID NO: 128).

The 3' UTR nucleic acid sequence may be selected from selected from the group consisting of hsACO2 (SEQ ID NO: 111), hsATP5B (SEQ ID NO: 112), hsAK2 (SEQ ID NO: 113), hsALDH2 (SEQ ID NO: 114), hsCOX10 (SEQ ID NO: 115), hsUQCRFS1 (SEQ ID NO: 116), hsNDUFV1 (SEQ ID NO: 117), hsNDUFV2 (SEQ ID NO: 118), hsSOD2 (SEQ ID NO: 119), hsCOX6c (SEQ ID NO: 120), hsIRP1 (SEQ ID NO: 121), hsMRPS12 (SEQ ID NO: 122), hsATP5J2 (SEQ ID NO: 123), rnSOD2 (SEQ ID NO: 124), and hsOXAlL (SEQ ID NO: 125). The 3' UTR nucleic acid sequence may also be a variant having at least 70%, 75%, 80%, 85%, 90%, and more preferably at least 95%, 96%, 97%, 98%, 99%, 99.5%, or 100% identity to any 3' UTR nucleic acid sequence listed herein. For example, the 3' UTR nucleic acid sequence may be SEQ ID NO: 13 or 14.

Also disclosed herein are recombinant nucleic acid sequences comprising a mitochondrial targeting sequence, a mitochondrial protein coding sequence, and a 3' UTR nucleic acid sequence. For example, the recombinant nucleic acid sequence may be selected from SEQ ID NOs: 15-84. The recombinant nucleic acid sequence may also be a variant having at least 70%, 75%, 80%, 85%, 90%, and more preferably at least 95%, 96%, 97%, 98%, 99%, 99.5%, or 100% identity to any the recombinant nucleic acid sequence listed herein.

### Promoters and regulatory sequences

The vector of the present invention also includes elements allowing for expression of the disclosed transgene in vitro or in vivo. Thus, the vector typically comprises a promoter sequence operably linked to the polynucleotide sequence encoding the transgene or a variant thereof.

Any suitable promoter may be used. The promoter sequence may be constitutively active, i.e., may be operational in any host cell background, or alternatively may be active only in a specific host cell environment, thereby allowing for targeted expression of the transgene in a particular cell type. A promoter may show inducible expression in response to presence of another factor, for example a factor present in a host cell. In any event, where the vector is administered for therapy, the promoter must be functional in a retinal cell background.

In some embodiments, it is preferred that the promoter shows retinal cell-specific expression in order to allow for the transgene to only be expressed in retinal cell populations. Thus, expression from the promoter may be retinal-specific, for example confined only to cells of the neurosensory retina and the retinal pigment epithelium.

Preferred promoters for the ND4 transgene include the chicken beta-actin (CBA) promoter, optionally in combination with a cytomegalovirus (CME) enhancer element. In some cases, the preferred promoters for the ND4 transgene comprise the CAG promoter. A particularly preferred promoter is a hybrid CBA/CAG promoter, such as the promoter used in the rAVE expression cassette. Examples of promoters based on human sequences that would induce retinal-specific gene expression include rhodopsin kinase for rods and cones (Allocca et. al, 2007, J Viol 81: 11372-80), PR2.1 for cones-only (Mancuso et. al, 2009, Nature), and/or RPE65 for the retinal pigment epithelium (Bainbridge et. al, 2008, N Eng J Med)*.*

In some embodiments, the promoter comprises a sequence having at least 70%, at least 75%, at least 80%, at least 85%, at least 90%, at least 95%, at least 97%, at least 99%, or 100% identity to the sequence as set forth in SEQ ID NO: 169. In some embodiments, the promoter comprises a sequence that differs from the sequence as set forth in SEQ ID NO: 169 by at most 1, 2, 3, 4, 5, 6, 7, 8, 9, or 10 nucleotides. In some embodiments, the promoter has a sequence of SEQ ID NO: 169.

The vectors of the present invention may also contain one or more additional regulatory sequences which may act pre- or post-transcriptionally. The regulatory sequence may be part of the native ND4 locus or may be a heterologous regulatory sequence. The vector of the present invention may comprise several portions of the 5' UTR or the 3' UTR from the native ND4 transcription.

Regulatory sequences are any sequences which facilitate the expression of the transgene, i.e., act to increase the expression of a transcription, improve nuclear export of mRNA, or enhance its stability. Such regulatory sequences include, for example, enhancer elements, postregulatory elements, and polyadenylation sites. In the context of the vector of the present invention, such regulatory sequences will be cis-acting. However, the present invention also contemplates the use of trans-acting regulatory sequences located on additional genetic constructs.

A preferred postregulatory element for use in a vector of the present invention is the woodchuck hepatitis virus postregulatory element (WPRE) or a variant thereof. Another regulatory sequence which may be used in a vector of the present invention is the scaffold-attachment region (SAR).

### Intron

Intron, also known as spacer sequence, refers to a segment of a gene or mRNA molecule that has no coding effect and is an intervening sequence in the DNA of a eukaryotic cell. These sequences are transcribed in the precursor RNA and removed by splicing, thus ultimately absent from the mature RNA molecule.

In some embodiments, a recombinant nucleic acid of the present invention comprises an intron. In some embodiments, the intron is located between the promoter and the Kozak sequence. In some embodiments, the intron is located between the promoter and the mitochondrial targeting sequence. In some embodiments, the intron is located between the promoter and the mitochondrial protein coding sequence.

In some embodiments, the intron comprises a sequence having at least 70%, at least 75%, at least 80%, at least 85%, at least 90%, at least 95%, at least 97%, at least 99%, or 100% identity to the sequence as set forth in SEQ ID NO: 170. In some embodiments, the intron comprises a sequence that differs from the sequence as set forth in SEQ ID NO: 170 by at most 1, 2, 3, 4, 5, 6, 7, 8, 9, or 10 nucleotides. In some embodiments, the intron has a sequence of SEQ ID NO: 170.

### Kozak sequence

The Kozak sequence (Kozak consensus sequence) is present in eukaryotic mRNA. Ribosomes are able to recognize this sequence on the mRNA and use it as a translation initiation site.

In some embodiments, a recombinant nucleic acid of the present invention comprises a kozak sequence. In some embodiments, the kozak sequence is SEQ ID NO: 171. In some embodiments, the kozak sequence differs from SEQ ID NO: 171 by at most 1, 2, or 3 nucleotides.

In some technical descriptions in the art, the kozak sequence is as set forth in SEQ ID NO: 171 immediately flanked by a translation initiation sequence of four nucleotides in length at the 3' end, and the complete flanked sequence is GCCACCATGG (SEQ ID NO: 177) with nucleotides -4 to -2 is the start codon ATG. Thus, one skilled in the art would understand that when a kozak sequence is ligated to a coding sequence (e.g., when a Kozak sequence is positioned before a mitochondrial targeting sequence), the Kozak sequence preceded the coding sequence does not contain a translation initiation sequence of this four nucleotides in length.

### PolyA tail

Polyadenylic acid (polyA) tail is usually located downstream of the coding region of the gene and serves to terminate the transcription. In some embodiments, the recombinant nucleic acid contains polyA tail. In some embodiments, the polyA tail has 150-200 adenylate residues.

In some embodiments, the recombinant nucleic acid comprises a simian vacuolating virus polyA tail (SV40 polyA). In some embodiments, the simian vacuolating virus polyA tail comprises a sequence having at least 70%, at least 75%, at least 80%, at least 85%, at least 90%, at least 95%, at least 97%, at least 99%, or 100% identity to the sequence as set forth in SEQ ID NO: 173. In some embodiments, the simian vacuolating virus polyA tail is a sequence that differs from the sequence as set forth in SEQ ID NO: 173 by at most 1, 2, 3, 4, 5, 6, 7, 8, 9, or 10 nucleotides. In some embodiments, the simian vacuolating virus polyA tail has a sequence of SEQ ID NO: 173.

In some embodiments, the recombinant nucleic acid comprises a bovine growth factor polyA tail (bGH polyA). In some embodiments, the bovine growth factor polyA tail comprises a sequence having at least 70%, at least 75%, at least 80%, at least 85%, at least 90%, at least 95%, at least 97%, at least 99%, or 100% identity to the sequence as set forth in SEQ ID NO: 172. In some embodiments, the bovine growth factor polyA tail is a sequence that differs from the sequence as set forth in SEQ ID NO: 172 by at most 1, 2, 3, 4, 5, 6, 7, 8, 9, or 10 nucleotides. In some embodiments, the bovine growth factor polyA tail has a sequence of SEQ ID NO: 172.

In some embodiments, the polyA signal sequence is no more than 122 base pairs (bp), no more than 125 base pairs, no more than 130 base pairs, no more than 140 base pairs, no more than 150 base pairs, no more than 160 base pairs, no more than 170 base pairs, no more than 180 base pairs, no more than 190 base pairs, no more than 200 base pairs, no more than 220 base pairs, no more than 240 base pairs, no more than 260 base pairs, no more than 280 base pairs, or no more than 300 base pairs in length.

### Preparation of vector

The vectors of the present invention may be prepared by standard means known in the art for provision of vectors for gene therapy. Thus, well-established public domain transfection, packaging, and purification methods can be used to prepare a suitable vector preparation.

As discussed above, a vector of the present invention may comprise the full genome of a naturally occurring AAV virus in addition to a polynucleotide sequence encoding ND4 or a variant thereof. However, commonly a derivatized genome, e.g., a derivative having at least one inverted terminal repeat (ITR) sequence but possibly lacking any AAV genes such as rep or cap, will be used.

In such embodiments, in order to provide for assembly of the derivatised genome into an AAV viral particle, additional genetic constructs providing AAV and/or helper virus functions will be provided in a host cell in combination with the derivatized genome. These additional constructs will typically contain genes encoding structural AAV capsid proteins, i.e. cap, VP1, VP2, VP3, and genes encoding other functions required for the AAV life cycle, such as rep. The selection of structural capsid proteins provided on the additional constructs will determine the serotype of the packaged viral vector.

A particularly preferred packaged viral vector for use in the present invention comprises a derivatized genome of AAV2 in combination with AAV5 or AAV8 capsid proteins. This packaged viral vectors typically comprise one or more AAV2 ITRs.

As mentioned above, AAV viruses are replication incompetent and so helper virus functions, preferably adenoviral helper functions will typically also be provided on one or more additional constructs to allow for AAV replication.

All of the above additional constructs may be provided as plasmids or other episomal elements in the host cell, or alternatively one or more constructs may be integrated into the genome of the host cell.

In these aspects, the present invention provides a method for production of the vectors of the present invention. The method includes providing a vector comprising an adeno-associated virus (AAV) genome or a derivative thereof and a polynucleotide sequence encoding ND4 or a variant thereof in a host cell, and providing means for replication and assembly of the vector into an AAV viral particle. Preferably, the method includes providing a vector comprising a derivative of the AAV genome and a polynucleotide sequence encoding ND4 or a variant thereof, together with one or more additional genetic constructs encoding AAV and/or helper virus functions. Typically, the derivative of an AAV genome comprises at least one ITR. Optionally, the method further includes a step of purifying the assembled viral particles. Additionally, the method may include a step of formulating the viral particles for therapeutic use.

### Methods of therapy and medical uses

As discussed above, the present inventors have surprisingly demonstrated that a vector of the present invention may be used to address the cellular dysfunction underlying LHON. In particular, they have shown that the use of the vectors can correct the defect associated with LHON. This provides a means whereby the degenerative process of the disease can be treated, arrested, palliated, or prevented.

The present invention therefore provides a method for treating or preventing LHON in a patient in need thereof, comprising administering a therapeutically effective amount of a vector encoding a mitochondrial protein as described herein to the patient by direct retinal, subretinal, or intravitreal injection. In some embodiments, the method further includes administering a steroid before, during, and/or after administering the vector encoding the mitochondrial protein. Accordingly, LHON is thereby treated or prevented in the patient.

Vectors suitable for use in the methods of the present invention include the vectors described herein, equivalent vectors encoding mitochondrial proteins, and biosimilars thereof. Equivalent vectors encoding mitochondrial proteins suitable for use in the methods according to the present invention include those described in the art, such as those described by Guy et. al in Ophthalmology 2017; 124:1621-1634 and Vignal et. al in Ophthalmology 2018; 6:945-947, each of which is incorporated by reference in its entirety. Biosimilars are biological products that are highly similar to existing FDA-approved reference products ("RP") and have no clinically meaningful differences. A "highly similar" product is a product having similar purity, chemical properties, and biological activity as the reference product. However, minor differences between the clinically inactive components of the reference products and the proposed biosimilar products are acceptable. For example, these may include minor differences in stabilizers or buffers compared to the stabilizers or buffers used in the reference products, and slight differences (i.e., acceptable intra-product variations) are expected during the production process. The FDA will carefully assess any differences between the proposed biosimilar product and the reference product to ensure that the biosimilar meets high FDA approval standards. By "no clinically meaningful difference" is meant that the biosimilar product has no clinically meaningful difference in safety, purity, and potency (safety and efficacy) from the reference product, as is typically demonstrated by human pharmacokinetic (exposure) and pharmacodynamic (response) studies, clinical immunogenicity assessments, and, if necessary, additional clinical studies.

In some embodiments, a patient in need of treatment according to the methods provided herein has one or more mitochondrial DNA (mtDNA) point mutations. In some embodiments, the patient has a point mutation in a gene encoding a protein of complex I in the oxidative phosphorylation chain of mitochondria. For example, the patient may have one or more point mutations in the MT-ND4 gene (also known as ND4, NCBI gene ID: 4538) encoding NADH dehydrogenase subunit-4 protein (ND4). In some embodiments, the patient has a point mutation at nucleotide position 11778 in the ND4 gene. In some embodiments, the point mutation is G11778A in the ND4 gene. In some embodiments, a patient in need of treatment according to the methods provided herein has a G11778A point mutation in the ND4 gene and is of Chinese and/or Argentine ancestry.

In some embodiments, a patient in need of treatment according to the methods provided herein has a G11778A point mutation in the ND4 gene and is of Argentine ancestry. In some embodiments, a patient in need of treatment according to the methods provided herein has a G11778A point mutation in the ND4 gene is of Chinese ancestry.

In a related aspect, the present invention provides for use of a vector of the present invention in a method for treating or preventing LHON by administering the vector to a patient by direct retinal, subretinal, or intravitreal injection. Additionally, the present invention provides the use of a vector of the present invention in the manufacture of a medicament for treating or preventing LHON by direct retinal, subretinal, or intravitreal injection.

In all of these embodiments, the vectors of the present invention may be administered in order to prevent the onset of one or more symptoms of LHON. The patient may be asymptomatic. The subject may have a predisposition to the disease. The method or use may include a step of identifying whether or not a subject is at risk of developing, or has, LHON. A prophylactically effective amount of the vector is administered to such a subject. A prophylactically effective amount is an amount which prevents the onset of one or more symptoms of the disease.

Alternatively, the vector may be administered once the symptoms of the disease have appeared in a subject developed, i.e., to cure existing symptoms of the disease. A prophylactically effective amount of the antagonist is administered to such a subject. A therapeutically effective amount is an amount which is effective to ameliorate one or more symptoms of the disease. Such an amount may also arrest, slow, or reverse some loss of the peripheral vision associated with LHON. Such an amount may also arrest, slow, or reverse the onset of LHON.

A typical single dose is between 10¹⁰ and 10¹² genomic particles, depending on the amount of remaining retinal tissue that requires transduction. Genomic particles are defined herein as AAV capsids containing single-stranded DNA molecules that can be quantified using a sequence-specific method, such as real-time PCR. That dose may be provided as a single dose, but may be repeated for the contralateral eye or in cases where the vector may not have targeted the correct region of retina for whatever reason (e.g., surgical complications). The treatment is preferably a single permanent treatment for each eye, but repeated injections, for example in future years and/or with different AAV serotypes may be considered.

The present invention also provides a method for monitoring treatment or prevention of LHON in a patient including measuring activity ex vivo in retinal cells obtained from said patient following administration of the AAV vector of the present invention by direct retinal, subretinal, or intravitreal injection. Such a method may allow for the efficacy of the treatment to be determined.

In some embodiments, the present disclosure provides a method of treating an ocular disorder (e.g., LHON), which comprises administering a therapeutically effective amount of a vector and a steroid as described herein. Exemplary steroids include, but are not limited to, alclometasone dipropionate, amcinonide, beclomethasone dipropionate, betamethasone, betamethasone benzoate, betamethasone dipropionate, betamethasone sodium phosphate, betamethasone sodium phosphate and sodium acetate, betamethasone valerate, clobetasol propionate, clocortolone pivalate, cortisol (hydrocortisone), cortisol acetate (hydrocortisone), cortisone butyrate (hydrocortisone), cortisol cypionate (hydrocortisone), cortisol (hydrocortisone) sodium phosphate, cortisol (hydrocortisone) sodium succinate, cortisol valerate (hydrocortisone), cortisone acetate, desonide, desoximetasone, dexamethasone, dexamethasone acetate, dexamethasone sodium phosphate, diflorasone diacetate, fludrocortisone acetate, flunisolide, fluocinonide, fluocinolone acetonide acetate, fluorometholone, flurandrenolide, halcinonide, medrysone, methylprednisolone, methylprednisolone acetate, methylprednisolone sodium succinate, mometasone furoate, paramethasone acetate, prednisolone, prednisolone acetate, prednisolone sodium phosphate, prednisolone butylacetate, prednisone, triamcinolone, triamcinolone acetonide, triamcinolone diacetate, and triamcinolone hexaacetonide, or synthetic analogs thereof, or combinations thereof. In some embodiments, the steroid is a glucocorticoid. In some embodiments, the steroid is selected from the group consisting of prednisone, methylprednisolone, and methylprednisolone sodium succinate.

In some embodiments, the steroid is methylprednisolone. In some embodiments, the methylprednisolone is formulated as tablets, e.g. MEDROL@ tablets, for oral administration. For example, in some embodiments, methylprednisolone is formulated as a tablet with one or more inactive ingredients such as calcium stearate, corn starch, sodium erythrosine, lactose, mineral oil, sorbic acid, sucrose, or FD&C Yellow No. 6. In some embodiments, methylprednisolone is formulated as a liquid for administration by injection, e.g., SOLU-MEDROL^{®}. For example, in some embodiments, methylprednisolone sodium succinate is formulated as a liquid with one or more inactive ingredients such as anhydrous sodium dihydrogen phosphate, anhydrous disodium hydrogen phosphate, or hydrated lactose, optionally with a preservative such as benzyl alcohol. In some embodiments, the steroid is MEDROL^{®} or SOLU-MEDROL^{®}, including general form thereof.

In some embodiments, the patient receives one or more doses of steroid ranging from about 1 mg/60 kg to about 100 mg/60 kg, from about 1 mg/60 kg to about 80 mg/60 kg, from about 1 mg/60 kg to about 60 mg/60 kg, from about 1 mg/60 kg to about 40 mg/60 kg, from about 1 mg/60 kg to about 20 mg/60 kg, from about 20 mg/60 kg to about 100 mg/60 kg, from about 20 mg/60 kg to about 80 mg/60 kg, from about 20 mg/60 kg to about 60 mg/60 kg, from about 20 mg/60 kg to about 40 mg/60 kg, from about 40 mg/60 kg to about 100 mg/60 kg, from about 40 mg/60 kg to about 80 mg/60 kg, from about 40 mg/60 kg to about 60 mg/60 kg, from about 60 mg/60 kg to about 100 mg/60 kg, from about 60 mg/60 kg to about 80 mg/60 kg, or from about 80 mg/60 kg to about 100 mg/60 kg. In some embodiments, the patient receives one or more doses of steroid of about 4 mg/60 kg, 6 mg/60 kg, 8 mg/60 kg, 10 mg/60 kg, 16 mg/60 kg, 20 mg/60 kg, 24 mg/60 kg, 32 mg/60 kg, 40 mg/60 kg, 48 mg/60 kg, 60 mg/60 kg, or 80 mg/60 kg. In some embodiments, the patient receives one or more doses of steroid ranging from about 1 mg to about 96 mg. In some embodiments, the patient receives one or more doses of steroid of at least about 1 mg. In some embodiments, the patient receives one or more doses of steroid of up to about 96 mg. In some embodiments, the patient receives one or more doses of steroid ranging from about 1 mg to about 2 mg, from about 1 mg to about 4 mg, from about 1 mg to about 8 mg, from about 1 mg to about 16 mg, from about 1 mg to about 32 mg, from about 1 mg to about 64 mg, from about 1 mg to about 96 mg, from about 2 mg to about 4 mg, from about 2 mg to about 8 mg, from about 2 mg to about 16 mg, from about 2 mg to about 32 mg, from about 2 mg to about 64 mg, from about 2 mg to about 96 mg, from about 4 mg to about 8 mg, from about 4 mg to about 16 mg, from about 4 mg to about 32 mg, from about 4 mg to about 64 mg, from about 4 mg to about 96 mg, from about 8 mg to about 16 mg, from about 8 mg to about 32 mg, from about 8 mg to about 64 mg, from about 8 mg to about 96 mg, from about 16 mg to about 32 mg, from about 16 mg to about 64 mg, from about 16 mg to about 96 mg, from about 32 mg to about 64 mg, from about 32 mg to about 96 mg, or from about 64 mg to about 96 mg. In some embodiments, the patient receives one or more doses of steroid of about 1 mg, about 2 mg, about 4 mg, about 8 mg, about 16 mg, about 32 mg, about 64 mg, or about 96 mg. In some embodiments, the steroid is methylprednisolone (e.g., MEDROL@). In some embodiments, the steroid is prednisone.

In some embodiments, one or more doses of steroid (i.e., one or more doses of pre-operative steroid) are administered prior to administration of a therapeutic vector. In some embodiments, a daily dose of steroid is delivered for at least 1 day, 2 days, 3 days, 4 days, 5 days, 6 days, 7 days, 8 days, 9 days, or at least 10 days prior to administration of a therapeutic vector. In some embodiments, the patient receives one or more doses of pre-operative steroid ranging from about 1 mg/60 kg to about 100 mg/60 kg, from about 1 mg/60 kg to about 80 mg/60 kg, from about 1 mg/60 kg to about 60 mg/60 kg, from about 1 mg/60 kg to about 40 mg/60 kg, from about 1 mg/60 kg to about 20 mg/60 kg, from about 20 mg/60 kg to about 100 mg/60 kg, from about 20 mg/60 kg to about 80 mg/60 kg, from about 20 mg/60 kg to about 60 mg/60 kg, from about 20 mg/60 kg to about 40 mg/60 kg, from about 40 mg/60 kg to about 100 mg/60 kg, from about 40 mg/60 kg to about 80 mg/60 kg, from about 40 mg/60 kg to about 60 mg/60 kg, from about 60 mg/60 kg to about 100 mg/60 kg, from about 60 mg/60 kg to about 80 mg/60 kg, or from about 80 mg/60 kg to about 100 mg/60 kg. In some embodiments, the patient receives one or more doses of pre-operative steroid of about 4 mg/60 kg, 6 mg/60 kg, 8 mg/60 kg, 10 mg/60 kg, 16 mg/60 kg, 20 mg/60 kg, 24 mg/60 kg, 32 mg/60 kg, 40 mg/60 kg, 48 mg/60 kg, 60 mg/60 kg, or 80 mg/60 kg. In some embodiments, the patient receives one or more doses of pre-operative steroid ranging from about 1 mg to about 96 mg. In some embodiments, the patient receives one or more doses of pre-operative steroid of at least about 1 mg. In some embodiments, the patient receives one or more doses of pre-operative steroid of up to about 96 mg. In some embodiments, the patient receives one or more doses of pre-operative steroid ranging from about 1 mg to about 2 mg, from about 1 mg to about 4 mg, from about 1 mg to about 8 mg, from about 1 mg to about 16 mg, from about 1 mg to about 32 mg, from about 1 mg to about 64 mg, from about 1 mg to about 96 mg, from about 2 mg to about 4 mg, from about 2 mg to about 8 mg, from about 2 mg to about 16 mg, from about 2 mg to about 32 mg, from about 2 mg to about 64 mg, from about 2 mg to about 96 mg, from about 4 mg to about 8 mg, from about 4 mg to about 16 mg, from about 4 mg to about 32 mg, from about 4 mg to about 64 mg, from about 4 mg to about 96 mg, from about 8 mg to about 16 mg, from about 8 mg to about 32 mg, from about 8 mg to about 64 mg, from about 8 mg to about 96 mg, from about 16 mg to about 32 mg, from about 16 mg to about 64 mg, from about 16 mg to about 96 mg, from about 32 mg to about 64 mg, from about 32 mg to about 96 mg, or from about 64 mg to about 96 mg. In some embodiments, the patient receives one or more doses of pre-operative steroid of about 1 mg, about 2 mg, about 4 mg, about 8 mg, about 16 mg, about 32 mg, about 64 mg, or about 96 mg. In some embodiments, the steroid is methylprednisolone (e.g., MEDROL@). In some embodiments, the steroid is prednisone.

In some embodiments, the patient receives one or more doses of pre-operative steroid ranging from about 20 mg/60 kg to about 45 mg/60 kg, from about 25 mg/60 kg to about 45 mg/60 kg, from about 30 mg/60 kg to about 45 mg/60 kg, from about 35 mg/60 kg to about 45 mg/60 kg, from about 40 mg/60 kg to about 45 mg/60 kg, from about 20 mg/60 kg to about 40 mg/60 kg, from about 25 mg/60 kg to about 40 mg/60 kg, from about 30 mg/60 kg to about 40 mg/60 kg, from about 35 mg/60 kg to about 40 mg/60 kg, from about 20 mg/60 kg to about 35 mg/60 kg, from about 25 mg/60 kg to about 35 mg/60 kg, from about 30 mg/60 kg to about 35 mg/60 kg, from about 20 mg/60 kg to about 30 mg/60 kg, from about 25 mg/60 kg to about 30 mg/60 kg, or from about 20 mg/60 kg to about 25 mg/60 kg. In some embodiments, the patient receives one or more doses of pre-operative steroid of about 25 mg/60 kg, about 26 mg/60 kg, about 27 mg/60 kg, about 28 mg/60 kg, about 29 mg/60 kg, about 30 mg/60 kg, about 31 mg/60 kg, about 32 mg/60 kg, about 33 mg/60 kg, about 34 mg/60 kg, or about 35 mg/60 kg. In some embodiments, the steroid is methylprednisolone (e.g., MEDROL@). In some embodiments, a daily dose of methylprednisolone (e.g., MEDROL^{®}) ranging from 25 mg/60 kg to about 45 mg/60 kg is delivered for at least 1 day, 2 days, 3 days, 4 days, 5 days, 6 days, 7 days, 8 days, 9 days, or at least 10 days prior to administration of a therapeutic vector. In some embodiments, a daily dose of methylprednisolone (e.g., MEDROL@) of about 32 mg/60 kg is delivered for at least 1 day, 2 days, 3 days, 4 days, 5 days, 6 days, 7 days, 8 days, 9 days, or at least 10 days prior to administration of a therapeutic vector.

In some embodiments, the patient receives one or more doses of pre-operative steroid ranging from about 50 mg/60 kg to about 70 mg/60 kg, from about 55 mg/60 kg to about 70 mg/60 kg, from about 60 mg/60kg to about 70 mg/60 kg, from about 65 mg/60 kg to about 70 mg/60 kg, from about 50 mg/60 kg to about 65 mg/60 kg, from about 55 mg/60 kg to about 65 mg/60 kg, from about 60 mg/60 kg to about 65 mg/60 kg, from about 50 mg/60 kg to about 60 mg/60 kg, from about 55 mg/60 kg to about 60 mg/60 kg, or from about 50 mg/60 kg to about 55 mg/60 kg. In some embodiments, the patient receives one or more pre-operative doses of about 55 mg/60 kg, about 56 mg/60 kg, about 57 mg/60 kg, about 58 mg/60 kg, about 59 mg/60 kg, about 60 mg/k, about 61 mg/60 kg, about 62 mg/60 kg, about 63 mg/60 kg, about 64 mg/60 kg, or about 65 mg/60 kg. In some embodiments, the steroid is prednisone. In some embodiments, a daily dose of prednisone ranging from about 50 mg/60 kg to about 70 mg/60 kg is delivered for at least 1 day, 2 days, 3 days, 4 days, 5 days, 6 days, 7 days, 8 days, 9 days, or at least 10 days prior to administration of a therapeutic vector. In some embodiments, a daily dose of prednisone of about 60 mg/60 kg is delivered for at least 1 day, 2 days, 3 days, 4 days, 5 days, 6 days, 7 days, 8 days, 9 days, or at least 10 days prior to administration of a therapeutic vector.

In some embodiments, one or more doses of steroid (i.e., one or more doses of post-operative steroid) are administered following administration of a therapeutic vector. In some embodiments, a daily dose of steroid is delivered for at least 1 day, 2 days, 3 days, 4 days, 5 days, 6 days, 7 days, 8 days, 9 days, or at least 10 days following administration of a therapeutic vector. In some embodiments, a daily dose of steroid is delivered for at least 1 week, 2 weeks, 3 weeks, 4 weeks, 5 days, 6 weeks, 7 weeks, 8 weeks, 9 weeks, 10 weeks, 11 weeks, 12 weeks, 13 weeks, 14 weeks, or at least 15 weeks following administration of a therapeutic vector. In some embodiments, the patient receives one or more doses of post-operative steroid ranging from about 1 mg/60 kg to about 100 mg/60 kg, from about 1 mg/60 kg to about 80 mg/60 kg, from about 1 mg/60 kg to about 60 mg/60 kg, from about 1 mg/60 kg to about 40 mg/60 kg, from about 1 mg/60 kg to about 20 mg/60 kg, from about 20 mg/60 kg to about 100 mg/60 kg, from about 20 mg/60 kg to about 80 mg/60 kg, from about 20 mg/60 kg to about 60 mg/60 kg, from about 20 mg/60 kg to about 40 mg/60 kg, from about 40 mg/60 kg to about 100 mg/60 kg, from about 40 mg/60 kg to about 80 mg/60 kg, from about 40 mg/60 kg to about 60 mg/60 kg, from about 60 mg/60 kg to about 100 mg/60 kg, from about 60 mg/60 kg to about 80 mg/60 kg, and from about 80 mg/60 kg to about 100 mg/60 kg. In some embodiments, the patient receives one or more doses of post-operative steroid of about 1 mg to about 96 mg. In some embodiments, the patient receives one or more doses of post-operative steroid of at least about 1 mg. In some embodiments, the patient receives one or more doses of post-operative steroid of up to about 96 mg. In some embodiments, the patient receives one or more doses of post-operative steroid ranging from about 1 mg to about 2 mg, from about 1 mg to about 4 mg, from about 1 mg to about 8 mg, from about 1 mg to about 16 mg, from about 1 mg to about 32 mg, from about 1 mg to about 64 mg, from about 1 mg to about 96 mg, from about 2 mg to about 4 mg, from about 2 mg to about 8 mg, from about 2 mg to about 16 mg, from about 2 mg to about 32 mg, from about 2 mg to about 64 mg, from about 2 mg to about 96 mg, from about 4 mg to about 8 mg, from about 4 mg to about 16 mg, from about 4 mg to about 32 mg, from about 4 mg to about 64 mg, from about 4 mg to about 96 mg, from about 8 mg to about 16 mg, from about 8 mg to about 32 mg, from about 8 mg to about 64 mg, from about 8 mg to about 96 mg, from about 16 mg to about 32 mg, from about 16 mg to about 64 mg, from about 16 mg to about 96 mg, from about 32 mg to about 64 mg, from about 32 mg to about 96 mg, or from about 64 mg to about 96 mg. In some embodiments, the patient receives one or more doses of post-operative steroid of about 1 mg, about 2 mg, about 4 mg, about 8 mg, about 16 mg, about 32 mg, about 64 mg, or about 96 mg. In some embodiments, the steroid is methylprednisolone (e.g., MEDROL@). In some embodiments, the steroid is prednisone.

In some embodiments, the patient receives one or more doses of post-operative steroid ranging from about 70 mg/60 kg to about 90 mg/60 kg, 75 mg/60 kg to about 90 mg/60 kg, from about 80 mg/60 kg to about 90 mg/60 kg, from about 85 mg/60 kg to about 90 mg/60 kg, from about 70 mg/60 kg to about 85 mg/60 kg, from about 75 mg/60 kg to about 85 mg/60 kg, from about 80 mg/60 kg to about 85 mg/60 kg, from about 70 mg/60 kg to about 80 mg/60 kg, from about 75 mg/60 kg to about 80 mg/60 kg, or, from about 70 mg/60 kg to about 75 mg/60 kg. In some embodiments, the patient receives one or more doses of post-operative steroid of about 75 mg/60 kg, about 76 mg/60 kg, about 77 mg/60 kg, about 78 mg/60 kg, about 79 mg/60 kg, about 80 mg/60 kg, about 81 mg/60 kg, about 82 mg/60 kg, about 83 mg/60 kg, about 84 mg/60 kg, or about 85 mg/60 kg. In some embodiments, the steroid is methylprednisolone sodium succinate (e.g., SOLU-MEDROL^{®}). In some embodiments, a daily dose of methylprednisolone sodium succinate (e.g., SOLU-MEDROL^{®}) ranging from about 70 mg to about 90 mg/60 kg is delivered for at least 1 day, 2 days, 3 days, 4 days, 5 days, 6 days, 7 days, 8 days, 9 days, or at least 10 days following administration of a therapeutic vector. In some embodiments, a daily dose of methylprednisolone sodium succinate (e.g., SOLU-MEDROL^{®}) of about 80 mg/60 kg is delivered for at least 1 day, 2 days, 3 days, 4 days, 5 days, 6 days, 7 days, 8 days, 9 days, or at least 10 days following administration of a therapeutic vector.

In some embodiments, the patient receives one or more doses of post-operative steroid ranging from about 30 mg/60 kg to about 50 mg/60 kg, 35 mg/60 kg to about 50 mg/60 kg, from about 40 mg/60 kg to about 50 mg/60 kg, from about 45 mg/60 kg to about 50 mg/60 kg, from about 30 mg/60 kg to about 45 mg/60 kg, from about 35 mg/60 kg to about 45 mg/60 kg, from about 40 mg/60 kg to about 45 mg/60 kg, from about 30 mg/60 kg to about 40 mg/60 kg, from about 35 mg/60 kg to about 40 mg/60 kg, or from about 30 mg/60 kg to about 35 mg/60 kg. In some embodiments, the patient receives one or more doses of post-operative steroid of about 35 mg/60 kg, about 36 mg/60 kg, about 37 mg/60 kg, about 38 mg/60 kg, about 39 mg/60 kg, about 40 mg/60 kg, about 41 mg/60 kg, about 42 mg/60 kg, about 43 mg/60 kg, about 44 mg/60 kg, or about 45 mg/60 kg. In some embodiments, the steroid is methylprednisolone (e.g., MEDROL@). In some embodiments, a daily dose of methylprednisolone (e.g., MEDROL@) ranging from about 30 mg/60 kg to about 50 mg/60 kg is delivered for at least 1 day, 2 days, 3 days, 4 days, 5 days, 6 days, 7 days, 8 days, 9 days, or at least 10 days following administration of a therapeutic vector. In some embodiments, a daily dose of methylprednisolone (e.g., MEDROL^{®}) of about 40 mg/60 kg is delivered for at least 1 day, 2 days, 3 days, 4 days, 5 days, 6 days, 7 days, 8 days, 9 days, or at least 10 days following administration of a therapeutic vector.

In some embodiments, the patient receives one or more doses of post-operative steroid ranging from about 20 mg/60 kg to about 45 mg/60 kg, 25 mg/60 kg to about 45 mg/60 kg, from about 30 mg/60 kg to about 45 mg/60 kg, from about 35 mg/60 kg to about 45 mg/60 kg, from about 40 mg/60 kg to about 45 mg/60 kg, from about 20 mg/60 kg to about 40 mg/60 kg, from about 25 mg/60 kg to about 40 mg/60 kg, from about 30 mg/60 kg to about 40 mg/60 kg, from about 35 mg/60 kg to about 40 mg/60 kg, from about 20 mg/60 kg to about 35 mg/60 kg, from about 25 mg/60 kg to about 35 mg/60 kg, from about 30 mg/60 kg to about 35 mg/60 kg, from about 20 mg/60 kg to about 30 mg/60 kg, from about 25 mg/60 kg to about 30 mg/60 kg, or from about 20 mg/60 kg to about 25 mg/60 kg. In some embodiments, the patient receives one or more doses of post-operative steroid of about 25 mg/60 kg, about 26 mg/60 kg, about 27 mg/60 kg, about 28 mg/60 kg, about 29 mg/60 k, about 30 mg/60 kg, about 31 mg/60 kg, about 32 mg/60 kg, about 33 mg/60 kg, about 34 mg/60 kg, or about 35 mg/60 kg. In some embodiments, the steroid is methylprednisolone (e.g., MEDROL@). In some embodiments, a daily dose of methylprednisolone (e.g., MEDROL@) ranging from about 20 mg/60 kg to about 45 mg/60 kg is delivered for at least 1 day, 2 days, 3 days, 4 days, 5 days, 6 days, 7 days, 8 days, 9 days, or at least 10 days following administration of a therapeutic vector. In some embodiments, a daily dose of methylprednisolone (e.g., MEDROL@) of about 32 mg/60 kg is delivered for at least 1 day, 2 days, 3 days, 4 days, 5 days, 6 days, 7 days, 8 days, 9 days, or at least 10 days following administration of a therapeutic vector.

In some embodiments, the patient receives one or more doses of post-operative steroid ranging from about 15 mg/60 kg to about 35 mg/60 kg, 20 mg/60 kg to about 35 mg/60 kg, about 25 mg/60 kg to about 35 mg/60 kg, about 30 mg/60 kg to about 35 mg/60 kg, about 15 mg/60 kg to about 30 mg/60 kg, about 20 mg/60 kg to about 30 mg/60 kg, about 25 mg/60 kg to about 30 mg/60 kg, about 15 mg/60 kg to about 25 mg/60 kg, about 20 mg/60 kg to about 25 mg/60 kg or about 15 mg/60 kg to about 20 mg/60 kg. In some embodiments, the patient receives one or more doses of post-operative steroid ranging from about 20 mg/60 kg, about 21 mg/60 kg, about 22 mg/60 kg, about 23 mg/60 kg, about 24 mg/60 kg, about 25 mg/60 kg, about 26 mg/60 kg, about 27 mg/60 kg, about 28 mg/60 kg, about 29 mg/60 kg or about 30 mg/60 kg. In some embodiments, the steroid is methylprednisolone (e.g., MEDROL@). In some embodiments, methylprednisolone (e.g., MEDROL@) with a daily dose of about 15 mg/60 kg to about 35 mg/60 kg is delivered for at least 1 day, 2 days, 3 days, 4 days, 5 days, 6 days, 7 days, 8 days, 9 days or at least 10 days following administration of a therapeutic vector. In some embodiments, methylprednisolone (e.g., MEDROL@) with a daily dose of about 24 mg/60 kg is delivered for at least 1 day, 2 days, 3 days, 4 days, 5 days, 6 days, 7 days, 8 days, 9 days or at least 10 days following administration of a therapeutic vector.

In some embodiments, the patient receives one or more doses of post-operative steroid ranging from about 5 mg/60 kg to about 25 mg/60 kg, 10 mg/60 kg to about 25 mg/60 kg, about 15 mg/60 kg to about 25 mg/60 kg, about 20 mg/60 kg to about 25 mg/60 kg, about 5 mg/60 kg to about 20 mg/60 kg, about 10 mg/60 kg to about 20 mg/60 kg, about 15 mg/60 kg to about 20 mg/60 kg, about 5 mg/60 kg to about 15 mg/60 kg, about 10 mg/60 kg to about 15 mg/60 kg or about 5 mg/60 kg to about 10 mg/60 kg. In some embodiments, the patient receives one or more doses of post-operative steroid ranging from about 10 mg/60 kg, about 11 mg/60 kg, about 12 mg/60 kg, about 13 mg/60 kg, about 14 mg/60 kg, about 15 mg/60 kg, about 16 mg/60 kg, about 17 mg/60 kg, about 18 mg/60 kg, about 19 mg/60 kg or about 20 mg/60 kg. In some embodiments, the steroid is methylprednisolone (e.g., MEDROL@). In some embodiments, methylprednisolone (e.g., MEDROL@) with a daily dose of about 5 mg/60 kg to about 25 mg/60 kg is delivered for at least 1 day, 2 days, 3 days, 4 days, 5 days, 6 days, 7 days, 8 days, 9 days or at least 10 days following administration of a therapeutic vector. In some embodiments, methylprednisolone (e.g., MEDROL@) with a daily dose of about 16 mg/60 kg is delivered for at least 1 day, 2 days, 3 days, 4 days, 5 days, 6 days, 7 days, 8 days, 9 days or at least 10 days following administration of a therapeutic vector.

In some embodiments, the patient receives one or more doses of post-operative steroid ranging from about 1 mg/60 kg to about 20 mg/60 kg, 5 mg/60 kg to about 20 mg/60 kg, from about 10 mg/60 kg to about 20 mg/60 kg, from about 15 mg/60 kg to about 20 mg/60 kg, from about 1 mg/60 kg to about 15 mg/60 kg, from about 5 mg/60 kg to about 15 mg/60 kg, from about 10 mg/60 kg to about 15 mg/60 kg, from about 1 mg/60 kg to about 10 mg/60 kg, from about 5 mg/60 kg to about 10 mg/60 kg, or from about 1 mg/60 kg to about 5 mg/60 kg. In some embodiments, the patient receives one or more doses of post-operative steroid ranging from about 1 mg/60 kg, about 2 mg/60 kg, about 3 mg/60 kg, about 4 mg/60 kg, about 5 mg/60 kg, about 6 mg/60 kg, about 7 mg/60 kg, about 8 mg/60 kg, about 9 mg/60 kg, about 10 mg/60 kg, about 11 mg/60 kg, about 12 mg/60 kg, about 13 mg/60 kg, about 14 mg/60 kg, or about 15 mg/60 kg. In some embodiments, the steroid is methylprednisolone (e.g., MEDROL@). In some embodiments, methylprednisolone (e.g., MEDROL@) with a daily dose of about 1 mg/60 kg to about 20 mg/60 kg is delivered for at least 1 day, 2 days, 3 days, 4 days, 5 days, 6 days, 7 days, 8 days, 9 days or at least 10 days following administration of a therapeutic vector. In some embodiments, methylprednisolone (e.g., MEDROL@) with a daily dose of about 8 mg/60 kg is delivered for at least 1 day, 2 days, 3 days, 4 days, 5 days, 6 days, 7 days, 8 days, 9 days or at least 10 days following administration of a therapeutic vector. In some embodiments, methylprednisolone (e.g., MEDROL@) with a daily dose of about 6 mg/60 kg is delivered for at least 1 day, 2 days, 3 days, 4 days, 5 days, 6 days, 7 days, 8 days, 9 days or at least 10 days following administration of a therapeutic vector. In some embodiments, methylprednisolone (e.g., MEDROL@) with a daily dose of about 4 mg/60 kg is delivered for at least 1 day, 2 days, 3 days, 4 days, 5 days, 6 days, 7 days, 8 days, 9 days or at least 10 days following administration of a therapeutic vector.

In some embodiments, the patient receives one or more doses of post-operative steroid ranging from about 30 mg/60 kg to about 50 mg/60 kg, 35 mg/60 kg to about 50 mg/60 kg, from about 40 mg/60 kg to about 50 mg/60 kg, from about 45 mg/60 kg to about 50 mg/60 kg, from about 30 mg/60 kg to about 45 mg/60 kg, from about 35 mg/60 kg to about 45 mg/60 kg, from about 40 mg/60 kg to about 45 mg/60 kg, from about 30 mg/60 kg to about 40 mg/60 kg, from about 35 mg/60 kg to about 40 mg/60 kg, or from about 30 mg/60 kg to about 35 mg/60 kg. In some embodiments, the patient receives one or more doses of post-operative steroid of about 35 mg/60 kg, about 36 mg/60 kg, about 37 mg/60 kg, about 38 mg/60 kg, about 39 mg/60 kg, about 40 mg/60 kg, about 41 mg/60 kg, about 42 mg/60 kg, about 43 mg/60 kg, about 44 mg/60 kg, or about 45 mg/60 kg. In some embodiments, the steroid is prednisone. In some embodiments, prednisone with a daily dose of about 30 mg/60 kg to about 50 mg/60 kg is delivered for at least 1 day, 2 days, 3 days, 4 days, 5 days, 6 days, 7 days, 8 days, 9 days or at least 10 days following administration of a therapeutic vector. In some embodiments, prednisone with a daily dose of about 40 mg/60 kg is delivered for at least 1 day, 2 days, 3 days, 4 days, 5 days, 6 days, 7 days, 8 days, 9 days or at least 10 days following administration of a therapeutic vector.

In some embodiments, the patient receives one or more doses of post-operative steroid ranging from about 10 mg/60 kg to about 30 mg/60 kg, 15 mg/60 kg to about 30 mg/60 kg, about 20 mg/60 kg to about 30 mg/60 kg, about 25 mg/60 kg to about 30 mg/60 kg, about 10 mg/60 kg to about 25 mg/60 kg, about 15 mg/60 kg to about 25 mg/60 kg, about 20 mg/60 kg to about 25 mg/60 kg, about 10 mg/60 kg to about 20 mg/60 kg, about 15 mg/60 kg to about 20 mg/60 kg or about 10 mg/60 kg to about 15 mg/60 kg. In some embodiments, the patient receives one or more doses of post-operative steroid ranging from about 15 mg/60 kg, about 16 mg/60 kg, about 17 mg/60 kg, about 18 mg/60 kg, about 19 mg/60 kg, about 20 mg/60 kg, about 21 mg/60 kg, about 22 mg/60 kg, about 23 mg/60 kg, about 24 mg/60 kg or about 25 mg/60 kg. In some embodiments, the steroid is prednisone. In some embodiments, prednisone with a daily dose of about 10 mg/60 kg to about 30 mg/60 kg is delivered for at least 1 day, 2 days, 3 days, 4 days, 5 days, 6 days, 7 days, 8 days, 9 days or at least 10 days following administration of a therapeutic vector. In some embodiments, prednisone with a daily dose of about 20 mg/60 kg is delivered for at least 1 day, 2 days, 3 days, 4 days, 5 days, 6 days, 7 days, 8 days, 9 days or at least 10 days following administration of a therapeutic vector.

In some embodiments, the patient receives one or more doses of post-operative steroid ranging from about 1 mg/60 kg to about 20 mg/60 kg, 5 mg/60 kg to about 20 mg/60 kg, from about 10 mg/60 kg to about 20 mg/60 kg, from about 15 mg/60 kg to about 20 mg/60 kg, from about 1 mg/60 kg to about 15 mg/60 kg, from about 5 mg/60 kg to about 15 mg/60 kg, from about 10 mg/60 kg to about 15 mg/60 kg, from about 1 mg/60 kg to about 10 mg/60 kg, from about 5 mg/60 kg to about 10 mg/60 kg, or from about 1 mg/60 kg to about 5 mg/60 kg. In some embodiments, the patient receives one or more doses of post-operative steroid ranging from about 1 mg/60 kg, about 2 mg/60 kg, about 3 mg/60 kg, about 4 mg/60 kg, about 5 mg/60 kg, about 6 mg/60 kg, about 7 mg/60 kg, about 8 mg/60 kg, about 9 mg/60 kg, about 10 mg/60 kg, about 11 mg/60 kg, about 12 mg/60 kg, about 13 mg/60 kg, about 14 mg/60 kg, or about 15 mg/60 kg. In some embodiments, the steroid is prednisone. In some embodiments, prednisone with a daily dose of about 1 mg/60 kg to about 20 mg/60 kg is delivered for at least 1 day, 2 days, 3 days, 4 days, 5 days, 6 days, 7 days, 8 days, 9 days or at least 10 days following administration of a therapeutic vector. In some embodiments, prednisone with a daily dose of about 10 mg/60 kg is delivered for at least 1 day, 2 days, 3 days, 4 days, 5 days, 6 days, 7 days, 8 days, 9 days or at least 10 days following administration of a therapeutic vector.

In some embodiments, the patient receives one or more doses of post-operative steroid ranging from about 1 g/60 kg to about 15 g/60 kg, about 5 g/60 kg to about 15 g/60 kg, about 10 g/60 kg to about 15 g/60 kg, about 1 g/60 kg to about 10 g/60 kg, about 5 g/60 kg to about 10 g/60 kg or about 1 g/60 kg to about 5 g/60 kg. In some embodiments, the patient receives one or more doses of post-operative steroid ranging from about 1 g/60 kg, about 2 g/60 kg, about 3 g/60 kg, about 4 g/60 kg, about 5 g/60 kg, about 6 g/60 kg, about 7 g/60 kg, about 8 g/60 kg, about 9 g/60 kg, about 10 g/60 kg, about 11 g/60 kg, about 12 g/60 kg, about 13 g/60 kg, about 14 g/60 kg or about 15 g/60 kg. In some embodiments, the steroid is creatine phosphate sodium. In some embodiments, creatine phosphate sodium with a daily dose of about 1 g/60 kg to about 15 g/60 kg is delivered for at least 1 day, 2 days, 3 days, 4 days, 5 days, 6 days, 7 days, 8 days, 9 days or at least 10 days following administration of a therapeutic vector. In some embodiments, creatine phosphate sodium with a daily dose of about 2 g/60 kg is delivered for at least 1 day, 2 days, 3 days, 4 days, 5 days, 6 days, 7 days, 8 days, 9 days or at least 10 days following administration of a therapeutic vector.

In some embodiments, the patient receives an intravenous dose of creatine phosphate sodium (2 g/60 kg) and an intravenous dose of methylprednisolone sodium succinate (e.g., SOL-MEDROL^{®}, 80 mg/60 kg) on the day when the therapeutic AAV vector is administered, and receive both the above drugs daily for consecutive 3 days following administration of the therapeutic AAV vector. On day 3 after administration of the therapeutic AAV vector, methylprednisolone (e.g., MEDROL@) tablets at a dose of 40 mg/60 kg are administered to patients for consecutive 4 days. On day 7 after administration of the therapeutic AAV vector, methylprednisolone (e.g., MEDROL@) tablets at a dose of 32 mg/60 kg are administered to patients for consecutive 7 days. On day 14 after administration of the therapeutic AAV vector, methylprednisolone (e.g., MEDROL@) tablets at a dose of 24 mg/60 kg are administered to patients for consecutive 7 days. On day 21 after administration of the therapeutic AAV vector, methylprednisolone (e.g., MEDROL@) tablets at a dose of 16 mg/60 kg are administered to patients for consecutive 7 days. On day 28 after administration of the therapeutic AAV vector, methylprednisolone (e.g., MEDROL@) tablets at a dose of 8 mg/60 kg are administered to patients for consecutive 7 days. On day 35 after administration of the therapeutic AAV vector, methylprednisolone (e.g., MEDROL@) tablets at a dose of 6 mg/60 kg are administered to patients for consecutive 7 days. On day 42 after administration of the therapeutic AAV vector, methylprednisolone (e.g., MEDROL@) tablets at a dose of 4 mg/60 kg are administered to patients for consecutive 7 days.

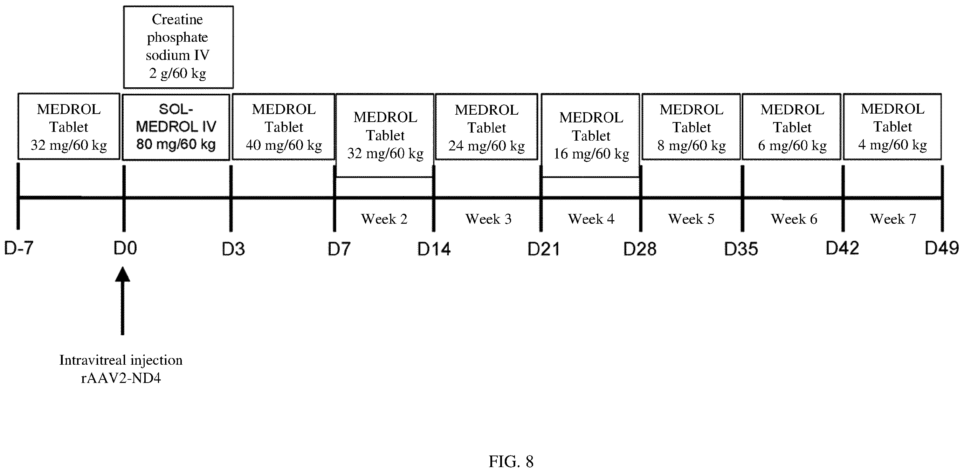

In some embodiments, 7 days before administration of the therapeutic AAV vector, the patient receives methylprednisolone (e.g., MEDROL@) tablets at a dose of 32 mg/60 kg. On the day when the therapeutic AAV vector is administered, the patient receives an intravenous dose of methylprednisolone sodium succinate (e.g., SOL-MEDROL^{®}, 80 mg/60 kg) daily for consecutive 3 days. On day 3 after administration of the therapeutic AAV vector, methylprednisolone (e.g., MEDROL@) tablets at a dose of 40 mg/60 kg are administered to patients for consecutive 4 days. On day 7 after administration of the therapeutic AAV vector, methylprednisolone (e.g., MEDROL@) tablets at a dose of 32 mg/60 kg are administered to patients for consecutive 7 days. On day 14 after administration of the therapeutic AAV vector, methylprednisolone (e.g., MEDROL@) tablets at a dose of 24 mg/60 kg are administered to patients for consecutive 7 days. On day 21 after administration of the therapeutic AAV vector, methylprednisolone (e.g., MEDROL@) tablets at a dose of 16 mg/60 kg are administered to patients for consecutive 7 days. On day 28 after administration of the therapeutic AAV vector, methylprednisolone (e.g., MEDROL@) tablets at a dose of 8 mg/60 kg are administered to patients for consecutive 7 days. On day 35 after administration of the therapeutic AAV vector, methylprednisolone (e.g., MEDROL@) tablets at a dose of 6 mg/60 kg are administered to patients for consecutive 7 days. On day 42 after administration of the therapeutic AAV vector, methylprednisolone (e.g., MEDROL@) tablets at a dose of 4 mg/60 kg are administered to patients for consecutive 7 days. An exemplary schematic diagram of a treatment regimen of LHON gene therapy is shown in FIG. 8.

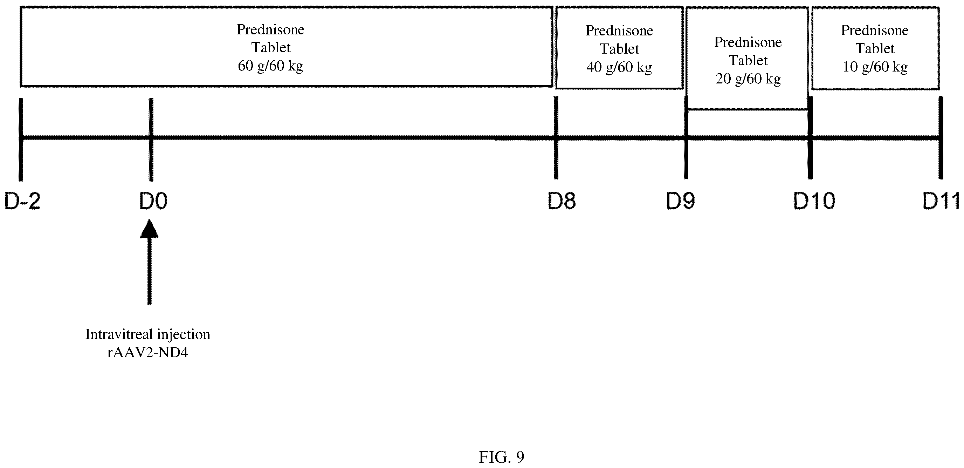

In some embodiments, the patient receives prednisone tablets at a dose of 60 mg/60 kg prior to administration of the therapeutic AAV vector and receive prednisone tablets daily for consecutive 7 days following administration of the therapeutic AAV vector. On day 8 after administration of the therapeutic AAV vector, prednisone tablets at a dose of 40 mg/kg are administered to the patients for 1 day. On day 9 after administration of the therapeutic AAV vector, prednisone tablets at a dose of 20 mg/kg are administered to the patients for 1 day. On day 10 after administration of the therapeutic AAV vector, prednisone tablets at a dose of 10 mg/kg are administered to the patients for 1 day. An exemplary schematic diagram of a treatment regimen of LHON gene therapy is shown in FIG. 9.

The dose and the interval can be adjusted individually to be sufficient to maintain the therapeutic effect. A person skilled in the art will be able to optimize the effective local dose without undue experiments.

In some embodiments, administration of steroids before, during and/or after administration of the therapeutic AAV vector described herein results in a higher average vision recovery in a population of, e.g., at least 10 patients, compared to administration of an equivalent therapeutic AAV vector free of steroids. In some embodiments, administration of steroids before, during, and/or after administration of the therapeutic AAV vector results in lower incidence of adverse events in a population of, e.g., at least 10 patients, compared to administration of an equivalent therapeutic AAV vector free of steroids. In some embodiments, the adverse events are selected from anterior chamber inflammation, vitritis, ocular hypertension, cataract removal, keratitis, vitreous hemorrhage, allergic conjunctivitis and ophthalmalgia.

In some embodiments, the higher average vision recovery and lower incidence of adverse events achieved according to the methods of the present invention are determined by comparison to a population of patients with ocular disorders treated by using a therapeutic AAV vector free of steroids before, during, and/or after administration of the therapeutic AAV vector. In some embodiments, a population of patients treated according to the methods of the present disclosure and a population of patients treated by using an equivalent therapeutic AAV vector are ethnically matched. In some embodiments, the population of patients is Chinese or Argentinean.

### Diagnostic method and kit

In some embodiments, the present disclosure provides a method for screening patients for treatment of ocular disorders. In such embodiments, the method comprises culturing a target cell population together with a composition comprising an AAV in the presence of a serum sample obtained from patients, wherein the AAV comprises a recombinant nucleic acid sequence encoding a detectable marker; and detecting the expression level of the detectable marker in target cells after culture, wherein the patients are selected for treatment if the expression level of the detectable marker in the target cells is higher than a predetermined threshold. In some embodiments, the method further comprises administering a pharmaceutical composition comprising an AAV to the patients, wherein the AAV comprises a recombinant nucleic acid sequence encoding a mitochondrial protein.

The method for screening patients for treatment of ocular disorders described herein utilizes the serum sample obtained from patients to assess the immune response of particular patients against a recombinant viral vector for the delivery of therapeutic proteins. Soluble factors (e.g., antibodies) present in the serum of patients can prevent viral infection of target cells, thereby reducing the delivery of therapeutic proteins and/or reducing the efficacy of the pharmaceutical composition. The method of the present disclosure utilizes AAV encoding a detectable marker, such that the level of infectivity of target cells can be measured by detecting the marker. In some embodiments, the present disclosure provides a method for identifying patients exhibiting low immunoreactivity to an AAV composition and for selecting such patients for treatment with a therapeutic AAV vector described herein. In some embodiments, the present disclosure provides a method for identifying patients exhibiting high immunoreactivity to an AAV composition and for excluding such patients from future treatment with a therapeutic AAV vector described herein.

In some embodiments, the expression levels of the detectable markers in the target cells are correlated with the immune response of the patients against the AAV vector. For example, serum from patients that exhibits high immunoreactivity to AAV contains soluble factors that prevent AAV encoding a detectable marker from infecting target cells and preventing expression of the detectable markers in the target cells. In such cases, the expression levels of the detectable markers in the target cells cultured in the presence of patient serum are reduced relative to the expression levels of the detectable markers in the target cells cultured in the absence of patient serum. Alternatively, serum from patients that exhibits low immunoreactivity to AAV contains less or no soluble factors that prevent AAV encoding a detectable marker from infecting target cells. In such cases, the expression levels of the detectable markers in the target cells cultured in the presence of patient serum is the same or not significantly reduced relative to the expression levels of the detectable markers in the target cells cultured in the absence of the patient serum.

The detectable marker may be any protein or nucleic acid molecule that is not endogenously expressed by target cells and/or AAV vectors. Examples of the detectable marker include, but are not limited to, FLAG tags, polyhistidine tags (e.g., 6xHis), SNAP tags, Halo tags, cMyc tags, glutathione-S-transferase tags, avidin, enzymes, fluorescent proteins, luminescent proteins, chemiluminescent proteins, bioluminescent proteins and phosphorescent proteins. In some embodiments, the fluorescent protein is selected from the group consisting of: blue/UV proteins (e.g., BFP, TagBFP, mTagBFP2, Azurite, EBFP2, mKalama1, Sirius, Sapphire and T-Sapphire); cyan proteins (e.g., CFP, eCFP, Cerulean, SCFP3A, mTurquoise, mTurquoise2, monomer Midorisishi-Cyan, TagCFP and mTFP1); green proteins (e.g., GFP, eGFP, meGFP (A208K mutation), Emerald, Superfolder GFP, monomeric Azami Green, TagGFP2, mUKG, mWasabi, Clover and mNeon Green); yellow proteins (such as YFP, eYFP, Citrine, Venus, SYFP2 and TagYFP); orange proteins (e.g., monomers Kusabira-Orange, mKO κ, mKO2, mOrange and mOrange2); red proteins (e.g., RFP, mRaspberry, mCherry, mStrawberry, mTangerine, tdTomato, TagRFP, TagRFP-T, mApple, mRuby and mRuby2); far-infrared proteins (e.g., mPlum, HcRed-Tandem, mKate2, mNeptune and NirFP); near-infrared proteins (e.g., TagRFP657, IFP1.4 and iRFP); long stokes shift proteins (e.g., mKeima Red, LSS-mKate1, LSS-mKate2 and mBeRFP); photoactivatable proteins (e.g., PA-GFP, PAmCherry1 and PATagRFP); photoconvertible proteins (e.g., Kaede (green), Kaede (red), KikGR1 (green), KikGR1 (red), PS-CFP2, PS-CFP2, mEos2 (green), mEos2 (red), mEos3.2 (green), mEos3.2 (red), PSmOrange and PSmOrange); and photoswitching proteins (such as Dronpa). In some embodiments, the detectable marker may be selected from AmCyan, AsRed, DsRed2, DsRed Express, E2-Crimson, HcRed, ZsGreen, ZsYellow, mCherry, mStrawberry, mOrange, mBanana, mPlum, mRasberry, tdTomato, DsRed monomer and/or AcGFP, all of which are available from Clontech. In a particular embodiment, the detectable marker is GFP.

The detectable marker can be detected by methods generally known in the art, including but not limited to flow cytometry, qPCR, Western blot, ELISA and immunohistochemistry. In a particular embodiment, the detection method is a high-throughput detection method, such as flow cytometry or qPCR, such that a plurality of patient samples can be analyzed simultaneously. In some embodiments, the detection method is flow cytometry. In some embodiments, the detection method is qPCR.

In some embodiments, a predetermined threshold of the expression level of the detectable marker is set for screening patients for treatment of ocular disorders. In some embodiments, patients that meet or exceed this threshold are selected for treatment with a therapeutic AAV vector described herein. In some embodiments, patients that do not meet this threshold are excluded from future treatment with a therapeutic AAV vector described herein, or must receive an immunosuppressive regimen before beginning treatment with a therapeutic AAV vector described herein.

In some embodiments, the predetermined threshold may be expressed as an absolute expression level of the detectable marker in the test sample. Patients with the expression level higher than the absolute expression level are characterized as suitable for gene therapy, and/or patients with the expression level lower than the absolute expression level are characterized as unsuitable for gene therapy. For example, in some embodiments where the detectable marker is detected by qPCR, the predetermined threshold is an absolute expression level greater than or equal to 0.2. In such embodiments, patients are characterized as suitable for gene therapy if the absolute expression level of the detectable marker is greater than or equal to 0.2, and are characterized as unsuitable for gene therapy if the absolute expression level of the detectable marker is less than 0.2. In some embodiments, the predetermined threshold is an absolute expression level greater than or equal to 0.6. In such embodiments, patients are characterized as suitable for gene therapy if the absolute expression level of the detectable marker is greater than or equal to 0.6, and are characterized as unsuitable for gene therapy if the absolute expression level of the detectable marker is less than 0.6.

In some embodiments where the detectable marker is detected by flow cytometry, the predetermined threshold is the absolute expression level of marker-positive target cells greater than or equal to 20% in the test sample (e.g., % GFP + cells ≥ 20%). In such embodiments, patients are characterized as suitable for gene therapy if the absolute expression level of the detectable marker is the absolute expression level of marker-positive target cells greater than or equal to 20%, and patients are characterized as unsuitable for gene therapy if the absolute expression level of the detectable marker is the absolute expression level of marker-positive target cells less than 20%. In some embodiments, the predetermined threshold is the absolute expression level of marker-positive target cells greater than or equal to 40% in the test sample (e.g., %GFP + cells ≥ 40%). In such embodiments, the patient is characterized as suitable for gene therapy if the absolute expression level of the detectable marker is the absolute expression level of marker-positive target cells greater than or equal to 40%, and the patient is characterized as unsuitable for gene therapy if the absolute expression level of the detectable marker is the absolute expression level of marker-positive target cells less than 40%.

In some embodiments, the predetermined threshold may be expressed as a relative expression level of the detectable marker in the test sample (i.e., detecting expression of the detectable marker in the test sample relative to the control sample). The patient with the expression level higher than the relative expression level is characterized as suitable for gene therapy, and/or the patient with the expression level lower than the relative expression level is characterized as unsuitable for gene therapy. In some embodiments where the detectable marker is detected by flow cytometry, the predetermined threshold is the relative expression level of marker-positive target cells greater than or equal to 40% in the test sample (e.g., %GFP + cells ≥ 40%). In such embodiments, the patient is characterized as suitable for gene therapy if the absolute expression level of the detectable marker is the absolute expression level of marker-positive target cells greater than or equal to 40%, and the patient is characterized as unsuitable for gene therapy if the absolute expression level of the detectable marker is the absolute expression level of marker-positive target cells less than 40%. In some embodiments where the detectable marker is detected by flow cytometry, the predetermined threshold is the relative expression level of marker-positive target cells greater than or equal to 80% in the test sample (e.g., %GFP + cells ≥ 80%). In such embodiments, the patient is characterized as suitable for gene therapy if the absolute expression level of the detectable marker is the absolute expression level of marker-positive target cells greater than or equal to 80%, and the patient is characterized as unsuitable for gene therapy if the absolute expression level of the detectable marker is the absolute expression level of marker-positive target cells less than 80%.

In some embodiments, the patient screened and/or selected for treatment according to the method described herein has one or more mtDNA point mutations. In some embodiments, the patient has a point mutation in a gene encoding a protein of complex I in the oxidative phosphorylation chain of mitochondria. For example, the patient may have one or more point mutations in the ND4 gene. In some embodiments, the patient has a point mutation at nucleotide position 11778 in the ND4 gene. In some embodiments, the point mutation is G11778A in the ND4 gene. In some embodiments, the patient screened and/or selected for treatment according to the method described herein has a G11778A point mutation in the ND4 gene and is of Chinese and/or Argentinean ancestry.

In some embodiments, the patient screened and/or selected for treatment according to the method described herein has a G11778A point mutation in the ND4 gene and is of Argentinean ancestry. In some embodiments, the patient screened and/or selected for treatment according to the method described herein has a G11778A point mutation in the ND4 gene and is of Chinese ancestry.

In some embodiments, the present disclosure provides a kit for screening patients for treatment of ocular disorders and/or for selecting patients for treatment of ocular disorders. In such embodiments, the kit comprises: an AAV comprising a recombinant nucleic acid encoding a detectable marker, and one or more reagents for detecting the detectable marker. In some embodiments, the one or more reagents for detecting the detectable marker are selected from antibodies that bind to the detectable marker and one or more primer oligonucleotides specific to the recombinant nucleic acid encoding the detectable marker.

In some embodiments, the kit further comprises one or more reagents for reconstituting and/or diluting the AAV vector and/or detecting the reagent components. In some embodiments, the kit further comprises one or more additional reagents, such as buffers, washing buffers and/or cell culture medium for introducing the AAV vector into cells. The components of the kit may be placed in separate containers or may be combined in a single container.

In addition to the components mentioned above, in some embodiments, the kit further comprises an instruction for using the components of the kit to implement the method of the present disclosure. The instruction for implementing the method is generally recorded on a suitable recording medium. For example, the instruction may be printed on a substrate such as paper or plastic. As such, the instruction may be present in the kit as a package insert, or in a label (i.e., associated with the package or sub-package) of the kit or the containers of components of the kit. In other embodiments, the instruction exists as an electronically stored data file on a suitable computer readable storage medium (e.g., CD-ROM, a magnetic disk and a flash drive). In yet other embodiments, the actual instruction is not present in the kit, but a means of obtaining the instruction from a remote source, e.g., via the internet, is provided. An example of this embodiment is a kit that includes a website where the instruction can be viewed and/or downloaded. As with the instruction, the means of obtaining the instruction is recorded on a suitable substrate.

### Pharmaceutical compositions and excipients

The vector of the present invention may be formulated into pharmaceutical compositions. In addition to the vector, these compositions may also contain pharmaceutically acceptable excipients, carriers, buffers, stabilizers or other materials well known to a person skilled in the art. Such materials should be non-toxic and should not interfere with the efficacy of active ingredients. The exact properties of the carriers or other materials can be determined by a person skilled in the art according to the route of administration (i.e., in this case, direct retinal, subretinal or intravitreal injection).

The pharmaceutical composition is typically in a liquid form. The liquid pharmaceutical composition typically includes a liquid carrier such as water, petroleum, animal or vegetable oil, mineral oil, or synthetic oil. A physiological saline solution, magnesium chloride, glucose or other sugar solutions, or glycols such as ethylene glycol, propylene glycol or polyethylene glycol may be included. In some cases, a surfactant, such as pluronic acid (PF68, also known as poloxamer 188), may be used.

When being injected to the ill site, active ingredients will be in the form of a pyrogen-free aqueous solution having suitable pH, isotonicity and stability. A person skilled in the art is fully enabled to use, for example, isotonic vehicle such as sodium chloride injection, Ringer's injection and lactated Ringer's injection to prepare a suitable solution. Preservatives, stabilizers, buffers, antioxidants and/or other additives may be included as desired.

For delayed release, the vector may be included in a pharmaceutical composition formulated for slow release, such as in microcapsules formed of a biocompatible polymer or in a liposome carrier system according to the method known in the art.

In another aspect, a pharmaceutical composition comprising an adeno-associated virus (AAV) is disclosed herein, wherein the adeno-associated virus comprises any recombinant nucleic acid disclosed herein. In some cases, the pharmaceutical composition further comprises a pharmaceutically acceptable excipient thereof.

In some cases, the pharmaceutically acceptable excipient comprises phosphate buffered saline (PBS), α,α-trehalose dehydrate, L-histidine monohydrochloride monohydrate, polysorbate 20, NaCl, NaH₂PO₄, Na₂HPO₄, KH₂PO₄, K₂HPO₄, poloxamer 188 or any combination thereof. In some cases, the pharmaceutically acceptable excipient is selected from the group consisting of phosphate buffered saline (PBS), α,α-trehalose dehydrate, L-histidine monohydrochloride monohydrate, polysorbate 20, NaCl, NaH₂PO₄, Na₂HPO₄, KH₂PO₄, K₂HPO₄, poloxamer 188 or any combination thereof.

In some cases, the pharmaceutically acceptable excipient comprises poloxamer 188. Poloxamer 188 is an ethylene oxide-polyoxypropylene glycol copolymer. As a surfactant, poloxamer 188 has the functions of dispersing, stabilizing and emulsifying. In some cases, the pharmaceutically acceptable excipient comprises 0.0001% to 0.01% of poloxamer 188. In some cases, the pharmaceutically acceptable excipient comprises 0.0005% to 0.005% of poloxamer 188. In some cases, the pharmaceutically acceptable excipient comprises 0.0007% to 0.002% of poloxamer 188. In some cases, the pharmaceutically acceptable excipient comprises 0.0008% to 0.0012% of poloxamer 188. The pharmaceutically acceptable excipient comprises 0.0009% to 0.0011% of poloxamer 188. In some cases, the pharmaceutically acceptable excipient comprises 0.001% (0.01 mg/mL) of poloxamer 188.

In some cases, the pharmaceutically acceptable excipient further comprises one or more salts. In some cases, the one or more salts comprise NaCl, NaH₂PO₄, Na₂HPO₄ and KH₂PO₄. In some cases, the one or more salts comprise 80 mM NaCl, 5 mM NaH₂PO₄, 40 mM Na₂HPO₄ and 5 mM KH₂PO₄. In some cases, the one or more salts comprise NaCl, Na₂HPO₄ and KH₂PO₄. In some cases, the one or more salts comprise 154 mM NaCl, 5.6 mM Na₂HPO₄ and 8.4 mM KH₂PO₄. In some cases, the one or more salts comprise NaCl, Na₂HPO₄ and KH₂PO₄. In some cases, the one or more salts are NaCl, Na₂HPO₄ and KH₂PO₄. In some cases, the NaCl has a concentration of 5-15 mg/mL. In some cases, the NaCl has a concentration of 9 mg/mL. In some cases, the KH₂PO₄ has a concentration of 0.1-0.5 mg/mL. In some cases, the KH₂PO₄ has a concentration of 0.144 mg/mL. In some cases, the Na₂HPO₄ has a concentration of 0.5-1 mg/mL. In some cases, the Na₂HPO₄ has a concentration of 0.795 mg/mL.

In some cases, the pharmaceutical composition has a pH of 6-8. In some cases, the pharmaceutical composition has a pH of 7.2-7.4. In some cases, the pharmaceutical composition has a pH of 7.3. In some cases, the pharmaceutical composition has a viral titer of at least 1.0×10¹⁰ vg/mL. In some cases, the pharmaceutical composition has a viral titer of at least 5.0×10¹⁰ vg/mL.

In some cases, the pharmaceutical composition is subjected to five freeze/thaw cycles and maintains at least 60%, 70%, 80% or 90% of the viral titer as compared to the viral titer prior to the five freeze/thaw cycles. In some cases, when administered to a patient having Leber's hereditary optic neuropathy, the pharmaceutical composition results in a higher average recovery of vision than a comparable pharmaceutical composition without the recombinant nucleic acid.

In some cases, the pharmaceutical composition is stored in a container of a particular material. In some examples, the container is made of a cycloolefin polymer.

### Samples

Samples suitable for use in the method described herein may be nucleic acid samples from a subject. As used herein, the "nucleic acid sample" may comprise RNA or DNA or a combination thereof. In another embodiment, the "polypeptide sample" (e.g., a peptide or protein or a fragment derived therefrom) can be used to determine information that an amino acid has changed due to a genetic variant. The nucleic acids and the polypeptides may be extracted from one or more samples including, but not limited to, blood, saliva, urine, mucosal scrapings of oral cavity lining, expectorants, serum, tears, skin, tissue or hair. Nucleic acid information analysis can be performed on the nucleic acid sample. As used herein, the "nucleic acid information" includes the nucleic acid sequence itself, the presence/absence of genetic variation in the nucleic acid sequence, physical properties (e.g., Tm) that vary according to the nucleic acid sequence, and the amount of nucleic acids (e.g., mRNA copy number). The "nucleic acid" means any one of DNA, RNA, DNA including artificial nucleotides or RNA including artificial nucleotides. As used herein, the "purified nucleic acid" includes cDNA, fragments of genomic nucleic acid, nucleic acids generated using polymerase chain reaction (PCR), nucleic acids formed by restriction enzyme treatment of genomic nucleic acids, recombinant nucleic acids, and chemically synthesized nucleic acid molecules. The "recombinant" nucleic acid molecule includes a nucleic acid molecule that is formed by artificially combining two sequence segments separated in other ways, e.g., nucleic acid fragments separated by chemical synthesis or by genetic engineering techniques. As used herein, the "polypeptide" includes proteins, protein fragments and peptides that are isolated from natural sources, produced by recombinant techniques or chemically synthesized. The polypeptide may have one or more modifications, such as post-translational modifications (e.g., glycosylation and phosphorylation) or any other modification (e.g., pegylation). The polypeptide may contain one or more non-naturally occurring amino acids (e.g., an amino acid with a side chain modification).

In some embodiments, the nucleic acid sample may comprise cells or tissues, such as cell lines. Exemplary cell types from which nucleic acids can be obtained using the method described herein include, but are not limited to, the following: blood cells, such as B lymphocytes, T lymphocytes, leukocytes, erythrocytes, macrophages or neutrophils; muscle cells, such as skeletal cells, smooth muscle cells or cardiac muscle cells; germ cells, such as sperm or eggs; epithelial cells; connective tissue cells, such as adipocytes and chondrocytes; fibroblasts or osteoblasts; neurons; astrocytes; stromal cells; organ-specific cells, such as kidney cells, pancreas cells, liver cells or keratinocytes; stem cells; or any cell developed therefrom. The cells from which nucleic acids can be obtained may be blood cells or a particular type of blood cell, including, for example, hematopoietic stem cells or cells produced from hematopoietic stem cells, such as erythrocytes, B lymphocytes, T lymphocytes, natural killer cells, neutrophils, basophils, eosinophils, monocytes, macrophages or platelets. In general, any type of stem cells may be used, including but not limited to embryonic stem cells, adult stem cells or pluripotent stem cells.

In some embodiments, the nucleic acid sample may be treated for RNA or DNA isolation. For example, RNA or DNA in a cell or tissue sample can be isolated from other components of the nucleic acid sample. Standard techniques may be used. For example, by centrifuging the cell sample, and resuspending the pelleted cells in, e.g., a buffer solution (e.g., phosphate buffered saline (PBS)), cells can be harvested from the nucleic acid sample. In some embodiments, after centrifuging the cell suspension to obtain cell pellets, the cells may be lysed to extract DNA. In some embodiments, the nucleic acid sample may be concentrated and/or purified to isolate DNA. All nucleic acid samples that are derived from a subject and include nucleic acid samples subjected to any kind of further treatment are considered to be derived from the subject. In some embodiments, standard techniques and kits known in the art may be used to extract RNA or DNA from the nucleic acid sample, include for example, phenol extraction, QIAAMP^{®} tissue kit (Qiagen, Chatsworth, Calif.), WIZARD^{®} genomic DNA purification kit (Promega) or Qiagen Autopure method using Puregene chemistry, and can purify highly stable DNA that is well suited for archiving.

In some embodiments, determining the identity of an allele or determining the copy number may, but does not need to, include obtaining a nucleic acid sample comprising RNA and/or DNA from a subject, and/or assessing the identity, copy number, presence or absence of one or more genetic variations within genomic DNA (i.e., the genome of the subject) derived from the nucleic acid sample and the chromosomal location thereof.

The individual or tissue undergoing the assay does not need to actually perform a physical analysis on the nucleic acid sample from the subject. In some embodiments, the method may include using the information obtained by analyzing the nucleic acid sample by the third party. In some embodiments, the method may include steps performed at more than one location. For example, the nucleic acid sample may be obtained from the subject at a first location, such as in a healthcare facility or at the home of the subject in the case of a self-test kit. The nucleic acid sample may be analyzed at the same site or at a second site (such as a laboratory or other test sites).

### Nucleic acids

The nucleic acids and polypeptides described herein can be used in the method and kits of the present disclosure. In some embodiments, aptamers that specifically bind to the nucleic acids and polypeptides described herein can be used in the method and kits of the present disclosure. As used herein, the nucleic acid may comprise deoxyribonucleotides (DNA) or ribonucleotides (RNA) (whether deoxyribonucleotides (DNA) or ribonucleotides (RNA) is single or in polymers, naturally occurring or non-naturally occurring, double-stranded or single-stranded, encoded (e.g., a translated gene) or non-encoded (e.g., a regulatory region)), or any fragment, derivative, simulant or complement thereof. In some embodiments, the nucleic acid may include an oligonucleotide, a nucleotide, a polynucleotide, a nucleic acid sequence, a genomic sequence, complementary DNA (cDNA), an antisense nucleic acid, a DNA region, a probe, a primer, a gene, a regulatory region, an intron, an exon, an open reading frame, a binding site, a target nucleic acid and an allele-specific nucleic acid.

The "probe" as used herein includes nucleic acid fragments used for examining nucleic acids in a sample by using a hybridization reaction based on the complementarity of the nucleic acids.

As used herein, the "hybrid" includes a double strand formed within nucleic acids of the same type as described above or across nucleic acids of different types, including DNA-DNA, DNA-RNA, RNA-RNA and the like.

As used herein, the "isolated" nucleic acid is one that is isolated from nucleic acids that are usually flanked by a gene or nucleotide sequence (e.g., in a genomic sequence) and/or that have been completely or partially purified from other transcribed sequences (e.g., in an RNA library). For example, the isolated nucleic acid of the present disclosure may be substantially isolated relative to the complex cellular environment where naturally occurring nucleic acids exist, the culture medium used when nucleic acids are produced by recombinant techniques or chemical precursors or other chemicals used when nucleic acids are chemically synthesized. In some cases, the isolated material may form part of a composition, such as a crude extract, a buffer system or a reagent mixture that contains other materials. In some embodiments, the material may be purified to be substantially homogeneous by using the method known in the art, for example, by polyacrylamide gel electrophoresis (PAGE) or column chromatography (e.g., HPLC). With respect to the genomic DNA (gDNA), the term "isolated" may also refer to a nucleic acid that is isolated from the chromosome with which the genomic DNA is naturally associated. For example, the isolated nucleic acid molecule may contain nucleotides less than about 250 kb, 200 kb, 150 kb, 100 kb, 75 kb, 50 kb, 25 kb, 10 kb, 5 kb, 4 kb, 3 kb, 2kb, 1 kb, 0.5 kb or 0.1 kb, and the nucleotides are flanked by the nucleic acid molecule in the gDNA of the cell from which the nucleic acid molecule is derived.

The nucleic acid may be fused to other coding or regulatory sequences and may be considered isolated. For example, the recombinant DNA contained in a vector is included in the definition of the "isolated" as used herein. In some embodiments, the isolated nucleic acid may include a recombinant DNA molecule in a heterologous host cell or heterologous organism and a partially or substantially purified DNA molecule in a solution. The isolated nucleic acid also encompasses in vivo and in vitro RNA transcripts of the DNA molecules of the present disclosure. The isolated nucleic acid molecule or nucleotide sequence may be synthesized chemically or synthesized by recombination. Such isolated nucleotide sequence may be used, for example, in the manufacture of the encoded polypeptide as a probe for isolating homologous sequences (e.g., from other mammalian species), for gene mapping (e.g., by in-situ hybridization with a chromosome), or for detecting gene expression in tissue (e.g., human tissue), e.g., by Northern blot analysis or other hybridization techniques disclosed herein. The present disclosure also relates to a nucleic acid sequence that hybridizes with the nucleotide sequence described herein under, e.g., highly stringent hybridization conditions for selective hybridization. Such nucleic acid sequence can be detected and/or isolated by allele-specific or sequence-specific hybridization (e.g., under highly stringent conditions). Stringent conditions and method for nucleic acid hybridization are well known to a person skilled in the art (see, e.g., Current Protocols in Molecular Biology, Ausubel, F. et al, John Wiley & Sons, (1998) and Kraus, M. and Aaronson, S., Methods Enzymol., 200:546-556 (1991)), which is incorporated herein by reference in its entirety.

Calculations of "identity" or "percent identity" between two or more nucleotides or amino acid sequences can be determined by aligning the sequences for optimal comparison purposes (e.g., gaps can be introduced in the sequence of a first sequence). The nucleotides at corresponding positions are then compared, and the percent identity between the two sequences is a function of the number of identical positions shared by the sequences (i.e. % identity = the number of identical positions/the total number of positions × 100). For example, a position in the first sequence is occupied by the nucleotide at the identical position as the corresponding position in the second sequence, then the molecules are identical at this position. The percent identity between the two sequences is a function of the number of identical positions shared by the sequences, taking into account the number of gaps, and the length of each gap, which need to be introduced for optimal alignment of the two sequences. As used herein, the terms "identical" and "identity", when used in describing the degree of sequence identity, are intended to be synonymous, unless otherwise indicated. For example, at least 99% identical to a particular sequence means having at least 99% identity to the sequence.

In some embodiments, the length of sequences aligned for comparison purposes is at least 30%, at least 40%, at least 50%, at least 60%, at least 70%, at least 80%, at least 90%, or at least 95% of the length of the reference sequence. The actual comparison of the two sequences can be accomplished by well-known methods, for example, using a mathematical algorithm. A non-limiting example of such a mathematical algorithm is described in Karlin, S. and Altschul, S., Proc. Natl. Acad. Sci. USA, 90, 5873-5877 (1993). Such an algorithm is incorporated into the NBLAST and XBLAST programs (version 2.0), as described in Altschul, S. et al., Nucleic Acids Res., 25:3389-3402 (1997). When utilizing BLAST and Gapped BLAST programs, any relevant parameters of the respective programs (e.g., NBLAST) may be used. For example, parameters for sequence comparison may be set at score= 100, word length= 12, or may be varied (e.g., W=5 or W=20). Other examples include the algorithm of Myers and Miller, CABIOS (1989), ADVANCE, ADAM, BLAT, and FASTA. In some embodiments, the percent identity between two amino acid sequences may be accomplished using, for example, the GAP program in the GCG software package (Accelrys, Cambridge, UK).

"Probes" or "primers" may be oligonucleotides that hybridize in a base-specific manner to a complementary strand of a nucleic acid molecule. Probes may include primers, which may be a single-stranded oligonucleotide probe that may act as a point of initiation of template-directed DNA synthesis using methods including but not limited to, polymerase chain reaction (PCR) and ligase chain reaction (LCR) for amplification of a target sequence. The oligonucleotides as described herein may include segments or fragments of nucleic acid sequences or their complements. In some embodiments, DNA segments may be contiguous bases between 5 and 10,000, and can range from 5, 10, 12, 15, 20, or 25 nucleotides to 10, 15, 20, 25, 30, 40, 50, 100, 200, 500, 1000 or 10,000 nucleotides. In addition to DNA and RNA, probes and primers may further include polypeptide nucleic acids (PNAs), as described in Nielsen, P. et al., Science 254: 1497-1500 (1991). A probe or primer may include a region of nucleotide sequence that hybridizes to at least about 15, generally about 20-25, and in certain embodiments about 40, 50, 60 or 75, consecutive nucleotides of a nucleic acid molecule.

The present disclosure further provides isolated nucleic acids, for example, probes or primers, containing a fragment or portion that can selectively hybridize to a nucleic acid that includes, or consists of a nucleotide sequence, wherein the nucleotide sequence may include at least one polymorphism or polymorphic allele contained in the genetic variations described herein or the wild-type nucleotide that is located at the identical position, or the complements thereof. In some embodiments, the probe or primer may have at least 70% identity, at least 80% identity, at least 85% identity, at least 90% identity, or at least 95% identity, to the contiguous nucleotide sequence or to the complement of the contiguous nucleotide sequence.

In some embodiments, a nucleic acid probe may be an oligonucleotide capable of hybridizing with a complementary region of a gene associated with a condition (e.g., LHON) containing a genetic variation described herein. The nucleic acid fragments of the present disclosure may be used as probes or primers in assays such as those described herein.

The nucleic acids of the present disclosure, such as those described above, can be identified and isolated using standard molecular biology techniques well known to the skilled person. In some embodiments, DNA may be amplified and/or may be labeled (e.g., radiolabeled, fluorescently labeled) and used as a probe for screening, for example, a cDNA library derived from an organism. cDNA may be derived from mRNA and may be contained in a suitable vector. For example, corresponding clones can be isolated, DNA can be obtained after in vivo excision, and the cloned insert can be sequenced in either or both directions by art-recognized methods to identify the correct reading frame encoding a polypeptide with the appropriate molecular weight. Using these or similar methods, the polypeptide and the DNA encoding the polypeptide can be isolated, sequenced and further characterized.

In some embodiments, nucleic acids may include one or more polymorphisms, variations, or mutations, for example, single nucleotide polymorphisms (SNPs), single nucleotide variations (SNVs), copy number variations (CNVs), for example, insertions, deletions, inversions, and translocations. In some embodiments, nucleic acids may include analogs, for example, phosphorothioates, phosphoramidates, methyl phosphonate, chiralmethyl phosphonates, 2'-O-methyl-ribonucleosides, or modified nucleic acids, for example, modified backbone residues or linkages, or nucleic acids combined with carbohydrates, lipids, polypeptide or other materials, or peptide nucleic acids (PNAs), for example, chromatin, ribosomes, and transcriptosomes. In some embodiments, nucleic acids may include nucleic acids in various structures, for example, A DNA, B DNA, Z-form DNA, siRNA, tRNA, and ribozymes. In some embodiments, nucleic acids may be naturally or non-naturally polymorphic, for example, having one or more sequence differences, for example, additions, deletions and/or substitutions, as compared with a reference sequence. In some embodiments, a reference sequence may be based on publicly available information, for example, the U.C. Santa Cruz Human Genome Browser Gateway (genome.ucsc.edu/cgi-bin/hgGateway) or the NCBI website (www.ncbi.nlm.nih.gov). In some embodiments, a reference sequence can be determined by a practitioner of the present disclosure using methods well known in the art, for example, by sequencing a reference nucleic acid.

In some embodiments, a probe can hybridize to an allele, SNP, SNV, or CNV as described herein. In some embodiments, the probe can bind to another marker sequence associated with LHON as described herein.

Those skilled in the art would know how to design a probe, so that sequence specific hybridization can occur only if a particular allele is present in a genomic sequence from a test nucleic acid sample. The present disclosure can also be simplified to practice using any convenient genotyping method, including commercially available techniques and methods for genotyping particular genetic variations.

Control probes can also be used, for example, a probe that binds a less variable sequence, for example, a repetitive DNA associated with a centromere of a chromosome can be used as a control. In some embodiments, probes can be obtained from commercial sources. In some embodiments, probes can be synthesized, for example, chemically or in vitro, or made from chromosomal or genomic DNA through standard techniques. In some embodiments, sources of DNA that can be used include genomic DNA, cloned DNA sequences, and somatic cell hybrids that contain one, or a part of one, human chromosome along with the normal chromosome complement of the host, and chromosomes purified by flow cytometry or microdissection. The region of interest can be isolated through cloning, or by site-specific amplification using PCR.

One or more nucleic acids, for example, a probe or primer, can also be labeled, for example, by direct labeling, to include a detectable label. A detectable label may include any label capable of being detected by a physical, chemical, or biological method, for example, a radioactive label such as 32P or 3H, a fluorescent label such as FITC, a chromophore label, an affinity-ligand label, an enzyme label such as alkaline phosphatase, horseradish peroxidase, or I2 galactosidase, an enzyme cofactor label, a hapten conjugate label such as digoxigenin or dinitrophenyl, a Raman signal generating label, a magnetic label, a spin label, an epitope label such as the FLAG or HA epitope, a luminescent label, a heavy atom label, a nanoparticle label, an electrochemical label, a light scattering label, a spherical shell label, a semiconductor nanocrystal label such as a quantum dot (described in U.S. Pat. No. 6,207,392) and a probe labeled with any other signal generating label known to those of skill in the art, wherein a label can allow the probe to be visualized with or without a secondary detection molecule. A nucleotide can be directly incorporated into a probe with standard techniques, for example, nick translation, random priming, and PCR labeling. A "signal", as used herein, includes a signal suitably detected and measured by appropriate means, including fluorescence, radioactivity, chemiluminescence, and the like.

Non-limiting examples of label parts for detection include but are not limited to: suitable enzymes such as horseradish peroxidase, alkaline phosphatase, beta-galactosidase, or acetylcholinesterase; members of a binding pair that are capable of forming complexes such as streptavidin/biotin, avidin/biotin or an antigen/antibody complex including, for example, rabbit IgG and anti-rabbit IgG; fluorophores such as umbelliferone, fluorescein, fluorescein isothiocyanate, rhodamine, tetramethyl rhodamine, eosin, green fluorescent protein, erythrosin, coumarin, methyl coumarin, pyrene, malachite green, stilbene, lucifer yellow, Cascade Blue, Texas Red, dichlorotriazinylamine fluorescein, dansyl chloride, phycoerythrin, fluorescent lanthanide complexes such as those including Europium and Terbium, cyanine dye family members, such as Cy3 and Cy5, molecular beacons and fluorescent derivatives thereof, as well as others known in the art as described, for example, in Principles of Fluorescence Spectroscopy, Joseph R. Lakowicz (Editor), Plenum Pub Corp, 2nd edition (July 1999) and the 6th Edition of the Molecular Probes Handbook by Richard P. Hoagland; a luminescent material such as luminol; light scattering or plasmon resonant materials such as gold or silver particles or quantum dots; or radioactive materials including 14C, 1231, 1241, 1251, Tc99m, 32P, 33P, 35S or 3H.

Other labels can also be used in the methods of the present disclosure, for example, backbone labels. The backbone labels include nucleic acid dyes that bind nucleic acids in a sequence independent manner. Non-limiting examples include intercalating dyes such as phenanthridine and acridine (for example, ethidium bromide, propidium iodide, hexidium iodide, dihydroethidium, ethidium homodimer-1 and -2, ethidium monoazide, and ACMA); some minor groove binders, such as indoles and imidazoles (for example, Hoechst 33258, Hoechst 33342, Hoechst 34580, and DAPI); and miscellaneous nucleic acid dyes such as acridine orange (which can also intercalate), 7-AAD, actinomycin D, LDS751 and hydroxystilbamidine. All of the aforementioned nucleic acid dyes are commercially available from suppliers such as Molecular Probes, Inc. Still other examples of nucleic acid dyes include the following dyes from Molecular Probes: cyanine dyes such as SYTOX Blue, SYTOX Green, SYTOX Orange, POPO-1, POPO-3, YOYO-1, YOYO-3, TOTO-1, TOTO-3, JOJO-1, LOLO-1, BOBO-1, BOBO-3, PO-PRO-1, PO-PRO-3, BO-PRO-1, BO-PRO-3, TO-PRO-1, TO-PRO-3, TO-PRO-5, JO-PRO-1, LO-PRO-1, YO-PRO-1, YO-PRO-3, PicoGreen, OliGreen, RiboGreen, SYBR Gold, SYBR Green I, SYBR Green II, SYBR DX, SYTO-40, -41, -42, -43, -44, -45 (blue), SYTO-13, -16, -24, -21, -23, -12, -11, -20, -22, -15, -14, -25 (green), SYTO-81, -80, -82, -83, -84, -85 (orange), SYTO-64, -17, -59, -61, -62, -60, and -63 (red).

In some embodiments, fluorophores of different colors can be chosen, for example, 7-amino-4-methylcoumarin-3-acetic acid (AMCA), 5-(and-6)-carboxy-X-rhodamine, lissamine rhodamine B, 5-(and-6)-carboxyfluorescein, fluorescein-5-isothiocyanate (FITC), 7-diethylaminocoumarin-3-carboxylic acid, tetramethylrhodamine-5-(and-6)-isothiocyanate, 5-(and-6)-carboxytetramethylrhodamine, 7-hydroxycoumarin-3-carboxylic acid, 6-[fluorescein 5-(and-6)-carboxamido]hexanoic acid, N-(4,4-difluoro-5,7-dimethyl-4-bora-3a,4a diaza-3-indacene)propionic acid, eosin-5-isothiocyanate, erythrosin-5-isothiocyanate, TRITC, rhodamine, tetramethylrhodamine, R-phycoerythrin, Cy-3, Cy-5, Cy-7, Texas Red, Phar-Red, allophycocyanin (APC),and CASCADETM blue acetylazide, such that each probe in or not in a set can be distinctly visualized. In some embodiments, fluorescently labeled probes can be observed with a fluorescence microscope and an appropriate filter for each fluorophore, or by using dual or triple band-pass filter sets to observe multiple fluorophores. In some embodiments, techniques such as flow cytometry can be used to examine the hybridization pattern of the probes.

In other embodiments, the probes can be indirectly labeled, for example, with biotin or digoxygenin, or labeled with radioactive isotopes such as 32P and/or 3H. As a non-limiting example, a probe indirectly labeled with biotin can be detected by avidin conjugated to a detectable marker. For example, avidin can be conjugated to an enzymatic marker such as alkaline phosphatase or horseradish peroxidase. In some embodiments, enzymatic markers can be detected using colorimetric reactions using a substrate and/or a catalyst for the enzyme. In some embodiments, catalysts for alkaline phosphatase such as 5-bromo-4-chloro-3-indolyl phosphate and nitro blue tetrazolium may be used. In some embodiments, a catalyst can be used for horseradish peroxidase, for example, diaminobenzoate.

### Formulations, routes of administration and effective doses

Yet another aspect of the present disclosure relates to formulations, routes of administration and effective doses for pharmaceutical compositions including an agent or combination of agents of the present disclosure. Such pharmaceutical compositions can be used to treat a condition (for example, LHON) as described above.

Compounds of the present disclosure can be administered as pharmaceutical formulations including those suitable for oral (including buccal and sub-lingual), rectal, nasal, topical, transdermal patch, pulmonary, vaginal, suppository, or parenteral (including intraocular, intravitreal, intramuscular, intraarterial, intrathecal, intradermal, intraperitoneal, subcutaneous and intravenous) administration or in a form suitable for administration by aerosolization, inhalation or insufflation. General information on drug delivery systems can be found in Ansel et al., Pharmaceutical Dosage Forms and Drug Delivery Systems (Lippencott Williams & Wilkins, Baltimore Md. (1999).

In various embodiments, the pharmaceutical composition includes carriers and excipients (including but not limited to buffers, carbohydrates, mannitol, polypeptides, amino acids, antioxidants, bacteriostats, chelating agents, suspending agents, thickening agents and/or preservatives), water, oils (including those of petroleum, animal, vegetable or synthetic origin, such as peanut oil, soybean oil, mineral oil and sesame oil), saline solutions, aqueous dextrose and glycerol solutions, flavoring agents, coloring agents, detackifiers and other acceptable additives, adjuvants, or binders, and other pharmaceutically acceptable auxiliary substances to approximate physiological conditions, such as pH buffering agents, tonicity adjusting agents, emulsifying agents and wetting agents. Examples of excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. In some embodiments, the pharmaceutical preparation is substantially free of preservatives. In other embodiments, the pharmaceutical preparation can contain at least one preservative. General methods on pharmaceutical dosage forms are found in Ansel et al., Pharmaceutical Dosage Forms and Drug Delivery Systems (Lippencott, Williams, & Wilkins, Baltimore Md. (1999)). It can be recognized that, while the compositions of the present disclosure can be administered by any suitable carrier known to those of ordinary skill in the art, the type of carrier can vary depending on the mode of administration.

Compounds can also be encapsulated within liposomes using well-known techniques. Biodegradable microspheres can also be employed as carriers for the pharmaceutical compositions of the present disclosure. Suitable biodegradable microspheres are disclosed, for example, in U.S. Pat. Nos. 4,897,268, 5,075,109, 5,928,647, 5,811,128, 5,820,883, 5,853,763, 5,814,344 and 5,942,252.

The compound can be administered in the form of liposomes or microspheres (or microparticles). Methods for preparing liposomes and microspheres for administration to a subject are well known to those skilled in the art. Methods for encapsulating biological materials in liposomes are described in U.S. Pat. No. 4,789,734 (the contents of which are hereby incorporated by reference). Basically, the material is dissolved in an aqueous solution, the appropriate phospholipids and lipids are added, along with surfactants if required, and the material is dialyzed or sonicated if necessary. A review of known methods is provided by G. Gregoriadis, Chapter 14, "Liposomes," Drug Carriers in Biology and Medicine, pp. 2.sup.87-341 (Academic Press, 1979).

Microspheres formed of polymers or polypeptides are well known to those skilled in the art, and can be tailored for directly entering into the blood stream through the gastrointestinal tract. Alternatively, the compound can be incorporated, and the microspheres or composite of microspheres are implanted for slow release over a period of time ranging from days to months. See, for example, U.S. Pat. Nos. 4,906,474, 4,925,673 and 3,625,214, and Jein, TIPS 19:155-157 (1998), the contents of which are hereby incorporated by reference.

The concentration of the drug and the pH of the solution buffered can be adjusted, and the isotonicity are adjusted to be compatible with intraocular or intravitreal injection.

The compounds of the present disclosure can be formulated as a sterile solution or suspension, in suitable vehicles. The pharmaceutical compositions can be sterilized by conventional, well-known sterilization techniques, or can be sterile filtered. The resulting aqueous solutions can be packaged for use as is, or lyophilized, and the lyophilized preparation is combined with a sterile solution prior to administration. Suitable formulations and additional carriers are described in Remington "The Science and Practice of Pharmacy" (20th Ed., Lippincott Williams & Wilkins, Baltimore MD).

The agents or pharmaceutically acceptable salts thereof can be provided alone or in combination with one or more other agents or in one or more other forms. For example, a formulation can include one or more agents in particular proportions, depending on the relative potency of each agent and the intended indication. For example, in compositions for targeting two different host targets, and where potencies are similar, about a 1:1 ratio of agents can be used. The two forms can be formulated together, in the same dosage unit, for example, in one cream, suppository, tablet, capsule, aerosol spray, or packet of powder to be dissolved in a beverage; or two forms can be formulated in separate units, for example, two creams, two suppositories, two tablets, two capsules, a tablet and a liquid for dissolving the tablet, two aerosol sprays, or a packet of powder and a liquid for dissolving the powder.

The term "pharmaceutically acceptable salt" means those salts that retain biological effectiveness and properties of agents used in the present disclosure and are not biologically or otherwise undesirable.

Typical salts are those of inorganic ions such as sodium, potassium, calcium, magnesium ions, or the like. Such salts include those formed with inorganic or organic acids such as hydrochloric, hydrobromic, phosphoric, nitric, sulfuric, methanesulfonic, p-toluenesulfonic, acetic, fumaric, succinic, lactic, mandelic, malic, citric, tartaric, or maleic acid. Furthermore, if the agent contains a carboxyl or other acidic group, it may be converted into a pharmaceutically acceptable addition salt using an inorganic or organic base. Examples of suitable bases include sodium hydroxide, potassium hydroxide, ammonia, cyclohexylamine, dicyclohexylamine, ethanolamine, diethanolamine, triethanolamine, or the like.

Pharmaceutically acceptable ester or amide refers to those that retain the biological effectiveness and properties of the agents used in the present disclosure and are not biologically or otherwise undesirable. Typical esters include ethyl ester, methyl ester, isobutyl ester, ethylene glycol ester, or the like. Typical amides include unsubstituted amide, alkyl amide, dialkyl amide, or the like.

In some embodiments, an agent may be administered in combination with one or more other compounds, forms, and/or agents, such as those described above. Pharmaceutical compositions containing one or more other active agents may be formulated to contain a certain molar ratio. For example, a molar ratio of a first active agent to the other active agent may be about 99:1 to about 1:99. In some subsets of embodiments, the molar ratio of the first active agent to the other active agent ranges from about 80:20 to about 20:80; about 75:25 to about 25:75, about 70:30 to about 30:70, about 66:33 to about 33:66, about 60:40 to about 40:60; about 50:50; or about 90: 10 to about 10:90. The molar ratio of the first active agent to the other active agent may be about 1:9, and may be about 1:1 in some embodiments. Two agents, forms and/or compounds may be formulated together in the same dosage unit, for example, in one cream, suppository, tablet, capsule or packet of powder to be dissolved in a beverage; alternatively, the two agents, forms and/or compounds may be formulated in separate units, such as two creams, suppositories, tablets, two capsules, tablets and liquids for dissolving the tablets, aerosol sprays, packets of powder and liquids for dissolving the powders, or the like.

The agent and/or combination of agents may be further administered with other agents, if necessary or desired. A choice of the agent that may be co-administered with the agent and/or combination of agents of the present disclosure may depend, at least in part, on a condition being treated.

The agent (or pharmaceutically acceptable salt, ester, or amide thereof) may be administered alone or in a form of a pharmaceutical composition, where the active agent is blended or mixed with one or more pharmaceutically acceptable carriers. The pharmaceutical composition as used herein may be any composition prepared for administration to a subject. The pharmaceutical composition used according to the present disclosure may be formulated in a conventional manner using one or more physiologically acceptable carriers including excipients, diluents and/or adjuvants, which, for example, facilitate processing of the active agent into an administrable formulation. The appropriate formulation may depend, at least in part, on the route of administration selected. The agent or pharmaceutically acceptable salt, ester, or amide thereof used in the present disclosure may be delivered to the subject using a variety of routes or modes of administration, including oral, buccal, topical, rectal, transdermal, transmucosal, subcutaneous, intravenous, intraocular, intravitreal, and intramuscular administration, as well as inhalation.

In some embodiments, the agent may be brought into a solution using an oil or non-aqueous solvent due to, for example, presence of a large lipophilic moiety. Alternatively, an emulsion, suspension or other formulations, such as a liposome formulation, may be used. With respect to the liposome formulation, any known method may be used to prepare a liposome for treating the condition. See, for example, Bangham et al, J. Mol. Biol. 23: 238-252 (1965) and Szoka et al, Proc. Natl Acad. Sci. USA 75: 4194-4198 (1978) incorporated herein by reference. Ligand may also be attached to the liposome to direct these compositions to a specific action site. The agent of the present disclosure may also be incorporated into food products, such as cream cheese, butter, salad dressing, or ice cream, to facilitate solubilization, administration, and/or compliance in certain populations of subjects.

The compounds of the present disclosure may be formulated for parenteral administration (e.g., by injection, such as intraocular or intravitreal injection) and may be presented in an unit dosage form in an ampoule, pre-filled syringe, small volume infusion or in a multi-dose container with an added preservative. The composition may take such forms as a suspension, solution or emulsion in an oily or aqueous vehicle, for example, a solution in aqueous polyethylene glycol.

For an injectable formulation, the vehicle may be selected from those known to be suitable in the art, including an aqueous solution or oily suspension or emulsion, as well as sesame oil, corn oil, cottonseed oil, or peanut oil, as well as elixir, mannitol, dextrose, or a sterile aqueous solution and similar pharmaceutical vehicles. The formulation may also comprise a biocompatible biodegradable polymer composition, such as poly(lactic-co-glycolic acid). These substances may be made into a microsphere or nanosphere, loaded with drugs and further coated or derived to provide excellent sustained-release performance. The vehicles suitable for periocular or intraocular injection include, for example, suspension of therapeutic agent in injection grade water, liposome, and a vehicle suitable for lipophilic substances. Other vehicles for the periocular or intraocular injection are well known in the art.

In some embodiments, the composition is formulated into a pharmaceutical composition suitable for intravenous administration to a human according to conventional procedures. Typically, the composition for the intravenous administration is a solution in a sterile isotonic aqueous buffer. If necessary, the composition may also include a solubilizer and local anesthetic such as lidocaine, to reduce a pain at an injection site. Typically, ingredients are provided in the unit dosage form individually or as a mixture, for example, as a lyophilized powder or anhydrous concentrate in a hermetically sealed container such as an ampoule or a sachet indicating the amount of active agent. In the case where the composition is to be administered by infusion, it may be dispensed in an infusion bottle containing sterile pharmaceutical grade water or saline. In the case of administrating the composition by injection, an ampoule of sterile water or saline for injection may be provided so that the ingredients may be mixed before the administration.

When administered by the injection, the active compound may be formulated in an aqueous solution, especially in a physiologically compatible buffer, such as Hanks' solution, Ringer's solution or physiological saline buffer. The solution may contain a formulating agent such as a suspending agent, stabilizer and/or dispersant. Alternatively, the active compound may be in a powder form and reconstituted with a suitable vehicle, such as sterile pyrogen-free water, before use. In some embodiments, the pharmaceutical composition does not comprise an adjuvant or any other substance added to enhance an immune response stimulated by a peptide. In some embodiments, the pharmaceutical composition comprises a substance inhibiting the immune response to the peptide. Formulation methods are known in the art and are disclosed, for example, in Remington's Pharmaceutical Sciences, latest edition, Mack Publishing Co., Easton P.

In some embodiments, an ophthalmic solution, suspension, ointment, or insert containing the agent or combination of agents of the present disclosure may be used to effectively treat an ocular disorder. The ophthalmic solution may be prepared by dissolving the active ingredients in a sterile aqueous solution, such as physiological saline, buffer solution, or the like, or by combining a powder composition to be dissolved before use. Other vehicles known in the art may be selected, including but not limited to: balanced salt solution, saline solution, water-soluble polyether such as polyethylene glycol, polyvinyl compound such as polyvinyl alcohol and povidone, cellulose derivative such as methyl cellulose and hydroxypropyl methyl cellulose, petroleum derivative such as mineral oil and white petrolatum, animal fat such as lanolin, polymer of acrylic acid such as carboxypolymethylene gel, vegetable fat such as peanut oil and polysaccharide such as dextran, and glycosaminoglycan such as sodium hyaluronate. If necessary, additives commonly used in the ophthalmic solution may be added. Such additives include an isotonic agent (e.g., sodium chloride, etc.), a buffer (e.g., boric acid, sodium monohydrogen phosphate, sodium dihydrogen phosphate, etc.), a preservative (e.g., benzalkonium chloride, benzethonium chloride, chlorobutanol, etc.), a thickening agent (e.g., sugar such as lactose, mannitol, maltose, etc., e.g., hyaluronic acid or salt thereof such as sodium hyaluronate, potassium hyaluronate, etc., e.g., mucopolysaccharides such as chondroitin sulfate, etc., e.g., sodium polyacrylate, carboxyvinyl polymer, cross-linked polyacrylate, polyvinyl alcohol, polyvinylpyrrolidone, methyl cellulose, hydroxypropylmethyl cellulose, hydroxyethyl cellulose, carboxymethyl cellulose, hydroxypropyl cellulose, or other agents known to those skilled in the art).

Solubility of components of the composition of the present invention may be enhanced by a surfactant or other suitable co-solvents in the composition. Such co-solvents include polysorbates 20, 60, and 80; Pluronic F68, F-84 and P-103; Cyclodextrins, or other agents known to those skilled in the art. A use level of such co-solvents may be about 0.01% to 2% by weight.

The composition of the present disclosure may be packaged in a multi-dose form. The preservative may preferably prevent microbial contamination during use. Suitable preservatives include: benzalkonium chloride, thimerosal, chlorobutanol, methyl paraben, propyl paraben, phenethyl alcohol, disodium edetate, sorbic acid, Onamer M, or other agents known to those skilled in the art. In ophthalmic products of the prior art, the use level of such preservatives may be 0.004% to 0.02%. In the composition of the present application, the preservative, preferably benzalkonium chloride, may be used at a level of 0.001% by weight to less than 0.01% by weight (for example, 0.001% by weight to 0.008% by weight, preferably about 0.005% by weight). It has been found that the benzalkonium chloride at a concentration of 0.005% may be sufficient to protect the composition of the present disclosure from a microbial attack.

In some embodiments, the agent of the present disclosure is delivered in a soluble rather than suspension form, which allows for faster and quantitative absorption to action site. In general, the formulation such as gel, cream, lotion, suppository, and ointment may provide an area of longer exposure to the agent of the present disclosure, while the formulation in a solution form, such as spray, provide a more immediate short-term exposure.

It is further expected that the compounds of the present disclosure may be attached to a biocompatible polymer in a releasable manner for use in a sustained-release formulation on, in or attached to an insert for topical, intraocular, periocular or systemic administration. A controlled release from the biocompatible polymer may also be utilized with a water-soluble polymer to form a formulation that is instillable. The controlled release from the biocompatible polymer such as a PLGA microsphere or nanosphere may be used in the formulation suitable for intraocular implantation or injection for a sustained-release administration, and any suitable biodegradable and biocompatible polymer may be used.

### Further numbered embodiments

Further embodiments of the present invention are provided in the following numbered items:
Item 1: A recombinant nucleic acid comprising (sequentially from the 5' end to the 3' end) a mitochondrial targeting sequence and a mitochondrial protein coding sequence, wherein optionally, the mitochondrial protein coding sequence encodes an ND4 protein; and optionally, the ND4 protein comprises an amino acid sequence having at least 90%, at least 95%, at least 96%, at least 97%, at least 98%, at least 99%, or 100% identity to SEQ ID NO: 160.
Item 2: The recombinant nucleic acid according to item 1, comprising a sequence having at least 90%, at least 95%, at least 96%, at least 97%, at least 98%, at least 99%, or 100% identity to any one of sequences as set forth in SEQ ID NOs: 180 and 174-176.
Item 3: The recombinant nucleic acid according to item 1 or 2, comprising a sequence having at least 90%, at least 95%, at least 96%, at least 97%, at least 98%, at least 99%, or 100% identity to SEQ ID NO: 180.
Item 4: The recombinant nucleic acid according to any one of items 1 to 3, comprising a sequence as set forth in SEQ ID NO: 180.
Item 5: The recombinant nucleic acid according to any one of items 1 to 4, comprising a Kozak sequence positioned before the 5' end of the mitochondrial targeting sequence, wherein optionally, the Kozak sequence is SEQ ID NO: 171; and optionally, no redundant nucleotides are present between the Kozak sequence and the mitochondrial targeting sequence.
Item 6: The recombinant nucleic acid according to any one of items 1 to 5, comprising an intron sequence, wherein optionally, the intron sequence is positioned before the 5' end of the mitochondrial targeting sequence; optionally, the intron sequence is positioned before the 5' end of the Kozak sequence; and optionally, the intron sequence comprises a sequence having at least 90%, at least 95%, at least 96%, at least 97%, at least 98%, at least 99%, or 100% identity to a sequence as set forth in SEQ ID NO: 170.
Item 7: The recombinant nucleic acid according to any one of items 1 to 6, comprising a promoter sequence, wherein optionally, the promoter sequence is positioned before the 5' end of the mitochondrial targeting sequence, the Kozak sequence, and/or the intron sequence; and optionally, the promoter sequence comprises a sequence having at least 90%, at least 95%, at least 96%, at least 97%, at least 98%, at least 99%, or 100% identity to a sequence as set forth in SEQ ID NO: 169.
Item 8: The recombinant nucleic acid according to any one of items 1 to 7, comprising a 3' UTR sequence, wherein optionally, the 3' UTR sequence is positioned after the 3' end of the mitochondrial protein coding sequence; and optionally, the 3' UTR sequence comprises a sequence having at least 90%, at least 95%, at least 96%, at least 97%, at least 98%, at least 99%, or 100% identity to a sequence as set forth in SEQ ID NO: 13.
Item 9: The recombinant nucleic acid according to any one of items 1 to 8, comprising a poly A signal sequence, wherein optionally, the poly A signal sequence is positioned after the 3' end of the mitochondrial protein coding sequence and/or the 3' UTR sequence; optionally, the polyA signal sequence comprises a sequence having at least 90%, at least 95%, at least 96%, at least 97%, at least 98%, at least 99%, or 100% identity to a sequence as set forth in SEQ ID NO: 172 or 173; and optionally, the polyA signal sequence comprises a sequence having at least 90%, at least 95%, at least 96%, at least 97%, at least 98%, at least 99%, or 100% identity to a sequence as set forth in SEQ ID NO: 173.
Item 10: The recombinant nucleic acid according to any one of items 1 to 9, comprising a sequence having at least 80%, at least 85%, at least 90%, at least 95%, or 100% identity to a spacer sequence as set forth in SEQ ID NO: 185 between the 3' UTR sequence and the polyA signal sequence.
Item 11: The recombinant nucleic acid according to any one of items 1 to 10, wherein
   the mitochondrial targeting sequence comprises a sequence having at least 90%, at least 95%, at least 96%, at least 97%, at least 98%, at least 99%, or 100% identity to a sequence as set forth in SEQ ID NO: 1; and/or
   the mitochondrial protein coding sequence comprises a sequence having at least 90%, at least 95%, at least 96%, at least 97%, at least 98%, at least 99%, or 100% identity to a sequence as set forth in SEQ ID NO: 6.
Item 12: The recombinant nucleic acid according to any one of items 1 to 11, further comprising a first inverted terminal repeat (ITR) sequence and a second ITR sequence, wherein optionally, the first ITR sequence comprises a sequence having at least 90%, at least 95%, at least 96%, at least 97%, at least 98%, at least 99%, or 100% identity to a sequence as set forth in SEQ ID NO: 178, and the second ITR sequence comprises a sequence having at least 90%, at least 95%, at least 96%, at least 97%, at least 98%, at least 99%, or 100% identity to a sequence as set forth in SEQ ID NO: 179.
Item 13: A recombinant nucleic acid comprising (sequentially from the 5' end to the 3' end):
   a mitochondrial targeting sequence, a mitochondrial protein coding sequence, a 3' UTR sequence, and a polyA signal sequence,
   wherein the polyA signal sequence comprises a sequence having at least 90%, at least 95%, at least 96%, at least 97%, at least 98%, at least 99%, or 100% identity to a sequence as set forth in SEQ ID NO: 173.
Item 14: The recombinant nucleic acid according to item 13, wherein the 3' UTR sequence comprises a sequence having at least 90%, at least 95%, at least 96%, at least 97%, at least 98%, at least 99%, or 100% identity to a sequence as set forth in SEQ ID NO: 13.
Item 15: The recombinant nucleic acid according to any one of items 9 to 14, wherein mRNA comprising the mitochondrial protein coding sequence produced by transcription of the recombinant nucleic acid has an expression level that is higher than mRNA of a control recombinant nucleic acid lacking the polyA signal sequence; preferably, has an expression level that is at least 10%, at least 15%, at least 20%, at least 30%, at least 40%, at least 50%, at least 60%, at least 70%, at least 80%, at least 90%, at least 100%, at least 150%, at least 200%, or at least 300% higher than mRNA of the control recombinant nucleic acid.
Item 16: The recombinant nucleic acid according to item 15, wherein the control recombinant nucleic acid is substituted at a position of the polyA signal sequence with a sequence comprising SEQ ID NO: 172; preferably, mRNA produced by transcription of the recombinant nucleic acid has an expression level that is at least 10%, at least 15%, or at least 20% higher than mRNA produced by the control recombinant nucleic acid.
Item 17: The recombinant nucleic acid according to any one of items 9 to 16, wherein the mitochondrial protein produced by translation of the recombinant nucleic acid has an expression level that is higher than the mitochondrial protein of the control recombinant nucleic acid lacking the polyA signal sequence; preferably, has an expression level that is at least 10%, at least 15%, at least 20%, at least 30%, at least 40%, at least 50%, at least 60%, at least 70%, at least 80%, at least 90%, at least 100%, at least 150%, at least 200%, or at least 300% higher than the mitochondrial protein of the control recombinant nucleic acid.
Item 18: The recombinant nucleic acid according to item 17, wherein the control recombinant nucleic acid is substituted at a position of the polyA signal sequence with a sequence comprising SEQ ID NO: 172; preferably, the mitochondrial protein produced by the translation of the recombinant nucleic acid has an expression level that is at least 10%, at least 15%, or at least 20% higher than the mitochondrial protein produced by the control recombinant nucleic acid.
Item 19: The recombinant nucleic acid according to any one of items 9 to 18, wherein the polyA signal sequence has a length of no more than 122 base pairs, no more than 125 base pairs, no more than 130 base pairs, no more than 140 base pairs, no more than 150 base pairs, no more than 160 base pairs, no more than 170 base pairs, no more than 180 base pairs, no more than 190 base pairs, or no more than 200 base pairs.
Item 20: The recombinant nucleic acid according to any one of items 13 to 19, further comprising a Kozak sequence positioned before the 5' end of the mitochondrial targeting sequence, wherein optionally, the Kozak sequence is SEQ ID NO: 171; and optionally, no redundant nucleotides are present between the Kozak sequence and the mitochondrial targeting sequence.
Item 21: The recombinant nucleic acid according to any one of items 13 to 20, comprising a sequence having at least 80%, at least 85%, at least 90%, at least 95%, or 100% identity to a spacer sequence as set forth in SEQ ID NO: 185 between the 3' UTR sequence and the polyA signal sequence.
Item 22: A recombinant nucleic acid comprising (sequentially from the 5' end to the 3' end):
   a Kozak sequence, a mitochondrial targeting sequence, a mitochondrial protein coding sequence, and a 3' UTR sequence, wherein:
   the Kozak sequence is SEQ ID NO: 171;
   the 3' UTR sequence comprises a sequence having at least 90%, at least 95%, at least 96%, at least 97%, at least 98%, at least 99%, or 100% identity to a sequence as set forth in SEQ ID NO: 13; and
   no redundant nucleotides are present between the Kozak sequence and the mitochondrial targeting sequence.
Item 23: The recombinant nucleic acid according to item 22, further comprising a polyA signal sequence, wherein the polyA signal sequence comprises a sequence having at least 90%, at least 95%, at least 96%, at least 97%, at least 98%, at least 99%, or 100% identity to a sequence as set forth in SEQ ID NO: 172 or 173.
Item 24: The recombinant nucleic acid according to any one of items 13 to 23, further comprising an intron sequence, wherein optionally, the intron sequence is positioned before the 5' end of the Kozak sequence; and optionally, the intron sequence comprises a sequence having at least 90%, at least 95%, at least 96%, at least 97%, at least 98%, at least 99%, or 100% identity to a sequence as set forth in SEQ ID NO: 170.
Item 25: The recombinant nucleic acid according to any one of items 13 to 24, further comprising a promoter sequence, wherein optionally, the promoter sequence comprises a sequence having at least 90%, at least 95%, at least 96%, at least 97%, at least 98%, at least 99%, or 100% identity to a sequence as set forth in SEQ ID NO: 169.
Item 26: The recombinant nucleic acid according to item 25, wherein the promoter sequence is positioned before the 5' end of the intron sequence.
Item 27: A recombinant nucleic acid comprising (sequentially from the 5' end to the 3' end):
   a promoter sequence, an intron sequence, a Kozak sequence, a mitochondrial targeting sequence, and a mitochondrial protein coding sequence, wherein
   the intron sequence comprises a sequence having at least 90%, at least 95%, at least 96%, at least 97%, at least 98%, at least 99%, or 100% identity to a sequence as set forth in SEQ ID NO: 170;
   optionally, the recombinant nucleic acid further comprises a 3' UTR sequence; and optionally, the recombinant nucleic acid further comprises a polyA signal sequence.
Item 28: The recombinant nucleic acid according to any one of items 13 to 27, further comprising a first inverted terminal repeat (ITR) sequence and a second ITR sequence, wherein optionally, the first ITR sequence comprises a sequence having at least 90%, at least 95%, at least 96%, at least 97%, at least 98%, at least 99%, or 100% identity to a sequence as set forth in SEQ ID NO: 178, and the second ITR sequence comprises a sequence having at least 90%, at least 95%, at least 96%, at least 97%, at least 98%, at least 99%, or 100% identity to a sequence as set forth in SEQ ID NO: 179.
Item 29: The recombinant nucleic acid according to any one of items 13 to 28, wherein no redundant nucleotides are present between the mitochondrial protein coding sequence and the 3' UTR sequence.
Item 30: The recombinant nucleic acid according to any one of items 13 to 29, wherein
   the mitochondrial targeting sequence comprises a sequence having at least 90%, at least 95%, at least 96%, at least 97%, at least 98%, at least 99%, or 100% identity to a sequence as set forth in SEQ ID NO: 1; and/or
   the mitochondrial protein coding sequence comprises a sequence having at least 90%, at least 95%, at least 96%, at least 97%, at least 98%, at least 99%, or 100% identity to a sequence as set forth in SEQ ID NO: 6.
Item 31: The recombinant nucleic acid according to any one of items 13 to 30, wherein no redundant nucleotides are present between the mitochondrial targeting sequence and the mitochondrial protein coding sequence.
Item 32: The recombinant nucleic acid according to any one of items 1 to 31, comprising (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence, wherein
   preferably, the first ITR sequence comprises a sequence having at least 90%, at least 95%, at least 96%, at least 97%, at least 98%, at least 99%, or 100% identity to a sequence as set forth in SEQ ID NO: 178; the promoter sequence comprises a sequence having at least 90%, at least 95%, at least 96%, at least 97%, at least 98%, at least 99%, or 100% identity to a sequence as set forth in SEQ ID NO: 169; the intron sequence comprises a sequence having at least 90%, at least 95%, at least 96%, at least 97%, at least 98%, at least 99%, or 100% identity to a sequence as set forth in SEQ ID NO: 170; the Kozak sequence is SEQ ID NO: 171; the mitochondrial targeting sequence comprises a sequence having at least 90%, at least 95%, at least 96%, at least 97%, at least 98%, at least 99%, or 100% identity to a sequence as set forth in SEQ ID NO: 1; the mitochondrial protein coding sequence comprises a sequence having at least 90%, at least 95%, at least 96%, at least 97%, at least 98%, at least 99%, or 100% identity to a sequence as set forth in SEQ ID NO: 6; the 3' UTR sequence comprises a sequence having at least 90%, at least 95%, at least 96%, at least 97%, at least 98%, at least 99%, or 100% identity to a sequence as set forth in SEQ ID NO: 13; the polyA signal sequence comprises a sequence having at least 90%, at least 95%, at least 96%, at least 97%, at least 98%, at least 99%, or 100% identity to a sequence as set forth in SEQ ID NO: 173; and the second ITR sequence comprises a sequence having at least 90%, at least 95%, at least 96%, at least 97%, at least 98%, at least 99%, or 100% identity to a sequence as set forth in SEQ ID NO: 179.
Item 33: The recombinant nucleic acid according to any one of items 1 to 32, comprising (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence, wherein
   preferably, the first ITR sequence comprises a sequence having at least 99%, at least 99.5%, or 100% identity to a sequence as set forth in SEQ ID NO: 178; the promoter sequence comprises a sequence having at least 99%, at least 99.5%, or 100% identity to a sequence as set forth in SEQ ID NO: 169; the intron sequence comprises a sequence having at least 99%, at least 99.5%, or 100% identity to a sequence as set forth in SEQ ID NO: 170; the Kozak sequence is SEQ ID NO: 171; the mitochondrial targeting sequence comprises a sequence having at least 99%, at least 99.5%, or 100% identity to a sequence as set forth in SEQ ID NO: 1; the mitochondrial protein coding sequence comprises a sequence having at least 99%, at least 99.5%, or 100% identity to a sequence as set forth in SEQ ID NO: 6; the 3' UTR sequence comprises a sequence having at least 99%, at least 99.5%, or 100% identity to a sequence as set forth in SEQ ID NO: 13; the polyA signal sequence comprises a sequence having at least 99%, at least 99.5%, or 100% identity to a sequence as set forth in SEQ ID NO: 173; and the second ITR sequence comprises a sequence having at least 99%, at least 99.5%, or 100% identity to a sequence as set forth in SEQ ID NO: 179.
Item 34: The recombinant nucleic acid according to any one of items 1 to 33, comprising (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence, wherein
   preferably, the first ITR sequence comprises a sequence as set forth in SEQ ID NO: 178; the promoter sequence comprises a sequence as set forth in SEQ ID NO: 169; the intron sequence comprises a sequence as set forth in SEQ ID NO: 170; the Kozak sequence is SEQ ID NO: 171; the mitochondrial targeting sequence comprises a sequence as set forth in SEQ ID NO: 1; the mitochondrial protein coding sequence comprises a sequence as set forth in SEQ ID NO: 6; the 3' UTR sequence comprises a sequence as set forth in SEQ ID NO: 13; the polyA signal sequence comprises a sequence as set forth in SEQ ID NO: 173; and the second ITR sequence comprises a sequence as set forth in SEQ ID NO: 179.
Item 35: The recombinant nucleic acid according to any one of items 13 to 34, comprising a sequence having at least 90%, at least 95%, at least 96%, at least 97%, at least 98%, at least 99%, or 100% identity to any one of sequences as set forth in SEQ ID NOs: 174-176, and 180.
Item 36: The recombinant nucleic acid according to any one of items 13 to 34, comprising a sequence having at least 90%, at least 95%, at least 96%, at least 97%, at least 98%, at least 99%, or 100% identity to a sequence as set forth in SEQ ID NO: 180, preferably, a sequence identical to SEQ ID NO: 180.
Item 37: A viral vector comprising the recombinant nucleic acid according to any one of items 1 to 36.
Item 38: The viral vector according to item 37, wherein the viral vector is a recombinant adeno-associated virus (rAAV) vector.
Item 39: The viral vector according to item 38, wherein the rAAV vector is an rAAV2 vector.
Item 40: A pharmaceutical composition comprising the recombinant nucleic acid according to any one of items 1 to 36.
Item 41: A pharmaceutical composition comprising a viral vector, wherein the viral vector comprises the recombinant nucleic acid according to any one of items 1 to 36.
Item 42: The pharmaceutical composition according to item 41, wherein the viral vector is an adeno-associated virus (AAV) vector.
Item 43: The pharmaceutical composition according to any one of items 40 to 42, further comprising a pharmaceutically acceptable excipient thereof.
Item 44: The pharmaceutical composition according to item 43, wherein the pharmaceutically acceptable excipient comprises phosphate buffered saline (PBS), α,α-trehalose dehydrate, L-histidine hydrochloride monohydrate, polysorbate 20, NaCl, NaH₂PO₄, Na₂HPO₄, KH₂PO₄, K₂HPO₄, poloxamer 188, or any combination thereof.
Item 45: The pharmaceutical composition according to item 43, wherein the pharmaceutically acceptable excipient is selected from phosphate buffered saline (PBS), α,α-trehalose dehydrate, L-histidine hydrochloride monohydrate, polysorbate 20, NaCl, NaH₂PO₄, Na₂HPO₄, KH₂PO₄, K₂HPO₄, poloxamer 188, and any combination thereof.
Item 46: The pharmaceutical composition according to any one of items 43 to 45, wherein the pharmaceutically acceptable excipient comprises poloxamer 188.
Item 47: The pharmaceutical composition according to item 46, wherein the pharmaceutically acceptable excipient comprises 0.0001%-0.01% poloxamer 188.
Item 48: The pharmaceutical composition according to item 46, wherein the pharmaceutically acceptable excipient comprises 0.001% poloxamer 188.
Item 49: The pharmaceutical composition according to any one of items 46 to 48, wherein the pharmaceutically acceptable excipient further comprises one or more salts.
Item 50: The pharmaceutical composition according to item 49, wherein the one or more salts comprise NaCl, Na₂HPO₄, and KH₂PO₄.
Item 51: The pharmaceutical composition according to item 50, comprising
   a) the NaCl at a concentration of 5-15 mg/mL; preferably, the NaCl at a concentration of 9 mg/mL;
   b) the KH₂PO₄ at a concentration of 0.1-0.5 mg/mL; preferably, the KH₂PO₄ at a concentration of 0.144 mg/mL; and/or
   c) the Na₂HPO₄ at a concentration of 0.5-1 mg/mL; preferably, the Na₂HPO₄ at a concentration of 0.795 mg/mL.
Item 52: The pharmaceutical composition according to any one of items 40 to 51, wherein the pharmaceutical composition has a pH of 7.2-7.4.
Item 53: The pharmaceutical composition according to item 52, wherein the pharmaceutical composition has a pH of 7.3.
Item 54: The pharmaceutical composition according to any one of items 40 to 53, wherein the pharmaceutical composition has a viral titer of at least 1.0×10¹⁰ vg/mL, at least 3.0×10¹⁰ vg/mL, or at least 6.0×10¹⁰ vg/mL.
Item 55: The pharmaceutical composition according to item 54, wherein the pharmaceutical composition has a viral titer of at least 9.0×10¹⁰ vg/mL.
Item 56: The pharmaceutical composition according to any one of items 40 to 55, wherein the pharmaceutical composition retains at least 60% of a viral titer after five freeze/thaw cycles as compared with the viral titer prior to the five freeze/thaw cycles.
Item 57: Use of the pharmaceutical composition according to any one of items 40 to 56 in the preparation of a medicament for treating an ocular disease.
Item 58: A method for treating an ocular disease, comprising administering to a patient an effective dose of the pharmaceutical composition according to any one of items 40 to 56.
Item 59: The use according to item 57 or the method according to item 58, wherein the ocular disease is Leber's hereditary optic neuropathy (LHON).
Item 60: The use or method according to any one of items 57 to 59, wherein the pharmaceutical composition is administered by intraocular or intravitreal injection.
Item 61: The use or method according to item 60, wherein the pharmaceutical composition is administered by intravitreal injection.
Item 62: The use or method according to any one of items 57 to 61, wherein 0.01-0.1 mL of the pharmaceutical composition is administered into each eye.
Item 63: The use or method according to item 62, wherein 0.045-0.055 mL of the pharmaceutical composition is administered into each eye.
Item 64: The use or method according to any one of items 57 to 63, wherein the adeno-associated virus is administered into each eye at a dose of at least 1.5×10⁹ vg; preferably, the adeno-associated virus is administered at a dose of at least 3.0×10⁹ vg.
Item 65: The use or method according to any one of items 57 to 63, wherein the adeno-associated virus is administered into each eye at a dose of at least 4.5×10⁹ vg.
Item 66: The use or method according to any one of items 57 to 65, further comprising administering a corticosteroid to the patient.
Item 67: The use or method according to item 66, wherein the corticosteroid comprises prednisone or methylprednisolone.
Item 68: The use or method according to item 67, wherein the corticosteroid is prednisone.
Item 69: The use or method according to any one of items 66 to 68, wherein the corticosteroid is administered beginning about 2 days prior to the administration of the pharmaceutical composition.
Item 70: The use or method according to any one of items 66 to 69, wherein the corticosteroid is administered orally.
Item 71: The use or method according to any one of items 66 to 70, wherein the corticosteroid is administered for about 28 consecutive days after the administration begins.
Item 72: The use or method according to any one of items 66 to 71, wherein the corticosteroid is administered daily after the administration begins, and at a dose that is reduced after each week of consecutive administration.
Item 73: The use or method according to any one of items 66 to 72, wherein the corticosteroid is administered at a dosage of: 40 mg daily for a week at the beginning of administration, then 30 mg daily for one week, then 20 mg daily for one week, and 10 mg daily for one week at last.
Item 74: The use or method according to any one of items 57 to 73, further comprising administering creatine phosphate sodium to the patient.
Item 75: The use or method according to item 74, wherein the creatine phosphate sodium is administered by intravenous injection.
Item 76: The use or method according to any one of items 57 to 75, wherein the administration of the pharmaceutical composition results in a higher average recovery level of vision as compared with administration of a pharmaceutical composition without the recombinant nucleic acid.

### Examples

The present invention is further described below in conjunction with the following exemplary examples. It should be understood that these examples are intended merely to illustrate the present invention, rather than to limit the scope of the present invention. Unless otherwise indicated, methods and conditions disclosed in, e.g., sambrook et al., molecular cloning: a laboratory manual (New York: cold spring harbor laboratory press, 1989), or conditions recommended by manufacturers may be used in the following examples.

### Example 1. Construction and optimization of ND4 plasmids

### I. Construction and preparation of plasmids

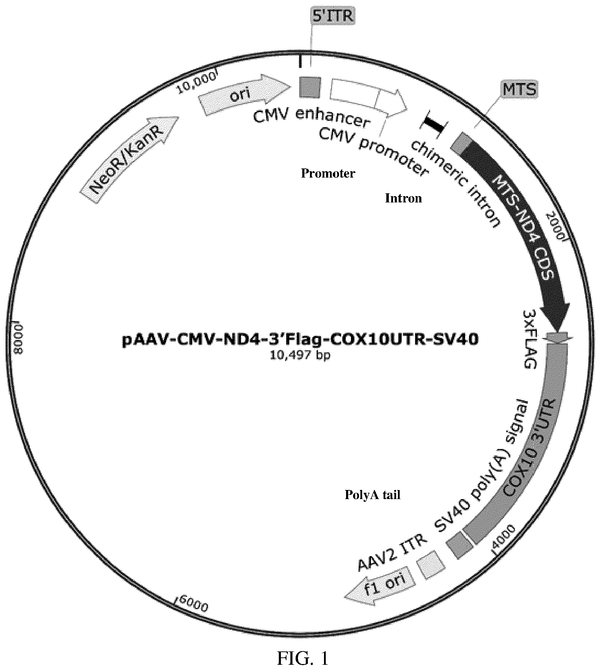

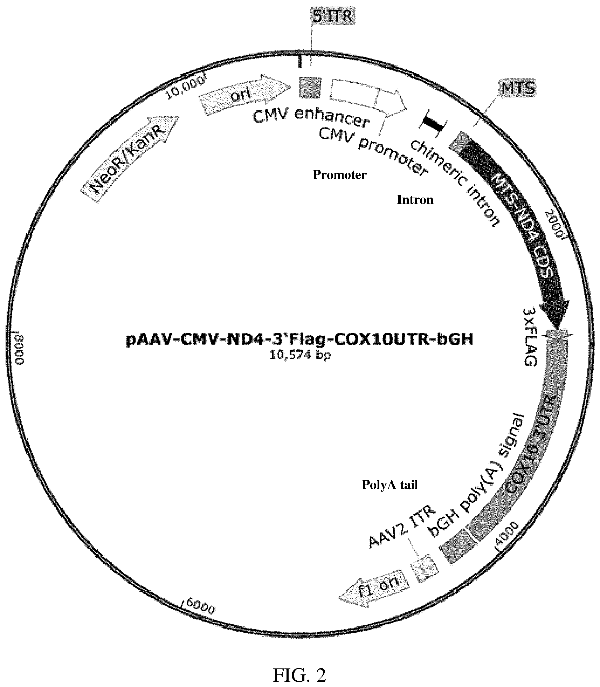

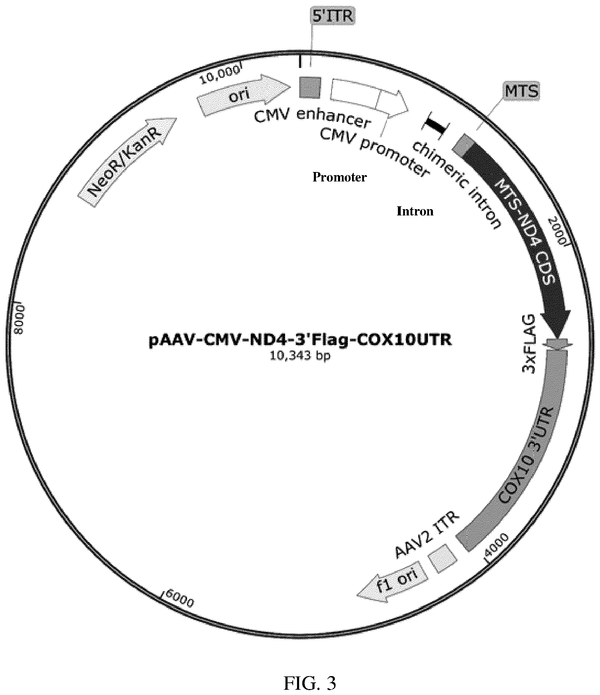

A mitochondrial targeting sequence (MTS) COX10 (SEQ ID NO: 1) was linked to a human ND4 coding sequence (SEQ ID NO: 6) and a 3' UTR sequence (SEQ ID NO: 13), an FLAG-labeled peptide was added before a terminator, a CMV promoter (SEQ ID NO: 169) and a chimeric intron (SEQ ID NO: 170) were added to the 5' end, a bGH (SEQ ID NO: 172) or SV40 (SEQ ID NO: 173) polyA tail (or not) was linked to the 3' end, and an AAV2 plasmid skeleton was inserted to obtain: pAAV-CMV-ND4-3' Flag-COX10UTR plasmid A (FIG. 3; SEQ ID NO: 174), pAAV-CMV-ND4-3' Flag-COX10UTR-bGH plasmid B (FIG. 2; SEQ ID NO: 175), and pAAV-CMV-ND4-3' Flag-COX10UTR-SV40 plasmid C (FIG. 1; SEQ ID NO: 176). Ligation products were transformed into *Escherichia coli*, and single colonies were selected for enzyme digestion verification and sequencing verification.

### II. Cell transfection with the plasmids

1. One day before transfection, HEK293T cells were trypsinized, counted, and then spread on a plate to have a convergence degree of 70%-80% on the day of transfection.
2. For each well of cells, 2.0 µg of plasmid DNA was diluted with 125 µL of a serum-free DMEM culture medium; and 6 µL of a PEI reagent (1 µg/µL) was diluted with 125 µL of a DMEM culture medium.
3. The diluted DNA and PEI were mixed, and incubated for 20 min at room temperature.
4. A compound obtained above was directly added to each well, and the culture plate was shaken gently for well mixing.
5. The cells were cultured at 37 °C in 5% of CO₂ for 48 h.
6. After the culture media were removed, the cells were washed with PBS, trypsinized, centrifuged, and then collected for later use.

### III. Determination of the content of mRNA with qPCR

1. Extraction of total RNA (extraction with a kit)
   1) 1 mL of a lysis solution RZ was added to per 10 cm² of the culture plate to lyse the cells, the cells were beaten with a sampler several times until a solution was transparent, and the solution was left to stand at 15-30 °C for 5 min to completely separate a nucleic acid-protein compound.
   2) 200 µL of chloroform was added, covered with a tube cover, shaken violently for 15 sec, left to stand at room temperature for 3 min, and then centrifuged at 12,000 rpm (about 13,400× g) at 4 °C for 10 min to obtain a sample that was separated into three layers including a yellow organic phase, an intermediate layer, and a colorless aqueous phase. The aqueous phase mainly included RNA, and had a volume of about 50% of the lysis solution RZ reagent used. The aqueous phase was transferred to a new tube for operation in the next step.
   3) 0.5 times volume of absolute ethanol was slowly added for well mixing (in this case, a precipitate might exist). An obtained solution and the precipitate were transferred into an adsorption column CR3, and centrifuged at 12,000 rpm (about 13,400× g) at 4 °C for 30 sec. When the solution and the mixture could not be completely added to the adsorption column CR3 at one time, the solution and the precipitate were transferred into the adsorption column CR3 in two portions, and centrifuged at 12,000 rpm (about 13,400× g) at 4 °C for 30 sec, and then a waste liquid in the collection tube was discharged.
   4) 500 µL of a deproteinized liquid RD was added to the adsorption column CR3, and centrifuged at 12,000 rpm (about 13,400× g) at 4 °C for 30 sec, a waste liquid was discharged, and the CR3 was put into a collection tube.
   5) 500 µL of a rinsing solution RW was added to the adsorption column CR3, left to stand at room temperature for 2 min, and centrifuged at 12,000 rpm (about 13,400× g) at 4 °C for 30 sec, a waste liquid was discharged, and the operation was repeated once.
   6) The adsorption column was put into in a 2 mL collection tube, and centrifuged at 12,000 rpm (about 13,400× g) at 4 °C for 2 min to remove residual liquid.
   7) The adsorption column CR3 was transferred into a new 1.5 mL centrifuge tube, 30-100 µL of RNase-Free ddH₂O was added, and the resulting mixture was left to stand at room temperature for 2 min, and centrifuged at 12,000 rpm (about 13,400× g) at 4 °C for 2 min.
   8) The concentration of RNA was measured by using an ultra-micro ultraviolet spectrophotometer.
2. Reverse transcription
   1) A solution containing 2 µg of RNA, and 4 µL of 5×FastKing-RT SuperMix were added into a PCR tube, and then ribonuclease-free deionized water was added to 20 µL.
   2) The tube was incubated on a PCR instrument at 42 °C for 15 min, and then incubated at 95 °C for 3 min to inactivate a reverse transcriptase.
3. Quantitative PCR
   1) A 0.2 mL PCR tube was used to prepare the following reaction system, and each reverse transcription product was prepared in 3 tubes. 10 µL of 2×qPCR Mix, 0.4 µL of Rox, 1 µL of a 10 µM gene primer, 2.0 µL of a reverse transcription product, and 5.6 µL of ddH₂O were used.

Amplification primers of a target gene flag:
a forward primer 5'-AGACCATGACGGTGAT-3' (SEQ ID NO: 181), and
a reverse primer 5'-CTTGTCATCGTCATCCT-3' (SEQ ID NO: 182).
Amplification primers of an internal reference gene actin:
a forward primer 5'-GGACTTCGAGCAAGAGATGG-3' (SEQ ID NO: 183), and
a reverse primer 5'-AGGAAGGAAGGCTGGAAGAG-3' (SEQ ID NO: 184).

### 2) PCR amplification

Pre-denaturation: 95 °C, 30 s,
40 cycles: 95 °C, 5 s→55 °C, 30 s→72 °C, 30 s,
a dissolution curve: 55 °C→95 °C, at a rate of 0.5 °C/10 s.

3) The ^{ΔΔ}CT method for processing of results was as follows: A = CT (a target gene, a sample to be tested) - CT (an internal standard gene, a sample to be tested); B = CT (a target gene, a control sample) - CT (an internal standard gene, a control sample); K = A - B; and an expression multiple = 2 - K.

### IV. Experimental results and discussions

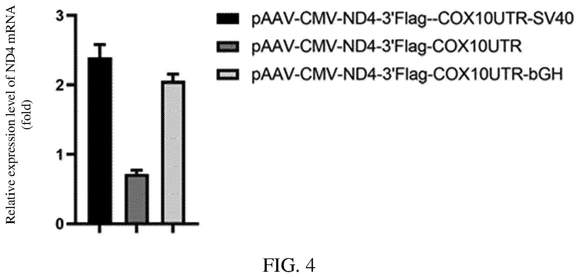

The expression of ND4 was detected at the mRNA level, and the result showed that compared with the pAAV2-CMV-rND4-COX10UTR plasmid A, the pAAV2-CMV-rND4-COX10UTR-bGHPolyA plasmid B and the pAAV2-CMV-rND4-COX10UTR-SV40 plasmid C (FIG. 4) greatly improved the abundance of ND4 mRNA in HEK293 cells, and the plasmid C was better than the plasmid B. The above results show that more ND4 protein can be expressed in cells by codon optimization and addition of different combinations of post-transcriptional regulatory elements, and a better effect is achieved when SV40 polyA is used as a polyA tail.

### Example 2. Virus packaging and production

1. HEK293T cells at a convergence degree of above 90% were passaged in a culture disk at a ratio of 1:3.
2. The serum was changed into a serum-free culture medium about 1-2 h before plasmid transformation, and a target gene plasmid and a helper plasmid were transferred into the HEK293T cells using a transfection reagent.
3. 24 h after plasmid transformation, the serum-free culture medium was refreshed.
4. 72 h after transfection, virus collection was performed. The cells were pipetted with the culture medium and centrifuged; and then a culture medium supernatant and a cell precipitate were collected separately. A virus in the culture medium supernatant was precipitated with PEG8000 overnight, and then a virus precipitate was collected.
5. A virus mixture was purified by density gradient centrifugation with iodixanol, and then concentrated in an ultrafiltration tube.

### Example 3. Formula development

### 3.1 Study on safety of main components

An injection includes the following adjuvants: anhydrous potassium dihydrogen phosphate, anhydrous disodium hydrogen phosphate, sodium chloride, water for injection, and poloxamer 188. Among them, the anhydrous potassium dihydrogen phosphate, the anhydrous disodium hydrogen phosphate, the sodium chloride and the water for injection has proved their practicability and safety as components of PBS in a formula of a traditional ophthalmic injection solution.

Data show that AAV2 virus particles have limited solubility and are likely to aggregate under high concentration and repeated freeze/thaw conditions. In addition, AAV virus particles have a strong adsorption effect on a surface of a container. Therefore, the poloxamer 188 was added to the injection by the applicant to maintain the injection in a relatively stable state.

The following samples were prepared: PBS + 0.01% poloxamer 188 + 10 mg/mL iodixanol; PBS + 0.01% poloxamer 188; PBS + 10 mg/mL iodixanol; PBS + 0.001% poloxamer 188 + 1 mg/mL iodixanol; and PBS + 0.001% poloxamer 188. The samples were intravitreally injected into eyes of New Zealand rabbits. Grouping results are shown in the following Table 2.

**Table 2. Grouping design**

| Groups | | Eyes | Treatment | Dose volume/µL | Number of animals |
|---|---|---|---|---|---|
| 1 | Injection operation group | Right eye | - | - | 3 |
| | | Left eye | - | - | |
| 2 | Test sample group 1 | Right eye | | | 3 |
| | | Left eye | PBS + 0.01% poloxamer 188 + 10 mg/mL iodixanol | 50 | |
| 3 | Test sample group 2 | Right eye | - | - | 3 |
| | | Left eye | PBS + 0.01% poloxamer 188 | 50 | |
| 4 | Test sample group 3 | Right eye | - | - | 3 |
| | | Left eye | PBS + 10 mg/mL iodixanol | 50 | |
| 5 | Test sample group 4 | Right eye | | | 3 |
| | | Left eye | PBS + +0.001% poloxamer 188 + +1mg/mL iodixanol | 50 | |
| 6 | Test sample group 5 | Right eye | - | - | 3 |
| | | Left eye | PBS + +0.001% poloxamer 188 | 50 | |

Clinical observation, intraocular pressure measurement, and general ophthalmic examination were performed on the injected New Zealand rabbits, and euthanasia, gross anatomy, and histopathological observation were performed on the experimental New Zealand rabbits on the day 22 of the experiment.
(1) Clinical observation results: during the experiment, all animals did not show systemic abnormal clinical manifestations except for ocular symptoms.
(2) Intraocular pressure: On the day 22 of the experiment, compared with the injection operation group, the intraocular pressure of the left eyes of female animals in the test group (PBS + 10 mg/mL iodixanol) was significantly reduced (p ≤ 0.05), and at other time points, all the test sample groups had no statistical difference with the injection operation group in the intraocular pressure of the same eyes of animals in the same sex. The difference had contingency without an obvious time-response relation, and thus was irrelevant to test samples.
(3) General ophthalmic examination:

### Conjunctiva:

On the day 1 after treatment, most of the animals developed mild bulbar conjunctival hyperemia in the left eyes, and were completely recovered on the day 8. The injection operation group might have bulbar conjunctival hyperemia, which had no dose relation with samples. Therefore, it was considered that the bulbar conjunctival hyperemia was related to injection operation and irrelevant to test samples.

It was observed that female animals in the groups 2, 3, and 6 had mild conjunctival edema in the left eyes with an incidence of 1/3 of eyes, and were completely recovered on the day 4. The conjunctival edema had low incidence and no obvious dose-response relation, and thus was irrelevant to test samples.

### Lens and vitreous humors:

It was observed that a male animal in the group 4 had uniformly sized greyish-white dotted particles in the anterior vitreous humor of the left eye on the day 8 to day 22, a female animal in the group 5 had greyish-white dotted particle deposits in the anterior lens capsule of the left eye and uniformly sized greyish-white dotted particles in the anterior vitreous humor on the day 8 to day 22, both of which were mild. The above abnormalities had low incidence and no obvious time/dose-response relation, and thus were irrelevant to test samples.

### (4) Gross anatomy and histopathology

On the day 22 of the experiment, euthanasia was performed. Several animals in the injection operation group and all the test sample groups had mild scattered inflammatory cell infiltration of corneal limbus in the left and/or right eye balls. Since this lesion was also observed in the animals in the injection operation group and in the right eyeball of several animals that has not been treated, it was considered that this lesion might be a background or spontaneous lesion of the test animals, and thus was irrelevant to test samples provided.

It was observed under a microscope that all animals had no other significant abnormal pathological changes in the eyeballs.

Conclusion: After the single intravitreal injection of the test samples PBS + 0.01% poloxamer 188 + 10 mg/mL iodixanol, PBS + 0.01% poloxamer 188, PBS + 10 mg/mL iodixanol, PBS + 0.001% poloxamer 188 + 1 mg/mL iodixanol, and PBS + 0.001% poloxamer 188 (50 µL/eye) into the New Zealand rabbits, the animals showed no ocular toxicity reactions related to test samples.

This proves the safety of 0.01% poloxamer 188 as an adjuvant used in an intraocular injection solution.

### 3.2 Study on stability of formulas

Specific formula design is shown in Table 3.

**Table 3. Formula design**

| No. | Formula |
|---|---|
| Formula 1 | pH 7.2-7.4 |
| | Potassium dihydrogen phosphate: 0.144 mg/mL |
| | Sodium chloride: 9.00 mg/mL |
| | Disodium hydrogen phosphate: 0.795 mg/mL |
| Formula 2 | pH 7.2-7.4 |
| | Potassium dihydrogen phosphate: 0.144 mg/mL |
| | Sodium chloride: 9.00 mg/mL |
| | Disodium hydrogen phosphate: 0.795 mg/mL |
| | Poloxamer 188: 0.01 mg/mL |

Products were produced according to the above formulas and tested for stability. Experimental schemes for testing the stability of formulas are shown in Tables 4 and 5.

**Table 4. Experimental scheme for testing the stability of formulas**

| Storage conditions | Sample amount/specification | Test time points |
|---|---|---|
| 2-8 °C | 20 vials; 0.2 mL/vial | Day 0, day 1, and day 5 |

The titer of viral genomes in the formulas 1 and 2 was determined separately on the day 0, day 1, and day 5 to compare the stability of the formulas.

**Table 5: Freeze/thaw experimental scheme for testing the stability of formulas**

| Storage conditions | Sample amount/specification | Test time points |
|---|---|---|
| -75±15 °C | 20 vials; 0.2 mL/vial | 0, 1, 2, 3, 4 and 5 freeze/thaw cycles |

The titer of viral genomes in the formulas 1 and 2 was determined separately after 0, 1, 2, 3, 4 and 5 freeze/thaw cycles to compare the stability of the formulas.

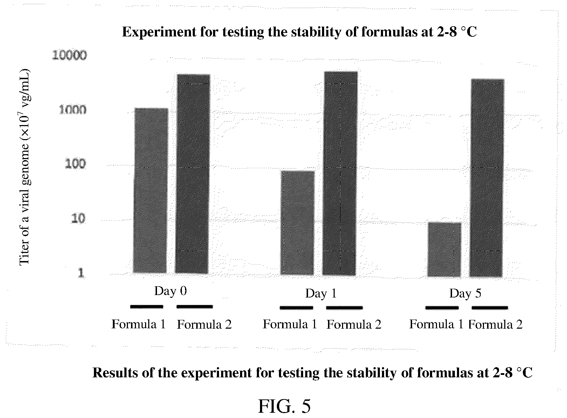

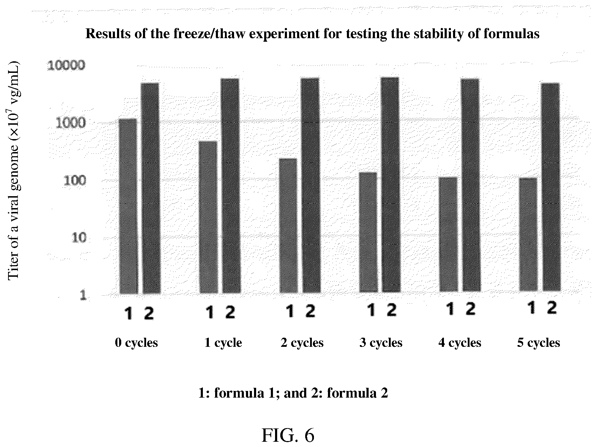

The study on stability of the formulas was performed according to the above two schemes. The results are shown in FIG. 5 (results of the experiment for testing the stability of formulas at 2-8 °C) and FIG. 6 (results of the freeze/thaw experiment for testing the stability of formulas).

**Table 6: Determination results of titer of viral genomes in an experiment for testing the stability of formulas (vg/mL)**

| Formula | Day 0 | Day 1 | Day 5 |
|---|---|---|---|
| Formula 1 | 1.13×10¹⁰ | 8.31×10⁸ | 1.01×10⁸ |
| Formula 2 | 4.72×10¹⁰ | 5.87×10¹⁰ | 4.53×10¹⁰ |

As shown in Table 6, in an experiment for testing the stability of formulas stored at 2-8 °C, the titer of a viral genome in the formula 1 was significantly reduced, and the titer of a viral genome in the formula 2 was not significantly changed. In addition, the titer of a viral genome in the formula 1 had an RSD of 153.67%, and the titer of a viral genome in the formula 2 had an RSD of 14.39%. Therefore, the formula 2 is more stable than the formula 1.

**Table 7: Determination results of titer of viral genomes in a freeze/thaw experiment for testing the stability of formulas (vg/mL)**

| Formula | 0 cycles | 1 cycle | 2 cycles | 3 cycles | 4 cycles | 5 cycles |
|---|---|---|---|---|---|---|
| Formula 1 | 1.13×10¹⁰ | 4.62×10⁹ | 2.25×10⁹ | 1.25×10⁹ | 1.01×10⁹ | 9.48×10⁸ |
| Formula 2 | 4.72×10¹⁰ | 5.48×10¹⁰ | 5.33×10¹⁰ | 5.33×10¹⁰ | 4.94×10¹⁰ | 4.08×10¹⁰ |

As shown in Table 7, in a freeze/thaw experiment, the titer of a viral genome in the formula 1 was significantly reduced, and the titer of a viral genome in the formula 2 was not significantly changed. In addition, the titer of a viral genome in the formula 1 had an RSD of 113.25%, and the titer of a viral genome in the formula 2 had an RSD of 10.53%. Therefore, the formula 2 is more stable than the formula 1.

In conclusion, a formula with the poloxamer 188 is more stable than a formula without the poloxamer 188.

### Example 4. A Phase I/II/III, single-arm, multi-center, and two-stage clinical study for evaluating the safety and efficacy of gene therapy of ND4 mutation-associated Leber's hereditary optic neuropathy (LHON)

The study is a phase I/II/III, single-arm, multi-center, and two-stage clinical study for evaluating the safety and efficacy of an NR082 ophthalmic injection in subjects with ND4 mutation-associated LHON. After admission, all subjects meeting inclusion criteria received a study drug therapy and a systemic immunomodulatory regimen, that is, oral administration of a prescribed dose of a corticosteroid (prednisone) for treatment for 28 days, to prevent or reduce ocular inflammation and possible immune reactions associated with the IVT injection of the NR082 ophthalmic injection. The specific dosing regimen for prednisone was as follows: 40 mg daily for one week starting from 2 days before the injection treatment of the subjects, then 30 mg daily for one week, then 20 mg daily for one week, then 10 mg daily for one week, and discontinuation at last.

The study duration for each subject included a screening period, a follow-up period for 52 (±4) weeks and a long-term safety follow-up period for 4 years. The study included the following visits:
screening period (day -42 to day -2),
hospitalization: grouping (day -2),
hospitalization: treatment (day 1),
hospitalization: safety observation (day 2 to day 8),
visits 1-6: follow-up (week 2 (day 15) to week 52), and
visits 7-14: long-term follow-up and end of study (once every 6 months for 4 years).

Treatment groups and duration were as follows:
the longest study duration for each subject is 52 (±4) weeks and 4 years of long-term follow-up, including the screening period.

The doses used were as follows:
1.5×10⁹ vg, 0.05 mL eye/dose (low dose), and
4.5×10⁹ vg, 0.05 mL eye/dose (high dose).

### Primary efficacy analysis

The primary efficacy endpoint was the proportion of subjects who had an improvement of ≥ 0.3 LogMAR in BCVA of an inject eye as compared with a baseline 52 (± 4) weeks after treatment.

### Secondary efficacy analysis

The proportion (%) of subjects who had an improvement of ≥ 0.3 LogMAR in BCVA of an injected eye as compared with a baseline was summarized by descriptive statistics (subject counts and associated percentages) of categorical variables according to treatment dose groups and visits (as applicable). The exact Cloner-Pearson 95% CI of the above reaction variables was calculated according to visits (as applicable) or calculated based on a normal approximation method (as applicable).

**Table 8. Secondary efficacy analysis-proportion (%) of subjects who had an improvement of ≥ 0.3 LogMAR in best corrected visual acuity (BCVA) as compared with a baseline**

| Full analysis set (FAS) | | | |
|---|---|---|---|
| Visit | Statistics | 1.5×10⁹ vg, 0.05 mL eye/dose | 4.5×10⁹ vg, 0.05 mL eye/dose |
| | | | (N = 6) |
| | | (N = 6) | |
| Studied eye | | | |
| Week 2 | n (%) | 1 (16.7) | 4 (66.7) |
| | 95 CI | 0.4, 64.1 | 22.3, 95.7 |
| Week 6 | n (%) | 1 (16.7) | 4 (66.7) |
| | 95 CI | 0.4, 64.1 | 22.3, 95.7 |
| Week 12 | n (%) | 2 (33.3) | 4 (66.7) |
| | 95 CI | 4.3, 77.7 | 22.3, 95.7 |

Percentages were calculated with the number of subjects in the full analysis set (FAS) as denominators.

The 95% confidence interval was estimated by a Clopper-Pearson method.

n: the number of cases; and 95% CI: the 95% confidence interval.

As shown in Table 8, subjects who had an improvement of ≥ 0.3 LogMAR in best corrected visual acuity (BCVA) as compared with a baseline included 1 case (16.7%), 1 case (16.7%), and 2 cases (33.3%) in the low dose group at weeks 2, 6, and 12, respectively, and 4 cases (66.7%), 4 cases (66.7%), and 4 cases (66.7%) in the high dose group at weeks 2, 6, and 12, respectively.

The change in BCVA (LogMAR) of an injected eye as compared with a baseline and the improvement in BCVA of an injected eye as compared with a minimum are summarized by descriptive statistics of continuous variables (including number of observations [n], mean, standard deviation [SD], median, minimum [min], and maximum [max]) according to treatment dose groups and visits (as applicable); and changes in visual field parameters (including visual field index, mean defect of visual field, and pattern standard deviation value), contrast sensitivity parameters, and visual evoked potential (VEP) waveform parameters of an injected eye and a non-injected eye as compared with a baseline are summarized.

**Table 9. Secondary efficacy analysis-mean change in BCVA (LogMAR) of a studied eye as compared with a baseline**

| Full analysis set (FAS) | | | | | |
|---|---|---|---|---|---|
| Visit | Statistics | 1.5×10⁹ vg, 0.05 mL eye/dose | | 4.5×10⁹ vg, 0.05 mL eye/dose | |
| | | Results (N = 6) | Change as compared with a baseline (N = 6) | Results (N = 6) | Change as compared with a baseline (N = 6) |
| Baseline | n | 6 | | 6 | |
| | Mean (SD) | 1.862 (0.3597) | | 2.150 (0.2324) | |
| | Median | 1.735 | | 2.300 | |
| | Min | 1.48 | | 1.85 | |
| | Max | 2.30 | | 2.30 | |

| Week 2 | n | 6 | 6 | 6 | 6 |
|---|---|---|---|---|---|
| | Mean (SD) | 1.782 (0.2747) | -0.080 (0.2656) | 1.783 (0.1035) | -0.367 (0.2978) |
| | Median | 1.680 | 0.020 | 1.850 | -0.450 |
| | Min | 1.52 | -0.62 | 1.64 | -0.66 |
| | Max | 2.30 | 0.06 | 1.85 | 0.00 |

| Week 6 | n | 6 | 6 | 6 | 6 |
|---|---|---|---|---|---|
| | Mean (SD) | 1.758 (0.2960) | -0.103 (0.2544) | 1.812 (0.0939) | -0.338 (0.2768) |
| | Median | 1.670 | 0.000 | 1.850 | -0.450 |
| | Min | 1.44 | -0.62 | 1.62 | -0.68 |
| | Max | 2.30 | 0.04 | 1.85 | 0.00 |

| Week 12 | n | 6 | 6 | 6 | 6 |
|---|---|---|---|---|---|
| | Mean (SD) | 1.617 (0.0898) | -0.245 (0.3133) | 1.742 (0.1202) | -0.408 (0.2707) |
| | Median | 1.640 | -0.105 | 1.755 | -0.450 |
| | Min | 1.44 | -0.66 | 1.60 | -0.70 |
| | Max | 1.68 | 0.02 | 1.85 | 0.00 |

| | | | | | |
|---|---|---|---|---|---|
| BCVA: best corrected visual acuity. n: the number of cases; Mean: the mean number; SD: the standard deviation; Median: the median number; Min: the minimum; and Max: the maximum. | | | | | |

As shown in Table 9, the mean of BCVA as compared with a baseline was increased by 0.080 LogMAR, 0.103 LogMAR, and 0.245 LogMAR in the low dose group at weeks 2, 6, and 12, respectively, and by 0.367 LogMAR, 0.338 LogMAR, and 0.408 LogMAR in the high dose group at weeks 2, 6, and 12, respectively.

**Table 10. Secondary efficacy analysis-mean change in visual field function parameters as compared with a baseline**

| Full analysis set (FAS) | | | | | |
|---|---|---|---|---|---|
| Visual field index (%) | | | | | |
| Visit | Statistics | 1.5×10⁹ vg, 0.05 mL eye/dose | | 4.5×10⁹ vg, 0.05 mL eye/dose | |
| | | Results (N = 6) | Change as compared with a baseline (N = 6) | Results (N = 6) | Change as compared with a baseline (N = 6) |
| Studied eye | | | | | |

| Baseline | n | 6 | | 6 | |
|---|---|---|---|---|---|
| | Mean (SD) | 9.7 (8.33) | | 4.3 (4.13) | |
| | Median | 6.0 | | 2.5 | |
| | Min | 2 | | 1 | |
| | Max | 22 | | 12 | |

| Week 2 | n | 6 | 6 | 6 | 6 |
|---|---|---|---|---|---|
| | Mean (SD) | 15.8 (9.81) | 6.2 (9.97) | 5.3 (4.32) | 1.0 (2.61) |
| | Median | 15.0 | 7.0 | 4.5 | 0.5 |
| | Min | 2 | -8 | 1 | -1 |
| | Max | 30 | 19 | 13 | 6 |

| Week 6 | n | 6 | 6 | 6 | 6 |
|---|---|---|---|---|---|
| | Mean (SD) | 13.8 (10.05) | 4.2 (10.63) | 16.5 (31.13) | 12.2 (27.41) |
| | Median | 10.0 | 3.0 | 4.5 | 1.0 |
| | Min | 4 | -11 | 2 | -1 |
| | Max | 29 | 21 | 80 | 68 |

| Week 12 | n | 6 | 6 | 6 | 6 |
|---|---|---|---|---|---|
| | Mean (SD) | 16.3 (10.31) | 6.7 (14.12) | 17.8 (34.42) | 13.5 (30.63) |
| | Median | 14.0 | 7.0 | 4.0 | 1.0 |
| | Min | 6 | -10 | 2 | 0 |
| | Max | 36 | 28 | 88 | 76 |

| | | | | | |
|---|---|---|---|---|---|
| n: the number of cases; Mean: the mean number; SD: the standard deviation; Median: the median number; Min: the minimum; and Max: the maximum. | | | | | |

As shown in Table 10, the mean of the visual field index as compared with a baseline was increased by 6.2%, 4.2%, and 6.7% in the low dose group at weeks 2, 6, and 12, respectively, and by 1.0%, 12.2%, and 13.5% in the high dose group at weeks 2, 6, and 12, respectively.

**Table 11 Secondary efficacy analysis-mean change in visual field function parameters as compared with a baseline**

| Full analysis set (FAS) | | | | | |
|---|---|---|---|---|---|
| Change in mean defect of visual field (dB) | | | | | |
| Visit | Statistics | 1.5×10⁹ vg, 0.05 mL eye/dose | | 4.5×10⁹ vg, 0.05 mL eye/dose | |
| | | Results (N = 6) | Change as compared with a baseline (N = 6) | Results (N = 6) | Change as compared with a baseline (N = 6) |
| Studied eye | | | | | |

| Baseline | n | 6 | | 6 | |
|---|---|---|---|---|---|
| | Mean (SD) | -30.237 | | -32.323 | |
| | Median | (3.3663) | | (1.8518) | |
| | Min | -31.795 | | -32.700 | |
| | Max | -32.96 | | -34.04 | |
| | | -24.88 | | -29.03 | |

| Week 2 | n | 6 | 6 | 6 | 6 |
|---|---|---|---|---|---|
| | Mean (SD) | -27.863 | 2.373 | -31.635 | 0.688 (1.4796) |
| | Median | (3.7715) | (3.8945) | (2.0544) | 0.080 |
| | Min | -27.715 | 2.550 | -32.165 | -0.29 |
| | Max | -33.36 | -3.08 | -33.88 | 3.59 |
| | | -22.57 | 7.25 | -28.16 | |

| Week 6 | n | 6 | 6 | 6 | 6 |
|---|---|---|---|---|---|
| | Mean (SD) | -28.730 | 1.507 | -27.910 | 4.413 (8.8705) |
| | Median | (3.6588) | (3.9301) | (10.4491) | 0.615 |
| | Min | -30.045 | 1.080 | -31.880 | -0.28 |
| | Max | -32.36 | -4.14 | -33.57 | 22.39 |
| | | -23.52 | 7.55 | -6.64 | |

| Week 12 | n | 6 | 6 | 6 | 6 |
|---|---|---|---|---|---|
| | Mean (SD) | -27.787 | 2.450 | -27.193 | 5.130 |
| | Median | (3.2752) | (5.1200) | (12.7169) | (11.0775) |
| | Min | -28.545 | 2.930 | -32.215 | 0.625 |
| | Max | -31.24 | -4.10 | -33.24 | 0.38 |
| | | -21.78 | 9.29 | -1.29 | 27.74 |

| | | | | | |
|---|---|---|---|---|---|
| n: the number of cases; Mean: the mean number; SD: the standard deviation; Median: the median number; Min: the minimum; and Max: the maximum. | | | | | |

As shown in Table 11, the mean of the mean defect of visual field as compared with a baseline was increased by 2.373 dB, 1.507 dB, and 2.450 dB in the low dose group at weeks 2, 6, and 12, respectively, and by 0.688 dB, 4.413 dB, and 5.130 dB in the high dose group at weeks 2, 6, and 12, respectively.

Although preferred embodiments of the present invention are shown and described herein, it is obvious to those skilled in the art that such embodiments are merely provided as examples. Numerous variations, changes, and replacements will occur to those skilled in the art without departing from the present invention. It should be understood that various alternatives to the embodiments of the present invention described herein may be employed in implementing the present invention. The following claims are intended to define the scope of the present invention, and methods and structures within the scope of the claims and equivalents thereof are covered thereby.

### Incorporation by reference

All references, articles, publications, patents, patent publications, and patent applications cited herein are incorporated by reference in entirety for all purposes. However, reference to any reference, article, publication, patent publication or patent application cited herein is not, and should not be taken as, an acknowledgment or any form of suggestion that they form part of the effective prior art or the common general knowledge in any country in the world.

## Claims

1. A recombinant nucleic acid comprising (sequentially from the 5' end to the 3' end) a mitochondrial targeting sequence and a mitochondrial protein coding sequence, wherein optionally, the mitochondrial protein coding sequence encodes an ND4 protein; and optionally, the ND4 protein comprises an amino acid sequence having at least 90%, at least 95%, at least 96%, at least 97%, at least 98%, at least 99%, or 100% identity to SEQ ID NO: 160.

2. The recombinant nucleic acid according to claim 1, comprising a sequence having at least 90%, at least 95%, at least 96%, at least 97%, at least 98%, at least 99%, or 100% identity to any one of sequences as set forth in SEQ ID NOs: 180 and 174-176.

3. The recombinant nucleic acid according to claim 1 or 2, comprising a sequence having at least 90%, at least 95%, at least 96%, at least 97%, at least 98%, at least 99%, or 100% identity to SEQ ID NO: 180.

4. The recombinant nucleic acid according to any one of claims 1 to 3, comprising a sequence as set forth in SEQ ID NO: 180.

5. The recombinant nucleic acid according to any one of claims 1 to 4, comprising a Kozak sequence positioned before the 5' end of the mitochondrial targeting sequence, wherein optionally, the Kozak sequence is SEQ ID NO: 171; and optionally, no redundant nucleotides are present between the Kozak sequence and the mitochondrial targeting sequence.

6. The recombinant nucleic acid according to any one of claims 1 to 5, comprising an intron sequence, wherein optionally, the intron sequence is positioned before the 5' end of the mitochondrial targeting sequence; optionally, the intron sequence is positioned before the 5' end of the Kozak sequence; and optionally, the intron sequence comprises a sequence having at least 90%, at least 95%, at least 96%, at least 97%, at least 98%, at least 99%, or 100% identity to a sequence as set forth in SEQ ID NO: 170.

7. The recombinant nucleic acid according to any one of claims 1 to 6, comprising a promoter sequence, wherein optionally, the promoter sequence is positioned before the 5' end of the mitochondrial targeting sequence, the Kozak sequence, and/or the intron sequence; and optionally, the promoter sequence comprises a sequence having at least 90%, at least 95%, at least 96%, at least 97%, at least 98%, at least 99%, or 100% identity to a sequence as set forth in SEQ ID NO: 169.

8. The recombinant nucleic acid according to any one of claims 1 to 7, comprising a 3' UTR sequence, wherein optionally, the 3' UTR sequence is positioned after the 3' end of the mitochondrial protein coding sequence; and optionally, the 3' UTR sequence comprises a sequence having at least 90%, at least 95%, at least 96%, at least 97%, at least 98%, at least 99%, or 100% identity to a sequence as set forth in SEQ ID NO: 13.

9. The recombinant nucleic acid according to any one of claims 1 to 8, comprising a poly A signal sequence, wherein optionally, the polyA signal sequence is positioned after the 3' end of the mitochondrial protein coding sequence and/or the 3' UTR sequence; optionally, the polyA signal sequence comprises a sequence having at least 90%, at least 95%, at least 96%, at least 97%, at least 98%, at least 99%, or 100% identity to a sequence as set forth in SEQ ID NO: 172 or 173; and optionally, the polyA signal sequence comprises a sequence having at least 90%, at least 95%, at least 96%, at least 97%, at least 98%, at least 99%, or 100% identity to a sequence as set forth in SEQ ID NO: 173.

10. The recombinant nucleic acid according to any one of claims 1 to 9, comprising a sequence having at least 80%, at least 85%, at least 90%, at least 95%, or 100% identity to a spacer sequence as set forth in SEQ ID NO: 185 between the 3' UTR sequence and the polyA signal sequence.

11. The recombinant nucleic acid according to any one of claims 1 to 10, wherein
the mitochondrial targeting sequence comprises a sequence having at least 90%, at least 95%, at least 96%, at least 97%, at least 98%, at least 99%, or 100% identity to a sequence as set forth in SEQ ID NO: 1; and/or
the mitochondrial protein coding sequence comprises a sequence having at least 90%, at least 95%, at least 96%, at least 97%, at least 98%, at least 99%, or 100% identity to a sequence as set forth in SEQ ID NO: 6.

12. The recombinant nucleic acid according to any one of claims 1 to 11, further comprising a first inverted terminal repeat (ITR) sequence and a second ITR sequence, wherein optionally, the first ITR sequence comprises a sequence having at least 90%, at least 95%, at least 96%, at least 97%, at least 98%, at least 99%, or 100% identity to a sequence as set forth in SEQ ID NO: 178, and the second ITR sequence comprises a sequence having at least 90%, at least 95%, at least 96%, at least 97%, at least 98%, at least 99%, or 100% identity to a sequence as set forth in SEQ ID NO: 179.

13. A recombinant nucleic acid comprising (sequentially from the 5' end to the 3' end):
a mitochondrial targeting sequence, a mitochondrial protein coding sequence, a 3' UTR sequence, and a polyA signal sequence,
wherein the polyA signal sequence comprises a sequence having at least 90%, at least 95%, at least 96%, at least 97%, at least 98%, at least 99%, or 100% identity to a sequence as set forth in SEQ ID NO: 173.

14. The recombinant nucleic acid according to claim 13, wherein the 3' UTR sequence comprises a sequence having at least 90%, at least 95%, at least 96%, at least 97%, at least 98%, at least 99%, or 100% identity to a sequence as set forth in SEQ ID NO: 13.

15. The recombinant nucleic acid according to any one of claims 13 to 14, further comprising a Kozak sequence positioned before the 5' end of the mitochondrial targeting sequence, wherein optionally, the Kozak sequence is SEQ ID NO: 171; and optionally, no redundant nucleotides are present between the Kozak sequence and the mitochondrial targeting sequence.

16. The recombinant nucleic acid according to any one of claims 13 to 15, comprising a sequence having at least 80%, at least 85%, at least 90%, at least 95%, or 100% identity to a spacer sequence as set forth in SEQ ID NO: 185 between the 3' UTR sequence and the polyA signal sequence.

17. A recombinant nucleic acid comprising (sequentially from the 5' end to the 3' end):
a Kozak sequence, a mitochondrial targeting sequence, a mitochondrial protein coding sequence, and a 3' UTR sequence, wherein:
the Kozak sequence is SEQ ID NO: 171;
the 3' UTR sequence comprises a sequence having at least 90%, at least 95%, at least 96%, at least 97%, at least 98%, at least 99%, or 100% identity to a sequence as set forth in SEQ ID NO: 13; and no redundant nucleotides are present between the Kozak sequence and the mitochondrial targeting sequence.

18. The recombinant nucleic acid according to claim 17, further comprising a polyA signal sequence, wherein the polyA signal sequence comprises a sequence having at least 90%, at least 95%, at least 96%, at least 97%, at least 98%, at least 99%, or 100% identity to a sequence as set forth in SEQ ID NO: 172 or 173.

19. The recombinant nucleic acid according to any one of claims 13 to 18, further comprising an intron sequence, wherein optionally, the intron sequence is positioned before the 5' end of the Kozak sequence; optionally, the intron sequence comprises a sequence having at least 90%, at least 95%, at least 96%, at least 97%, at least 98%, at least 99%, or 100% identity to a sequence as set forth in SEQ ID NO: 170.

20. The recombinant nucleic acid according to any one of claims 13 to 19, further comprising a promoter sequence, wherein optionally, the promoter sequence comprises a sequence having at least 90%, at least 95%, at least 96%, at least 97%, at least 98%, at least 99%, or 100% identity to a sequence as set forth in SEQ ID NO: 169.

21. The recombinant nucleic acid according to claim 20, wherein the promoter sequence is positioned before the 5' end of the intron sequence.

22. A recombinant nucleic acid comprising (sequentially from the 5' end to the 3' end):
a promoter sequence, an intron sequence, a Kozak sequence, a mitochondrial targeting sequence, and a mitochondrial protein coding sequence, wherein
the intron sequence comprises a sequence having at least 90%, at least 95%, at least 96%, at least 97%, at least 98%, at least 99%, or 100% identity to a sequence as set forth in SEQ ID NO: 170;
optionally, the recombinant nucleic acid further comprises a 3' UTR sequence; and optionally, the recombinant nucleic acid further comprises a polyA signal sequence.

23. The recombinant nucleic acid according to any one of claims 13 to 22, further comprising a first inverted terminal repeat (ITR) sequence and a second ITR sequence, wherein optionally, the first ITR sequence comprises a sequence having at least 90%, at least 95%, at least 96%, at least 97%, at least 98%, at least 99%, or 100% identity to a sequence as set forth in SEQ ID NO: 178, and the second ITR sequence comprises a sequence having at least 90%, at least 95%, at least 96%, at least 97%, at least 98%, at least 99%, or 100% identity to a sequence as set forth in SEQ ID NO: 179.

24. The recombinant nucleic acid according to any one of claims 13 to 23, wherein no redundant nucleotides are present between the mitochondrial protein coding sequence and the 3' UTR sequence.

25. The recombinant nucleic acid according to any one of claims 13 to 24, wherein
the mitochondrial targeting sequence comprises a sequence having at least 90%, at least 95%, at least 96%, at least 97%, at least 98%, at least 99%, or 100% identity to a sequence as set forth in SEQ ID NO: 1; and/or
the mitochondrial protein coding sequence comprises a sequence having at least 90%, at least 95%, at least 96%, at least 97%, at least 98%, at least 99%, or 100% identity to a sequence as set forth in SEQ ID NO: 6.

26. The recombinant nucleic acid according to any one of claims 13 to 25, wherein no redundant nucleotides are present between the mitochondrial targeting sequence and the mitochondrial protein coding sequence.

27. The recombinant nucleic acid according to any one of claims 1 to 26, comprising (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence, wherein
preferably, the first ITR sequence comprises a sequence having at least 90%, at least 95%, at least 96%, at least 97%, at least 98%, at least 99%, or 100% identity to a sequence as set forth in SEQ ID NO: 178; the promoter sequence comprises a sequence having at least 90%, at least 95%, at least 96%, at least 97%, at least 98%, at least 99%, or 100% identity to a sequence as set forth in SEQ ID NO: 169; the intron sequence comprises a sequence having at least 90%, at least 95%, at least 96%, at least 97%, at least 98%, at least 99%, or 100% identity to a sequence as set forth in SEQ ID NO: 170; the Kozak sequence is SEQ ID NO: 171; the mitochondrial targeting sequence comprises a sequence having at least 90%, at least 95%, at least 96%, at least 97%, at least 98%, at least 99%, or 100% identity to a sequence as set forth in SEQ ID NO: 1; the mitochondrial protein coding sequence comprises a sequence having at least 90%, at least 95%, at least 96%, at least 97%, at least 98%, at least 99%, or 100% identity to a sequence as set forth in SEQ ID NO: 6; the 3' UTR sequence comprises a sequence having at least 90%, at least 95%, at least 96%, at least 97%, at least 98%, at least 99%, or 100% identity to a sequence as set forth in SEQ ID NO: 13; the polyA signal sequence comprises a sequence having at least 90%, at least 95%, at least 96%, at least 97%, at least 98%, at least 99%, or 100% identity to a sequence as set forth in SEQ ID NO: 173; and the second ITR sequence comprises a sequence having at least 90%, at least 95%, at least 96%, at least 97%, at least 98%, at least 99%, or 100% identity to a sequence as set forth in SEQ ID NO: 179.

28. The recombinant nucleic acid according to any one of claims 1 to 26, comprising (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence, wherein
preferably, the first ITR sequence comprises a sequence having at least 99%, at least 99.5%, or 100% identity to a sequence as set forth in SEQ ID NO: 178; the promoter sequence comprises a sequence having at least 99%, at least 99.5%, or 100% identity to a sequence as set forth in SEQ ID NO: 169; the intron sequence comprises a sequence having at least 99%, at least 99.5%, or 100% identity to a sequence as set forth in SEQ ID NO: 170; the Kozak sequence is SEQ ID NO: 171; the mitochondrial targeting sequence comprises a sequence having at least 99%, at least 99.5%, or 100% identity to a sequence as set forth in SEQ ID NO: 1; the mitochondrial protein coding sequence comprises a sequence having at least 99%, at least 99.5%, or 100% identity to a sequence as set forth in SEQ ID NO: 6; the 3' UTR sequence comprises a sequence having at least 99%, at least 99.5%, or 100% identity to a sequence as set forth in SEQ ID NO: 13; the polyA signal sequence comprises a sequence having at least 99%, at least 99.5%, or 100% identity to a sequence as set forth in SEQ ID NO: 173; and the second ITR sequence comprises a sequence having at least 99%, at least 99.5%, or 100% identity to a sequence as set forth in SEQ ID NO: 179.

29. The recombinant nucleic acid according to any one of claims 1 to 26, comprising (sequentially from the 5' end to the 3' end): a first ITR sequence, a promoter sequence, an intron sequence, a Kozak sequence, a mitochondrial targeting sequence, a mitochondrial protein coding sequence, a 3' UTR sequence, a polyA signal sequence, and a second ITR sequence, wherein
preferably, the first ITR sequence comprises a sequence as set forth in SEQ ID NO: 178; the promoter sequence comprises a sequence as set forth in SEQ ID NO: 169; the intron sequence comprises a sequence as set forth in SEQ ID NO: 170; the Kozak sequence is SEQ ID NO: 171; the mitochondrial targeting sequence comprises a sequence as set forth in SEQ ID NO: 1; the mitochondrial protein coding sequence comprises a sequence as set forth in SEQ ID NO: 6; the 3' UTR sequence comprises a sequence as set forth in SEQ ID NO: 13; the polyA signal sequence comprises a sequence as set forth in SEQ ID NO: 173; and the second ITR sequence comprises a sequence as set forth in SEQ ID NO: 179.

30. The recombinant nucleic acid according to any one of claims 13 to 29, comprising a sequence having at least 90%, at least 95%, at least 96%, at least 97%, at least 98%, at least 99%, or 100% identity to any one of sequences as set forth in SEQ ID NOs: 174-176, and 180.

31. The recombinant nucleic acid according any one of claims 13 to 29, comprising a sequence having at least 90%, at least 95%, at least 96%, at least 97%, at least 98%, at least 99%, or 100% identity to SEQ ID NO: 180.

32. A viral vector comprising the recombinant nucleic acid according to any one of claims 1 to 31.

33. The viral vector according to claim 32, wherein the viral vector is a recombinant adeno-associated virus (rAAV) vector.

34. The viral vector according to claim 33, wherein the rAAV vector is an rAAV2 vector.

35. A pharmaceutical composition comprising an adeno-associated virus (AAV), wherein the AAV comprises the recombinant nucleic acid according to any one of claims 1 to 31.

36. The pharmaceutical composition according to claim 35, further comprising a pharmaceutically acceptable excipient thereof.

37. The pharmaceutical composition according to claim 36, wherein the pharmaceutically acceptable excipient comprises phosphate buffered saline (PBS), α,α-trehalose dehydrate, L-histidine hydrochloride monohydrate, polysorbate 20, NaCl, NaH₂PO₄, Na₂HPO₄, KH₂PO₄, K₂HPO₄, poloxamer 188, or any combination thereof.

38. The pharmaceutical composition according to claim 36, wherein the pharmaceutically acceptable excipient is selected from phosphate buffered saline (PBS), α,α-trehalose dehydrate, L-histidine hydrochloride monohydrate, polysorbate 20, NaCl, NaH₂PO₄, Na₂HPO₄, KH₂PO₄, K₂HPO₄, poloxamer 188, and any combination thereof.

39. The pharmaceutical composition according to any one of claims 36 to 38, wherein the pharmaceutically acceptable excipient comprises poloxamer 188.

40. The pharmaceutical composition according to claim 39, wherein the pharmaceutically acceptable excipient comprises 0.0001%-0.01% poloxamer 188.

41. The pharmaceutical composition according to claim 39, wherein the pharmaceutically acceptable excipient comprises 0.001% poloxamer 188.

42. The pharmaceutical composition according to any one of claims 39 to 41, wherein the pharmaceutically acceptable excipient further comprises one or more salts.

43. The pharmaceutical composition according to claim 42, wherein the one or more salts comprise NaCl, Na₂HPO₄, and KH₂PO₄.

44. The pharmaceutical composition according to claim 43, comprising
a) the NaCl at a concentration of 5-15 mg/mL; preferably, the NaCl at a concentration of 9 mg/mL;
b) the KH₂PO₄ at a concentration of 0.1-0.5 mg/mL; preferably, the KH₂PO₄ at a concentration of 0.144 mg/mL; and/or
c) the Na₂HPO₄ at a concentration of 0.5-1 mg/mL; preferably, the Na₂HPO₄ at a concentration of 0.795 mg/mL.

45. The pharmaceutical composition according to any one of claims 35 to 44, wherein the pharmaceutical composition has a pH of 7.2-7.4.

46. The pharmaceutical composition according to claim 45, wherein the pharmaceutical composition has a pH of 7.3.

47. The pharmaceutical composition according to any one of claims 35 to 46, wherein the pharmaceutical composition has a viral titer of at least 1.0×10¹⁰ vg/mL, at least 3.0×10¹⁰ vg/mL, or at least 6.0×10¹⁰ vg/mL.

48. The pharmaceutical composition according to claim 47, wherein the pharmaceutical composition has a viral titer of at least 9.0×10¹⁰ vg/mL.

49. The pharmaceutical composition according to any one of claims 35 to 48, wherein the pharmaceutical composition retains at least 60% of a viral titer after five freeze/thaw cycles as compared with the viral titer prior to the freeze/thaw cycles.

50. Use of the pharmaceutical composition according to any one of claims 35 to 49 in the preparation of a medicament for treating an ocular disease.

51. The use according to claim 50, wherein the ocular disease is Leber's hereditary optic neuropathy (LHON).

52. The use according to claim 50 or 51, wherein the pharmaceutical composition is administered by intraocular or intravitreal injection.

53. The use according to claim 52, wherein the pharmaceutical composition is administered by intravitreal injection.

54. The use according to any one of claims 50 to 53, wherein the pharmaceutical composition is administered at a dose of 0.01-0.1 mL/eye.

55. The use according to claim 54, wherein the pharmaceutical composition is administered at a dose of 0.045-0.055 mL/eye.

56. The use according to any one of claims 50-55, wherein the adeno-associated virus is administered at a dose of at least 1.5×10⁹ vg/eye; preferably, the adeno-associated virus is administered at a dose of at least 3.0×10⁹ vg/eye.

57. The use according to any one of claims 50 to 55, wherein the adeno-associated virus is administered at a dose of at least 4.5×10⁹ vg/eye.

58. The use according to any one of claims 50 to 57, further comprising administering a corticosteroid to a patient.

59. The use according to claim 58, wherein the corticosteroid comprises prednisone or methylprednisolone.

60. The use according to claim 59, wherein the corticosteroid is prednisone.

61. The use according to any one of claims 58 to 60, wherein the corticosteroid is administered beginning about 2 days prior to the administration of the pharmaceutical composition.

62. The use according to any one of claims 58 to 61, wherein the corticosteroid is administered orally.

63. The use according to any one of claims 58 to 62, wherein the corticosteroid is administered for about 28 consecutive days after the administration begins.

64. The use according to any one of claims 58 to 63, wherein the corticosteroid is administered daily after the administration begins, and at a dose that is reduced after each week of continuous administration.

65. The use according to any one of claims 58 to 64, wherein the corticosteroid is administered at a dosage of: 40 mg daily for one week at the beginning of administration, then 30 mg daily for one week, then 20 mg daily for one week, and 10 mg daily for one week at last.

66. The use according to any one of claims 50 to 65, further comprising administering creatine phosphate sodium to the patient.

67. The use according to claim 66, wherein the creatine phosphate sodium is administered by intravenous injection.

68. The use according to any one of claims 50 to 67, wherein the administration of the pharmaceutical composition results in a higher average recovery level of vision as compared with administration of a pharmaceutical composition without the recombinant nucleic acid.
